# EUROPEAN PATENT SPECIFICATION

(11) **EP 2 211 840 B1**
(45) Date of publication and mention of the grant of the patent: **04.12.2019**
(21) Application number: 08837321.2
(22) Date of filing: 12.10.2008
(51) Int. Cl.: A61K 9/127, A61K 8/14, A61K 8/63, A61Q 19/00, C07J 43/00, A61K 8/55, C07J 41/00, A61K 31/00

(54) **AMPHOTERIC LIPOSOMES COMPRISING NEUTRAL LIPIDS**
AMPHOTERE LIPOSOME MIT NEUTRALEN LIPIDEN
LIPOSOMES AMPHOTÈRES COMPRENANT DES LIPIDES NEUTRES

(30) Priority: 12.10.2007 WO PCT/EP2007/008917; 12.10.2007 US 974350; 11.04.2008 EP 08007302; 20.06.2008 WO PCT/EP2008/005221
(43) Date of publication of application: 04.08.2010
(73) Proprietor: Novosom Verwaltungs GmbH, 06120 Halle (DE)
(72) Inventor: PANZNER, Steffen, 06114 Halle (DE); LUTZ, Silke, 06114 Halle (DE); SIEPI, Evgenios, 5350 Frenaros (CY); MULLER, Claudia, 04685 Nerchau (DE); VINZENS, Ute, 06114 Halle (DE)
(74) Representative: J A Kemp LLP
(86) International application number: PCT/EP2008/008621
(87) International publication number: WO 2009/047006

(56) References cited:
- WO-A-98/51278
- WO-A-2004/047792
- WO-A-2005/094783
- WO-A-2006/048329
- WO-A-2006/053646
- WO-A-2007/012191
- WO-A2-2007/031333
- US-A- 5 296 231
- US-A- 5 302 389
- US-A1- 2003 099 697

## Description

### Field of the invention

The present invention relates to improvements in or relating to amphoteric liposomes comprising neutral lipids.

### Background to the invention

Amphoteric liposomes have been found to exhibit excellent biodistribution and to be well tolerated in animals. They can encapsulate active agents, including nucleic acid molecules, with high efficiency.

In contrast to zwitterionic structures, amphoteric liposomes advantageously have an isoelectric point and are negatively charged at higher pH values and positively charged at lower pH values. Amphoteric liposomes belong to the larger group of pH -sensitive liposomes that were introduced by Straubinger, et al. (FEBS Lett., 1985, 179(1), 148-154). Typical pH -responsive elements in pH -sensitive liposomes are cholesterol hemisuccinate (CHEMS), palmitoylhomocysteine, dioleoylglycerol hemisuccinate (DOG-Succ) and the like. CHEMS can stabilise dioleoylphosphatidylethanolamine (DOPE), a lipid which preferentially adopts the inverted hexagonal phase at temperatures above 10°C, into the lamellar phase at pH 7.4. Lamellar CHEMS/DOPE systems can be prepared at neutral or slightly alkaline pH but these systems become unstable and fuse at acidic pH (Hafez and Cullis, Biochim. Biophys. Acta, 2000, 1463, 107-114).

Fusogenic liposomes are very useful in pharmaceutical applications, especially for the intracellular delivery of drugs, e.g., nucleic acids, such, for example, as plasmids and oligonucleotides. After the uptake of a liposome into a cell by endocytosis the release of the drug from the endosome is a crucial step for the delivery of a drug into the cytosol of cells. The pH within an endosome is slightly acidic and therefore pH sensitive liposomes can fuse with the endosomal membrane and thereby allowing the release of the drug from the endosome. This means that destabilisation of the lipid phase, e.g., by enhanced fusogenicity, facilitates endosome escape and intracellular delivery. Also other environments of low pH can trigger the fusion of such liposomes, e.g., the low pH found in tumors or sites of inflammation.

Hafez, et al. (Biophys. J. 2000, 79(3), 1438-1446) were unsatisfied with the limited control over the pH at which such fusion occurs and demonstrated a rational approach to fine-tune the fusion point by adding cationic lipids. Such mixtures have true amphoteric properties in that they exist in a cationic state at low pH and as anionic particles at higher pH, typically at physiological pH. According to Hafez, et al. fusion starts at pH values where the net charge of the particles is zero (their isoelectric point), and once such point is crossed (the pH is lower to any extent) fusion is a continuous process. This view is shared by Li and Schick (Biophys. J., 2001, 80, 1703-1711) who analysed the fusion tendency for amphoteric lipid mixtures using a mathematical model.

Israelachvili and Mitchell in 1975 (Biochim. Biophys. Acta, 1975, 389, 13-19) introduced the molecular shape concept which assumes that the overall form of lipid molecules determines the structure of the hydrated lipid membrane. This means that the lipid geometry and more specifically the size ratio between the polar head-group and the hydrophobic membrane anchor is the key parameter determining the lipid phase (Israelachvili, et al. Biochim Biophys Acta. 1977 17;470(2):185-201). The original theory however did not consider counterions being a steric part of the polar head-group, but this was contributed by Li and Schick (Biophys. J., 2001, 80, 1703-1711). In their description of the DODAC/CHEMS system, the sodium ion enlarges the head-group of CHEMS at neutral pH, but dissociates as the pH drops, thus minimising the head-group volume and promoting a hexagonal phase; DODAC as a strong cation is assumed to be in constant association with its respective counterion, irrespective of the pH. The model predicts fusion at some pH and below.

Lipid phases according to the molecular shape concept (Israelachvili et al., 1980, Q. Rev. Biophys., 13(2), 121-200):

| **Shape** | **Organisation** | **Lipid phase** | **Examples** |
|---|---|---|---|
| Inverted cone | Micelles | Isotropic Hexagonal I | Detergents Lysophopholipids |
| Cylinder | Bilayer | Lamellar (Cubic) | PC, PS, PI, SM |
| Cone | Reverse micelles | Hexagonal II | PE, PA at low pH or with Ca2+, Cholesterol, Cardiolipin |

The addition of neutral lipids to amphoteric lipid mixtures has been found to have little impact on the isoelectric point of amphoteric liposomes. WO 02/066012 (Panzner, et al.) discloses certain amphoteric liposomes comprising neutral lipids with a stable size at both low and neutral pHs. WO 02/066012 also describes a method of loading such particles with nucleic acids starting from a low pH.

WO 05/094783 of Endert et al. discloses amphoteric liposome formulations comprising a mixture of phosphatidylcholines and cholesterol as neutral lipids, whereas the molar amount of cholesterol is between 35 and 40 mol%.

WO 07/031333 of Panzner et al. discloses amphoteric liposomes comprising a mixture of phosphatidylcholine and phosphatidylethanolamine as neutral lipids.

WO 98/51278 describes methods for the preparation of a lipid-nucleic acid composition. WO 2004/047792 describes a liposomal formulation comprising water-soluble glucocorticoids. WO 2006/048329 describes a pharmaceutical composition comprising an oligonucleotide as an active agent, and an amphoteric liposome as an excipient. WO 2006/053646 describes a pharmaceutical composition for local application, comprising a nucleic acid as a therapeutic agent, an excipient comprising an amphoteric liposome having an isoelectric point between 4 and 7.4, and a pharmaceutically acceptable vehicle therefor. WO 2007/012191 describes an apparatus and processes for producing liposomes.

US 5,302,389 describes a method for the treatment of UV induced suppression of the T-cell mediated immune response in a human comprising topically administering a therapeutically effective amount of liposome encapsulated T4 endonuclease V (T4N5) DNA repair enzyme. US 5,296,231 describes a process for producing a liposome-encapsulated DNA repair enzyme.

Amphoteric liposomes are complex structures and comprise at least a complementary pair of charged lipids. The inclusion of one or more such neutral lipids significantly adds to the complexity of the mixture, especially since the individual amounts of the components may vary.

### Object of the invention

It is an object of the present invention therefore to provide improved formulations of amphoteric liposomes comprising neutral lipids.

Another object of the invention is to provide improved formulations of amphoteric liposomes that allow transfection of cells.

Yet another object of the invention is to provide pharmaceutical compositions comprising such liposomes as a carrier for the delivery of active agents or ingredients, including drugs such as nucleic acid drugs, e.g., oligonucleotides and plasmids into cells or tissues.

### Summary of the invention

The scope of the present invention is defined by the appended claims. Any other disclosure in the present description, regardless of whether it is indicated as preferred or exemplified, does not form part of the present invention.

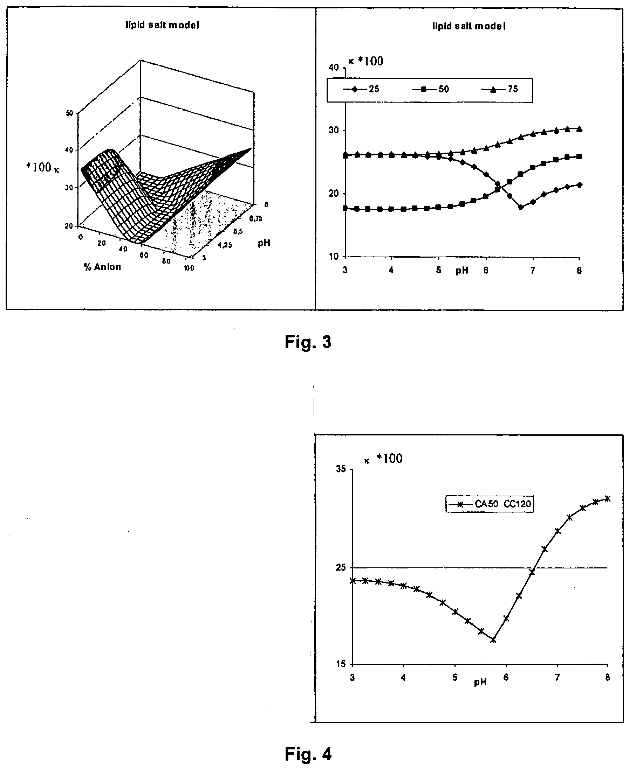

Tables 11-14 and Figs. 4 (first figure, now an empty space), 6, 7, 8 (second figure, now an empty space), 9 (second figure, now an empty space) and 16 have been removed from the description due to them being illegible in the originally filed application. These tables and figures, along with the corresponding legends, have been provided in the separate Supplementary Technical Information file, but, although being referred to occasionally in the present description, do not form part of the disclosure of the present application. Consequently, references to said tables and figures do not serve to incorporate said tables and figures by reference.

The present invention provides an amphoteric liposome comprising:
(a) neutral lipids selected from cholesterol or a mixture of cholesterol and at least one neutral or zwitterionic lipid, wherein κ(neutral) of said mixture is 0.25 or less and wherein κ(neutral) is calculated by the following formula: $κ \left(neutral\right) = κ \left(Lipid 1\right) * c \left(Lipid 1\right) + κ \left(Lipid 2\right) * c \left(Lipid 2\right) + … κ \left(Lipid i\right) * c \left(Lipid i\right)$ wherein κ(Lipid) is the κ value of the appropriate neutral or zwitterionic lipid, c(Lipid) is the concentration of said lipid and i is the running variable,
   wherein κ is the volume ratio between the polar and apolar section of a lipid: $κ = molecular volume \left(head\right) / molecular volume \left(tail\right) ;$ wherein the mixture of cholesterol and at least one neutral or zwitterionic lipid is selected from the group consisting of:
   (i) cholesterol/phosphatidylcholine;
   (ii) cholesterol/phosphatidylethanolamine; and
   (iii)cholesterol/phosphatidylethanolamine/phosphatidylcholine; and
(b) a mixture of lipid components comprising:
   (i) a stable cationic lipid and a chargeable anionic lipid, referred to as amphoter I mixture; or
   (ii) a chargeable cationic lipid and a chargeable anionic lipid, referred to as amphoter II mixture; or
   (iii)a chargeable cationic lipid and a stable anionic lipid, referred to as amphoter III mixture;
further wherein the mixture of lipid components comprises at least one lipid ion which is a pH-sensitive, weak acid or base which is chargeable; and
further wherein the minimum value of κ(total) of the mixture is smaller than 0.25.

Preferably κ(neutral) of said mixture of cholesterol and at least one neutral or zwitterionic lipid is less than 0.2 and most preferably less than 0.15. Suitably said phosphatidylethanolamines may be selected from the group of DOPE, POPE, DPhyPE, DLinPE, DMPE, DPPE, DSPE or natural equivalents thereof, wherein DOPE is the most preferred one.

The phosphtaidylcholines may be selected from the group POPC, DOPC, DMPC, DPPC, DSPC or natural equivalents thereof, such as soy bean PC or egg-PC wherein POPC or DOPC are the preferred ones.

In one embodiment of the invention the isoelectric point of the amphoteric liposomes is between 4 and 7, preferably between 4.5 and 6.5 and most preferred between 5 and 6.

Said anionic lipids may be selected from, but are not limited to, the group consisting of diacylglycerolhemisuccinates, e.g. DOGS, DMGS, POGS, DPGS, DSGS; diacylglycerolhemimalonates, e.g. DOGM or DMGM; diacylglycerolhemiglutarates, e.g. DOGG, DMGG; diacylglycerolhemiadipates, e.g. DOGA, DMGA; diacylglycerolhemicyclohexane-1,4-dicarboxylic acids, e.g. DO-cHA, DM-cHA; (2,3-Diacyl-propyl)amino}-oxoalkanoic acids e.g. DOAS, DOAM, DOAG, DOAA, DMAS, DMAM, DMAG, DMAA; Diacyl-alkanoic acids, e.g. DOP, DOB, DOS, DOM, DOG, DOA, DMP, DOB, DMS, DMM, DMG, DMA; Chems and derivatives therof, e.g. Chol-C2, Chol-C3, Chol-C5, Chol-C6, Chol-C7 or Chol-C8; Chol-Cl, CholC3N or Cholesterolhemidicarboxylic acids and Cholesteryloxycarbonylaminocarboxylic acids, e.g. Chol-C12 or CholC13N, fatty acids, e.g. Oleic acid, Myristic Acid, Palmitic acid, Stearic acid, Nervonic Acid, Behenic Acid; DOPA, DMPA, DPPA, POPA, DSPA, Chol-S04, DOPG, DMPG, DPPG, POPG, DSPG or DOPS, DMPS, DPPS, POPS, DSPS or Cetyl-phosphate.

Said cationic lipids may be selected from, but are not limited to, the group consisting of consisting of DOTAP, DMTAP, DPTAP, DSTAP, POTAP, DODAP, PODAP, DMDAP, DPDAP, DSDAP, DODMHEAP or DORI, PODMHEAP or PORI, DMDMHEAP or DMRI, DPDMHEAP or DPRI, DSDMHEAP or DSRI, DOMDHEAP, POMDHEAP, DMMDHEAP, DPMDHEAP, DSMDHEAP, DOMHEAP, POMHEAP, DMMHEAP, DPMHEAP, DSMHEAP, DODHEAP, PODHEAP, DMDHEAP, DPDHEAP, DSDHEAP, DDAB, DODAC, DOEPC, DMEPC, DPEPC, DSEPC, POEPC, DORIE, DMRIE, DOMCAP, DOMGME, DOP5P, DOP6P, DC-Chol, TC-Chol, DAC-Chol, Chol-Betaine, N-methyl-PipChol, CTAB, DOTMA, MoChol, HisChol, Chim, MoC3Chol, Chol-C3N-Mo3, Chol-C3N-Mo2, Chol-C4N-Mo2, Chol-DMC3N-Mo2, CholC4Hex-Mo2, DmC4Mo2, DmC3Mo2, C3Mo2, C3Mo3, C5Mo2, C6Mo2, C8Mo2, C4Mo4, PipC2-Chol, MoC2Chol, PyrroC2Chol, ImC3Chol, PyC2Chol, MoDO, MoDP, DOIM or DPIM.

In addition or alternatively the inventive amphoteric liposomes may comprise one or more compounds with Cpd.No. 1-97 listed in tables 59 and 60 of this disclosure.

In one embodiment of the invention the amphoteric liposomes are an amphoter I mixture and κ(min) of said mixtures is between 0.07 and 0.22, preferably between 0.09 and 0.15.

In another embodiment of the invention the amphoteric liposomes are an amphoter II mixture and κ(min) of these mixtures is less 0.23, preferably less than 0.18.

In another aspect of the invention, the liposome may comprise a lipid mixture other than one having the following specific combination of amphiphiles: DC-Chol/DOPA/Chol 40:20:40 (molar ratio).

In still other aspects of the invention the amphoteric liposome may be other than one comprising a mixture of cholesterol and phosphatidylcholine in a molar amount of 50 mol% or more.

In another particular aspect of the present invention, the amphoteric liposomes encapsulate at least one active agent. Said active agent may comprise a drug. In some embodiments said active agent may comprises a nucleic acid.

Without being limited to such use, the amphoteric liposomes described in the present invention are well suited for use as carriers for nucleic acid-based drugs such for example as oligonucleotides, polynucleotides and DNA plasmids. These drugs are classified into nucleic acids that encode one or more specific sequences for proteins, polypeptides or RNAs and into oligonucleotides that can specifically regulate protein expression levels or affect the protein structure through inter alia interference with splicing and artificial truncation.

In some embodiments of the present invention, therefore, the nucleic acid-based therapeutic may comprise a nucleic acid that is capable of being transcribed in a vertebrate cell into one or more RNAs, which RNAs may be mRNAs, shRNAs, miRNAs or ribozymes, wherein such mRNAs code for one or more proteins or polypeptides. Such nucleic acid therapeutics may be circular DNA plasmids, linear DNA constructs, like MIDGE vectors (Minimalistic Immunogenically Defined Gene Expression) as disclosed in WO 98/21322 or DE 19753182, or mRNAs ready for translation (e.g., EP 1392341).

In another embodiment of the invention, oligonucleotides may be used that can target existing intracellular nucleic acids or proteins. Said nucleic acids may code for a specific gene, such that said oligonucleotide is adapted to attenuate or modulate transcription, modify the processing of the transcript or otherwise interfere with the expression of the protein. The term "target nucleic acid" encompasses DNA encoding a specific gene, as well as all RNAs derived from such DNA, being pre-mRNA or mRNA. A specific hybridisation between the target nucleic acid and one or more oligonucleotides directed against such sequences may result in an inhibition or modulation of protein expression. To achieve such specific targeting, the oligonucleotide should suitably comprise a continuous stretch of nucleotides that is substantially complementary to the sequence of the target nucleic acid.

Oligonucleotides fulfilling the abovementioned criteria may be built with a number of different chemistries and topologies. The oligonucleotides may comprise naturally occurring or modified nucleosides comprising but not limited to DNA, RNA, locked nucleic acids (LNA's), 2'O-methyl RNA (2'Ome), 2' O-methoxyethyl RNA (2'MOE) in their phosphate or phosphothioate forms or Morpholinos or peptide nucleic acids (PNA's). Oligonucleotides may be single stranded or double stranded.

Oligonucleotides are polyanionic structures having 8-60 charges. In most cases these structures are polymers comprising nucleotides. The present invention is not limited to a particular mechanism of action of the oligonucleotides and an understanding of the mechanism is not necessary to practice the present invention.

The mechanisms of action of oligonucleotides may vary and might comprise inter alia effects on splicing, transcription, nuclear-cytoplasmic transport and translation.

In a preferred embodiment of the invention single stranded oligonucleotides may be used, including, but not limited to DNA-based oligonucleotides, locked nucleic acids, 2'-modified oligonucleotides and others, commonly known as antisense oligonucleotides. Backbone or base or sugar modifications may include, but are not limited to, Phosphothioate DNA (PTO); 2'O-methyl RNA (2'Ome), 2'Fluoro RNA (2'F), 2' O- methoxyethyl-RNA (2'MOE), peptide nucleic acids (PNA), N3'-P5' phosphoamidates (NP), 2'fluoroarabino nucleic acids (FANA), locked nucleic acids (LNA), Morpholine phosphoamidate (Morpholino), Cyclohexene nucleic acid (CeNA), tricyclo-DNA (tcDNA) and others. Moreover, mixed chemistries are known in the art, being constructed from more than a single nucleotide species as copolymers, blockcopolymers or gapmers or in other arrangements.

In addition to the aforementioned oligonucleotides, protein expression can also be inhibited using double stranded RNA molecules containing the complementary sequence motifs. Such RNA molecules are known as siRNA molecules in the art (e.g., WO 99/32619 or WO 02/055693). Other siRNAs comprise single stranded siRNAs or double stranded siRNAs having one non-continuous strand. Again, various chemistries were adapted to this class of oligonucleotides. Also, DNA / RNA hybrid systems are known in the art.

In another embodiment of the present invention, decoy oligonucleotides can be used. These double stranded DNA molecules and chemical modifications thereof do not target nucleic acids but transcription factors. This means that decoy oligonucleotides bind sequence-specific DNA-binding proteins and interfere with the transcription (e.g., Cho-Chung, et al. in Curr. Opin. Mol. Ther., 1999).

In a further embodiment of the invention oligonucleotides that may influence transcription by hybridizing under physiological conditions to the promoter region of a gene may be used. Again various chemistries may adapt to this class of oligonucleotides.

In a still further alternative of the invention, DNAzymes may be used. DNAzymes are single-stranded oligonucleotides and chemical modifications therof with enzymatic activity. Typical DNAzymes, known as the "10-23" model, are capable of cleaving single-stranded RNA at specific sites under physiological conditions. The 10-23 model of DNAzymes has a catalytic domain of 15 highly conserved deoxyribonucleotides, flanked by 2 substrate-recognition domains complementary to a target sequence on the RNA. Cleavage of the target mRNAs may result in their destruction and the DNAzymes recycle and cleave multiple substrates.

In yet another embodiment of the invention, ribozymes can be used. Ribozymes are single-stranded oligoribonucleotides and chemical modifications thereof with enzymatic activity. They can be operationally divided into two components, a conserved stem-loop structure forming the catalytic core and flanking sequences which are reverse complementary to sequences surrounding the target site in a given RNA transcript. Flanking sequences may confer specificity and may generally constitute 14-16 nt in total, extending on both sides of the target site selected.

In a still further embodiment of the invention aptamers may be used to target proteins. Aptamers are macromolecules composed of nucleic acids, such as RNA or DNA, and chemical modifications thereof that bind tightly to a specific molecular target and are typically 15-60 nt long. The chain of nucleotides may form intramolecular interactions that fold the molecule into a complex three-dimensional shape. The shape of the aptamer allows it to bind tightly against the surface of its target molecule including but not limited to acidic proteins, basic proteins, membrane proteins, transcription factors and enzymes. Binding of aptamer molecules may influence the function of a target molecule.

All of the above-mentioned oligonucleotides may vary in length between as little as 5 or 10, preferably 15 and even more preferably 18, and 50, preferably 30 and more preferably 25, nucleotides per strand. More specifically, the oligonucleotides may be antisense oligonucleotides of 8 to 50 nucleotides length that catalyze RNAseH mediated degradation of their target sequence or block translation or re-direct splicing or act as antogomirs; they may be siRNAs of 15 to 30 basepairs length; they may further represent decoy oligonucleotides of 15 to 30 basepairs length; can be complementary oligonucleotides influencing the transcription of genomic DNA of 15 to 30 nucleotides length; they might further represent DNAzymes of 25 to 50 nucleotides length or ribozymes of 25 to 50 nucleotides length or aptamers of 15 to 60 nucleotides length. Such subclasses of oligonucleotides are often functionally defined and can be identical or different or share some, but not all features of their chemical nature or architecture without substantially affecting the teachings of this invention. The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one or more mismatches within the said continuous stretch of base pairs, although this is less preferred. In general the type and chemical composition of such nucleic acids is of little impact for the performance of the inventive liposomes as vehicles be it in vivo or in vitro and the skilled artisan may find other types of oligonucleotides or nucleic acids suitable for combination with the inventive amphoteric liposomes.

In one aspect the amphoteric liposomes according to the present invention are useful to transfect cells *in vitro, in vivo* or *ex vivo.*

In another aspect of the invention the amphoteric liposomes according to the invention may comprise cell targeting ligands on the surface which bind to a target receptor of the cell surface. Ligands may include, but are not limited to, antibodies or their fragments, sugars, hormones, vitamins, peptides, such as arg-gly-asp (RGD), growth factors, bilirubin, transferrin, folate or other components.

In still other aspects of the invention the amphoteric liposomes may comprise membrane forming or membrane situated molecules which sterically stabilize the particles. Such molecules are known in the art and include amphipathic dextranes, polysialic acids, hydroxyethyl starches, hyaluronic acids, polyethylenglycols, Tween 80 or GM1 gangliosides (e.g. Woodle et al., Biochim. Biophys. Acta, 1113(2), 171-179, (1992); Allen et al., Biochim. Biophys. Acta, 981(1), 27-35, (1989)), without being limited to said substances. The abovementioned molecules are of amphipathic character and comprise at least one hydrophilic domain that can be selected from the moieties above and further comprise at least one hydrophobic domain, which is very often a lipid, one or more alkyl chains comprising 12 or more carbon atoms or one or more acyl chains comprising 12 or more carbon atoms. Amphipathic molecules that are most frequently used comprise DSPE-mPEG, DMPE-mPEG and polyethylenglycols coupled to ceramides having an N-acyl chain length between 8 and 24 carbon atoms. It is known to the skilled artisan that the size of the hydrophobic portion is related to the diffusion time of these sterically shielding moieties, as demonstrated in (Mok,K.W.et al.(1999). Biochim. Biophys. Acta 1419, 137-150; Silvius, J.R. and Zuckermann,M.J. (1993). Biochemistry 32, 3153-3161.;Webb,M.S. et al. (1998). Biochim. Biophys. Acta 1372, 272-282; Wheeler,J.J. et al. (1999). Gene Ther. 6, 271-281; Zhang,Y.P. et al. (1999). Gene Ther. 6, 1438-1447). The steric shielding may therefore be of constant or transient nature within the limits of the circulation time of such particles in a vertebrate or mammal. Also, said sterically stabilizing polymers may be grafted to both the exofacial and endofacial side of the lipid bilayer or may be limited to only the exofacial side. This can be achieved through different techniques of insertion of said moieties, as demonstrated in (Shi F et al. (2002), Biochemical Journal 366:333-341).

Amongst other effects steric stabilizers minimize the uptake of the particles by the RES (reticuloendothelial system) upon injection of the particles into the blood stream.

Amphoteric liposomes comprising cell targeting ligands and molecules which sterically stabilize the particles are also within the scope of the present invention. Drug delivery systems comprising both ligands and molecules which sterically stabilize are known in the art, e.g. Hu-Lieskovan et al., Cancer Res., 65(19), 8984-8992,(2005) or Schiffelers et al., Nucleic Acid Research, 32(19), (2004).

A further aspect of the invention relates to pharmaceutical compositions comprising the inventive amphoteric liposomes as a carrier for the delivery or targeted delivery of active agents or ingredients, including drugs such as nucleic acid drugs, e.g., oligonucleotides and plasmids. The pharmaceutical composition of the present invention may be formulated in a suitable pharmacologically acceptable vehicle. Vehicles such as water, saline, phosphate buffered saline and the like are well known to those skilled in the art for this purpose.

Said pharmaceutical compositions may be used for the treatment or prophylaxis of inflammatory, immune or autoimmune disorders, cancer and/or metabolic diseases of humans or non-human animals.

### Brief description of the drawings

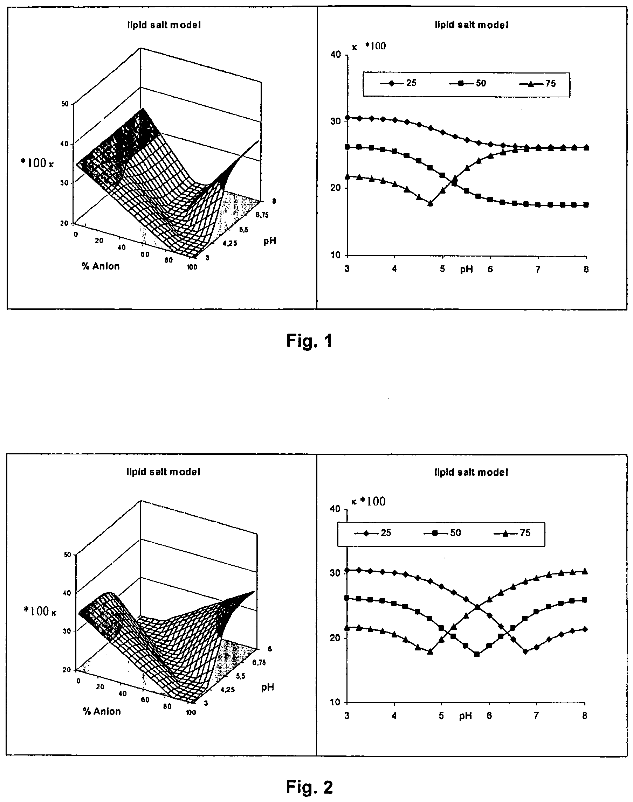

Figs. 1 and 2 are graphical representations of the calculation of κ for different ratios between anionic and cationic model lipids in amphoter I or amphoter II systems, respectively. Left panel: Surface plot for κ in response to pH and percentage of anionic lipid. Right panel: Detailed analysis of the pH response for selected amounts of anionic lipids.
Fig. 3 is a graphical representation of the calculation of κ for different ratios between anionic and cationic model lipids in amphoter III systems. Left panel: Surface plot for κ in response to pH and percentage of anionic lipid. Right panel: Detailed analysis of the pH response for selected amounts of anionic lipids.
Fig. 4 shows the stabilisation of the anionic or cationic state of an amphoter II mixture through various counterion sizes. The graph shows exclusive stabilisation of the anionic state through larger cationic counterions. CA - counter-anion; CC - counter-cation; the numbers in the legend indicate molecular volumes in Å³

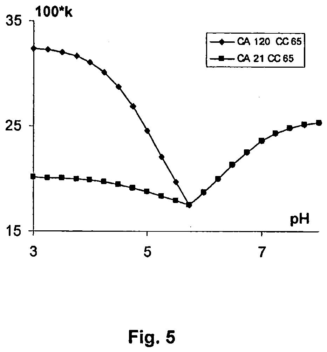

Fig. 5 illustrates the asymmetric stabilisation of a cationic amphoter II lipid phase through various counter-anions. During production, the cationic lipid phase is stabilised with larger anions (CA120). Liposomes are adjusted to a neutral pH and the buffer composition is changed for a smaller counter-anion (CA21). Liposomes that now encounter acidic pH are prone to fusion since the lipid phase has much lower values of κ. CA - counter-anion; CC - counter-cation; the numbers in the legend indicate molecular volumes in Å³.

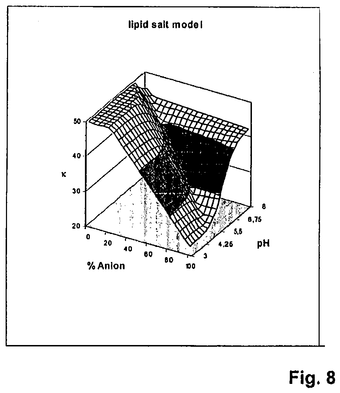

Fig. 8 shows the fusion behaviour of an amphoter II system comprising a MoChol and CHEMS. The graph shows a calculation of κ values for the system. The percentage in the legend stands for the amount of CHEMS in the mixture.

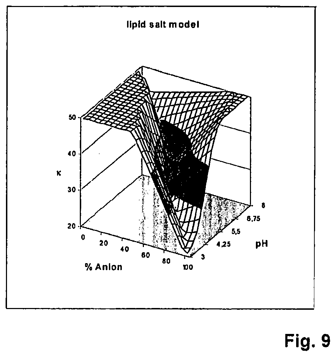

Fig. 9 shows the fusion behaviour of an amphoter II system comprising a monoalkyl lipid. The graph shows a calculation of κ values for the system. The percentage in the legend stands for the amount of oleic acid in the mixture.

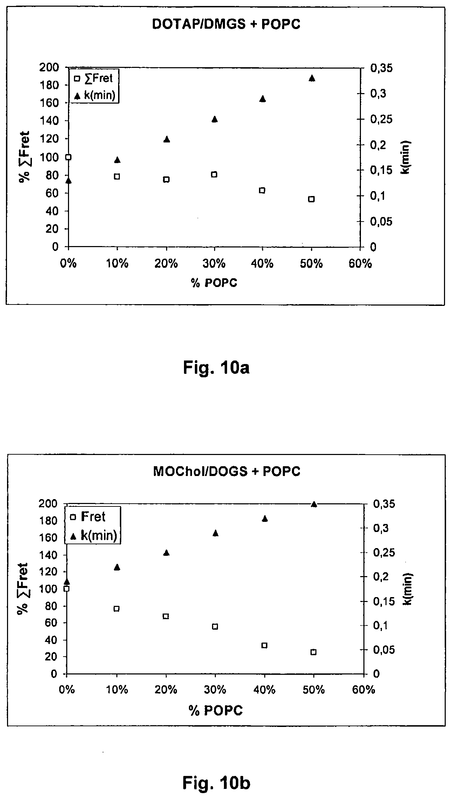

Figs. 10a and 10b show plots of the intensity of fusion (expressed as %∑FRET in the matrix C/A=0.17-0.75 for DOTAP/DMGS; C/A=0.33-3 for MoChol/DOGS vs. pH) for liposomes from DOTAP/DMGS or MoChol/DOGS against κ(min) for mixtures with 0% - 50% POPC. The reference κ(min) was modelled for C/A=0.66 (DOTAP/DMGS) or C/A=1(MoChol/DOGS). The %∑FRET for 0% POPC is set to 100.

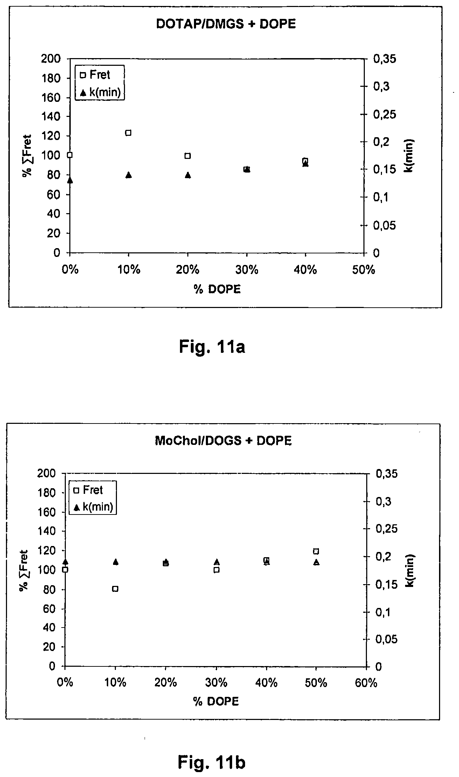

Figs. 11a and 11b show plots of the intensity of fusion (expressed as %∑FRET in the matrix C/A=0.17-0.75 for DOTAP/DMGS; C/A=0.33-3 for MoChol/DOGS vs. pH) for liposomes from DOTAP/DMGS or MoChol/DOGS against κ(min) for mixtures with 0% - 50% DOPE. The reference κ(min) was modelled for C/A=0.66 (DOTAP/DMGS) or C/A=1(MoChol/DOGS). The %∑FRET for 0% DOPE is set to 100.

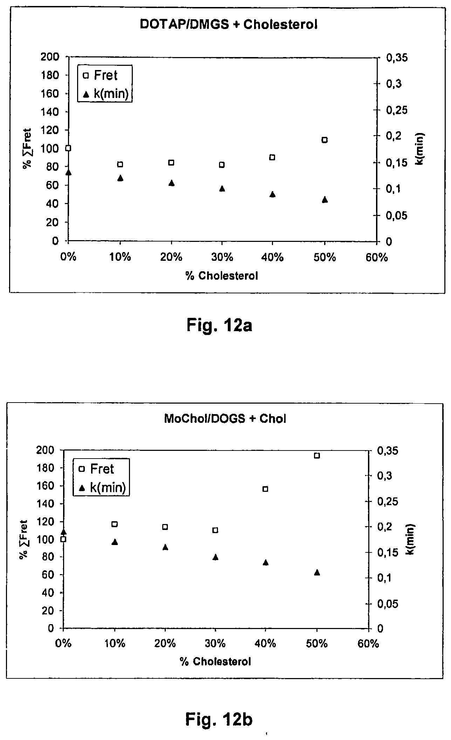

Figs. 12a and 12b show plots of the intensity of fusion (expressed as %∑FRET in the matrix C/A=0.17-0.75 for DOTAP/DMGS; C/A=0.33-3 for MoChol/DOGS vs. pH) for liposomes from DOTAP/DMGS or MoChol/DOGS against κ(min) for mixtures with 0% - 50% cholesterol. The reference κ(min) was modelled for C/A=0.66 (DOTAP/DMGS) or C/A=1(MoChol/DOGS). The %∑FRET for 0% cholesterol is set to 100.

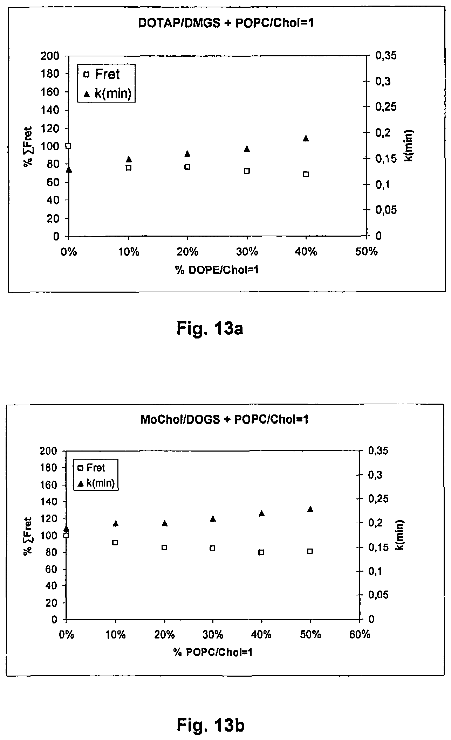

Figs. 13a and 13b show plots of the intensity of fusion (expressed as %∑FRET in the matrix C/A=0.17-0.75 for DOTAP/DMGS; C/A=0.33-3 for MoChol/DOGS vs. pH) for liposomes from DOTAP/DMGS or MoChol/DOGS against κ(min) for mixtures with 0% - 50% of a mixture POPC/cholesterol 1:1. The reference κ(min) was modelled for C/A=0.66 (DOTAP/DMGS) or C/A=1(MoChol/DOGS). The %∑FRET for 0% POPC/cholesterol is set to 100.

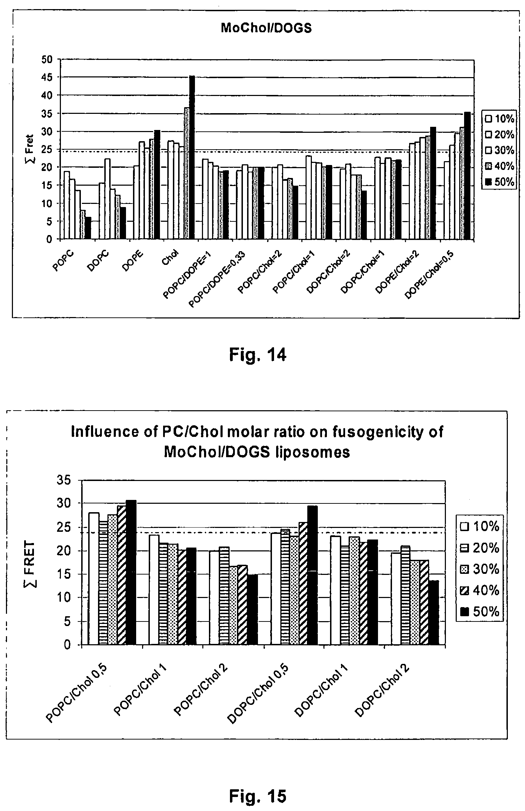

Fig. 14 shows the intensity of fusion (expressed as ∑FRET in the matrix C/A=0.33-3 vs. pH) of liposomes comprising MoChol/DOGS and 10% - 50% of different neutral or zwitterionic lipids. The dotted line indicates the intensity of fusion of the liposomes with 0% neutral or zwitterionic lipid.
Fig. 15 shows the intensity of fusion (expressed as ∑FRET in the matrix C/A=0.33-3 vs. pH) of liposomes comprising MoChol/DOGS and 10% - 50% of different POPC/Chol mixtures. The dotted line indicates the intensity of fusion of the liposomes with 0% neutral or zwitterionic lipid.

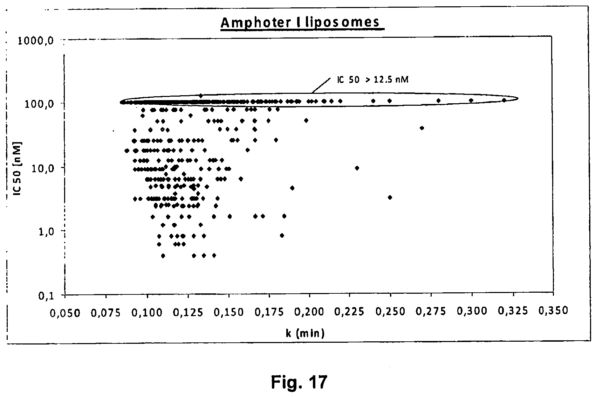

Fig. 17 shows a plot of IC50 values vs. κ(min) values of all amphoter I liposomes including neutral and/or zwitterionic lipids from table 76 encapsulating siRNA targeting Plk-1 (IC50 values derived from the in vitro transfection of Hela cells as described in example 8)

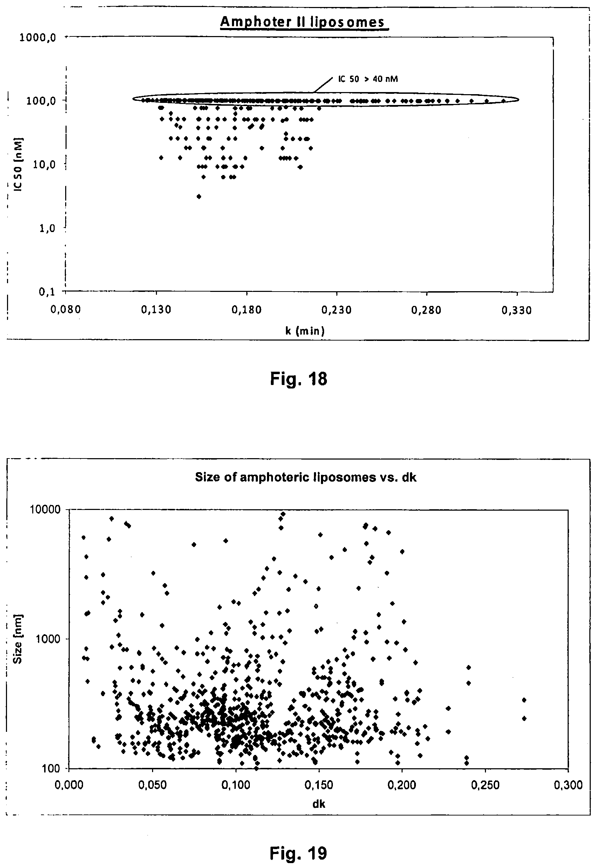

Fig. 18 shows a plot of IC50 values vs. κ(min) values of all amphoter II liposomes including neutral and/or zwitterionic lipids from table 77 encapsulating siRNA targeting Plk-1 (IC50 values derived from the in vitro transfection of Hela cells as described in example 8)
Fig. 19 shows a plot of the size vs. dx(pH 8) of all amphoteric liposomes comprising neutral and/or zwitterionic lipids from table 76 and 77.

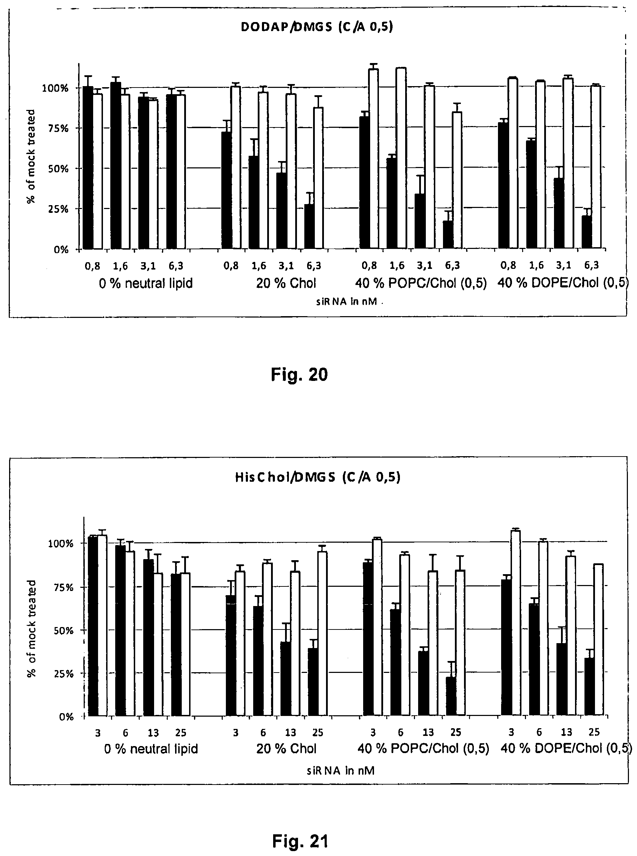

Fig. 20 shows the % cell viability (normalized to mock treated cells) of Hela cells transfected with different DODAP/DMGS (C/A=0.5) amphoteric liposomes encapsulating siRNA targeting Plk-1 (black bars) or non-targeting scrambled siRNA (grey bars) and comprising either no or different neutral and/or zwitterionic lipids in the molar amounts as indicated.
Fig: 21 shows the % cell viability (normalized to mock treated cells) of Hela cells transfected with different HisChol/DMGS (C/A=0.5) amphoteric liposomes encapsulating siRNA targeting Plk-1 (black bars) or non-targeting scrambled siRNA (grey bars) and comprising either no or different neutral and/or zwitterionic lipids in the molar amounts as indicated.
Fig. 22 shows the % cell viability (normalized to mock treated cells) of Hela cells transfected with different Chim/DMGS (C/A=0.5) amphoteric liposomes encapsulating siRNA targeting Plk-1 (black bars) or non-targeting scrambled siRNA (grey bars) and comprising increasing molar amounts of POPC/Chol mixtures (molar ratio 0.5) as neutral or zwitterionic lipid.
Fig. 23 shows the % cell viability (normalized to mock treated cells) of Hela cells transfected with different DC-Chol/DMGS (C/A=0.5) amphoteric liposomes encapsulating siRNA targeting Plk-1 (black bars) or non-targeting scrambled siRNA (grey bars) and comprising increasing molar amounts of POPC/Chol mixtures (molar ratio 0.5) as neutral or zwitterionic lipid.
Fig. 24 shows different plots of IC50 values vs. IP values from two different amphoteric lipid mixtures (HisChol/DMGS and DODAP/DMGS) with different IPs which comprises different neutral and/or zwitterionic lipids in the molar amounts as indicated and encapsulating siRNA targeting Plk-1.
Fig. 25 shows the relative ApoB expression in % (compared to untreated cells) of primary mouse hepatocytes transfected with DOTAP/DOGS/Chol 15:45:40 amphoteric liposome formulations encapsulating siRNA targeting ApoB100 or non-targeting scrambled (scr)siRNA, respectively.
Fig. 26 shows the relative ApoB expression in % (compared to untreated cells) of primary mouse hepatocytes transfected with DODAP/DMGS/Chol 24:36:40 amphoteric liposome formulations encapsulating siRNA targeting ApoB100 or non-targeting scrambled (scr)siRNA, respectively.
Fig. 27 shows the signals of Cy5.5 labelled siRNA (as average intensity) of cryosections from liver and spleen of mice 2h after tail vein injection of liposomal formulations F5, F7 and F8.

### Detailed description of the invention

By "chargeable" is meant that the amphiphile has a pK in the range pH 4 to pH 8. A chargeable amphiphile may therefore be a weak acid or base. A "stable" amphiphile is a strong acid or base, having a substantially stable charge on the range pH 4 to pH 8.

By "amphoteric" herein is meant a substance, a mixture of substances or a supra-molecular complex (e.g., a liposome) comprising charged groups of both anionic and cationic character wherein:
1) at least one, and optionally both, of the cation and anionic amphiphiles is chargeable, having at least one charged group with a pK between 4 and 8,
2) the cationic charge prevails at pH 4, and
3) the anionic charge prevails at pH 8.

As a result the substance or mixture of substances has an isoelectric point of neutral net charge between pH 4 and pH 8. Amphoteric character is by this definition different from zwitterionic character, as zwitterions do not have a pK in the range mentioned above. In consequence, zwitterions are essentially neutrally charged over a range of pH values; phosphatidylcholines and phosphatidylethanolamines are neutral lipids with zwitterionic character.

By "C/A" or "C/A ratio" or "C/A molar ratio" herein is meant the molar ratio of cationic amphiphiles to anionic amphiphiles in a mixture of amphiphiles.

By "*κ*(min)" herein is meant the minimum of the function *^{κ}*total^{(pH)}

By "*κ*(neutral)" herein is meant the *κ* value of a neutral or zwitterionic lipid or mixtures thereof.

By "IC50" herein is meant the inhibitory concentration of an oligonucleotide leading to a 50 % knockdown of a target mRNA or in case of a proliferation assay to a 50 % inhibition of cell viability.

The following list of lipids includes specific examples of neutral, zwitterionic, anionic, cationic or amphoteric lipids. The lipid list by no means limits the scope of this disclosure.

The abbreviations for the lipids are used herein, the majority of which abbreviations are in standard use in the literature:

**Neutral or zwitterionic lipids:**

| | |
|---|---|
| PC | Phosphatidylcholine (unspecified membrane anchor) |
| PE | Phosphatidylethanolamine(unspecifiedmembrane anchor) |
| SM | Sphingomyelin (unspecified membrane anchor) |
| DMPC | Dimyristoylphosphatidylcholine |
| DPPC | Dipalmitoylphosphatidylcholine |
| DSPC | Distearoylphosphatidylcholine |
| POPC | 1-Palmitoyl-2-oleoylphosphatidylcholine |
| DOPC | Dioleoylphosphatidylcholine |
| DOPE | Dioleoylphosphatidylethanolamine |
| DMPE | Dimyristoylphosphatidylethanolamine |
| DPPE | Dipalmitoylphosphatidylethanolamine |
| DPhyPE | Diphytanoylphosphatidylethanolamine |
| DlinPE | Dilinoleoylphosphatidylethanolamine |
| Chol | Cholesterol |

Any dialkyl derivatives of the neutral or zwitterionic lipids comprising diacyl groups listed above are also within the scope of the present invention.

### Anionic lipids:

- CHEMS: Cholesterolhemisuccinate
- Chol-COOH or Chol-Cl: Cholesteryl-3-carboxylic acid
- Chol-C2: Cholesterolhemioxalate
- Chol-C3: Cholesterolhemimalonate
- Chol-C3N: N-(Cholesteryl-oxycarbonyl)glycine
- Chol-C5: Cholesterolhemiglutarate
- Chol-C6: Cholesterolhemiadipate
- Chol-C7: Cholesterolhemipimelate
- Chol-C8: Cholesterolhemisuberate
- Chol-C12: Cholesterolhemidodecane dicarboxylic acid
- Chol-C13N: 12-Cholesteryloxycarbonylaminododecanoic acid

Cholesterolhemidicarboxylic acids and Cholesteryloxycarbonylaminocarboxylic acids of following general formula: 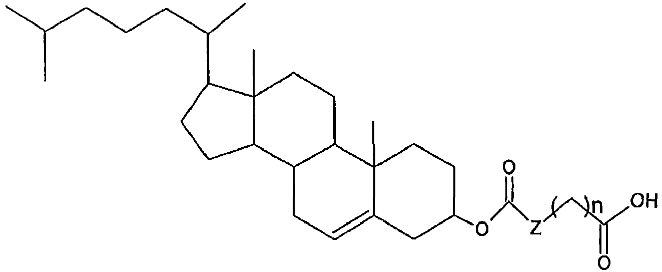 wherein Z is C or -NH- and n is any of between 1 and 29.

DGS or DG-Succ Diacylglycerolhemisuccinate (unspecified membrane anchor)
- DOGS or DOG-Succ: Dioleoylglycerolhemisuccinate
- DMGS or DMG-Succ: Dimyristoylglycerolhemisuccinate
- DPGS or DPG-Succ: Dipalmitoylglycerolhemisuccinate
- DSGS or DSG-Succ: Distearoylglycerolhemisuccinate
- POGS or POG-Succ: 1-Palmitoyl-2-oleoylglycerolhemisuccinate
- DOGM: Dioleoylglycerolhemimalonate
- DOGG: Dioleoylglycerolhemiglutarate
- DOGA: Dioleoylglycerolhemiadipate
- DMGM: Dimyristoylglycerolhemimalonate
- DMGG: Dimyristoylglycerolhemiglutarate
- DMGA: Dimyristoylglycerolhemiadipate
- DOAS: 4-{(2,3-Dioleoyl-propyl)amino}-4-oxobutanoic acid
- DOAM: 3-{(2,3-Dioleoyl-propyl)amino}-3-oxopropanoic acid
- DOAG: 5-{(2,3-Dioleoyl-propyl)amino}-5-oxopentanoic acid
- DOAA: 6-{(2,3-Dioleoyl-propyl)amino}-6-oxohexanoic acid
- DMAS: 4-{(2,3-Dimyristoyl-propyl)amino}-4-oxobutanoic acid
- DMAM: 3-{(2,3-Dimyristoyl-propyl)amino}-3-oxopropanoic acid
- DMAG: 5-{(2,3-Dimyristoyl-propyl)amino}-5-oxopentanoic acid
- DMAA: 6-{(2,3-Dimyristoyl-propyl)amino}-6-oxohexanoic acid
- DOP: 2,3-Dioleoyl-propanoic acid
- DOB: 3,4-Dioleoyl-butanoic acid
- DOS: 5,6-Dioleoyl-hexanoic acid
- DOM: 4,5-Dioleoyl-pentanoic acid
- DOG: 6,7-Dioleoyl-heptanoic acid
- DOA: 7,8-Dioleoyl-octanoic acid
- DMP: 2,3-Dimyristoyl-propanoic acid
- DMB: 3,4-Dimyristoyl-butanoic acid
- DMS: 5,6-Dimyristoyl-hexanoic acid
- DMM: 4,5-Dimyristoyl-pentanoic acid
- DMG: 6,7-Dimyristoyl-heptanoic acid
- DMA: 7,8-Dimyristoyl-octanoic acid
- DOG-GluA: Dioleoylglycerol-glucoronic acid (1- or 4-linked)
- DMG-GluA: Dimyristoylglycerol-glucoronic acid (1- or 4-linked)
- DO-cHA: Dioleoylglycerolhemicyclohexane-1,4-dicarboxylic acid
- DM-cHA: Dimyristoylglycerolhemicyclohexane-1,4-dicarboxylic acid
- PS: Phosphatidylserine (unspecified membrane anchor)
- DOPS: Dioleoylphosphatidylserine
- DPPS: Dipalmitoylphosphatidylserine
- PG: Phosphatidylglycerol (unspecified membrane anchor)
- DOPG: Dioleoylphosphatidylglycerol
- DPPG: Dipalmitoylphosphatidylglycerol
- Chol-SO4: Cholesterol sulphate
- PA: phosphatidic acid (unspecified membrane anchor)
- DOPA: Dioleoylphosphatidic acid
- SDS: Sodium dodecyl sulphate
- Cet-P: Cetylphosphate
- MA: Myristic Acid
- PA: Palmitic Acid
- OA: Oleic Acid
- LA: Linoleic Acid
- SA: Stearic Acid
- NA: Nervonic Acid
- BA: Behenic Acid

Any dialkyl derivatives of the anionic lipids comprising diacyl groups listed above are also within the scope of the present invention.

### Cationic lipids:

- MoChol: 4-(2-Aminoethyl)-Morpholino-Cholesterolhemisuccinate
- HisChol: Histaminyl-Cholesterolhemisuccinate
- CHIM: Cholesterol-(3-imidazol-1-yl propyl)carbamate
- DmC4Mo2: 4-(2-Aminoethyl)-Morpholino-Cholesterol-2,3-dimethylhemisuccinate
- DmC3Mo2: 4-(2-Aminoethyl)-Morpholino-Cholesterol-2,2-dimethylhemimalonate
- C3Mo2: 4-(2-Aminoethyl)-Morpholino-Cholesterol-hemimalonate
- C3Mo3: 4-(2-Aminopropyl)-Morpholino-Cholesterol-hemimalonate
- C4Mo4: 4-(2-Aminobutyl)-Morpholino-Cholesterol-hemisuccinate
- C5Mo2: 4-(2-Aminoethyl)-Morpholino-Cholesterol-hemiglutarate
- C6Mo2: 4-(2-Aminoethyl)-Morpholino-Cholesterol- hemiadipate
- C8Mo2: 4-(2-Aminoethyl)-Morpholino-Cholesterol- hemiadipate
- Chol-C3N-Mo3: [(3-Morpholine-4-yl-propylcarbamoyl)-methyl]-carbamic acid cholesteryl ester
- Chol-C3N-Mo2: [(2-Morpholine-4-yl-ethylcarbamoyl)methyl]-carbamic acid cholesteryl ester
- Chol-C4N-Mo2: [(2-Morpholine-4-yl-ethylcarbamoyl)-ethyl]-carbamic acid cholesteryl ester
- Chol-DMC3N-Mo2: [1-Methyl-2-(2-morpholine-4-yl-ethylcarbamoyl)-propyl]-carbamic acid cholesteryl ester
- Chol-C4Hex-Mo2: 2-(2-Morpholine-4-yl-ethylcarbamoyl)-cyclohexane carboxylic acid cholesteryl ester
- Chol-Betaine: Cholesteryl-oxycarbonyl-methyl-trimethylammonium chloride
- DDAB: Dimethyldioctadecylammonium bromide

1,2-Diacyl-3-Trimethylammonium-Propane
e.g.
- DOTAP: 1,2-Dioleoyl-3-Trimethylammonium-Propane
- DMTAP: 1,2-Dimyristoyl-3-Trimethylammonium-Propane
- DPTAP: 1,2-Dipalmitoyl-3-Trimethylammonium-Propane
- DSTAP: 1,2-Distearoyl-3-Trimethylammonium-Propane
- POTAP: Palmitoyloleoyl-3-Trimethylammonium-Propane

1,2-Diacyl-3-Dimethylhydroxyethylammonium-Propane
e.g.
- DODMHEAP or DORI: 1,2-Dioleoyl-3-dimethylhydroxyethyl-ammonium-Propane
- DMDMHEAP or DMRI: 1,2-Dimyristoyl-3-dimethylhydroxyethyl-ammonium-Propane
- DPDMHEAP or DPRI: 1,2-Dipalmitoyl-3-dimethylhydroxyethyl-ammonium-Propane
- DSDMHEAP or DSRI: 1,2-Distearoyl-3-dimethylhydroxyethyl-ammonium-Propane
- PODMHEAP or PORI: Palmitoyloleoyl-3-dimethylhydroxyethyl-ammonium-Propane

1,2-Diacyl-3-methyldihydroxyethylammonium-Propane
e.g.
- DOMDHEAP: 1,2-Dioleoyl-3-methyldihydroxyethylammonium-Propane
- DMMDHEAP: 1,2-Dimyristoyl-3-methyldihydroxyethylammonium-Propane
- DPMDHEAP: 1,2-Dipalmitoyl-3-methyldihydroxyethylammonium-Propane
- DSMDHEAP: 1,2-Distearoyl-3-methyldihydroxyethylammonium-Propane
- POMDHEAP: Palmitoyloleoyl-3-methyldihydroxyethyl-ammonium-Propane

1,2-Diacyl-3-Dimethylammonium-Propane
e.g.
- DODAP: 1,2-Dioleoyl-3-Dimethylammonium-Propane
- DMDAP: 1,2-Dimyristoyl-3-Dimethylammonium-Propane
- DPDAP: 1,2-Dipalmitoyl-3-Dimethylammonium-Propane
- DSDAP: 1,2-Distearoyl-3-Dimethylammonium-Propane
- PODAP: Palmitoyloleoyl-3-Dimethylammonium-Propane

1,2-Diacyl-3-methylhydroxyethylammonium-Propane
e.g.
- DOMHEAP: 1,2-Dioleoyl-3-methylhydroxyethylammonium-Propane
- DMMHEAP: 1,2-Dimyristoyl-3-methylhydroxyethylammonium-Propane
- DPMHEAP: 1,2-Dipalmitoyl-3-methylhydroxyethylammonium-Propane
- DSMHEAP: 1,2-Distearoyl-3-methylhydroxyethylammonium-Propane
- POMHEAP: Palmitoyloleoyl-3-methylhydroxyethylammonium-Propane

1,2-Diacyl-3-dihydroxyethylammonium-Propane
e.g.
- DODHEAP: 1,2-Dioleoyl-3-dihydroxyethylammonium-Propane
- DMDHEAP: 1,2-Dimyristoyl-3-dihydroxyethylammonium-Propane
- DPDHEAP: 1,2-Dipalmitoyl-3-dihydroxyethylammonium-Propane
- DSDHEAP: 1,2-Distearoyl-3-dihydroxyethylammonium-Propane
- PODHEAP: Palmitoyloleoyl-3-dihydroxyethylammonium-Propane

1,2-Diacyl-sn-Glycero-3-Ethylphosphocholine
e.g.
- DOEPC: 1,2-Dioleoyl-sn-Glycero-3-Ethylphosphocholine
- DMEPC: 1,2-Dimyristoyl-sn-Glycero-3-Ethylphosphocholine
- DPEPC: 1,2-Dipalmitoyl-sn-Glycero-3-Ethylphosphocholine
- DSEPC: 1,2-Distearoyl-sn-Glycero-3-Ethylphosphocholine
- POEPC: Palmitoyloleoyl-sn-Glycero-3-Ethylphosphocholine
- DOTMA: N-[1-(2,3-dioleyloxy)propyl]-N,N,N-trimethyl ammonium chloride
- DOTIM: 1-[2-(oleoyloxy)ethyl]-2-oleyl-3-(2-hydroxyethyl) imidazolinium chloride
- TMAG: N-(a-trimethylammonioacetyl)-didodecyl-D-glutamate chloride
- BCAT: O-(2R-1,2-di-O-(19Z,99Z-octadecadienyl)-glycerol)-N-(bis-2-aminoethyl) carbamate
- DODAC: Dioleyldimethylammonium chloride
- DORIE: 1,2-dioleyl-3-dimethyl-hydroxyethyl ammonium propane
- DMRIE: 1,2-dimyristyl-3-dimethyl-hydroxyethyl ammonium propane
- DOSC: 1,2-dioleoyl-3-succinyl-sn-glycerol choline ester
- DHMHAC: N,N-di-n-hexadecyl-N,N-dihydroxyethylammoniumbromide
- DHDEAB: N,N-di-n-hexadecyl-N-methyl,N-(2-hydroxyethyl)ammonium chloride
- DMHMAC: N,N-myristyl-N-(1-hydroxyprop-2-yl)-N-methylammoniumchloride
- DOTB: 1,2-dioleoyl-3-(4'-trimethylammonio)butanoyl-sn-glycerol
- DOSPA: 2,3-Dioleyloxy-N-[2-(sperminecarboxamido)ethyl]-N,N-dimethyl-1-propanaminium trifluoroacetate
- DOGS*: Dioctadecylamido-glycylspermine
- DOGSDSO: 1,2-dioleoyl-sn-glycero-3-succinyl-2-hydroxyethyl disulfide ornithine
- SAINT lipids: Synthetic Amphiphiles INTerdisciplinary
- DPIM, DOIM: 4,(2,3-bis-acyloxy-propyl)-1-methyl-1H-imidazole (unspecified membrane anchor)
- MoDP: 1,2-Dipalmitoyl-3-N-morpholine-propane
- MoDO: 1,2-Dioleoyl-3-N-morpholine-propane
- DPAPy: 2,3-bis-palmitoyl-propyl-pyridin-4-yl-amine
- DC-Chol: 3b-[N-(N9,N9-dimethylaminoethane)carbamoyl] cholesterol
- TC-Chol: 3b-[N-(N9,N9-trimethylaminoethane) carbamoyl] cholesterol
- DAC-Chol: 3b(N-(N,N'-Dimethylaminoethan)-carbamoyl)cholesterol
- PipC2Chol: 4{N-2-ethylamino[(3'-β-cholesteryl) carbamoyl]} piperazine
- MoC2Chol: {N-2-ethylamino[(3'-β-cholesteryl) carbamoyl]} morpholine
- MoC3Chol: (N-2-propylamino[(3'-β-cholesteryl) carbamoyl]} morpholine
- N-methyl-PipChol: N-methyl{4-N-amino[(3'-β-cholesteryl) carbamoyl]}piperazine
- PyrroC2Chol: {N-2-ethylamino[(3'-β-cholesteryl) carbamoyl]}pyrrolidine
- PipeC2Chol: {N-2-ethylamino[(3'-β-cholesteryl) carbamoyl] }piperidine
- ImC3Chol: {N-2-propylamino[(3'-β-cholesteryl) carbamoyl]}imidazole
- PyC2Chol: {N-2-ethylamino[(3'-β-cholesteryl) carbamoyl] }pyridine
- CTAB: Cetyltrimethylammonium bromide
- NeoPhectin™: cationic cardiolipins (e.g. [1,3-Bis-(1,2-bis-tetradecyloxy-propyl-3-dimethyl-ethoxyammoniumbromide)-propane-2-ol]

Any dialkyl derivatives of the cationic lipids comprising diacyl groups listed above are also within the scope of the present invention.

### Amphoteric lipids:

- HistChol: Nα-Histidinyl-Cholesterol-hemisuccinate
- HistDG: 1,2-Dipalmitoylglycerol-hemisuccinat-N_-Histidinyl-hemisuccinate, & Distearoyl-,Dimyristoyl, Dioleoyl or palmitoyl-oleoylderivatives
- IsoHistSuccDG: 1,2-Dipalmitoylglycerol-O-Histidinyl-Nα-hemisuccinat, & Distearoyl-, Dimyristoyl, Dioleoyl or palmitoyl-oleoylderivatives
- AC: Acylcarnosine, Stearyl- & Palmitoylcarnosine
- HCChol: Nα-Histidinyl-Cholesterolcarbamate

Any dialkyl derivatives of the amphoteric lipids comprising diacyl groups listed above are also within the scope of the present invention.
- MoChol: 4-(2-Aminoethyl)-Morpholino-Cholesterolhemisuccinate: 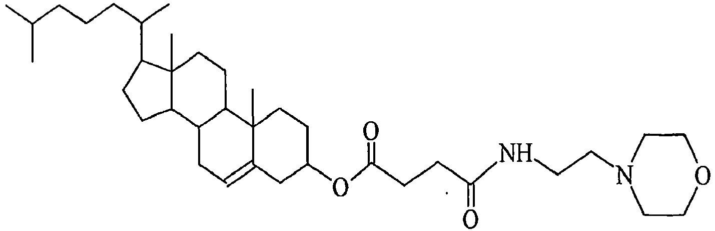
- HisChol: Histaminyl-Cholesterolhemisuccinate:

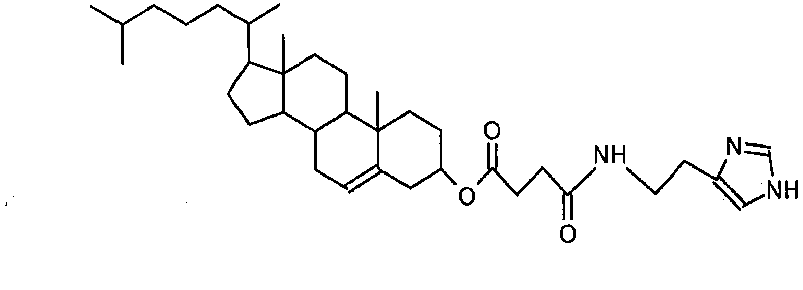

Disclosed herein are lipids which may be useful to prepare liposomes, especially amphoteric liposomes. These lipids may have the following general formula: 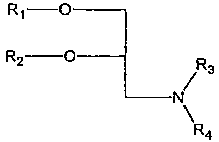 wherein R₁ and R₂ are independently C₈-C₃₀ alkyl or acyl chains with 0, 1 or 2 ethylenically unsaturated bonds or one of R₁ or R₂ may be H and wherein R3 is a non-branched, branched or cyclic alkyl, alkenyl, alkylene or alkynyl or an aryl group with 1 to 8 C-atoms, optionally substituted with -OH and wherein R4 is selected from one of the following structures:
or 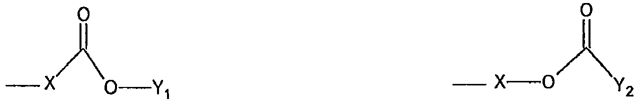 wherein X and Y₁ and Y₂ are independently non-branched, branched or cyclic alkyl, alkenyl; alkylene or alkynyl or a aryl group with 1 to 8 C-atoms, optionally substituted with -OH or Y₂ may be H.

Chemical representations of this class of lipids may include, but are not limited to:

| |
|---|
| 1,2-Diacyl-3-ethyl-(methoxycarbonyl-ethyl)ammonium-Propane |
| 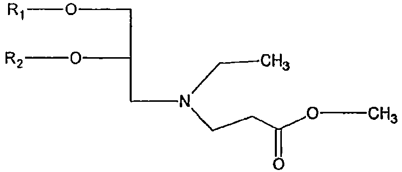 |
| 1,2-Diacyl-3-methyl-(methoxycarbonyl-ethyl)ammonium-Propane |
| 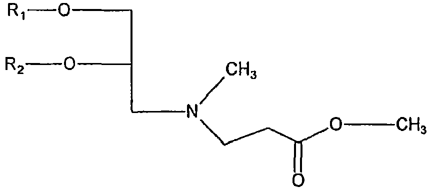 |
| 2-[(2,3-diacyloxypropyl)(methyl)amino]ethyl acetate |
| 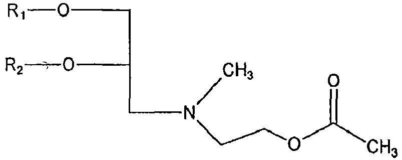 |

R1 and R2 are independently C₈-C₃₀ acyl chains with 0, 1 or 2 ethylenically unsaturated bonds or one of R₁ or R₂ may be H.

Specific lipids of said class of lipids include for example 1,2-Dioleoyl-3-methyl-(methoxycarbonyl-ethyl)ammonium-Propane (DOMCAP) or 1,2-Dioleoyl-3-methyl-(methoxycarbonylmethyl)ammonium-Propane (DOMGME).

The lipids may alternatively have one of the following general formulae: 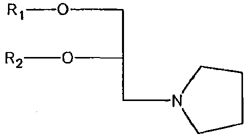 or 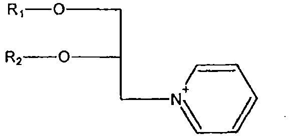 wherein R₁ and R₂ are independently C₈-C₃₀ alkyl or acyl chains with 0, 1 or 2 ethylenically unsaturated bonds or one of R₁ or R₂ may be H.

Specific lipids of said classes of lipids are for example 1,2-Dioleoyl-3-N-pyrrolidine-propane (DOP5P) or 1,2-Dioleoyl-3-N-pyrridinium-propane, bromide salt (DOP6P).

### Molecular volumes

Lipid shape theory is built on a shape balance between the hydrophobic part and the polar head-group of a given amphiphile rather than on absolute values for the two molecular portions. In accordance with the present invention, κ is the volume ratio between the polar and apolar section of a lipid. $κ = molecular volume \left(head\right) / molecular volume \left(tail\right)$

Various different ways are available to those skilled in the art to calculate molecular volumes and alternative methods and sources are discussed for example in Connolly, M. J. Am. Chem. Soc. (1985) 107, 1118 - 1124 and the references therein or are given at: http://www.ccl.net/cca/documents/molecular-modeling/node5.html

Molecular volume is commonly calculated by assigning a value called a van der Waals radius, rⁱ_{vdW}, to each atom type in such a way that the sum of these quantities for a given atom pair, i and j, is equal to their closest possible distance (dij): ${{r}^{i}}_{vdw} + {{r}^{j}}_{vdw} \leq dij$

Many different tables of "best" van der Waals radii exist, even though the values for corresponding atoms coming from different authors are similar. In geometric terms, the van der Waals radius may be imagined as a spherical "shield" surrounding the atom, and the closest distance between two non-bonded atoms is when their respective shields touch. However, the shields of covalently bonded atoms intersect since bond lengths are shorter than the sum of the van der Waals radii partaking atoms. A molecular van der Waals surface, also called a van der Waals envelope, is composed of the spheres for individual atoms with their intersecting sections removed.

For a single molecule (i.e., molecule for which there is a path between any two atoms along covalent bonds), the van der Waals envelope is a closed surface, and hence, it contains volume. This volume is called the molecular volume, or van der Waals volume and is usually given in Å³. The straightforward way of calculating molecular volume on a computer is by numerical integration.

In some embodiments, molecular volumes for lipid molecules and the respective head and tail fragments may be calculated using DS Viewer Pro 5.0 (Accelrys Inc., San Diego, CA) and volumes within the respective van der Waals radii were calculated.

Typical membrane fragments are 1,2-diacyl-ethyleneglycols that represent the hydrophobic section for common phospholipids, leaving the 3' carbon atom of the original glycerol with the phosphocholine head-group. The same fragment is also found in the common cationic lipid DOTAP and its derivatives but also in diacylglycerols with other polar head-groups such as dimyristoylglycerol hemisuccinate and the like.

For the cholesterol derivatives, the entire sterol, but not the 3' oxygene, is defined as the hydrophobic section and the head-group being complementary to that.

Likewise, for cationic or anionic alkyl derivatives the polar head-group is defined as the polar fragment involving the C1 carbon of the alkyl chain. Consequently, the residual chain with n-1 carbon atoms represents the hydrophobic apolar part.

Molecular volumes depend on the constants used for the calculations and may be affected by the conformation of the molecule. Typical values obtained for the hydrophobic apolar fragments are and were used for further calculations:

**Table:1**

| **Membrane fragment** | **Volume in Å³** | |
|---|---|---|
| di-lauroylethyleneglycol | 356 | |
| di-myristoylethyleneglycol | 407 | |
| di-palmitoylethyleneglycol | 458 | |
| di-stearoylethyleneglycol | 509 | |
| di-oleoylethyleneglycol | 501 | |
| Palmitoyl-oleoylethyleneglycol | 478 | |
| di-phytanoylethylenglycol | 566 | |
| di-oleylethyleneglycol (e.g., in DOTMA) | 495 | |
| di-palmitylethylenglycol | 452 | |
| Didoceyl-D-glutamate (e.g., in TMAG) | 395 | |
| Cholesteryl | 334 | |
| C11 hydrophobic part in lauryl derivatives | 132 | |
| C13 hydrophobic part in myristyl derivatives | 158 | |
| C15 hydrophobic part in palmityl derivatives | 184 | |
| C17 hydrophobic part in stearyl derivatives | 210 | |
| C17 hydrophobic part in oleyl derivatives | 208 | |
| Sphingomyelin / Ceramide | 467 | |
| backbone | | |

Molecular volumes for most counter-anions were derived the same way, but for Na+ or K+ the strongly bound hydration sphere is taken into account. The following values were used for further calculations:

**Table 2:**

| **Counterion** | **Volume in Å³** | |
|---|---|---|
| Acetate⁻ | 40 | |
| Citrate⁻ | 121 | |
| Phosphate²⁻ | 49 | |
| Chloride⁻ | 21 | |
| Formiate⁻ | 29 | |
| PF₆ ⁻ | 51 | |
| Methylsulfate⁻ | 64 | |
| Trifluoroacetate⁻ | 56 | |
| Barbituric acid | 79 | |
| Pyrophosphate⁴⁻ | 88 | |
| Sodium⁺ | 65¹⁾ to 88²⁾ | Hydrated radii are 2,5A and 2,76A, respectively |
| Potassium⁺ | 24¹⁾ to 52²⁾ | Hydrated radii are 1,8A and 2,32A, respectively |
| Lithium⁺ | 164 ²⁾ | |
| Imidazolium⁺ | 52 | |
| Morpholinium⁺ | 69 | |
| Tris(hydroxymethyl)-aminoethan⁺ | 91 | |
| Tris(hydroxyethyl)-aminoethan⁺ | 130 | |
| Bis(hydroxymethyl)-aminoethan⁺ | 74 | |
| Hydroxymethyl-aminoethan⁺ | 50 | |
| Bis(hydroxymethyl)hydroxyethyl-aminoethan⁺ | 107 | |
| Bis(hydroxyethyl)hydroxymethyl-aminoethan⁺ | 123 | |
| Triethylamine⁺ | 92 | |
| Diethyl-hydroxyethyl-amine⁺ | 100 | |
| Arginine⁺ | 135 | |
| Glucoronic acid⁻ | 129 | |
| Malonic acid⁻ | 66 | |
| Tartaric acid⁻ | 97 | |
| Glucosamine⁺ | 129 | |

| | | |
|---|---|---|
| 1) Gerald H. Pollack: Cells, Gels and the Engines of Life, Ebner and Sons Publishers, 2001 2) http://www.bbc.co.uk/dna/h2g2/A1002709#footnote1 | | |

The charged polar head-groups have different representations and the molecular volumes are given below in this description in tables 59, 60 and 61 for some individual members of this group.

**Table: 3**

| **Polar head-groups (neutral or zwitterionic)** | **Volume in Å³** | |
|---|---|---|
| Phosphocholine | 133 | |
| Phosphoethanolamine | 97 | |
| Cholesterol head group | 30 | |

It is possible to use other methods to determine molecular volumes for the lipids. Also, some parameters such as the exact split-point between membrane tail and polar head; number of water molecules in the hydration cage or the van der Waals radii can be varied without affecting the general applicability of the model. With the same understanding more subtle changes in the molecular volumes may be disregarded, in particular those arising from the dissociation of protons or from conformational changes. In some embodiments the molecular volumes recited in Tables 1, 2, 3, 59, 60 and 61 may be used in the present invention.

The counterions fall into the same category of sizes than the actual polar head-groups. As such, it has been found that the addition or withdrawal of counterions from lipid polar regions has a substantial effect on the total head-group size and in consequence on the head/tail balance κ. As an example, the CHEMS sodium salt has a head-group size of 141 Å³ which is reduced to 76 A³ in the undissociated form at pH 4. κ varies between 0.42 and 0.23, respectively. CHEMS does form a lamellar phase at pH 7.5 and higher but adopts a hexagonal phase at low pH.

Other lipids with known phase behaviour can be used to select κ values for discrimination between the lamellar and hexagonal phase; an example is given in Table 4 below. PE head-groups can form an intramolecular ring structure with hydrogen bonding between the terminal amino group and the oxygen in the phosphoester group (betaine structure)(e.g. Pohle et al., J. Mol. Struct.,408/409, (1997), 273-277). PC head-groups are sterically hindered and instead recruit counterions to their respective charged groups.

**Table 4:**

| **Lipid or mixture** | **κ.** | **Phase behaviour** |
|---|---|---|
| POPC | 0.51 | Lamellar |
| DOPE | 0.19 | Hexagonal |
| Cholesterol | 0,09 | Hexagonal |

### pH induced changes of molecular volumes in amphoteric lipid mixtures

In a first model no lipid salt formation occurs between charged anionic and cationic lipids. This reflects the assumptions of Li and Schick (Biophys. J., 2001, 80, 1703-1711) and might be the case for lipids that are sterically hindered to form lipid salts (independent ion model).

The lipid species in the membrane comprise undissociated anions and cations as well as the dissociated anions and cations, the latter being complexed with their respective counterions. The κ value for such a mixture is assumed to be the weighted sum of its components: $κ = κ \left({anion}^{0}\right) * c \left({anion}^{0}\right) + κ \left({anion}^{0}\right) * c \left({anion}^{0}\right) + κ \left({anion}^{-}\right) * c \left({anion}^{-}\right) + κ \left({cation}^{+}\right) * c \left({cation}^{+}\right) ;$ wherein anion° or cation° denotes the uncharged species and anion⁻ or cation⁺ denotes the respective charged species; and wherein c herein denotes concentration.

The amounts of the individual species present under such assumption can be calculated from known equilibrium constants K for the acid or base dissociation: $c \left({anion}^{-}\right) = c \left({anion}^{tot}\right) / \left({c}_{H +}/K+1\right)$ $c \left({anion}^{0}\right) = c \left({anion}^{tot}\right) - c ( {anion}^{- )}$ $c \left({cation}^{+}\right) = c \left({cation}^{tot}\right) / \left(K/{c}_{H +}+l\right)$ $c \left({cation}^{0}\right) = c \left({cation}^{tot}\right) - c \left({cation}^{+}\right) ;$ wherein anion° is the undissociated anion, anion⁻ the negatively charged molecule and anion^{tot} the total concentration of the respective anion. Cations follow the same nomenclature and c_{H+} and K describe the proton concentration and the equilibrium constant for the acid or base, respectively.

However, taking possible interaction between a cationic and anionic amphiphile into account the lipid salt occurs as a fifth species in the mixture: $κ = κ \left({anion}^{0}\right) * c \left({anion}^{0}\right) + κ \left({anion}^{0}\right) * c \left({anion}^{0}\right) + κ \left({anion}^{-}\right) * c \left({anion}^{-}\right) + κ \left({cation}^{+}\right) * c \left({cation}^{+}\right) + κ \left(salt\right) * c \left(salt\right)$

In a lipid salt, the cationic amphiphile serves as a counterion to the anionic amphiphile and vice versa thus displacing the small counterions like sodium or phosphate from the head-group. The lipid salt is net uncharged and its geometry has to be assumed to be the sum of both parts without the small counterions. Therefore: $κ \left(salt\right) = \left({v}_{head}\left(cation\right)+{v}_{head}\left(anion\right)\right) / \left({v}_{apolar}\left(cation\right)+{v}_{apolar}\left(anion\right)\right)$

Salt formation is limited by the charged amphiphile that is present in the lowest concentration: $c \left(salt\right) = MIN \left(c\left({cation}^{+}\right);c\left({anion}^{-}\right)\right)$

Salt formation between the two charged amphiphiles is assumed to be complete within this model, but of course, an incomplete salt formation may be assumed. The following calculations further reflect the fact that the salt comprises two lipid molecules. It is of course possible to assume further some membrane contraction upon lipid salt formation and to put a different weight on the contribution of k(salt).

### Model Calculations

To achieve amphoteric character of a lipid mix; at least one of the lipid ions needs to be a pH -sensitive, weak acid or base ("chargeable"). A detailed disclosure is found in WO 02/066012

Being different in character, three basic systems are possible and are analysed here:
"Amphoter I" strong cation and weak anion,
"Amphoter II" weak cation and weak anion,
"Amphoter III" weak cation and strong anion.

### a. Amphoter I systems

Amphoter I systems need an excess of the pH-sensitive anion to achieve amphoteric character. At pH 7 to 8 the anionic lipid is fully charged and salt formation occurs until all cationic lipids are consumed. In an example with 70 mol.% anionic lipid and 30 mol.% cationic lipid, all cationic lipid and a corresponding 30 mol.% of the anionic lipid would exist as lipid salt while 40 mol.% of the anionic lipid is unbound and recruits its counterion to the head-group.

Starting from neutral conditions, a reduction of the pH discharges the anionic lipid, the κ value becomes smaller owing to loss of the counterion and reaches a minimum when the portion of still-charged anionic lipid is equal to the amount of cationic lipid. Therefore, κ is minimal at the isoelectric point of the amphoteric lipid mixture. If the pH is further lowered, an increasingly smaller portion of the anionic lipid remains charged. This means dissociation of the lipid salt and recruitment of counterions, now to the cationic lipid liberated from the lipid salt.

The left panel in Fig. 1 of the accompanying drawings illustrates the complex behaviour of κ in dependence from pH and the amount of anionic lipid in the mixture. A "valley of fusogenicity" appears, and any amphoteric mixture having more than 55 mol.% and less than 85 mol.% anionic lipid is expected to fuse under slightly acidic conditions but to be stable both at neutrality and under more acidic conditions.

Amphoter I mixtures with less than 50 mol.% anionic lipid are no longer amphoteric since the anion can modulate, but not overcompensate, the charge on the cationic lipid. These mixtures might undergo a pH -dependent fusion, but do not provide a second stable phase at low pH. A 1:1 complex adopts a lamellar phase only at low pH and undergoes fusion at neutrality.

The parameters used for the calculations illustrated in Fig. 1 are given in Table 5 below; volumes in Å³.

**Table 5:**

| | |
|---|---|
| Anion head volume | 70 |
| Anion tail volume | 400 |
| Anion pK | 5 |
| Cation head volume | 70 |
| Cation tail volume | 400 |
| Cation pK | 15 |
| Counterion+ | 70 |
| Counterion- | 70 |

### b. Amphoter II systems

Amphoter II systems have the distinct advantage to be amphoteric over the entire range of anion: cation ratios and no charge overcompensation for the strong ion is needed as in Amphoter I or Amphoter III systems. A calculation for a model system is shown in Fig. 2.

The parameters used for the calculation are given in Table 6 below; all volumes in Å³.

**Table 6:**

| | |
|---|---|
| Anion head volume | 70 |
| Anion tail volume | 400 |
| Anion pK | 5 |
| Cation head volume | 70 |
| Cation tail volume | 400 |
| Cation pK | 6.5 |
| Counterion+ volume | 70 |
| Counterion-volume | 70 |

Again, the lipid salt model predicts stable states at neutral to slightly alkaline pH but also at slightly acidic pH and a pronounced valley of instability or fusogenicity in between.

In contrast to amphoter I systems, fusogenic states can be reached across a wide range of different lipid ratios between the anionic and cationic components. That is, the valley of fusogenicity extends across a wider range of anion/cation ratios, allowing a greater degree of control over the pH at which a given system is fusogenic.

### c. Amphoter III mixtures

Amphoter III mixtures comprising a stable anion and a pH - sensitive cation cannot form lipid salts at neutral pH, since little to no charged cationic lipid exists at this pH. It needs ongoing acidification to first create the cation which then may undergo salt formation. Calculation for a model system is shown in Fig. 3.

The parameters used for the calculation are given in Table 7 below; all volumes in Å³.

**Table 7:**

| | |
|---|---|
| Anion head volume | 70 |
| Anion tail volume | 400 |
| Anion pK | 1 |
| Cation head volume | 70 |
| Cation tail volume | 400 |
| Cation pK | 6.5 |
| Counterion+ volume | 70 |
| Counterion-volume | 70 |

As can be seen from Figs. 1 and 3, amphoter III systems behave like the mirror image of amphoter I systems. They provide a valley of fusogenicity as long as the weak lipid ion is present in excess and over-compensates the constant charge on the opposite ion. In contrast to amphoter I systems the pH for fusion locates higher than the pK of the pH-sensitive lipid ion.

Experimental evidence for the fusion valley is given in the Examples 1 to 4 and provides confirmation for the central hypothesis of lipid salt formation in amphoteric liposomes.

The algorithm described here allows prediction of fusion behaviour of a wide range of amphoteric lipid mixtures. The prediction rules are derived from a simple geometrical description of the interacting lipids and are independent from the actual chemical representation of the molecules. As such, existing and novel lipid combinations can be easily tested by those skilled in the art, and the intended fusion behaviour can be predicted in a rational way. The following key parameters may illustrate such selection process, but other priorities might be set dependent on the respective goals of the application.

### κ of the lipid salt

κ of the lipid salt is calculated in equation (7) above and may suitably be lower than 0.34 or 0.35 to predict reasonably a fusogenic hexagonal phase. In some embodiments κ may be lower than 0.3; preferably lower than 0.25. κ(salt) is low when the combined polar head-groups are small and the combined hydrophobic portions are large. The preferred sum of head-group volumes is about 300 Å³ or smaller; in a more preferred embodiment this volume is smaller than 220 Å³, and an even more preferred value is smaller than 170 Å³. According to the selection made above, preferred sums for the tail group volumes are larger than 650Å³ and may be as large as about 1000Å³, wherein combinations of proper head and tail groups are governed by the preferred κ(salt) values.

### Amplitude of change (d(κ)/d(pH))

A lipid salt with a low value for κ may be stabilised below or above its isoelectric point by recruitment of counterions. In a preferred embodiment of the invention larger counterions are used to stabilise either the cationic or the anionic state of the amphoteric lipid mixture. Fig. 4 illustrates such dependence from counterions size for an amphoter II system. The parameters used for the calculation of Fig. 4 are given in Table 8 below.

**Table 8:**

| | |
|---|---|
| Anion head volume | 70 |
| Anion tail volume | 400 |
| Anion pK | 5 |
| Cation head volume | 70 |
| Cation tail volume | 400 |
| Cation pK | 6.5 |
| Counterion+ | See Fig. 4 |
| Counterion- | See Fig. 4 |

It becomes apparent from Fig. 4 that such stabilisation may be asymmetric, e.g., providing rather limited stabilisation for the cationic phase and more stabilisation of the anionic phase of the amphoteric lipid mix. Also, counterions that do not naturally exist in physiological body fluid may be used to improve stability during storage; exchange of such storage ions with the sodium ions present in the body fluids may be advantageous for discharging the cargo from the liposomes *in vivo.* Proper ion volumes for the individual or common stabilisation of a lipid phase may be selected. Such stabilisation is of particular use for the manufacturing and storage of amphoteric liposomes.

In some embodiments of the present invention larger counter-cations are used to stabilise the amphoteric liposomes at neutral conditions. In a preferred embodiment such counter-cations have a molecular volume of 50 Å³ or more, in a more preferred embodiment this volume exceeds 75 Å³ and said neutral pH is between pH 7 and pH 8, more preferred about the physiological pH of 7.4.

If amphoteric liposomes are produced for pharmaceutical purposes, compatibility of the used ions with the application route needs to be obeyed. Suitable counter-cations can be selected from Table 2 above describing the ion sizes. Preferred counter-cations for pharmaceutical compositions are sodium or the respective ionized forms of tris(hydroxymethyl)aminomethan, tris-hydroxyethylaminomethan, triethylamine, arginine, in particular L-arginine and the like.

In an embodiment of the invention the amphoteric liposomes may be manufactured at a low pH in their cationic state. Under these conditions, the liposomes can bind polyanions such as proteins, peptides or nucleic acids, whether as large plasmids or smaller oligonucleotides. Such binding is useful for improvement of the encapsulation efficacy of said materials into the amphoteric liposomes.

It is advantageous to use a lipid phase with a low κ at acidic pH. Selection of large counter-anions facilitates stabilisation of said lipid phase, e.g., for the production of such liposomes and the encapsulation of cargo under these conditions.

Suitable large counter-anions have a molecular volume larger than 50 Å³, preferred large counterions have a molecular volume larger than 75 Å³. Suitable counter-anions can be selected from Table 2 above. Preferred counter-anions are citrate, pyrophosphate, barbiturate, methyl sulphate and the like.

After having contacted the lipid phase with the cargo to be encapsulated under acidic conditions, the liposomes are then neutralized and non-encapsulated cargo can optionally be removed. Typically, non-encapsulated cargo detaches from the lipid membrane since both carry the same charge under neutral conditions. The amphoteric liposomes are negatively charged above their isoelectric point, e.g., at a pH between 7 and 8 and the cargo molecules exist as polyanions at such a pH. This is in particular the case with nucleic acids that carry one negative charge *per* nucleobase. Such liposomes can undergo effective destabilisation when exposed to the low pH in combination with a smaller counter-anion. This is for example the case after systemic administration and cellular uptake and endocytosis of such liposomes. Chloride or phosphate are the most common counter-anions in the body fluids of animals, be it any animal, a mammal or humans. Phosphate, but even more so chloride, are small counterions with little or no hydration shell and molecular volumes < 60 Å³.

Fig. 5 illustrates a cycle of liposome generation and use which illustrates selective stabilisation and destabilisation of the lipid phase under acidic conditions through asymmetric counterion use. The parameters used for the calculation of Fig. 5 are given in Table 9 below; volumes in Å³.

**Table 9:**

| | |
|---|---|
| Anion head volume | 70 |
| Anion tail volume | 400 |
| Anion pK | 5 |
| Cation head volume | 70 |
| Cation tail volume | 400 |
| Cation pK | 6.5 |
| Counterion+ | See Fig. 5 |
| Counterion- | See Fig. 5 |

### Isoelectric point

A mathematical description for the isoelectric point of amphoteric liposomes is been given in the WO 02/066012. The isoelectric point of the amphoteric liposomes can be adjusted to a wide range of conditions, and there is sufficient chemical representation for individual lipids with different pK dissociation constants that allows the skilled artisan to select useful components and combinations for the making of amphoteric liposomes. In addition, the isoelectric point for a given amphoteric lipid composition can be easily tuned through the molar ratio between the anionic and the cationic lipid as presented in Hafez et al., Biophys. J., 79, (2000), 1438-1446.

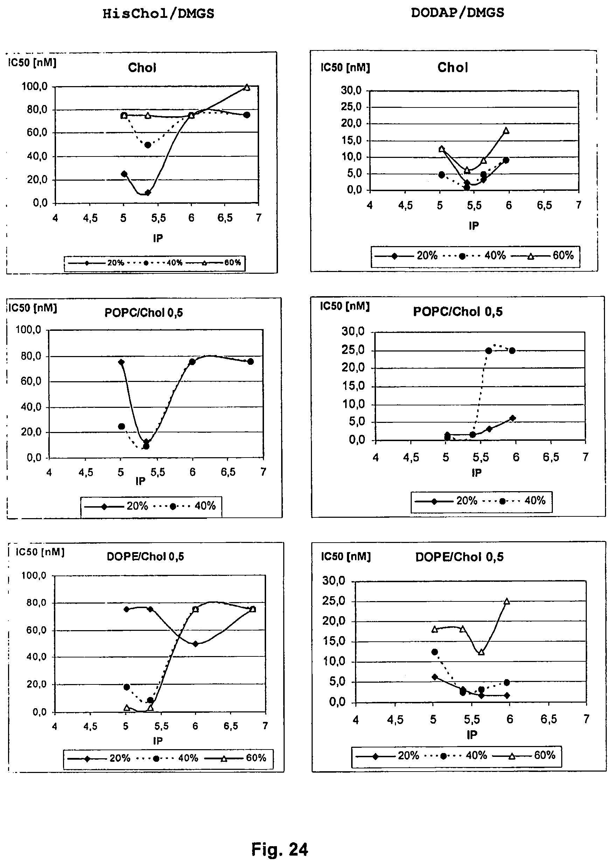

It has been found that the transfection efficieny of the inventive amphoteric liposomes depends on the isoelectric point of the amphoteric lipid mixtures. This is demonstrated in Fig.24 which surprisingly show that the inventive amphoteric liposomes including cholesterol or mixtures of cholesterol and PE or PC are more efficiently transfect cells within a specific range of isoelectric points.

In one embodiment of the present invention the isoelectric point of the inventive amphoteric liposomes is between 4 and 7, preferably between 4.5 and 6.5 and most preferred between 5 and 6.

The algorithm presented above provides structure-activity relationships between lipid chemistry and stability of the resulting membrane, in particular in response to the pH of the environment. Experimental data further illustrate this relationship and justify the model predictions (e.g. Examples 2, 3, 4 and corresponding Figs. 7, 8 and 9. In addition the model predicts fusion around the isoelectric point of the lipid mixture. Such correlation can be demonstrated in the experiment and is analyzed in Fig. 16.

The data provided above show a high degree of predictability from model calculations. The algorithm, starting from molecular volume considerations and rather long range interactions of electrical charges, does not reflect steric fit or misfit of the components; it also does not take phase transition temperatures and the associated molecular movements into account which might occur in isolated cases.

### In silico screening of amphoteric systems

The quantitative structure-activity relationships taught by the algorithm described above facilitate *in silico* screening and support rational selection and optimization. Such screening may be used on its own or in combination with empirical verification, e.g., by the inclusion of selected data points within a series of lipid homologues or use of experimental parameters.

The algorithm enables the selection of amphoteric liposomes for a number of technical purposes. A more detailed analysis is given below of the use of such amphoteric liposomes in pharmaceutical applications. Amongst such pharmaceutical applications, parenteral administration and direct administration into the blood stream of a human or non-human animal, preferably a mammal is of particular importance. Amphoteric liposomes have specific applicability *inter alia* in the intracellular delivery of cargo molecules. As described above, during uptake into the cells, liposomes are exposed to an acidic environment in the endosome or lysosome of cells. Destabilisation of the lipid phase, e.g., by enhanced fusogenicity is known to facilitate endosome escape and intracellular delivery. It is possible that other environments of low pH will also trigger said fusion, e.g., the low pH conditions found in tumors or at sites of inflammation. Amphoteric liposomes with a preferred low value of κ(salt) have been found to respond advantageously to acidification by destabilisation or formation of a fusogenic phase as intended.

The difference between κ(salt) and κ(total) for acidic conditions is of less importance, since an unstable lipid phase under acidic conditions does not interfere with cellular uptake. In addition, methods to stabilise such lipid phase for production have been described above.

The analysis is sensitive to counter-cation size and the proportion of anionic lipid in the mixture. As mentioned above, larger counter-cations make the selection less stringent, since this parameter directly improves the dκ(pH8) which means that systems with a low amplitude become more functional. Although resulting in a more or less stringent selection, the counter-cation size does not change the observed overall pattern of selected systems. This fact effectively compensates the variability of counter-cation sizes that can be found in the literature.

The present invention aims to provide alternative formulations of amphoteric liposomes comprising neutral lipids.

Neutral lipids comprise structures such as phosphatidylcholine, phosphatidylethanolamine, sphingolipids or cholesterol and the like. As these lipids do not have pH responsive elements that would react between pH 3 and 8, no changes in the molecular geometry occur in this range. Depending on the individual κ values of the neutral lipids, dilution of the bistable behaviour of the amphoteric lipid pair occurs and the steepness of d(K)/d(pH) becomes smaller, as shown in Fig. 6. In addition, the curve in the phase diagram is shifted towards lower or higher values of κ, depending on the neutral lipid used for dilution of the charged lipids. The parameters used for the calculation of Fig. 6 are given in Table 10 below; volumes in Å³.

**Table 10:**

| | |
|---|---|
| Anion head volume | 70 |
| Anion tail volume | 400 |
| Anion pK | 5 |
| Cation head volume | 70 |
| Cation tail volume | 400 |
| Cation pK | 6.5 |
| Counterion+ volume | 70 |
| Counterion-volume | 70 |

Fig. 6 illustrates this behaviour for the addition of different neutral lipids with κ values of 0.5, 0.3 or 0.19, respectively, in combination with the amphoter II model system described above. The amplitude of the system is reduced from Δκ = 0.089 to 0.044, while the minimum value follows the κ for the individual neutral components.

The addition of neutral lipids may extend the zone of fusogenic behaviour and to this end neutral lipids with low values of κ may be employed. Such lipids have κ values of 0.25 or less; more preferred lipids have κ values of about 0.2. Typical examples of such lipids are phosphatidylethanolamines. Phosphatidylethanolamines are assumed to form internal salt bridges (betaine structures) between the terminal amino group and the phosphate; therefore no counterions are recruited to the head-groups.

Phosphatidylethanolamines with C14 to C18 alkyl chains are preferred lipids to modulated the fusogenicity of the amphoteric liposomes.

Cholesterol is another example of a lipid having low κ and might therefore extend the fusogenic behaviour of an amphoteric lipid system.

It is of course possible to use mixtures of different neutral lipids to optimize the balance between fusogenicity and stability of such systems.

The algorithm described above facilitates quantitative predictions to be made on the effect of neutral lipid admixtures to amphoteric lipid systems. Such admixtures may result in improved stability of the liposome; they might further result in better resistance against serum proteins or enhanced uptake into cells. Optimization of amphoteric systems is a challenging task on its own, owing to the large number of useful components. This task becomes even more complicated with the addition of further components and rational approaches are urgently needed.

For the in silico screening, amphoteric lipid systems with lipid head-group sizes between 40 and 190 A³ and lipid hydrophobic tail sizes of 340, 410 or 500 A³ have been analyzed in the presence of a counter-cation, specifically sodium (65 A³). The counter-anion is of less relevance for the presented screen, since the ion (i) does not participate in the lipid salt and (ii) does essentially not bind to the membrane at pH8.

For the purpose of this in silico analysis, the parameter k(salt) is replaced by its functional equivalent k(salt)n. Likewise, the parameter dk(pH8) is replaced with dk(pH8)n to indicate its use for the analysis of systems comprising neutral lipids.

To be stable under storage conditions or while in the blood stream, a certain difference between κ(total) at neutral pH and κ(salt)n is necessary. In preferred embodiments, such difference, referred to herein as dκ(pH8)n, may be greater than or to equal 0.08. As noted above, κ(salt)n is the dominant predictor for fusogenicity, whereas dκ(pH8)n >=0.08 is a necessary, but not sufficient condition. A scoring of selected systems was done using 1/κ(salt)n as a metric. High values indicate systems with good fusion and sufficient stability amplitude.

The following *in silico* screens of amphoter I and amphoter II and III systems provide a more general and experimentally unbiased selection of fusogenic amphoteric liposomes further including neutral lipids with low k. The calculations allow one skilled in the art to deduce amphiphiles with preferred head and tail sizes and subsequently to identify improved amphoteric lipid mixtures.

### Amphoter I systems further comprising neutral lipids

For amphoter I systems, full dissociation of the anionic amphiphile was assumed at pH 8. A library of 324 amphoter I lipid systems having a C/A=0.333 was constructed and preferred lipid systems having κ(salt)n <0.34 and dκ(pH 8)n >= 0.08 were selected from the entire population. Fitness of the selected systems is presented as 1/κ(salt)n in the table 11 for the addition of 30% cholesterol to the library.

Systems with the best fitness have small headgroups for the lipid anion and the lipid cation. Large lipid anion tails are restricted by dκ(pH8)n, while the cation tail size has less of an impact.

Addition of a strongly lamellar lipid such as POPC or DOPC results in more stringent selection without qualitative impact on the selection rules presented before.

### b. Amphoter II systems further comprising neutral lipids

For amphoter II systems, full dissociation of the anionic amphiphile was assumed at pH 8 and essentially no dissociation of the cationic amphiphile was assumed at this pH. Such selections also apply to amphoter III systems, as long as they contain 50% or less of the anionic amphiphile.

Libraries of cation-rich amphoter II or amphoter III systems (C/A=3) were constructed as described previously and highly functional systems were selected using κ(salt)n<0.34 and dκ(pH8)n>0.08 as criteria. Fitness of the selected systems is presented as 1/κ(salt)n in Table 12 for the addition of 30% cholesterol to the library.

The addition of cholesterol results in a selection that is substantially biased towards cationic lipids with large headgroups and this feature is sensitive towards κ(salt)n; smaller values of κ(salt)n shift this optimum towards smaller head groups. Preferred lipid anions have small headgroups.

Again, the addition of a lamellar lipid such as POPC or DOPC results in more stringent selection without qualitative impact on the selection rules presented before.

Libraries of equilibrated amphoter II systems (C/A=1) were also constructed and introduced into the selection scheme in the presence of 30% cholesterol in this library (table 13).

While the corresponding amphoter II library (C/A=1) from mixtures without neutral lipids has numerous positive systems, the addition of 30% cholesterol resulted in a very stringent selection. This is counterintuitive to the addition of a lipid that promotes fusion and illustrates the impact of dκ(pH8)n as a selection criterium. Sensitivity analysis reveals dK(pH8)n as a very stringent variable and reduction of this value rapidly eliminates the selection pressure.

In this group, the addition of a lamellar lipid such as POPC or DOPC had similar impact than the addition of cholesterol.

Libraries of anion-rich amphoter II systems (C/A=0.33) were also constructed and introduced into the selection scheme in the presence of 30% cholesterol in this library (table 14).

Here, some bias of the positive candidates towards larger anion head groups can be observed. However, this needs to be interpreted carefully since the fusion activity is always improving in the presence of small anionic headgroups.

Addition of lamellar lipids such as POPC or DOPC implies more stringent selection criteria, but do not qualitatively change the pattern of positive candidates.

### Selection of amphoteric liposomes comprising neutral or zwitterionic lipids

The fusogenicity of different amphoteric liposome mixtures comprising charged amphiphiles can be investigated using lipid fusion assays, particle growth or other methods known in the art, thereby allowing the identification of preferred mixtures. Lipid mixing can be tested with fluorescence resonance energy transfer (FRET), and experimental details are described in Example 5 wherein the fusion of amphoteric lipid mixtures was monitored within a pH range of between pH 2.5 and pH 7.5.

A further experimental approach for the identification of preferred mixtures of amphoteric liposome formulations includes the transfection of cells using different amphoteric liposome formulations as delivery vehicles, as described in examples 8, 9 and 10. The delivery of active agents, such as nucleic acid active agents, into cells or tissues *in vitro* and *in vivo* is still a challenge and there is a need in the art for improved delivery vehicles that are efficient in transfection, safe for pharmaceutical use and easy to manufacture.

The algorithm described before also applies to amphoteric lipid mixtures further comprising neutral lipids and the quantitative impact of such admixtures is shown in Example 6 and corresponding Figs. 10 to 13. In brief, the inclusion of neutral lipids may decrease the fusion intensity of a given amphoteric system whenever κ(neutral) is higher than κ(min)of a mixture solely of charged lipids. Figs. 10a,b and 13a,b demonstrate this experimentally. The opposite case can also be found, as demonstrated in the Figs. 12a,b. Eventually, some systems are less affected by the introduction of neutral lipids, as shown in Figs. 11a, b. Since experimental optimisation of systems with a higher number of components becomes increasingly difficult and laborious, analysis of the impact of various constituents and numerical prediction becomes even more important and allows rapid and efficacious prediction.

In practical terms, the presence of neutral lipids in the membrane of amphoteric liposomes has an effect on the fusogenicity of the liposomes and may improve or impair the fusion or the functionality of the liposomes, such as the delivery of active agents into cells and tissues. It is apparent from the algorithm, that the nature of such effect is largely dictated by the relation between κ(salt) of the amphoteric system and κ(neutral), the membrane constant of the neutral lipid or a mixture of neutral lipids. If, for example κ(salt), is higher than κ(neutral), then the addition of such neutral lipids may stimulate fusion or expand the width of the fusion zone. Of course, κ(total) has to reach a certain minimum for this. 'Such minimum is smaller than 0.25.

Experimental evidence is given in Example 6 and Fig. 14, where different neutral lipids in different amounts were mixed into the membrane of an amphoter II system (MoChol/DOGS). Furthermore, the influence of neutral lipids on the fusogenicity of other amphoteric systems was tested in Example 6 and results are summarized in tables 72 and 73.

Cholesterol as neutral lipid has either no effect on the fusogenicity of amphoteric lipid systems or may even lead to an improvement in fusability. A similar behaviour was observed for the lipid DOPE. Cholesterol and phosphatidylethanolamines are neutral or zwitterionic lipids that have κ values below 0.3 and adopt hexagonal phases, whereas the κ value of cholesterol is even lower than that of phosphatidylethanolamine.

For optimising the balance between fusogenicity and stability it may be advantageous to use a mix of neutral or zwitterionic lipids as neutral component in the amphoteric liposomes.

It has also been found that neutral lipids may extend fusability to further C/A ratios as compared to mixture solely of charged amphiphiles. For example, the addition of 40 mol% cholesterol expands the C/A ratio of DOTAP/Chems for fusion to occur from C/A= >0 - 0.4 to C/A= >0 - 0.67. Further data can be found in tables 72 and 73 of example 6.

Neutral lipids may also have impact on other characteristics of amphoteric liposomes, such as colloidal stability or stability in body fluids. For example, the use of amphoteric liposomes in pharmaceutical applications requires stability of the liposomes during storage and travelling through the bloodstream.

Example 7 shows that neutral lipids may stabilise amphoteric liposomes. The amphoteric lipid mixture DOTAP/Oleic acid for example is at physiological pH and high C/A ratios colloidal instable and forms aggregates. The addition of certain amounts of e.g. cholesterol as neutral lipid can stabilise these mixture at physiological pH.

One aspect of the invention relates to amphoteric liposomes comprising cholesterol or a mixture of cholesterol with one or more neutral or zwitterionic lipids as neutral lipids.

In this aspect κ(neutral) of said mixture of cholesterol with one or more neutral or zwitterionic lipids is less than 0.25, preferably less than 0.2 and most preferred less than 0.15.

In some embodiments of this aspect the amphoteric liposome is other than one comprising a mixture of cholesterol and phosphatidylcholine in a molar amount of 50 mol% or more.

κ(neutral) can be calculated by the following formula: $κ \left(neutral\right) = κ \left(Lipid 1\right) * c \left(Lipid 1\right) + κ \left(Lipid 2\right) * c \left(Lipid 2\right) + … … κ \left(Lipid i\right) * c \left(Lipid i\right)$ wherein κ(Lipid) is the κ value of the appropriate neutral or zwitterionic lipid and c(Lipid) is the concentration of said lipid in the mixture of neutral lipids and i is the running variable.

For example, κ(neutral) values for different mixtures of cholesterol with zwitterionic lipids are shown in tables 15 - 17.

**Table 15:**

| | --> concentration of lipids | | | | | | | | |
|---|---|---|---|---|---|---|---|---|---|
| Chol | 0.1 | 0.2 | 0.3 | 0.4 | 0.5 | 0.6 | 0.7 | 0.8 | 0.9 |
| DOPE | 0.9 | 0.8 | 0.7 | 0.6 | 0.5 | 0.4 | 0.3 | 0.2 | 0.1 |
| | | | | | | | | | |
| K(neutral) | 0.182 | 0.172 | 0.162 | 0.152 | 0.142 | 0.132 | 0.122 | 0.112 | 0.102 |

**Table 16:**

| | --> concentration of lipids | | | | | | | | |
|---|---|---|---|---|---|---|---|---|---|
| Chol | 0.1 | 0.2 | 0.3 | 0.4 | 0.5 | 0.6 | 0.7 | 0.8 | 0.9 |
| POPC | 0.9 | 0.8 | 0.7 | 0.6 | 0.5 | 0.4 | 0.3 | 0.2 | 0.1 |
| | | | | | | | | | |
| K(neutral) | 0.4673 | 0.4256 | 0.3839 | 0.3422 | 0.3005 | 0.2588 | 0.2171 | 0.1754 | 0.1337 |

**Table 17:**

| | --> concentration of lipids | | | | | | | |
|---|---|---|---|---|---|---|---|---|
| Chol | 0.1 | 0.1 | 0.1 | 0.1 | 0.1 | 0.1 | 0.1 | 0.1 |
| DOPE | 0.1 | 0.2 | 0.3 | 0.4 | 0.5 | 0.6 | 0.7 | 0.8 |
| POPC | 0.8 | 0.7 | 0.6 | 0.5 | 0.4 | 0.3 | 0.2 | 0.1 |
| | | | | | | | | |
| K(neutral) | 0.4356 | 0.4039 | 0.3722 | 0.3405 | 0.3088 | 0.2771 | 0.2454 | 0.2137 |

| | --> concentration of lipids | | | | | | | |
|---|---|---|---|---|---|---|---|---|
| Chol | 0.2 | 0.2 | 0.2 | 0.2 | 0.2 | 0.2 | 0.2 | |
| DOPE | 0.1 | 0.2 | 0.3 | 0.4 | 0.5 | 0.6 | 0.7 | |
| POPC | 0.7 | 0.6 | 0.5 | 0.4 | 0.3 | 0.2 | 0.1 | |
| | | | | | | | | |
| K(neutral) | 0.3939 | 0.3622 | 0.3305 | 0.2988 | 0.2671 | 0.2354 | 0.2037 | |
| Chol | 0.3 | 0.3 | 0.3 | 0.3 | 0.3 | 0.3 | | |
| DOPE | 0.1 | 0.2 | 0.3 | 0.4 | 0.5 | 0.6 | | |
| POPC | 0.6 | 0.5 | 0.4 | 0.3 | 0.2 | 0.1 | | |
| | | | | | | | | |
| K(neutral) | 0.3522 | 0.3205 | 0.2888 | 0.2571 | 0.2254 | 0.1937 | | |

| | --> concentration of lipids | | | | | | | |
|---|---|---|---|---|---|---|---|---|
| Chol | 0.4 | 0.4 | 0.4 | 0.4 | 0.4 | | | |
| DOPE | 0.1 | 0.2 | 0.3 | 0.4 | 0.5 | | | |
| POPC | 0.5 | 0.4 | 0.3 | 0.2 | 0.1 | | | |
| | | | | | | | | |
| K(neutral) | 0.3105 | 0.2788 | 0.2471 | 0.2154 | 0.1837 | | | |

| | --> concentration of lipids | | | | | | | |
|---|---|---|---|---|---|---|---|---|
| Chol | 0.5 | 0.5 | 0.5 | 0.5 | | | | |
| DOPE | 0.1 | 0.2 | 0.3 | 0.4 | | | | |
| POPC | 0.4 | 0.3 | 0.2 | 0.1 | | | | |
| | | | | | | | | |
| K(neutral) | 0.2688 | 0.2371 | 0.2054 | 0.1737 | | | | |

| | --> concentration of lipids | | | | | | | |
|---|---|---|---|---|---|---|---|---|
| Chol | 0.6 | 0.6 | 0.6 | | | | | |
| DOPE | 0.1 | 0.2 | 0.3 | | | | | |
| POPC | 0.3 | 0.2 | 0.1 | | | | | |
| | | | | | | | | |
| K(neutral) | 0.2271 | 0.1954 | 0.1637 | | | | | |

| | --> concentration of lipids | | | | | | | |
|---|---|---|---|---|---|---|---|---|
| Chol | 0.7 | 0.7 | | | | | | |
| DOPE | 0.1 | 0.2 | | | | | | |
| POPC | 0.2 | 0.1 | | | | | | |
| | | | | | | | | |
| K(neutral) | 0.1854 | 0.1537 | | | | | | |

| | --> concentration of lipids | | | | | | | |
|---|---|---|---|---|---|---|---|---|
| Chol | 0.8 | | | | | | | |
| DOPE | 0.1 | | | | | | | |
| POPC | 0.1 | | | | | | | |
| | | | | | | | | |
| K(neutral) | 0.1437 | | | | | | | |

The mixture of cholesterol with one or more neutral or zwitterionic lipids is selected from the group consisting of:
a. cholesterol/phosphatidylcholine;
b. cholesterol/phosphatidylethanolamine ; and
c. cholesterol/phosphatidylethanolamine/ phosphatidylcholine.

In a preferred embodiment of the invention, cholesterol or a mixture of cholesterol and phosphatidylethanolamines are present in the amphoteric liposomes as sole neutral lipids, meaning that essentially no neutral lipids with κ(neutral)>0,25 such as phosphatidylcholines are present. Preferably not more than 80 mol%, more preferably not more than 65 mol%, and most preferred not more than 50 mol%, of these lipids are used as sole neutral lipids in the amphoteric liposomes.

It has been found that cholesterol or a mixture of cholesterol and phosphatidylethanolamine can improve the transfection efficiency of amphoteric lipid mixtures as shown in examples 8 and 9.

In one embodiment of the invention the molar ratio of the mixtures of cholesterol and phosphatidylethanolamine is 4 or less, preferably between 4 and 0.25, preferred between 3 and 0.5 and most preferred between 2 and 1.

The membrane tails of said phosphatidylethanolamines may be selected without limitation from the group of C14 to C20 linear saturated or unsaturated acyls or alkyls, which may further comprise methyl side chains such as in phytanoic acid, thereby forming lipids such as DOPE, POPE, DPhyPE, DLinPE, DMPE, DPPE, DSPE or natural equivalents thereof. Mixtures of different phosphatidylethanolamines are also within the scope of the present invention. In a preferred embodiment of the invention the phosphatidylethanolamine is DOPE.

In a further embodiment of the invention a mixture of cholesterol, phosphatidylethanolamines and phosphatidylcholines may be present in the amphoteric liposomes as neutral lipids. Preferably, as indicated in table 17 above, said mixtures include not more than 40 mol % phosphatidylcholines, a zwitterionic lipid component with κ(neutral) > 0.25.

Preferred are mixtures of phosphatidylcholines and cholesterol. The molar ratio of PC/Chol may be between 4 and 0.25 or between 3 and 0.33. Preferred are molar ratios of PC/Chol between 1.5 and 0.25, more preferred between 1 and 0.25. These neutral lipid mixes may be added to the salt-forming charged lipids in the amount 80 mol% or less or 65 mol% or less, preferred in an amount of 50 mol% or less.

In some embodiments, additions of PC/Chol in a molar amount of less than 50 mol%, preferably less than 40 mol% may be preferred.

In contrast, amphoteric liposome formulations as disclosed in WO 05/094783 of Endert et al. comprise mixtures of cholesterol and PC in either a total amount of more than 50 mol% or with molar ratios of PC/Chol of 2 or more and κ(neutral) > 0.3.

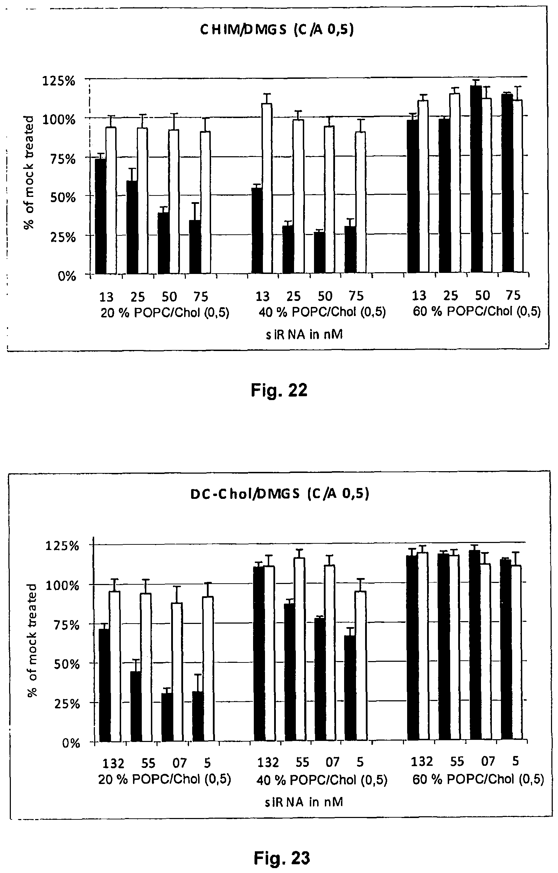

Figs. 22 and 23 show that increasing amounts of a mixture of POPC/Chol (molar ratio 0.5) diminshes the transfection efficiency of amphoteric liposome formulations. Similarly, as shown in Fig.15 increasing molar ratios of PC/Chol reduce the fusogenicity of amphoteric liposome formulations.

The phosphatidylcholines may be selected without limitation from the group POPC, DOPC, DMPC, DPPC, DSPC or natural equivalents thereof, such as soy bean PC or egg-PC. Mixtures of different phosphatidylcholines are also within the scope of the present invention. In a preferred embodiment of the invention the phosphatidylcholine is selected from POPC or DOPC.

It is possible to find other chemical representations of neutral lipids with κ(neutral)<0,25. Diacylglycerols carrying unsubstituted hydroxyls at the glycerol backbone can be considered for use as neutral lipids. However, some of these compounds function as a second messenger and signal into the protein kinase C pathway (Alberts et al.; Molecular Biology of the Cell, 3rd edition (1994, 747ff, Garland Publishing, London), which may limit their use.

For long-chain alcohols such limitations may not apply and linear, saturated or unsaturated alcohols having 14 to 30 C-atoms can for example be used for practicing the invention. Other neutral lipids may include tocopherols, other sterols, neutral or zwitterionic lysolipids, monoacyl- or monoalkylglycerols or dialkylglycerols.

The amphoteric liposomes comprising neutral lipids according to the invention may comprise one or more or a plurality of charged amphiphiles which in combination with one another have amphoteric character, being negatively charged or neutral at pH 7.4 and positively charged at pH 4 or less.

In one embodiment of the invention the amphoteric liposomes comprise an amphoteric lipid which may be selected from, but is not limited to, the group HistChol, HistDG, isoHistSuccDG, Acylcarnosin and HC-Chol.

The amphoteric liposomes of the invention comprise a mixture of charged lipids wherein at least one such charged lipid is pH responsive.

This mixture of charged lipid components comprises (i) a stable cationic lipid and a pH responsive, chargeable anionic lipid, referred to as amphoter I mixture (ii) a chargeable cationic lipid and a chargeable anionic lipid, referred to as amphoter II mixture or (iii) a stable anionic lipid and a chargeable cationic lipid, referred to as amphoter III mixture. The amphoteric liposomes according to the present invention may comprise one or more cationic lipids which may be selected from, but are not limited to, the group consisting of DOTAP, DMTAP, DPTAP, DSTAP, POTAP, DODAP, PODAP, DMDAP, DPDAP, DSDAP, DODMHEAP or DORI, PODMHEAP or PORI, DMDMHEAP or DMRI, DPDMHEAP or DPRI, DSDMHEAP or DSRI, DOMDHEAP, POMDHEAP, DMMDHEAP, DPMDHEAP, DSMDHEAP, DOMHEAP, POMHEAP, DMMHEAP, DPMHEAP, DSMHEAP, DODHEAP, PODHEAP, DMDHEAP, DPDHEAP, DSDHEAP, DDAB, DODAC, DOEPC, DMEPC, DPEPC, DSEPC, POEPC, DORIE, DMRIE, DOMCAP, DOMGME, DOP5P, DOP6P, DC-Chol, TC-Chol, DAC-Chol, Chol-Betaine, N-methyl-PipChol, CTAB, DOTMA, MoChol, HisChol, Chim, MoC3Chol, Chol-C3N-Mo3, Chol-C3N-Mo2, Chol-C4N-Mo2, Chol-DMC3N-Mo2, CholC4Hex-Mo2, DmC4Mo2, DmC3Mo2, C3Mo2, C3Mo3, C5Mo2, C6Mo2, C8Mo2, C4Mo4, PipC2-Chol, MoC2Chol, PyrroC2Chol, ImC3Chol, PyC2Chol, MoDO, MoDP, DOIM or DPIM.

In some embodiments the one or more cationic lipid may be selected from the group consisting of DOTAP, DODAP, DODMHEAP or DORI, DDAB, DOEPC, DC-Chol, MoChol, HisChol, Chim, Chol-C3N-Mo2, Chol-C4N-Mo2, MoDO, DOMCAP, DOP5P, DOP6P, DOIM or DPIM.

The amphoteric liposomes according to the present invention may comprise one or more anionic lipids which may be selected from, but are not limited to, the group consisting of diacylglycerolhemisuccinates, e.g. DOGS, DMGS, POGS, DPGS, DSGS; diacylglycerolhemimalonates, e.g. DOGM or DMGM; diacylglycerolhemiglutarates, e.g. DOGG, DMGG; diacylglycerolhemiadipates, e.g. DOGA, DMGA; diacylglycerolhemicyclohexane-1,4-dicarboxylic acids, e.g. DO-cHA, DM-cHA; (2,3-Diacyl-propyl)amino}-oxoalkanoic acids e.g. DOAS, DOAM, DOAG, DOAA, DMAS, DMAM, DMAG, DMAA; Diacyl-alkanoic acids, e.g. DOP, DOB, DOS, DOM, DOG, DOA, DMP, DOB, DMS, DMM, DMG, DMA; Chems and derivatives therof, e.g. Chol-C2, Chol-C3, Chol-C5, Chol-C6, Chol-C7 or Chol-C8; Chol-Cl, CholC3N or Cholesterolhemidicarboxylic acids and Cholesteryloxycarbonylaminocarboxylic acids, e.g. Chol-C12 or CholC13N, fatty acids, e.g. Oleic acid, Myristic Acid, Palmitic acid, Stearic acid, Nervonic Acid, Behenic Acid; DOPA, DMPA, DPPA, POPA, DSPA, Chol-SO4, DOPG, DMPG, DPPG, POPG, DSPG or DOPS, DMPS, DPPS, POPS, DSPS or Cetyl-phosphate.

In some embodiments the one or more anionic lipid may be selected from the group consisting of DOGS, DMGS, Chems, Chol-C3, Chol-C5, Chol-C6, Chol-C7, Chol-C8, Chol-Cl, CholC3N, Chol-C12, CholC13N or other Cholesterolhemidicarboxylic acids or Cholesteryloxycarbonylaminocarboxylic acids.

In addition or alternatively the inventive amphoteric liposomes may comprise one or more compounds with Cpd.No. 1-97 listed in tables 59 and 60.

As mentioned above κ(total) of an amphoteric lipid mixture comprising neutral lipids has to reach a certain minimum κ(min) to allow fusion of the liposomes.

In one embodiment of the invention the amphoteric liposome formulation is an amphoter I mixture and the neutral lipids are cholesterol or mixtures of cholesterol and neutral or zwitterionic lipids such as phosphatidylethanolamine or phosphatidylcholine and κ(min) of these mixtures is between 0.07 and 0.22, preferably between 0.09 and 0.15. This was surprisingly found and means that the transfection efficiency of the inventive amphoter I liposome formulations shows an optimum at a specific range of κ(min)values.

Fig.17 shows such relationship of the transfection efficiency of different amphoter I systems, expressed as IC50 vs. κ(min).

In a further embodiment of the invention amphoter I liposome formulations including cholesterol or mixtures of cholesterol and neutral or zwitterionic lipids such as phosphatidylethanolamine or phosphatidylcholine as neutral lipid components may be selected from the following mixtures:

**Table 18:**

| **Lipid 1** | **Mol%** | **Lipid 2** | **Mol%** | **Lipid 3** | **Mol%** | **Lipid 4** | **Mol%** | **Lipid 5** | **Mol%** |
|---|---|---|---|---|---|---|---|---|---|
| | | DOPE | 7 | DOTAP | 20 | DMGS | 60 | Chol | 13 |
| | | DOPE | 7 | DOTAP | 27 | DMGS | 53 | Chol | 13 |
| | | DOPE | 7 | DOTAP | 32 | DMGS | 48 | Chol | 13 |
| POPC | 7 | | | DOTAP | 20 | DMGS | 60 | Chol | 13 |
| POPC | 7 | | | DOTAP | 27 | DMGS | 53 | Chol | 13 |
| POPC | 7 | | | DOTAP | 32 | DMGS | 48 | Chol | 13 |
| | | | | DOTAP | 20 | DMGS | 60 | Chol | 20 |
| | | | | DOTAP | 24 | DMGS | 56 | Chol | 20 |
| | | | | DOTAP | 27 | DMGS | 53 | Chol | 20 |
| | | | | DOTAP | 32 | DMGS | 48 | Chol | 20 |
| | | | | DOTAP | 36 | DMGS | 44 | Chol | 20 |
| | | DOPE | 13 | DOTAP | 15 | DMGS | 45 | Chol | 27 |
| | | DOPE | 13 | DOTAP | 20 | DMGS | 40 | Chol | 27 |
| | | DOPE | 13 | DOTAP | 24 | DMGS | 36 | Chol | 27 |
| POPC | 13 | | | DOTAP | 15 | DMGS | 45 | Chol | 27 |
| POPC | 13 | | | DOTAP | 20 | DMGS | 40 | Chol | 27 |
| POPC | 13 | | | DOTAP | 24 | DMGS | 36 | Chol | 27 |
| POPC | 13 | | | DOTAP | 27 | DMGS | 33 | Chol | 27 |
| | | | | DOTAP | 18 | DMGS | 52 | Chol | 30 |
| | | | | DOTAP | 23 | DMGS | 47 | Chol | 30 |
| | | | | DOTAP | 28 | DMGS | 42 | Chol | 30 |
| | | | | DOTAP | 31 | DMGS | 39 | Chol | 30 |
| | | | | DOTAP | 22 | DMGS | 45 | Chol | 33 |
| | | | | DOTAP | 15 | DMGS | 45 | Chol | 40 |
| | | | | DOTAP | 20 | DMGS | 40 | Chol | 40 |
| | | | | DOTAP | 24 | DMGS | 36 | Chol | 40 |
| | | | | DOTAP | 27 | DMGS | 33 | Chol | 40 |
| | | | | DOTAP | 17 | DMGS | 43 | Chol | 40 |
| | | DOPE | 20 | DOTAP | 10 | DMGS | 30 | Chol | 40 |
| | | DOPE | 20 | DOTAP | 13 | DMGS | 27 | Chol | 40 |
| | | DOPE | 20 | DOTAP | 16 | DMGS | 24 | Chol | 40 |
| | | | | DOTAP | 13 | DMGS | 37 | Chol | 50 |
| | | | | DOTAP | 17 | DMGS | 33 | Chol | 50 |
| | | | | DOTAP | 20 | DMGS | 30 | Chol | 50 |
| | | | | DOTAP | 23 | DMGS | 27 | Chol | 50 |
| | | | | DOTAP | 10 | DMGS | 30 | Chol | 60 |
| | | | | DOTAP | 13 | DMGS | 27 | Chol | 60 |
| | | | | DOTAP | 16 | DMGS | 24 | Chol | 60 |
| | | | | DOTAP | 18 | DMGS | 22 | Chol | 60 |
| | | | | | | | | | |
| | | DOPE | 7 | DOTAP | 20 | DOGS | 60 | Chol | 13 |
| | | DOPE | 7 | DOTAP | 27 | DOGS | 53 | Chol | 13 |
| POPC | 7 | | | DOTAP | 20 | DOGS | 60 | Chol | 13 |
| POPC | 7 | | | DOTAP | 27 | DOGS | 53 | Chol | 13 |
| | | | | DOTAP | 20 | DOGS | 60 | Chol | 20 |
| | | | | DOTAP | 24 | DOGS | 56 | Chol | 20 |
| | | | | DOTAP | 27 | DOGS | 53 | Chol | 20 |
| | | DOPE | 13 | DOTAP | 15 | DOGS | 45 | Chol | 27 |
| POPC | 13 | | | DOTAP | 20 | DOGS | 40 | Chol | 27 |
| | | | | DOTAP | 18 | DOGS | 53 | Chol | 30 |
| | | | | DOTAP | 23 | DOGS | 47 | Chol | 30 |
| | | | | DOTAP | 15 | DOGS | 45 | Chol | 40 |
| | | | | DOTAP | 17 | DOGS | 43 | Chol | 40 |
| | | | | DOTAP | 20 | DOGS | 40 | Chol | 40 |
| | | DOPE | 20 | DOTAP | 10 | DOGS | 30 | Chol | 40 |
| | | | | DOTAP | 13 | DOGS | 38 | Chol | 50 |
| | | | | DOTAP | 17 | DOGS | 33 | Chol | 50 |
| | | | | DOTAP | 10 | DOGS | 30 | Chol | 60 |
| | | | | DOTAP | 13 | DOGS | 27 | Chol | 60 |
| | | | | | | | | | |
| | | | | DOTAP | 15 | OA | 45 | Chol | 40 |
| | | | | DOTAP | 17 | OA | 43 | Chol | 40 |
| | | | | DOTAP | 20 | OA | 40 | Chol | 40 |
| | | | | | | | | | |
| | | DOPE | 7 | DOTAP | 20 | CHEMS | 60 | Chol | 13 |
| | | DOPE | 7 | DOTAP | 27 | CHEMS | 53 | Chol | 13 |
| | | DOPE | 7 | DOTAP | 32 | CHEMS | 48 | Chol | 13 |
| | | DOPE | 7 | DOTAP | 36 | CHEMS | 44 | Chol | 13 |
| POPC | 7 | | | DOTAP | 20 | CHEMS | 60 | Chol | 13 |
| POPC | 7 | | | DOTAP | 27 | CHEMS | 53 | Chol | 13 |
| POPC | 7 | | | DOTAP | 32 | CHEMS | 48 | Chol | 13 |
| POPC | 7 | | | DOTAP | 36 | CHEMS | 44 | Chol | 13 |
| | | | | DOTAP | 27 | CHEMS | 53 | Chol | 20 |
| | | | | DOTAP | 32 | CHEMS | 48 | Chol | 20 |
| | | | | DOTAP | 36 | CHEMS | 44 | Chol | 20 |
| | | DOPE | 13 | DOTAP | 27 | CHEMS | 33 | Chol | 27 |
| POPC | 13 | | | DOTAP | 20 | CHEMS | 40 | Chol | 27 |
| POPC | 13 | | | DOTAP | 24 | CHEMS | 36 | Chol | 27 |
| POPC | 13 | | | DOTAP | 27 | CHEMS | 33 | Chol | 27 |
| | | | | DOTAP | 23 | CHEMS | 47 | Chol | 30 |
| | | | | DOTAP | 28 | CHEMS | 42 | Chol | 30 |
| | | | | DOTAP | 31 | CHEMS | 39 | Chol | 30 |
| | | | | DOTAP | 17 | Chems | 48 | Chol | 35 |
| | | | | DOTAP | 20 | Chems | 40 | Chol | 40 |
| | | | | DOTAP | 24 | Chems | 36 | Chol | 40 |
| | | | | DOTAP | 27 | CHEMS | 33 | Chol | 40 |
| | | | | DOTAP | 20 | CHEMS | 30 | Chol | 50 |
| | | | | DOTAP | 23 | CHEMS | 28 | Chol | 50 |
| | | | | DOTAP | 16 | CHEMS | 24 | Chol | 60 |
| | | | | DOTAP | 18 | CHEMS | 22 | Chol | 60 |
| | | | | | | | | | |
| | | | | DOTAP | 32 | Chol-C5 | 48 | Chol | 20 |
| | | | | DOTAP | 36 | Chol-C5 | 44 | Chol | 20 |
| | | | | DOTAP | 23 | Chol-C5 | 47 | Chol | 30 |
| | | | | DOTAP | 28 | Chol-C5 | 42 | Chol | 30 |
| | | | | DOTAP | 32 | Chol-C6 | 48 | Chol | 20 |
| | | | | DOTAP | 36 | Chol-C6 | 44 | Chol | 20 |
| | | | | DOTAP | 27 | Chol-C6 | 33 | Chol | 40 |
| | | | | | | | | | |
| | | | | DOTAP | 28 | Chol-Cl | 42 | Chol | 30 |
| | | | | | | | | | |
| | | | | DOTAP | 20 | Chol-C12 | 60 | Chol | 20 |
| | | | | DOTAP | 27 | Chol-C12 | 53 | Chol | 20 |
| | | | | DOTAP | 15 | Chol-C12 | 45 | Chol | 40 |
| | | | | DOTAP | 20 | Chol-C12 | 40 | Chol | 40 |
| | | | | | | | | | |
| | | | | DOTAP | 15 | Chol-C13N | 45 | Chol | 40 |
| | | | | DOTAP | 20 | Chol-C13N | 40 | Chol | 40 |
| | | | | | | | | | |
| | | | | DODAP | 36 | DMGS | 54 | Chol | 10 |
| | | DOPE | 7 | DODAP | 20 | DMGS | 60 | Chol | 13 |
| | | DOPE | 7 | DODAP | 27 | DMGS | 53 | Chol | 13 |
| | | DOPE | 7 | DODAP | 32 | DMGS | 48 | Chol | 13 |
| | | DOPE | 7 | DODAP | 36 | DMGS | 44 | Chol | 13 |
| POPC | 7 | | | DODAP | 20 | DMGS | 60 | Chol | 13 |
| POPC | 7 | | | DODAP | 27 | DMGS | 53 | Chol | 13 |
| POPC | 7 | | | DODAP | 32 | DMGS | 48 | Chol | 13 |
| POPC | 7 | | | DODAP | 36 | DMGS | 44 | Chol | 13 |
| | | | | DODAP | 38 | DMGS | 47 | Chol | 15 |
| | | | | DODAP | 20 | DMGS | 60 | Chol | 20 |
| | | | | DODAP | 27 | DMGS | 53 | Chol | 20 |
| | | | | DODAP | 32 | DMGS | 48 | Chol | 20 |
| | | | | DODAP | 36 | DMGS | 44 | Chol | 20 |
| | | | | DODAP | 30 | DMGS | 45 | Chol | 25 |
| | | DOPE | 13 | DODAP | 15 | DMGS | 45 | Chol | 27 |
| | | DOPE | 13 | DODAP | 20 | DMGS | 40 | Chol | 27 |
| | | DOPE | 13 | DODAP | 24 | DMGS | 36 | Chol | 27 |
| | | DOPE | 13 | DODAP | 27 | DMGS | 33 | Chol | 27 |
| POPC | 13 | | | DODAP | 15 | DMGS | 45 | Chol | 27 |
| POPC | 13 | | | DODAP | 20 | DMGS | 40 | Chol | 27 |
| | | | | DODAP | 18 | DMGS | 53 | Chol | 30 |
| | | | | DODAP | 23 | DMGS | 47 | Chol | 30 |
| | | | | DODAP | 28 | DMGS | 42 | Chol | 30 |
| | | | | DODAP | 32 | DMGS | 39 | Chol | 30 |
| | | | | DODAP | 15 | DMGS | 45 | Chol | 40 |
| | | | | DODAP | 20 | DMGS | 40 | Chol | 40 |
| | | | | DODAP | 24 | DMGS | 36 | Chol | 40 |
| | | | | DODAP | 27 | DMGS | 33 | Chol | 40 |
| | | DOPE | 20 | DODAP | 10 | DMGS | 30 | Chol | 40 |
| | | DOPE | 20 | DODAP | 13 | DMGS | 27 | Chol | 40 |
| | | DOPE | 20 | DODAP | 16 | DMGS | 24 | Chol | 40 |
| | | DOPE | 20 | DODAP | 18 | DMGS | 22 | Chol | 40 |
| | | | | DODAP | 13 | DMGS | 38 | Chol | 50 |
| | | | | DODAP | 17 | DMGS | 33 | Chol | 50 |
| | | | | DODAP | 20 | DMGS | 30 | Chol | 50 |
| | | | | DODAP | 23 | DMGS | 28 | Chol | 50 |
| | | | | DODAP | 10 | DMGS | 30 | Chol | 60 |
| | | | | DODAP | 13 | DMGS | 27 | Chol | 60 |
| | | | | DODAP | 16 | DMGS | 24 | Chol | 60 |
| | | | | DODAP | 18 | DMGS | 22 | Chol | 60 |
| | | | | | | | | | |
| | | DOPE | 7 | DODAP | 20 | DOGS | 60 | Chol | 13 |
| | | DOPE | 7 | DODAP | 27 | DOGS | 53 | Chol | 13 |
| | | DOPE | 7 | DODAP | 32 | DOGS | 48 | Chol | 13 |
| POPC | 7 | | | DODAP | 32 | DOGS | 48 | Chol | 13 |
| | | | | DODAP | 27 | DOGS | 53 | Chol | 20 |
| | | | | DODAP | 32 | DOGS | 48 | Chol | 20 |
| | | DOPE | 13 | DODAP | 15 | DOGS | 45 | Chol | 27 |
| | | DOPE | 13 | DODAP | 20 | DOGS | 40 | Chol | 27 |
| | | DOPE | 13 | DODAP | 24 | DOGS | 36 | Chol | 27 |
| | | | | DODAP | 23 | DOGS | 47 | Chol | 30 |
| | | | | DODAP | 28 | DOGS | 42 | Chol | 30 |
| | | | | DODAP | 24 | DOGS | 36 | Chol | 40 |
| | | | | DODAP | 27 | DOGS | 33 | Chol | 40 |
| | | | | DODAP | 20 | DOGS | 30 | Chol | 50 |
| | | | | DODAP | 23 | DOGS | 28 | Chol | 50 |
| | | | | DODAP | 16 | DOGS | 24 | Chol | 60 |
| | | | | | | | | | |
| | | DOPE | 7 | DODAP | 27 | CHEMS | 53 | Chol | 13 |
| | | DOPE | 7 | DODAP | 32 | CHEMS | 48 | Chol | 13 |
| | | DOPE | 7 | DODAP | 36 | CHEMS | 44 | Chol | 13 |
| POPC | 7 | | | DODAP | 20 | CHEMS | 60 | Chol | 13 |
| POPC | 7 | | | DODAP | 27 | CHEMS | 53 | Chol | 13 |
| POPC | 7 | | | DODAP | 32 | CHEMS | 48 | Chol | 13 |
| POPC | 7 | | | DODAP | 36 | CHEMS | 44 | Chol | 13 |
| | | | | DODAP | 32 | CHEMS | 48 | Chol | 20 |
| | | | | DODAP | 36 | CHEMS | 44 | Chol | 20 |
| | | DOPE | 13 | DODAP | 24 | CHEMS | 36 | Chol | 27 |
| | | DOPE | 13 | DODAP | 27 | CHEMS | 33 | Chol | 27 |
| POPC | 13 | | | DODAP | 15 | CHEMS | 45 | Chol | 27 |
| POPC | 13 | | | DODAP | 20 | CHEMS | 40 | Chol | 27 |
| POPC | 13 | | | DODAP | 24 | CHEMS | 36 | Chol | 27 |
| POPC | 13 | | | DODAP | 27 | CHEMS | 33 | Chol | 27 |
| | | | | DODAP | 17 | CHEMS | 53 | Chol | 30 |
| | | | | DODAP | 25 | CHEMS | 45 | Chol | 30 |
| | | | | DODAP | 28 | CHEMS | 42 | Chol | 30 |
| | | | | DODAP | 32 | CHEMS | 39 | Chol | 30 |
| | | | | DODAP | 15 | Chems | 45 | Chol | 40 |
| | | | | DODAP | 24 | CHEMS | 36 | Chol | 40 |
| | | | | DODAP | 20 | CHEMS | 30 | Chol | 50 |
| | | | | DODAP | 10 | CHEMS | 30 | Chol | 60 |
| | | | | | | | | | |
| | | | | DODAP | 27 | Chol-C6 | 53 | Chol | 20 |
| | | | | DODAP | 32 | Chol-C6 | 48 | Chol | 20 |
| | | | | DODAP | 36 | Chol-C6 | 44 | Chol | 20 |
| | | | | DODAP | 28 | Chol-C6 | 42 | Chol | 30 |
| | | | | DODAP | 31 | Chol-C6 | 39 | Chol | 30 |
| | | | | DODAP | 16 | Chol-C6 | 24 | Chol | 60 |
| | | | | DODAP | 18 | Chol-C6 | 22 | Chol | 60 |
| | | | | | | | | | |
| | | | | DODAP | 24 | NA | 36 | Chol | 40 |
| | | | | | | | | | |
| | | DOPE | 7 | DC-Chol | 27 | DMGS | 53 | Chol | 13 |
| | | DOPE | 7 | DC-Chol | 32 | DMGS | 48 | Chol | 13 |
| POPC | 7 | | | DC-Chol | 27 | DMGS | 53 | Chol | 13 |
| POPC | 7 | | | DC-Chol | 32 | DMGS | 48 | Chol | 13 |
| POPC | 7 | | | DC-Chol | 36 | DMGS | 44 | Chol | 13 |
| | | | | DC-Chol | 20 | DMGS | 60 | Chol | 20 |
| | | | | DC-Chol | 27 | DMGS | 53 | Chol | 20 |
| | | | | DC-Chol | 36 | DMGS | 44 | Chol | 20 |
| | | DOPE | 13 | DC-Chol | 15 | DMGS | 45 | Chol | 27 |
| | | DOPE | 13 | DC-Chol | 20 | DMGS | 40 | Chol | 27 |
| | | DOPE | 13 | DC-Chol | 24 | DMGS | 36 | Chol | 27 |
| | | DOPE | 13 | DC-Chol | 27 | DMGS | 33 | Chol | 27 |
| POPC | 13 | | | DC-Chol | 15 | DMGS | 45 | Chol | 27 |
| | | | | DC-Chol | 26 | DMGS | 39 | Chol | 35 |
| | | DOPE | 20 | DC-Chol | 10 | DMGS | 30 | Chol | 40 |
| | | DOPE | 20 | DC-Chol | 13 | DMGS | 27 | Chol | 40 |
| | | DOPE | 20 | DC-Chol | 16 | DMGS | 24 | Chol | 40 |
| | | | | DC-Chol | 20 | DMGS | 40 | Chol | 40 |
| | | | | DC-Chol | 20 | DMGS | 20 | Chol | 60 |
| | | | | DC-Chol | 21 | DMGS | 20 | Chol | 59 |
| | | | | | | | | | |
| | | | | DC-Chol | 22 | Chems | 43 | Chol | 35 |
| | | | | DC-Chol | 20 | Chems | 40 | Chol | 40 |
| | | | | | | | | | |
| | | | | DORI | 20 | CHEMS | 60 | Chol | 20 |
| | | | | DORI | 27 | CHEMS | 53 | Chol | 20 |
| | | | | DORI | 32 | CHEMS | 48 | Chol | 20 |
| | | | | DORI | 36 | CHEMS | 44 | Chol | 20 |
| | | | | DORI | 23 | CHEMS | 47 | Chol | 30 |
| | | | | DORI | 28 | CHEMS | 42 | Chol | 30 |
| | | | | DORI | 31 | CHEMS | 39 | Chol | 30 |
| | | | | DORI | 20 | Chems | 40 | Chol | 40 |
| | | | | DORI | 24 | CHEMS | 36 | Chol | 40 |
| | | | | DORI | 27 | CHEMS | 33 | Chol | 40 |
| | | | | DORI | 17 | CHEMS | 33 | Chol | 50 |
| | | | | DORI | 20 | CHEMS | 30 | Chol | 50 |
| | | | | DORI | 23 | CHEMS | 27 | Chol | 50 |
| | | | | DORI | 13 | CHEMS | 27 | Chol | 60 |
| | | | | DORI | 16 | CHEMS | 24 | Chol | 60 |
| | | | | DORI | 18 | CHEMS | 22 | Chol | 60 |
| | | | | | | | | | |
| | | | | DORI | 20 | DMGS | 60 | Chol | 20 |
| | | | | DORI | 27 | DMGS | 53 | Chol | 20 |
| | | | | DORI | 32 | DMGS | 48 | Chol | 20 |
| | | | | DORI | 36 | DMGS | 44 | Chol | 20 |
| | | | | DORI | 15 | DMGS | 45 | Chol | 40 |
| | | | | DORI | 20 | DMGS | 40 | Chol | 40 |
| | | | | DORI | 24 | DMGS | 36 | Chol | 40 |
| | | | | DORI | 27 | DMGS | 33 | Chol | 40 |
| | | | | | | | | | |
| | | | | DORI | 20 | DOGS | 60 | Chol | 20 |
| | | | | DORI | 27 | DOGS | 53 | Chol | 20 |
| | | | | DORI | 15 | DOGS | 45 | Chol | 40 |
| | | | | DORI | 20 | DOGS | 40 | Chol | 40 |
| | | | | DORI | 24 | DOGS | 36 | Chol | 40 |
| | | | | | | | | | |
| | | | | DOP5P | 20 | DMGS | 60 | Chol | 20 |
| | | | | DOP5P | 32 | DMGS | 48 | Chol | 20 |
| | | | | DOP5P | 36 | DMGS | 44 | Chol | 20 |
| | | | | DOP5P | 15 | DMGS | 45 | Chol | 40 |
| | | | | DOP5P | 20 | DMGS | 40 | Chol | 40 |
| | | | | DOP5P | 24 | DMGS | 36 | Chol | 40 |
| | | | | DOP5P | 27 | DMGS | 33 | Chol | 40 |
| | | | | | | | | | |
| | | | | DOP5P | 20 | Chems | 60 | Chol | 20 |
| | | | | DOP5P | 27 | Chems | 53 | Chol | 20 |
| | | | | DOP5P | 36 | Chems | 44 | Chol | 20 |
| | | | | DOP5P | 17 | Chems | 53 | Chol | 30 |
| | | | | DOP5P | 13 | Chems | 37 | Chol | 50 |
| | | | | | | | | | |
| | | | | DOP6P | 20 | DMGS | 60 | Chol | 20 |
| | | | | DOP6P | 32 | DMGS | 48 | Chol | 20 |
| | | | | | | | | | |
| | | | | DOP6P | 20 | Chems | 60 | Chol | 20 |
| | | | | DOP6P | 32 | Chems | 48 | Chol | 20 |
| | | | | DOP6P | 36 | Chems | 44 | Chol | 20 |
| | | | | DOP6P | 23 | Chems | 27 | Chol | 50 |
| | | | | DOP6P | 18 | Chems | 22 | Chol | 60 |

In another embodiment of the invention the amphoteric liposome formulation is an amphoter II mixture and the neutral lipids are cholesterol or mixtures of cholesterol and neutral or zwitterionic lipids such as phosphatidylethanolamine or phosphatidylcholine and κ(min) of these mixtures is less 0.23, preferably less than 0.18. Fig.18 shows the correlation of the transfection efficiency of different amphoter II systems, expressed as IC50 vs. κ(min).

In a further embodiment of the invention amphoter II liposome formulations including cholesterol or mixtures of cholesterol and neutral or zwitterionic lipids such as phosphatidylethanolamine or phosphatidylcholine as neutral lipid components may be selected from the following mixtures:

**Table 19:**

| **Lipid 1** | **Mol%** | **Lipid 2** | **Mol%** | **Lipid 3** | **Mol%** | **Lipid 4** | **Mol%** | **Lipid 5** | **Mol%** |
|---|---|---|---|---|---|---|---|---|---|
| | | DOPE | 7 | HisChol | 27 | DMGS | 53 | Chol | 13 |
| | | DOPE | 7 | HisChol | 40 | DMGS | 40 | Chol | 13 |
| POPC | 7 | | | HisChol | 27 | DMGS | 53 | Chol | 13 |
| POPC | 7 | | | HisChol | 40 | DMGS | 40 | Chol | 13 |
| | | | | HisChol | 20 | DMGS | 60 | Chol | 20 |
| | | | | HisChol | 27 | DMGS | 53 | Chol | 20 |
| | | DOPE | 13 | HisChol | 15 | DMGS | 45 | Chol | 27 |
| | | DOPE | 13 | HisChol | 20 | DMGS | 40 | Chol | 27 |
| | | DOPE | 13 | HisChol | 30 | DMGS | 30 | Chol | 27 |
| POPC | 13 | | | HisChol | 15 | DMGS | 45 | Chol | 27 |
| POPC | 13 | | | HisChol | 20 | DMGS | 40 | Chol | 27 |
| | | | | HisChol | 18 | DMGS | 53 | Chol | 30 |
| | | | | HisChol | 23 | DMGS | 47 | Chol | 30 |
| | | | | HisChol | 20 | DMGS | 40 | Chol | 40 |
| | | | | HisChol | 15 | DMGS | 45 | Chol | 40 |
| | | DOPE | 20 | HisChol | 10 | DMGS | 30 | Chol | 40 |
| | | DOPE | 20 | HisChol | 13 | DMGS | 27 | Chol | 40 |
| | | DOPE | 20 | HisChol | 20 | DMGS | 20 | Chol | 40 |
| | | | | HisChol | 30 | DMGS | 20 | Chol | 50 |
| | | | | HisChol | 13 | DMGS | 27 | Chol | 60 |
| | | | | HisChol | 27 | DMGS | 13 | Chol | 60 |
| | | | | HisChol | 20 | DMGS | 20 | Chol | 60 |
| POPC | 7 | DOPE | 28 | HisChol | 25 | DMGS | 30 | Chol | 10 |
| | | | | | | | | | |
| | | | | HisChol | 20 | DOGS | 60 | Chol | 20 |
| | | | | HisChol | 40 | DOGS | 20 | Chol | 40 |
| | | | | HisChol | 17 | DOGS | 53 | Chol | 30 |
| | | | | HisChol | 23 | DOGS | 47 | Chol | 30 |
| | | | | HisChol | 35 | DOGS | 35 | Chol | 30 |
| | | | | HisChol | 15 | DOGS | 45 | Chol | 40 |
| | | | | HisChol | 20 | DOGS | 20 | Chol | 60 |
| | | | | HisChol | 13 | DOGS | 27 | Chol | 60 |
| | | DOPE | 7 | HisChol | 20 | DOGS | 60 | Chol | 13 |
| | | DOPE | 7 | HisChol | 27 | DOGS | 53 | Chol | 13 |
| | | DOPE | 13 | HisChol | 15 | DOGS | 45 | Chol | 27 |
| | | DOPE | 13 | HisChol | 20 | DOGS | 40 | Chol | 27 |
| | | | | | | | | | |
| | | DOPE | 7 | MoChol | 27 | DMGS | 53 | Chol | 13 |
| | | DOPE | 7 | MoChol | 40 | DMGS | 40 | Chol | 13 |
| | | | | MoChol | 27 | DMGS | 53 | Chol | 20 |
| | | | | MoChol | 20 | DMGS | 60 | Chol | 20 |
| | | DOPE | 13 | MoChol | 15 | DMGS | 45 | Chol | 27 |
| | | DOPE | 13 | MoChol | 20 | DMGS | 40 | Chol | 27 |
| POPC | 13 | | | MoChol | 15 | DMGS | 45 | Chol | 27 |
| POPC | 13 | | | MoChol | 20 | DMGS | 40 | Chol | 27 |
| | | | | MoChol | 17 | DMGS | 53 | Chol | 30 |
| | | | | MoChol | 15 | DMGS | 45 | Chol | 40 |
| | | DOPE | 20 | MoChol | 10 | DMGS | 30 | Chol | 40 |
| | | DOPE | 20 | MoChol | 13 | DMGS | 27 | Chol | 40 |
| | | | | | | | | | |
| | | DOPE | 7 | CHIM | 40 | DMGS | 40 | Chol | 13 |
| | | DOPE | 7 | CHIM | 53 | DMGS | 27 | Chol | 13 |
| POPC | 7 | | | CHIM | 27 | DMGS | 53 | Chol | 13 |
| POPC | 7 | | | CHIM | 40 | DMGS | 40 | Chol | 13 |
| | | | | CHIM | 20 | DMGS | 60 | Chol | 20 |
| | | | | CHIM | 27 | DMGS | 53 | Chol | 20 |
| | | DOPE | 13 | CHIM | 15 | DMGS | 45 | Chol | 27 |
| | | DOPE | 13 | CHIM | 20 | DMGS | 40 | Chol | 27 |
| | | DOPE | 13 | CHIM | 30 | DMGS | 30 | Chol | 27 |
| POPC | 13 | | | CHIM | 15 | DMGS | 45 | Chol | 27 |
| POPC | 13 | | | CHIM | 20 | DMGS | 40 | Chol | 27 |
| | | | | CHIM | 23 | DMGS | 47 | Chol | 30 |
| | | | | CHIM | 15 | DMGS | 45 | Chol | 40 |
| | | | | CHIM | 30 | DMGS | 30 | Chol | 40 |
| | | | | CHIM | 40 | DMGS | 20 | Chol | 40 |
| | | | | CHIM | 45 | DMGS | 15 | Chol | 40 |
| | | DOPE | 20 | CHIM | 10 | DMGS | 30 | Chol | 40 |
| | | DOPE | 20 | CHIM | 13 | DMGS | 27 | Chol | 40 |
| | | | | CHIM | 20 | DMGS | 20 | Chol | 60 |
| | | | | | | | | | |
| | | | | | | | | | |
| | | DOPE | 7 | CholC4N-Mo2 | 40 | DMGS | 40 | Chol | 13 |
| POPC | 7 | | | CholC4N-Mo2 | 27 | DMGS | 53 | Chol | 13 |
| POPC | 7 | | | CholC4N-Mo2 | 40 | DMGS | 40 | Chol | 13 |
| | | | | CholC4N-Mo2 | 20 | DMGS | 60 | Chol | 20 |
| | | | | CholC4N-Mo2 | 27 | DMGS | 53 | Chol | 20 |
| | | | | CholC4N-Mo2 | 40 | DMGS | 40 | Chol | 20 |
| | | DOPE | 13 | CholC4N-Mo2 | 20 | DMGS | 40 | Chol | 27 |
| | | DOPE | 13 | CholC4N-Mo2 | 30 | DMGS | 30 | Chol | 27 |
| POPC | 13 | | | CholC4N-Mo2 | 15 | DMGS | 45 | Chol | 27 |
| POPC | 13 | | | CholC4N-Mo2 | 20 | DMGS | 40 | Chol | 27 |
| | | | | CholC4N-Mo2 | 17 | DMGS | 53 | Chol | 30 |
| | | | | CholC4N-Mo2 | 23 | DMGS | 47 | Chol | 30 |
| | | | | CholC4N-Mo2 | 15 | DMGS | 45 | Chol | 40 |
| | | | | CholC4N-Mo2 | 20 | DMGS | 40 | Chol | 40 |
| - | | DOPE | 20 | CholC4N-Mo2 | 13 | DMGS | 27 | Chol | 40 |
| | | | | CholC4N-Mo2 | 13 | DMGS | 37 | Chol | 50 |
| | | | | CholC4N-Mo2 | 17 | DMGS | 33 | Chol | 50 |
| | | | | CholC4N-Mo2 | 13 | DMGS | 27 | Chol | 60 |
| | | | | | | | | | |
| | | DOPE | 7 | CholC3N-Mo2 | 40 | DMGS | 40 | Chol | 13 |
| POPC | 7 | | | CholC3N-Mo2 | 27 | DMGS | 53 | Chol | 13 |
| POPC | 7 | | | CholC3N-Mo2 | 40 | DMGS | 40 | Chol | 13 |
| | | | | CholC3N-Mo2 | 20 | DMGS | 60 | Chol | 20 |
| | | | | CholC3N-Mo2 | 27 | DMGS | 53 | Chol | 20 |
| | | | | CholC3N-Mo2 | 40 | DMGS | 40 | Chol | 20 |
| | | DOPE | 13 | CholC3N-Mo2 | 20 | DMGS | 40 | Chol | 27 |
| POPC | 13 | | | CholC3N-Mo2 | 20 | DMGS | 40 | Chol | 27 |
| | | | | CholC3N-Mo2 | 17 | DMGS | 53 | Chol | 30 |
| | | | | CholC3N-Mo2 | 15 | DMGS | 45 | Chol | 40 |
| | | DOPE | 20 | CholC3N-Mo2 | 13 | DMGS | 27 | Chol | 40 |
| | | | | CholC3N-Mo2 | 13 | DMGS | 37 | Chol | 50 |
| | | | | CholC3N-Mo2 | 17 | DMGS | 33 | Chol | 50 |
| | | | | CholC3N-Mo2 | 10 | DMGS | 30 | Chol | 60 |
| | | | | CholC3N-Mo2 | 13 | DMGS | 27 | Chol | 60 |
| | | | | | | | | | |
| POPC | 7 | | | DOMCAP | 53 | DMGS | 27 | Chol | 13 |
| | | DOPE | 13 | DOMCAP | 40 | DMGS | 20 | Chol | 27 |
| POPC | 13 | | | DOMCAP | 20 | DMGS | 40 | Chol | 27 |
| POPC | 13 | | | DOMCAP | 30 | DMGS | 30 | Chol | 27 |
| | | DOPE | 18 | DOMCAP | 28 | Chol-Cl | 42 | Chol | 12 |
| | | | | | | | | | |
| | | DOPE | 7 | DOMCAP | 20 | Chol-C3 | 60 | Chol | 13 |
| | | DOPE | 7 | DOMCAP | 27 | Chol-C3 | 53 | Chol | 13 |
| POPC | 7 | | | DOMCAP | 20 | Chol-C3 | 60 | Chol | 13 |
| POPC | 7 | | | DOMCAP | 27 | Chol-C3 | 53 | Chol | 13 |
| | | | | DOMCAP | 20 | Chol-C3 | 60 | Chol | 20 |
| | | | | DOMCAP | 27 | Chol-C3 | 53 | Chol | 20 |
| | | | | DOMCAP | 40 | Chol-C3 | 40 | Chol | 20 |
| | | DOPE | 13 | DOMCAP | 15 | Chol-C3 | 45 | Chol | 27 |
| | | DOPE | 13 | DOMCAP | 20 | Chol-C3 | 40 | Chol | 27 |
| | | DOPE | 13 | DOMCAP | 30 | Chol-C3 | 30 | Chol | 27 |
| POPC | 13 | | | DOMCAP | 15 | Chol-C3 | 45 | Chol | 27 |
| POPC | 13 | | | DOMCAP | 20 | Chol-C3 | 40 | Chol | 27 |
| | | | | DOMCAP | 18 | Chol-C3 | 53 | Chol | 30 |
| | | | | DOMCAP | 23 | Chol-C3 | 47 | Chol | 30 |
| | | | | DOMCAP | 15 | Chol-C3 | 45 | Chol | 40 |
| | | | | DOMCAP | 20 | Chol-C3 | 40 | Chol | 40 |
| | | DOPE | 20 | DOMCAP | 13 | Chol-C3 | 27 | Chol | 40 |
| | | | | DOMCAP | 13 | Chol-C3 | 38 | Chol | 50 |
| | | | | DOMCAP | 10 | Chol-C3 | 30 | Chol | 60 |
| | | | | | | | | | |
| | | DOPE | 7 | MoDO | 20 | Chol-C3 | 60 | Chol | 13 |
| | | DOPE | 7 | MoDO | 27 | Chol-C3 | 53 | Chol | 13 |
| POPC | 7 | | | MoDO | 20 | Chol-C3 | 60 | Chol | 13 |
| POPC | 7 | | | MoDO | 27 | Chol-C3 | 53 | Chol | 13 |
| | | | | MoDO | 20 | Chol-C3 | 60 | Chol | 20 |
| | | | | MoDO | 27 | Chol-C3 | 53 | Chol | 20 |
| | | DOPE | 13 | MoDO | 15 | Chol-C3 | 45 | Chol | 27 |
| | | DOPE | 13 | MoDO | 20 | Chol-C3 | 40 | Chol | 27 |
| POPC | 13 | | | MoDO | 15 | Chol-C3 | 45 | Chol | 27 |
| POPC | 13 | | | MoDO | 20 | Chol-C3 | 40 | Chol | 27 |
| | | | | MoDO | 18 | Chol-C3 | 53 | Chol | 30 |
| | | | | MoDO | 23 | Chol-C3 | 47 | Chol | 30 |
| | | | | MoDO | 15 | Chol-C3 | 45 | Chol | 40 |
| | | | | MoDO | 20 | Chol-C3 | 40 | Chol | 40 |
| | | | | MoDO | 13 | Chol-C3 | 38 | Chol | 50 |
| | | | | MoDO | 10 | Chol-C3 | 30 | Chol | 60 |

The amphoteric liposomes according to the invention may be manufactured using suitable methods that are known to those skilled in the art. Such methods include, but are not limited to, extrusion through membranes of defined pore size, injection of an alcoholic lipid solution into a water phase containing the cargo to be encapsulated, or high pressure homogenisation.

A solution of the drug (e.g. an oligonucleotide) may be contacted with the lipid phase at a neutral pH, thereby resulting in volume inclusion of a certain percentage of the solution. High concentrations of the lipids, ranging from about 50 mM to about 150 mM, are preferred to achieve substantial encapsulation of the active agent.

Amphoteric liposomes offer the distinct advantage of binding nucleic acids at or below their isoelectric point, thereby concentrating these active agents at the liposome membrane. This process, called advanced loading procedure, is described in more detail in WO 02/066012 the content of which in incorporated herein by reference.

In one embodiment of the invention the amphoteric liposomes may be prepared by using said advanced loading procedure combined with a lipid film extrusion process.

In another embodiment of the invention the amphoteric liposomes may be prepared by using said advanced loading procedure combined with an injection of an alcoholic lipid solution into a water phase containing for example a nucleic acid. This process is described in more detail in WO 07/107304 (Panzner et al.)

Irrespective of the actual production process used to make the amphoteric liposomes of the invention, in some embodiments, non-encapsulated drug may be removed from the liposomes after the initial production of the liposomes. Again, the technical literature and the references included herein describe such methodology in detail and suitable process steps may include, but are not limited to, size exclusion chromatography, sedimentation, dialysis, ultrafiltration and diafiltration.

However, the removal of any non-encapsulated drug is not required for performance of the invention, and in some embodiments the liposomal formulations may comprise free as well as entrapped drug.

In one aspect of the invention the size of the liposomes may vary between 50 and 1000 nm, preferably between 50 and 500 nm and more preferred between 70 and 250 nm.

In other aspects the size of the liposomes may vary between 70 and 150 nm and in still other aspects the size of the liposomes may vary between 130 and 250 nm.

It has been mentioned throughout this invention that a certain minimum value for dκ(pH)8 is needed to achieve formation of a stable membrane phase at neutral pH. Analysis of the experimental data obtained in example 8 revealed that a higher frequency of very small particles is produced whenever dκ(pH8) is higher than 0.08; indicating the formation of stable liposomes. In numerous examples of this experiment, a surprisingly small dκ(pH8) of 0,04 was still sufficient for the formation of small particles. However, the frequency of formation of small particles is lower for lower values of dκ(pH8), since these particles did not escape the fusion zone. This analysis is shown in Fig.19.

The experimental data provided herein allow the more general description of successful carriers by screening libraries of amphoteric lipids *in silico* with κ(min) between 0,09 and 0,15 for amphoter I systems and κ(min) < 0,2 for amphoter II systems in combination with dκ(pH8)>0,04. While the easily accessible parameter κ(salt) or κ(salt)n has been used in the screens performed above, the function for κ(min) can be written and for amphoter I systems, there is: $κ \left(pH8\right) = {x}_{cat} * κ \left(salt\right) + \left({x}_{an}-{x}_{cat}\right) * \left({V}_{AH}+{V}_{CC}\right) / {V}_{AT}$ $κ \left(min\right) = {x}_{cat} * κ \left(salt\right) + \left({x}_{an}-{x}_{cat}\right) * {V}_{AH} / {V}_{AT}$ $\begin{matrix}dk \left(pH8\right) = κ \left(pH8\right) - κ \left(min\right) \\ = \left({x}_{an}-{x}_{cat}\right) * {V}_{CC} / {V}_{AT}\end{matrix}$

For amphoter II systems, the respective formulas are: $κ \left(pH8\right) = {x}_{cat} * {V}_{CH} / {V}_{CT} + {x}_{an} * \left({V}_{AH}+{V}_{CC}\right) / {V}_{AT}$ $κ \left(min\right) = {x}_{cat} * κ \left(salt\right) + \left({x}_{an}-{x}_{cat}\right) * {V}_{AH} / {V}_{AT}$ for systems with anion excess, but $κ \left(min\right) = {x}_{an} * κ \left(salt\right) + \left({x}_{cat}-{x}_{an}\right) * {V}_{CH} / {V}_{CT}$ for systems with cation excess, and $dk \left(pH8\right) = κ \left(pH8\right) + κ \left(min\right)$

For amphoter III systems, the following equations apply: $κ \left(pH8\right) = {x}_{cat} * {V}_{CH} / {V}_{CT} + {x}_{an} * \left({V}_{AH}+{V}_{CC}\right) / {V}_{AT}$ $κ \left(min\right) = {x}_{an} * κ \left(salt\right) + \left({x}_{cat}-{x}_{an}\right) * {V}_{CH} / {V}_{CT}$ $dk \left(pH8\right) = κ \left(pH8\right) - κ \left(min\right)$ In the equations, V_{AH}, V_{CH}, V_{AT} and V_{CT} denote the volumes of the anionic and cationic head and tail groups, respectively and xₐₙ and x_{cat} are the fractions of the anionic and cationic component. V_{cc} is the volume of the counterion.

In amphoter I systems at neutral pH, all of the cationic lipid and the same amount of the anionic lipid form the lipid salt and the remainder of the anionic lipid is charged as in (1a). A reduction in the pH results in protonation of the lipid anion and loss of its counterion until the availability of the charged lipid anion limits the lipid salt formation. All lipid anion is then essentially devoid of counterions either through ongoing protonation or due to its binding in the lipid salt; this is reflected in (2a). The same constraint applies for anion-rich amphoter II systems: κ(min) is found at the left flank of the lipid salt zone at a pH not too far from the pK of the lipid anion and equation (5a) is therefore identical with (2a).

The cation-rich amphoter II or the genuine amphoter III systems invert these features in that k(min) is found at the upper end of the lipid salt zone. This is where limiting amounts of the anionic lipid form the lipid salt with the ionized portion of the cationic lipid; the remainder of the cationic lipid being uncharged as in the equations (6a) and (9a). Any reduction in pH would increase κ through further ionization of the cationic lipid and recruitment of counteranions to the membrane. At somewhat higher pH the ionized lipid cation would not suffice to maintain lipid salt and the then liberated lipid anions would recruit their counterions to the bilayer.

At pH 8 both the charged lipid anion and the uncharged lipid cation coexist; again there is no difference between cation-rich amphoter II systems and amphoter III.

Amphoter II systems having an even distribution of anionic and cationic lipids do behave like other amphoter II systems at pH8. As far as κ(min) is concerned, a full salt formation is possible and not limited by either compound. It is therefore $κ \left(min\right) = κ \left(salt\right) .$

The individual pK values, while determining the actual place of the fusion zone, are dispensable as far as κ(min) and κ(pH8) are concerned and the pK of the lipid anion is 2 or more units lower than the pK of the lipid cation to facilitate near completion of the lipid salt formation. Smaller differences between the respective pK values result in incomplete lipid salt formation and κ(total) of the membrane then comprises larger portions of the non-partnered lipid species, thus raising κ(min) and reducing the system amplitude dκ(pH8). The effect is limited towards amphoter II systems and most pronounced in situations where both lipids are present in nearly equimolar amounts. A specific calculation is made under the amphoter II section below.

The only non-lipid variable left is the volume of the countercation and this is set at sodium, 65 Å³, for most purposes. The addition of neutral lipids in the screening library was done using a linear mixing of the amphoteric and the neutral lipid part.

### The library

Combinatorial libraries were created as tools for the comprehensive analysis of the formulation space using the calculations and parameters from above. For that, the four most typical lipid tail volumes (C24 alkyl=280 Å³, cholesterol=340 Å³, dimyristoylglycerol=410 Å³ and dioleoylglycerol=500 Å³) were systematically combined with eight different head groups representing volumes from 40 Å³ up to 200 Å³. The resulting 32 lipids were allowed to adopt all 1024 possible combinations to form charged lipid pairs and further layers of complexity were added in that the molar ratio between the anionic and cationic lipid was kept flexible and in that the then resulting sets of charged amphiphiles were optionally blended with variable amounts of neutral lipids having a κ(neutral)<0,25. Four of such libraries were established to accommodate the differences of the amphoter I, anion-rich amphoter II, equilibrated amphoter II and the cation-rich amphoter II/amphoter III systems.

### General description of preferred amphoter I systems with κ(min)>0,09, κ(min)<0,15, and dκ(pH8)>0,04

A library of lipids was constructed as described and the interaction between lipid anion and cation follow the amphoter I specification. The following tables 20-24 identify positively screened species comprising 0, 20, 30, 40 or 50% cholesterol. Values given in the table represent κ(min); AH, AT, CH and CT denote the anion and cation head and tail groups, respectively.

### Tables 20-24:

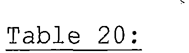

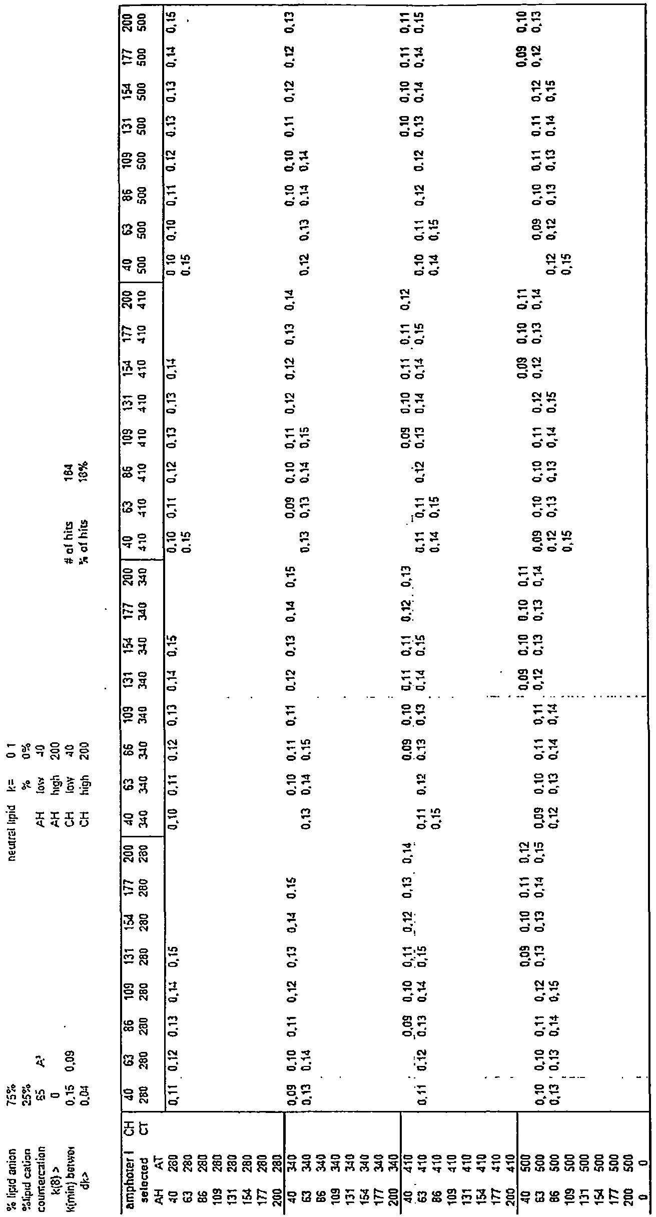

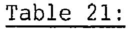

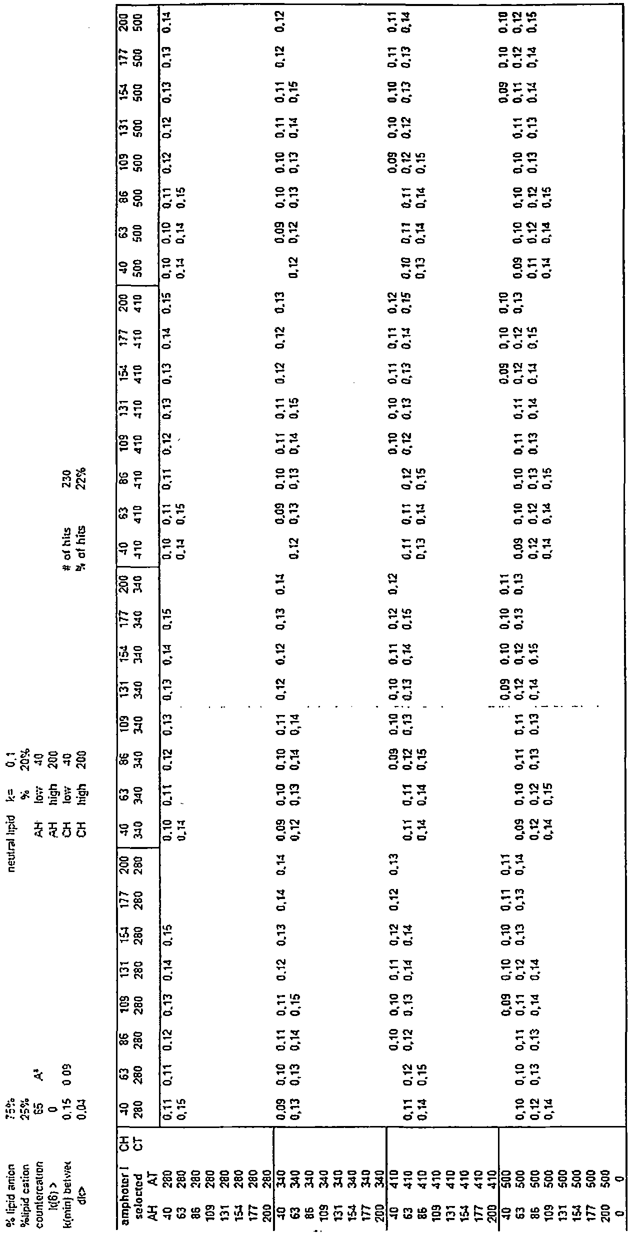

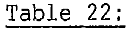

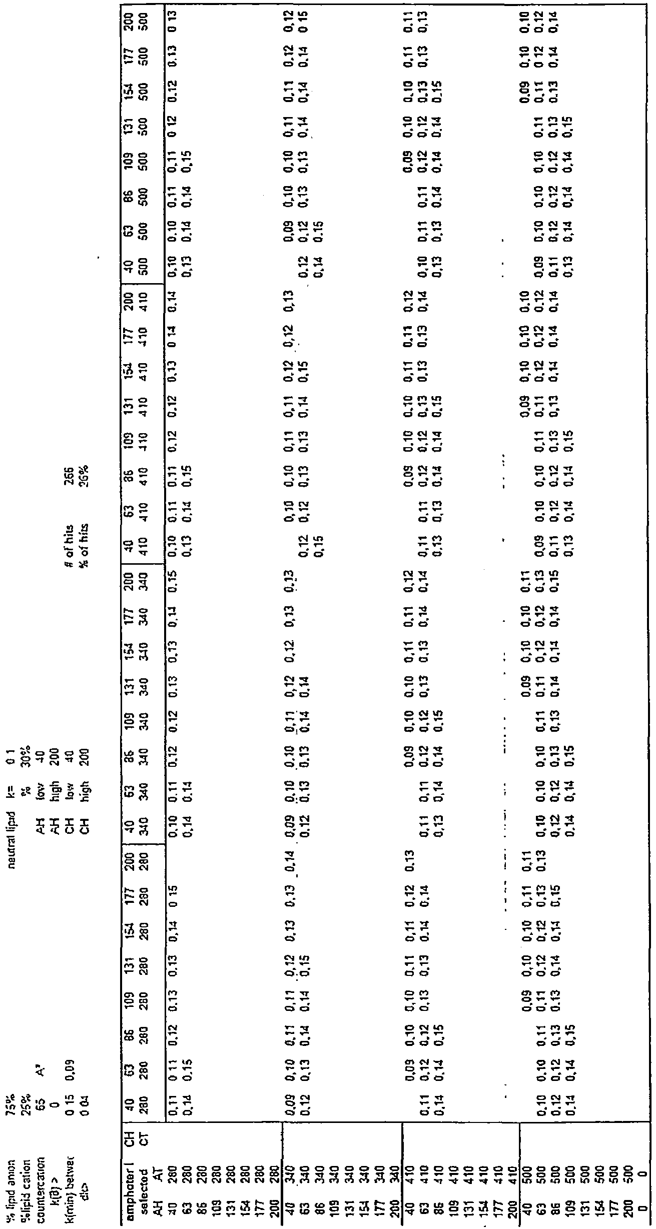

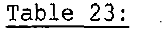

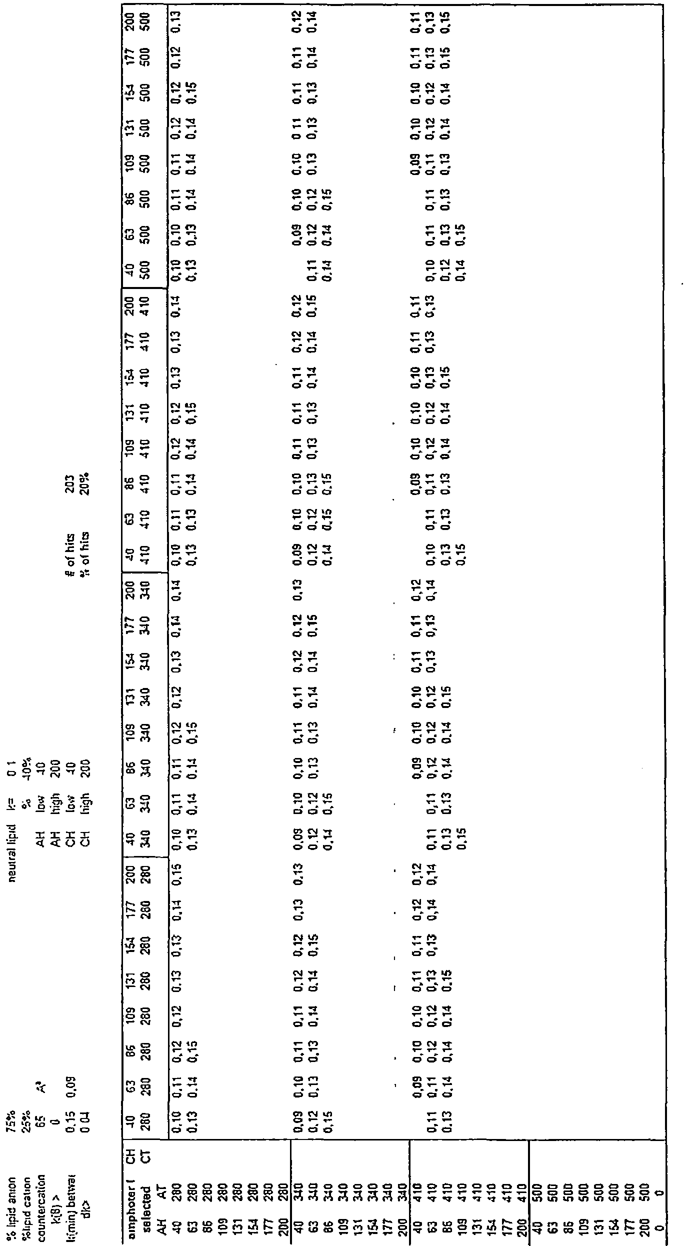

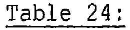

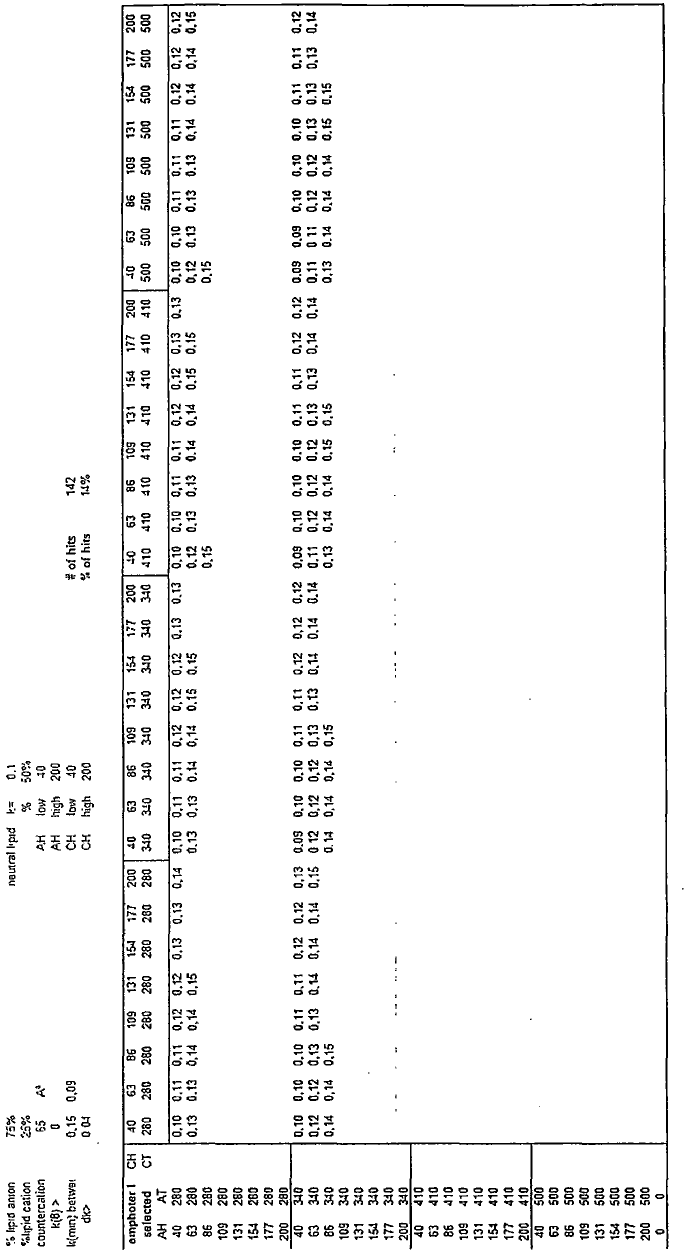

No positive amphoter I species were found with 60% cholesterol or more.

An increase in κ(neutral) yields additional selection pressure towards κ(min). Positive system for neutral lipids having κ(neutral) = 0.15, 0.2 or 0.25 are shown in the tables 25-27 below that provide such analysis for a neutral lipid content of 30%.

### Tables 25-27:

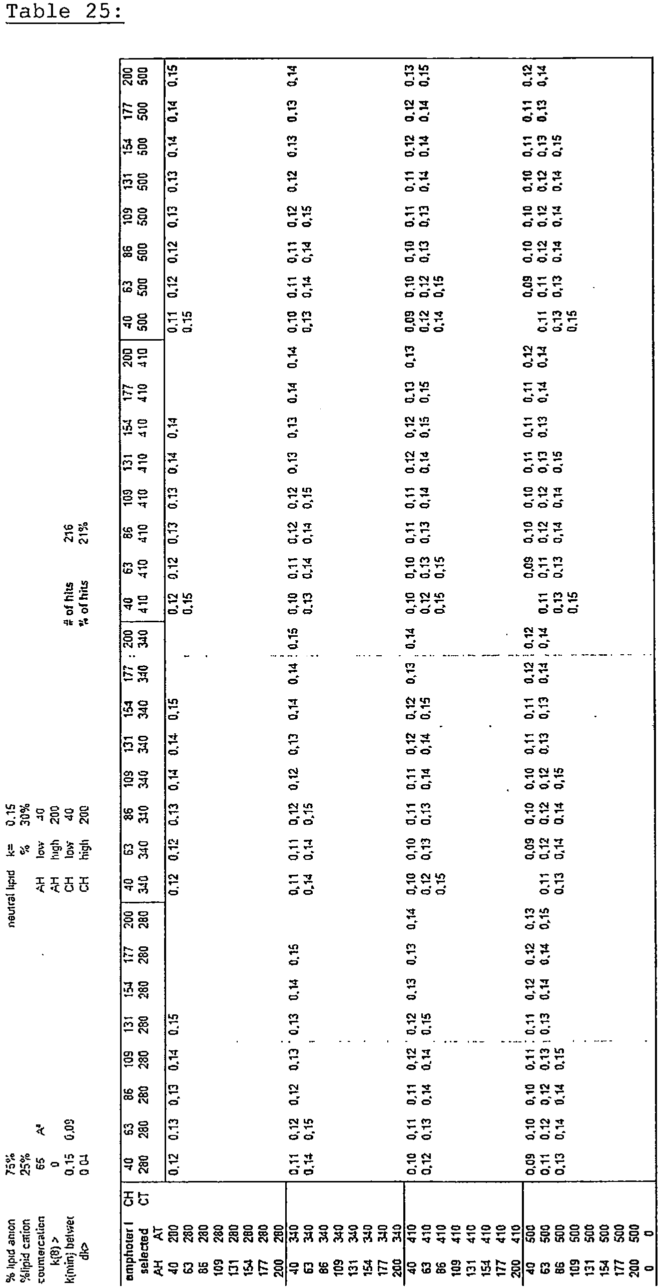

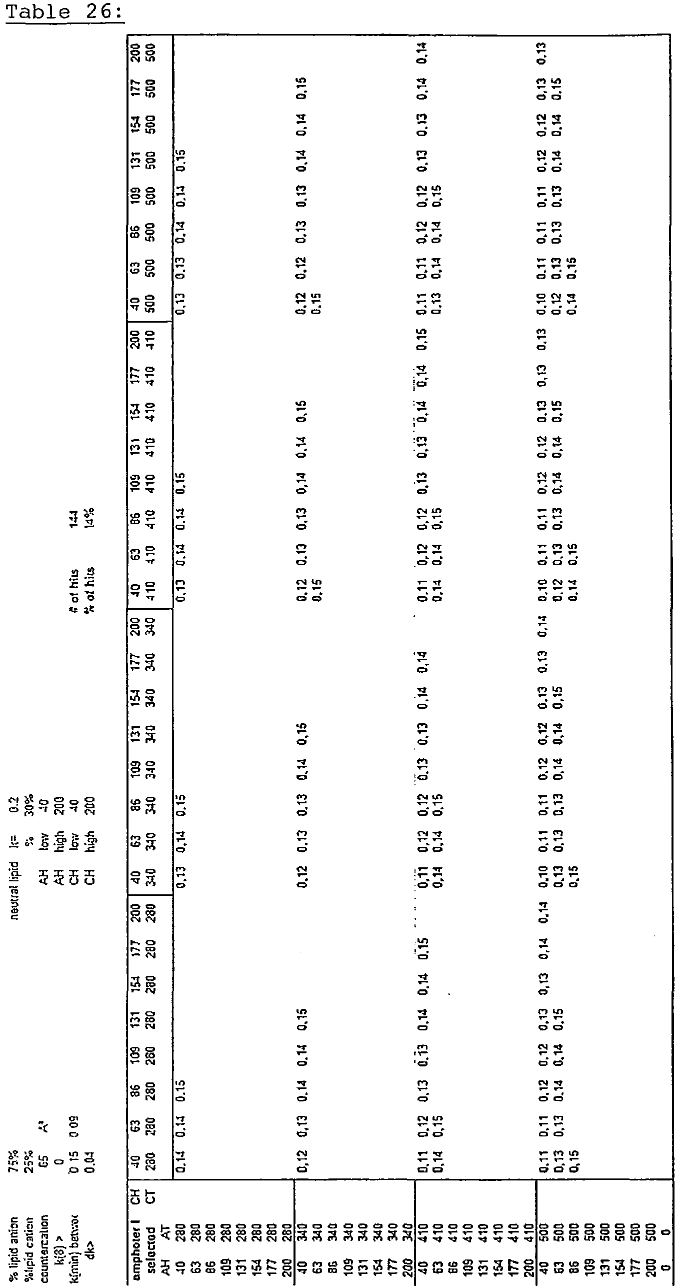

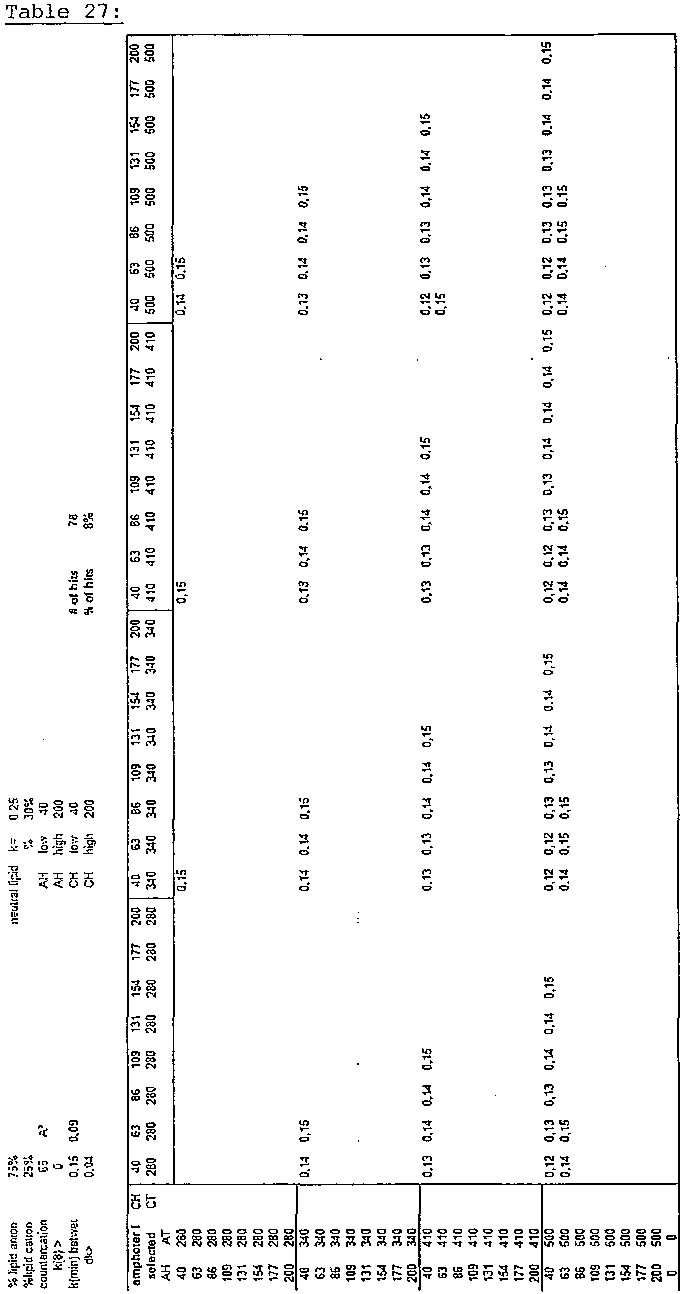

The selection pressure does also increase with higher amounts of the lipid cation, as the more extensive formation of the lipid salt reduces the system amplitude dK(pH8). Table 28 below demonstrates the reduced frequency of positive species for amphoter I systems with C/A=0,5 and 30% cholesterol and a C/A of about 0,66 represents the limit for this setup.

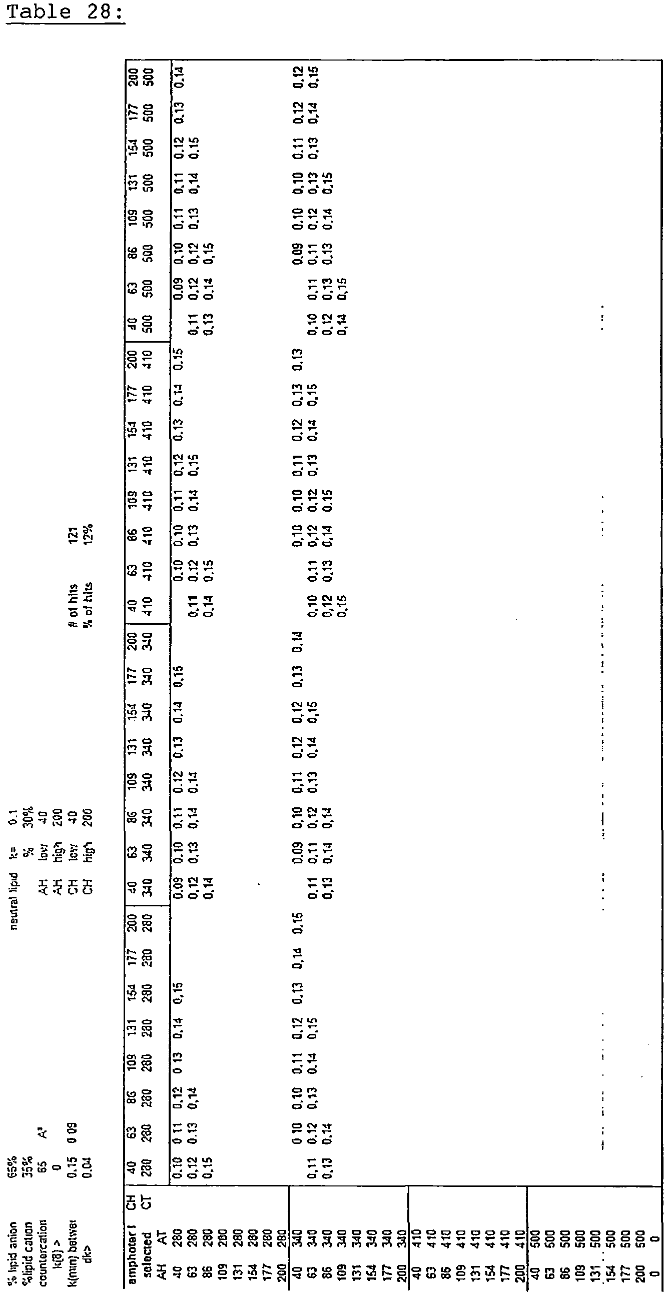

### General description of preferred anion-rich and equilibrated amphoter II systems with κ(min)<0,18 and dκ(pH8)>0,08

A library of lipids was constructed as described and the interaction between lipid anion and cation follow the amphoter II specification having an excess of the lipid anion or equal amounts of the lipid anion and lipid cation. Since no lipid salt formation limits the system amplitude at neutral pH, a more rigorous screen using dκ(pH8) is demonstrated here. It is of course possible to also screen the libraries with lower selection pressure as done for the amphoter I systems.

The following tables 29-34 identify positively screened species comprising 0, 20, 30, 40, 50 or 60% cholesterol. Values given in the table represent k(min); AH, AT, CH and CT denote the anion and cation head and tail groups, respectively.

### Tables 29-34:

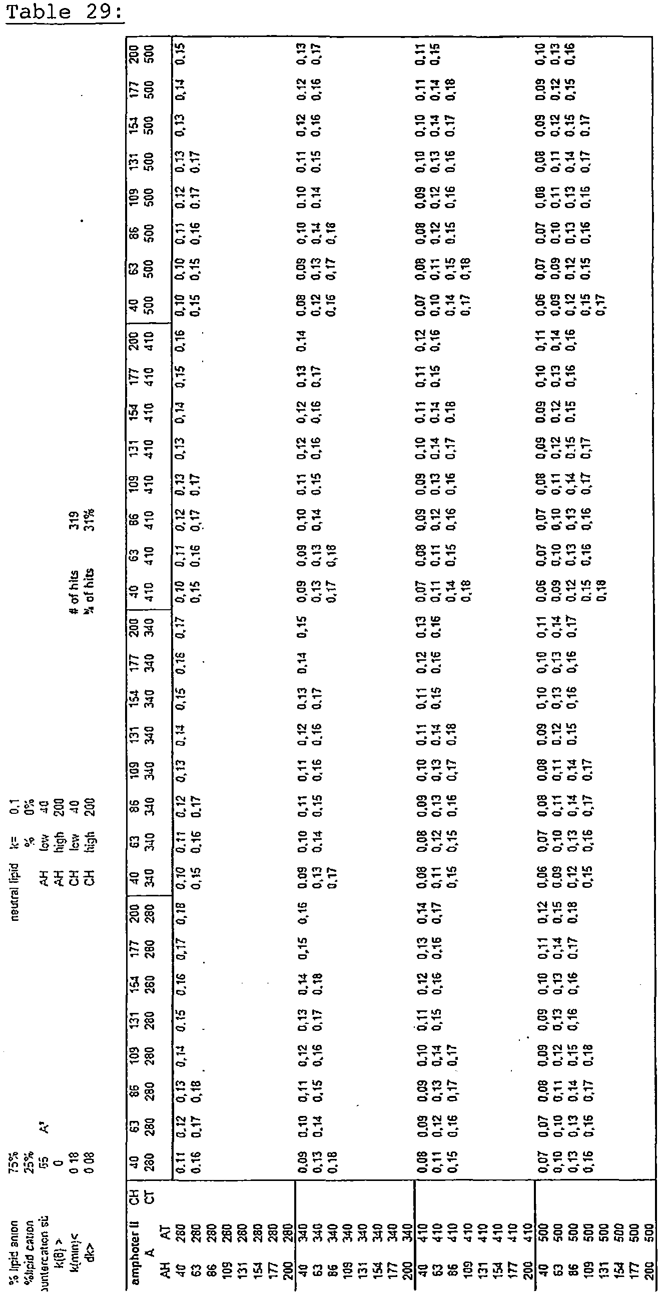

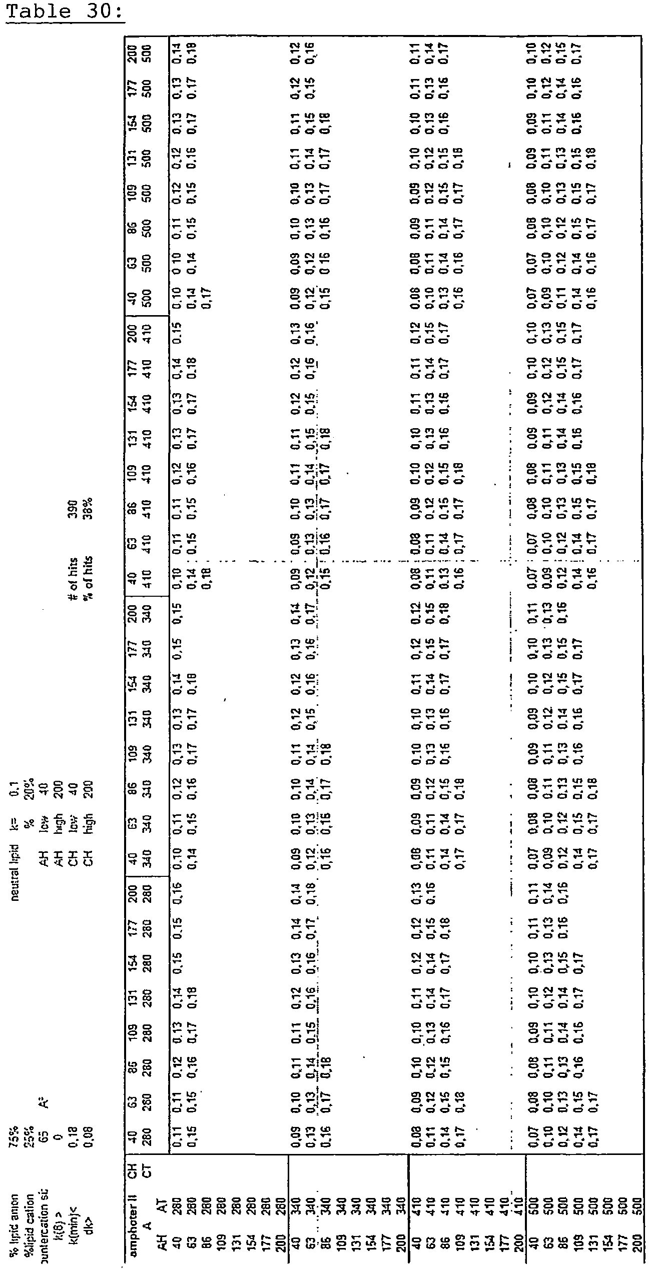

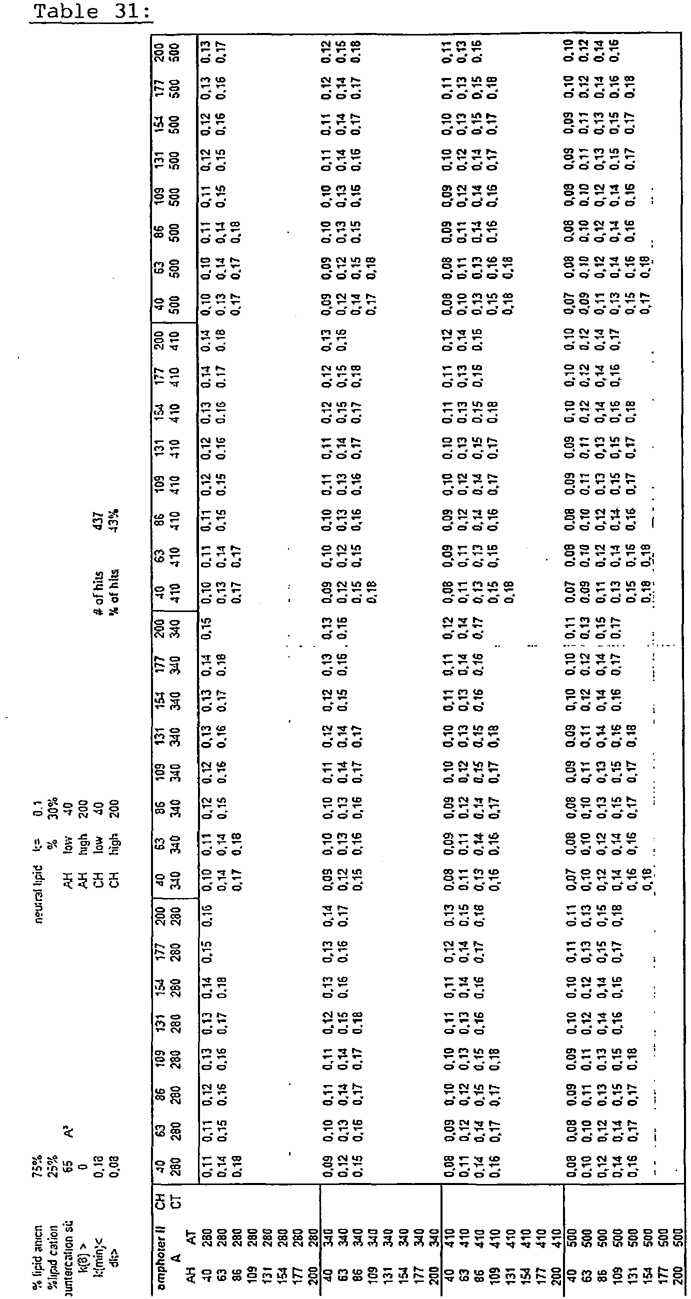

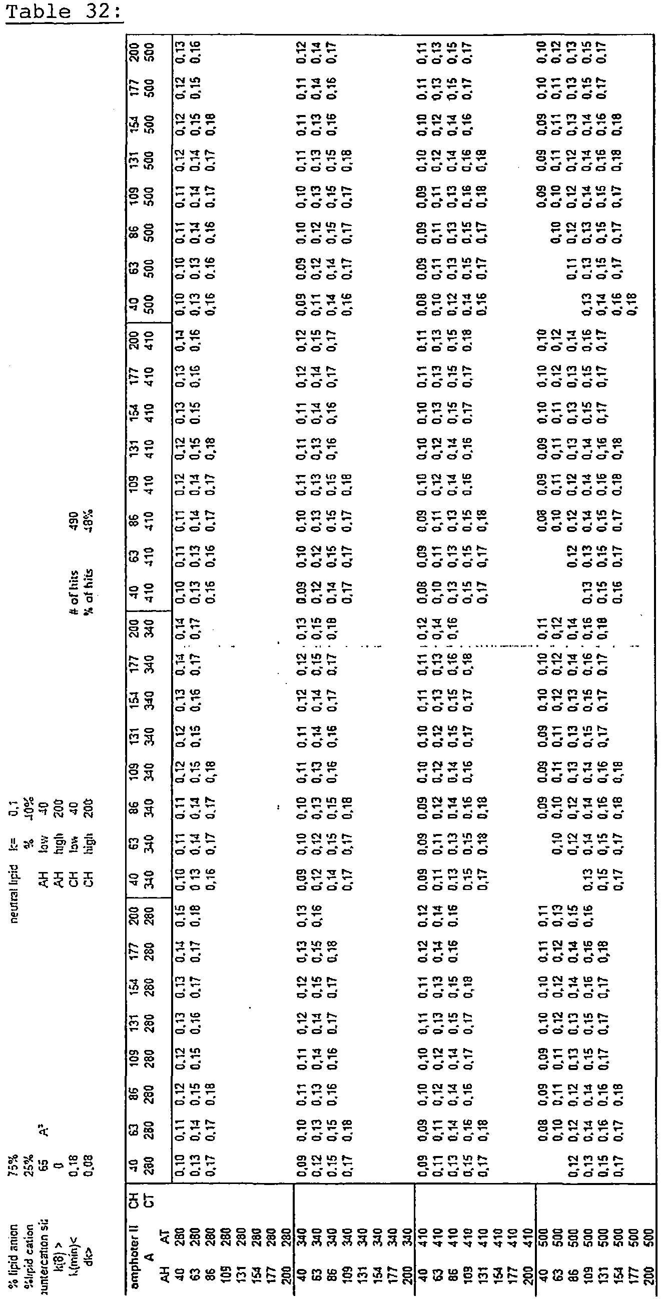

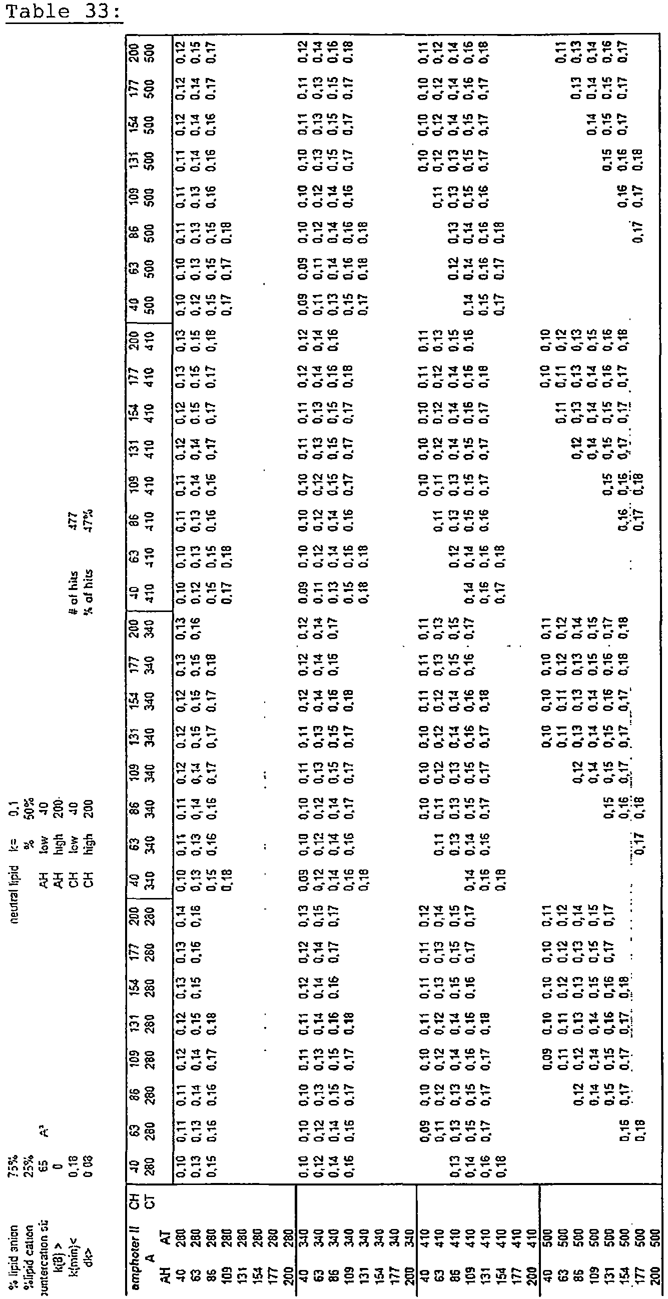

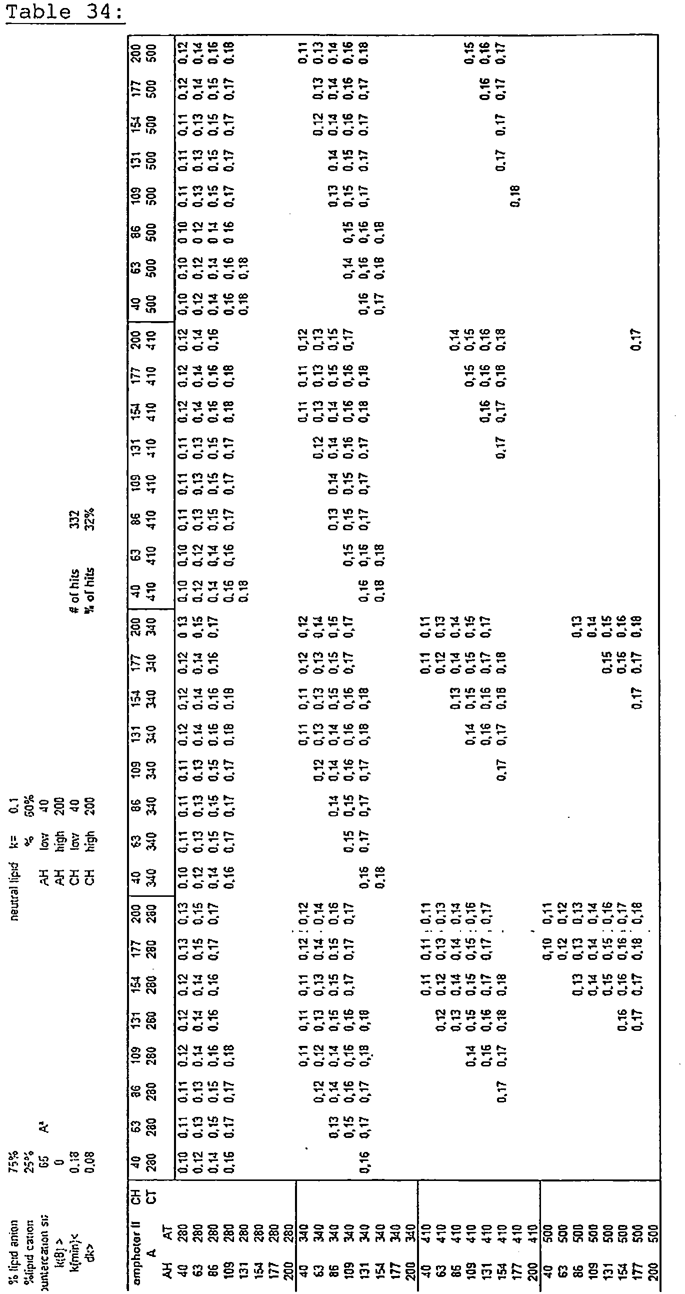

Use of neutral lipids with somewhat higher κ(neutral) is feasible and results for such mixtures comprising 30% of the neutral lipid component with κ(neutral)= 0.15, 0.2 or 0.25 are shown below in table 35-37:

### Tables 35-37:

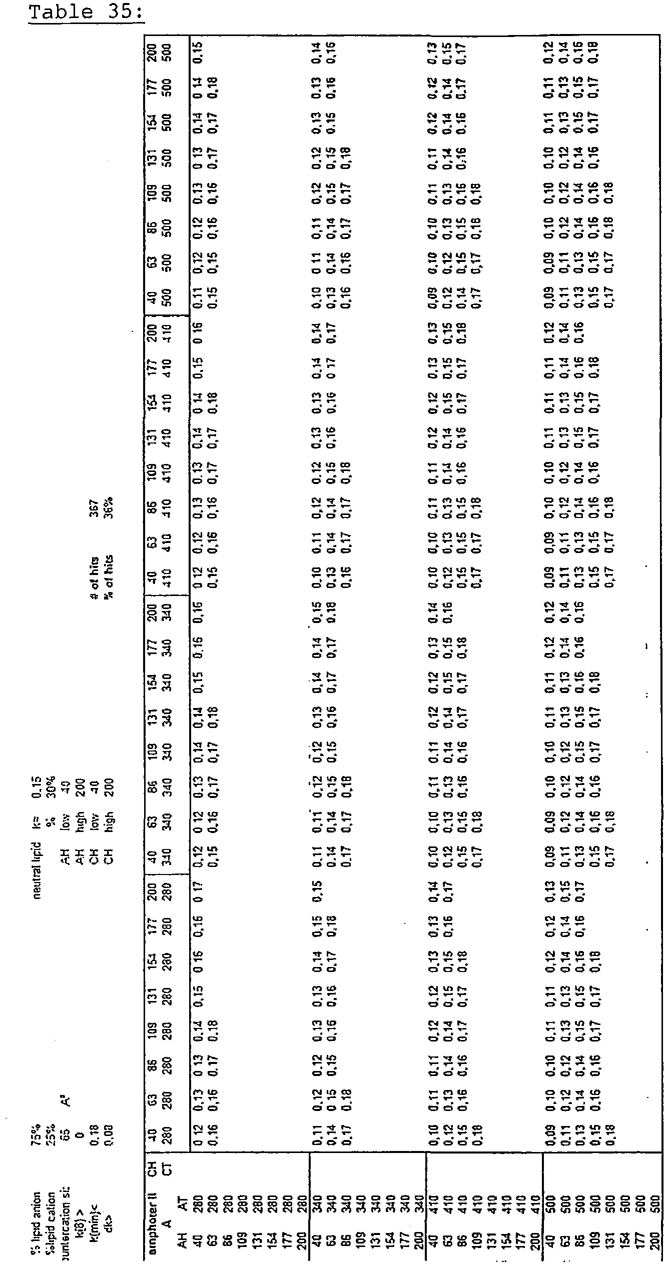

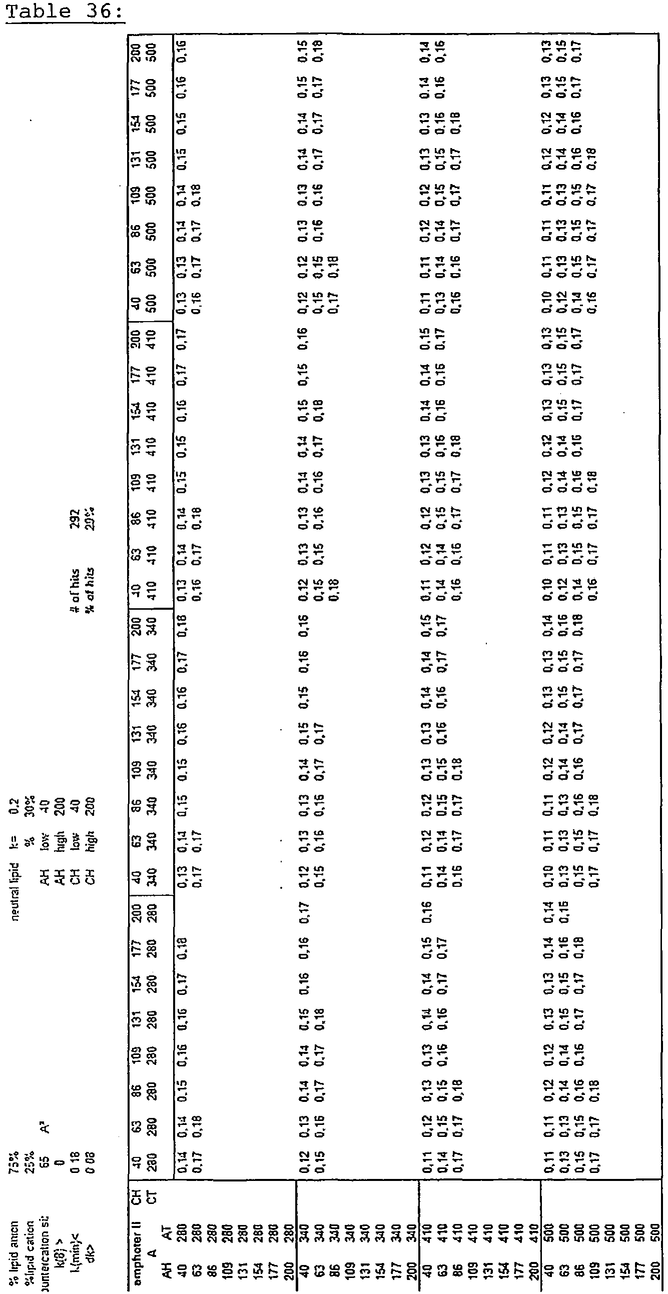

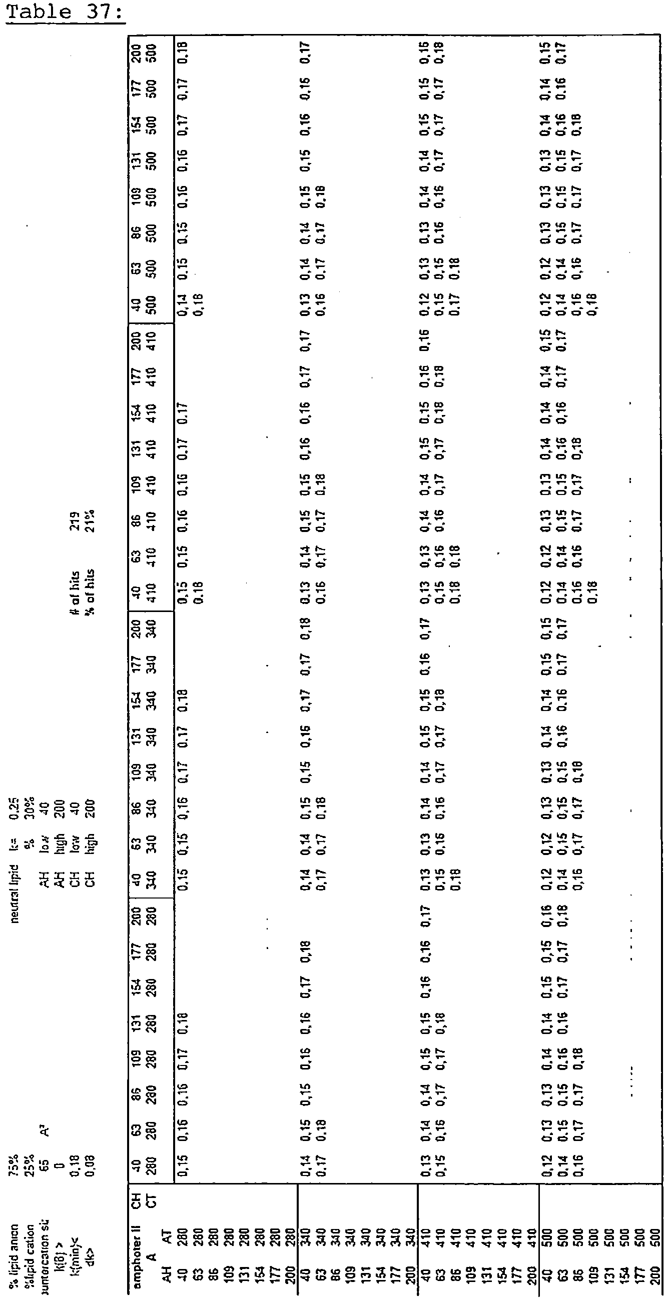

In contrast to amphoter I systems, a further increase in the amount of the cationic lipid component does not reduce the system amplitude dκ(pH8), as no lipid salt formation occurs at neutral pH. As such, the system becomes even more permissive and results in a higher frequency of positively screened species as shown below in table 38-40 for anion-rich amphoter II systems comprising 65, 60 or 50% lipid anion and 30% cholesterol.

### Tables 38-40:

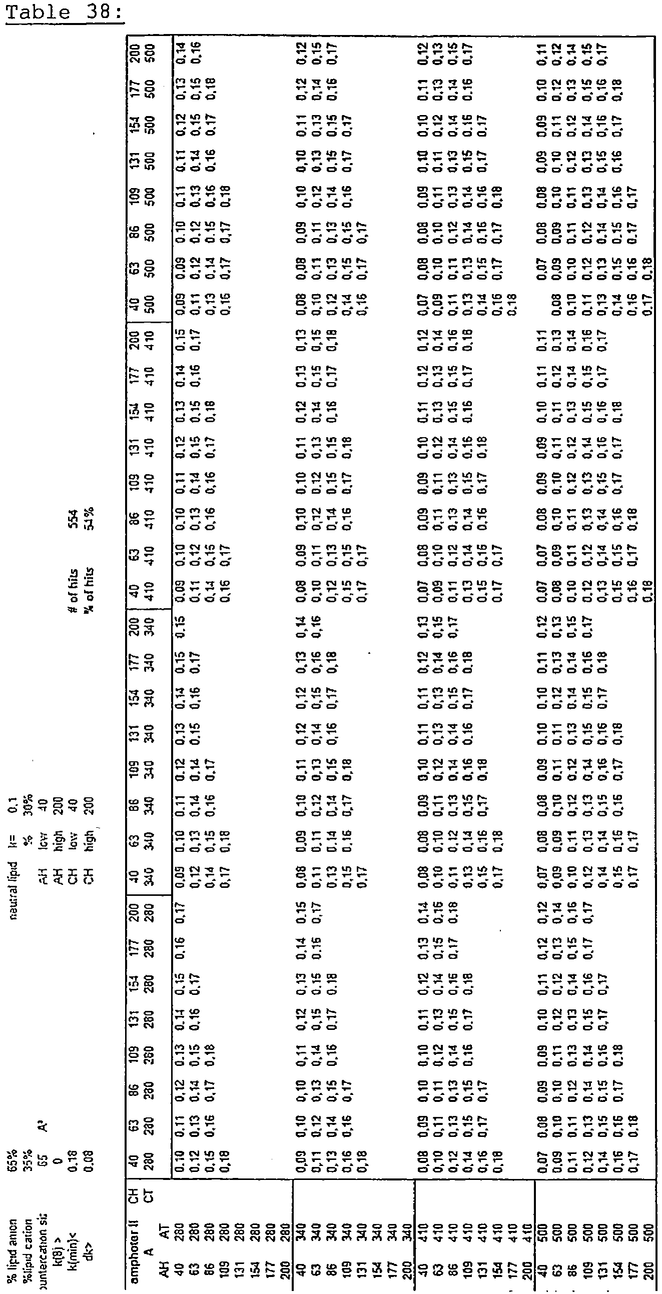

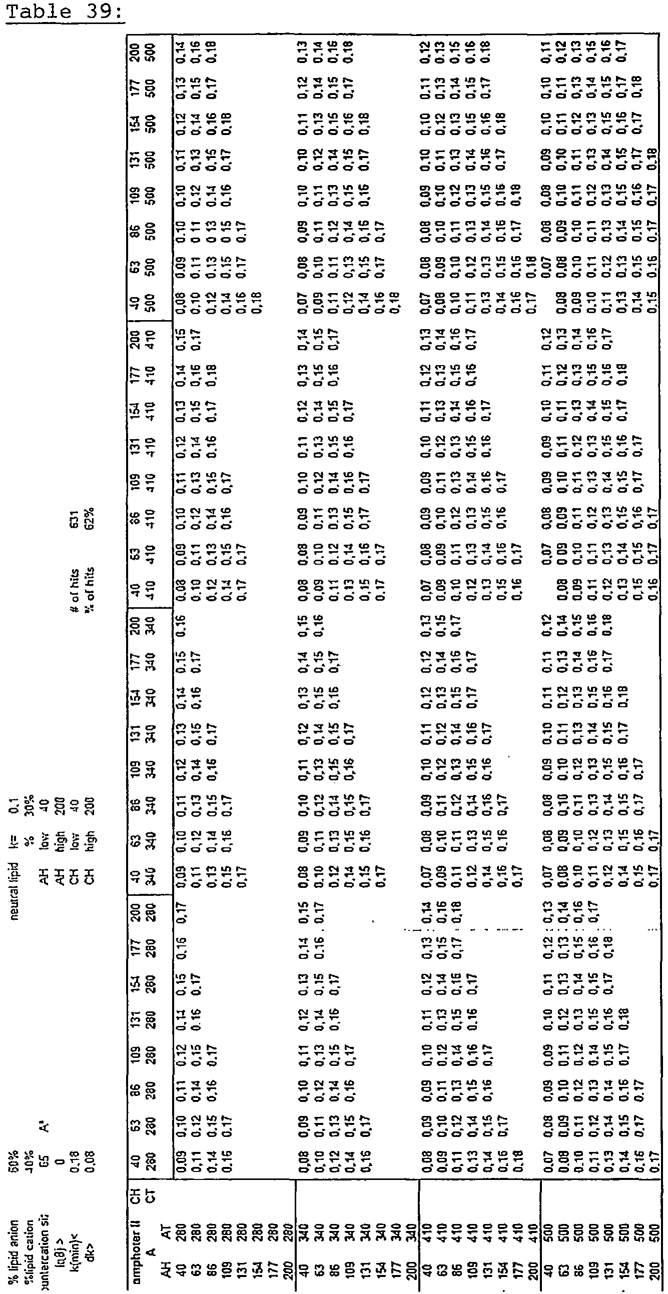

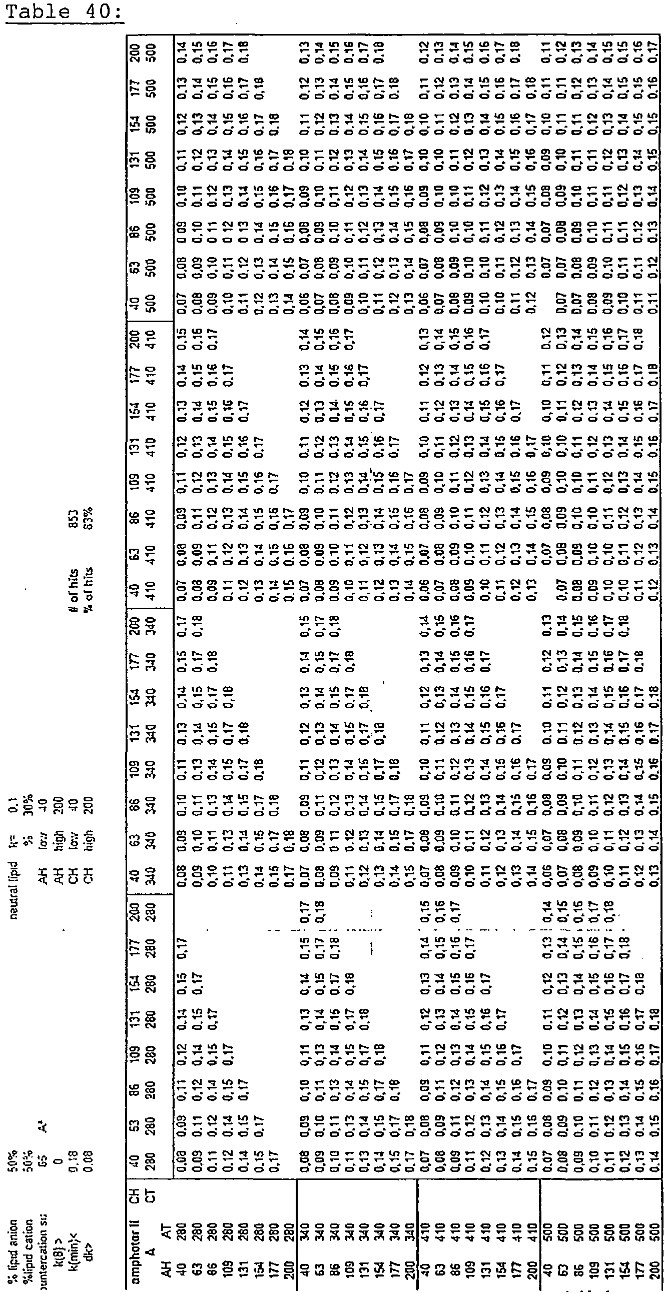

For amphoter II systems a provision with respect to the difference of the pK values has been made above. The extent of this limitation is shown below in table 41:

**Table 41:**

| pK(cation) - pK(anion) | % salt formation |
|---|---|
| 3 | 97 |
| 2 | 91 |
| 1,5 | 83 |
| 1 | 76 |
| 0,5 | 61 |
| 0 | 50 |
| -0,5 | 33 |
| -1 | 24 |
| -1,5 | 14 |
| -2 | 9 |

The effect is most pronounced for systems having equal amounts of the lipid anion and lipid cation and the equation for κ(min) of equilibrated amphoter II systems having a limiting difference in the pK values is then: $κ \left(min\right) = sf * κ \left(salt\right) + \left(1-sf\right) * \left({V}_{AH}/{V}_{AT}+{V}_{CH}/{V}_{CT}\right) ;$ wherein sf denotes the extent of salt formation is shown in table 41.

The reduced formation of the lipid salt leads both to a higher κ(min) and, in consequence, to a reduced dκ(pH8), since κ(pH8) is not affected. A small reduction in the ability of the lipid salt formation therefore results in a rather substantial reduction of fitness of such systems, as shown in tables 42 A-F below for (A) sf = 83% and 30% cholesterol; (B)sf= 76% and 30% cholesterol; (C) sf=83% and 30% of a neutral lipid having a k(neutral) of 0,2; (D) sf =76% and 30% of a neutral lipid having a k(neutral) of 0,2; (E) sf=76% and 15% cholesterol and (F) sf=83% and 15% cholesterol.

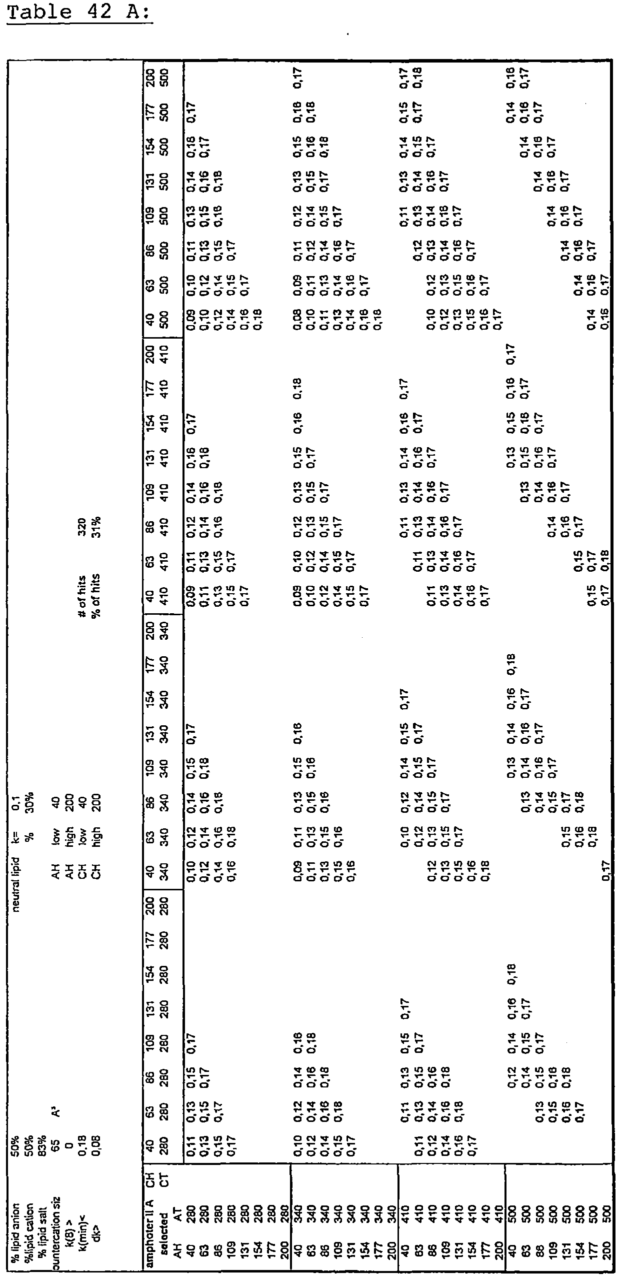

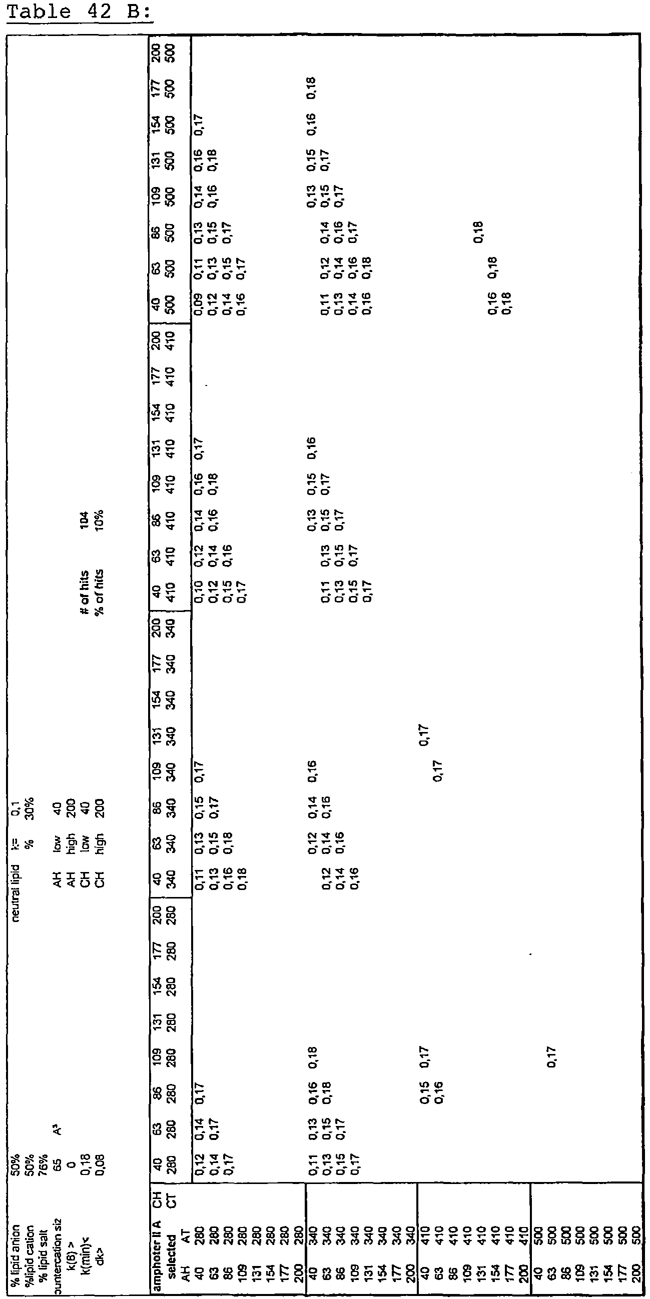

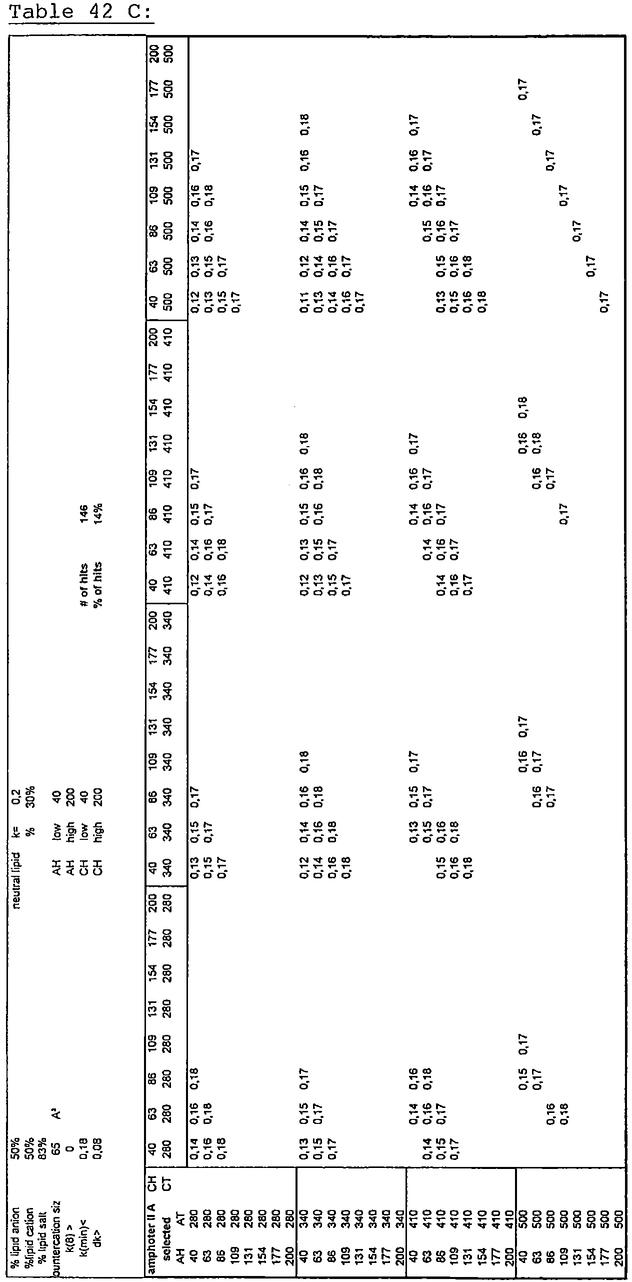

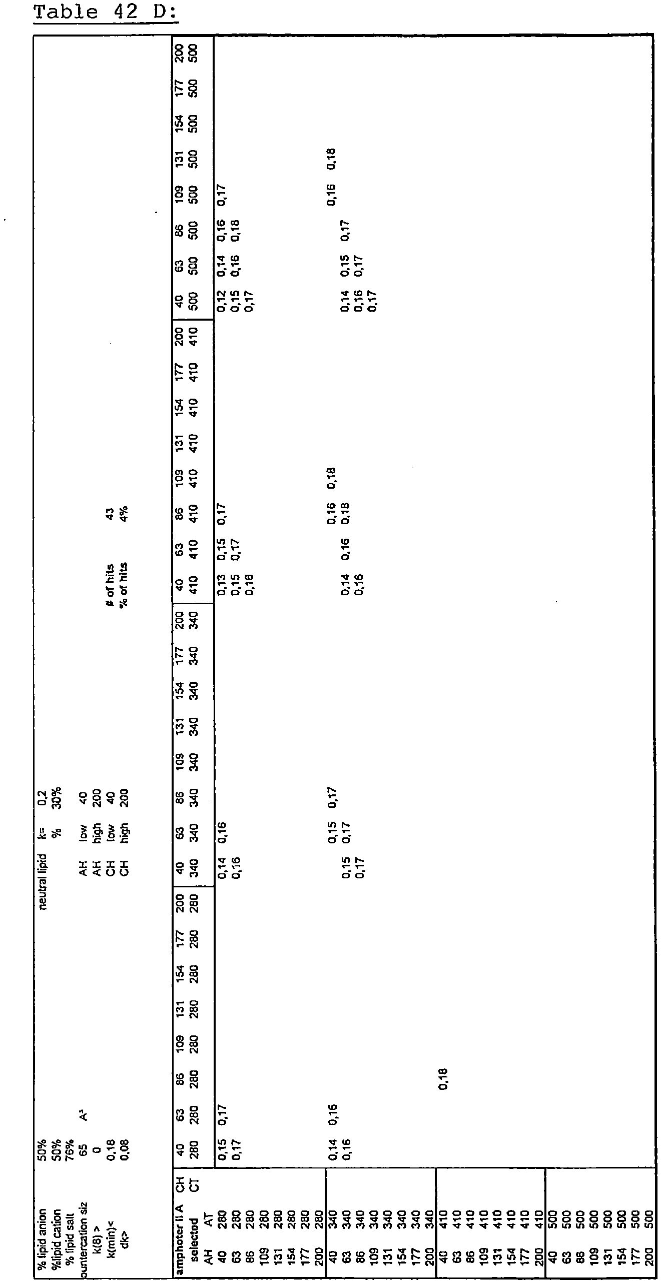

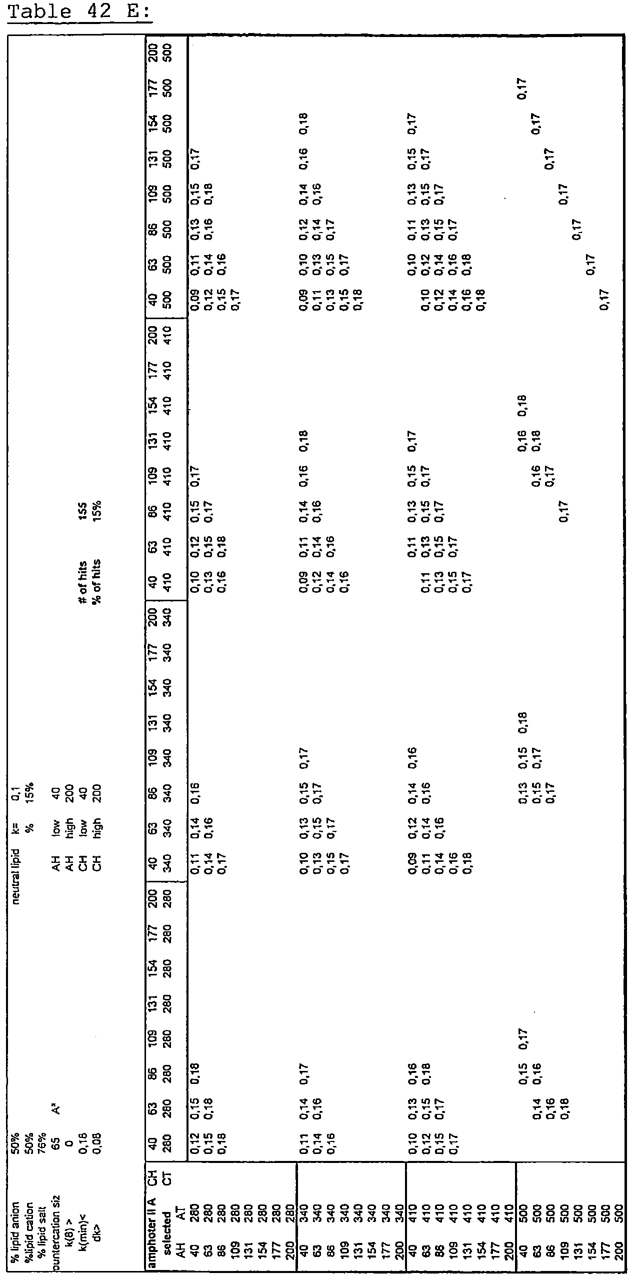

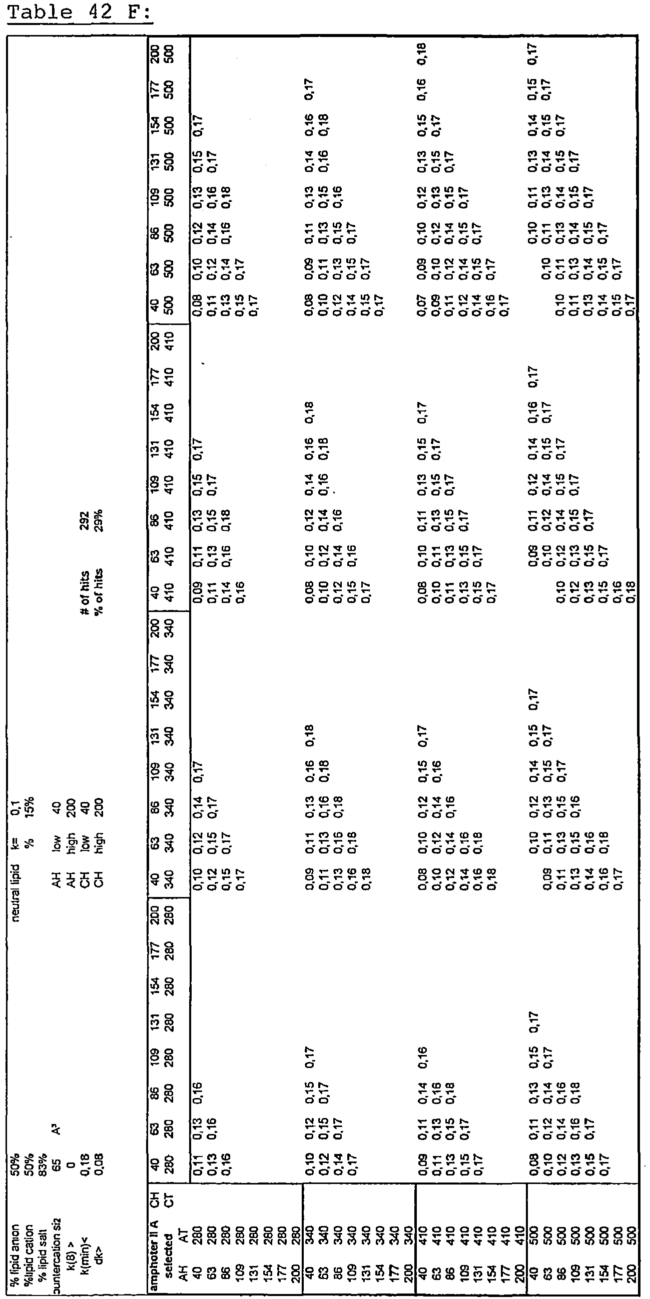

### General description of preferred cation-rich amphoter II and amphoter III systems with k(min)<0,18 and dk(pH8)>0,08

A library of lipids was constructed as described and the interaction between lipid anion and cation follow the amphoter II specification having an excess of the lipid cation. As with other amphoter II systems, there is no lipid salt formation limiting the system amplitude dκ(pH8) and the more stringent value of 0,08 was used for the screen. Amphoter III systems are guided by the same formulas and the results apply accordingly.

The following tables 43-48 identify positively screened species comprising 0, 20, 30, 40, 50 or 60% cholesterol. Values given in the table represent k(min); AH, AT, CH and CT denote the anion and cation head and tail groups, respectively.

### Tables 43-48:

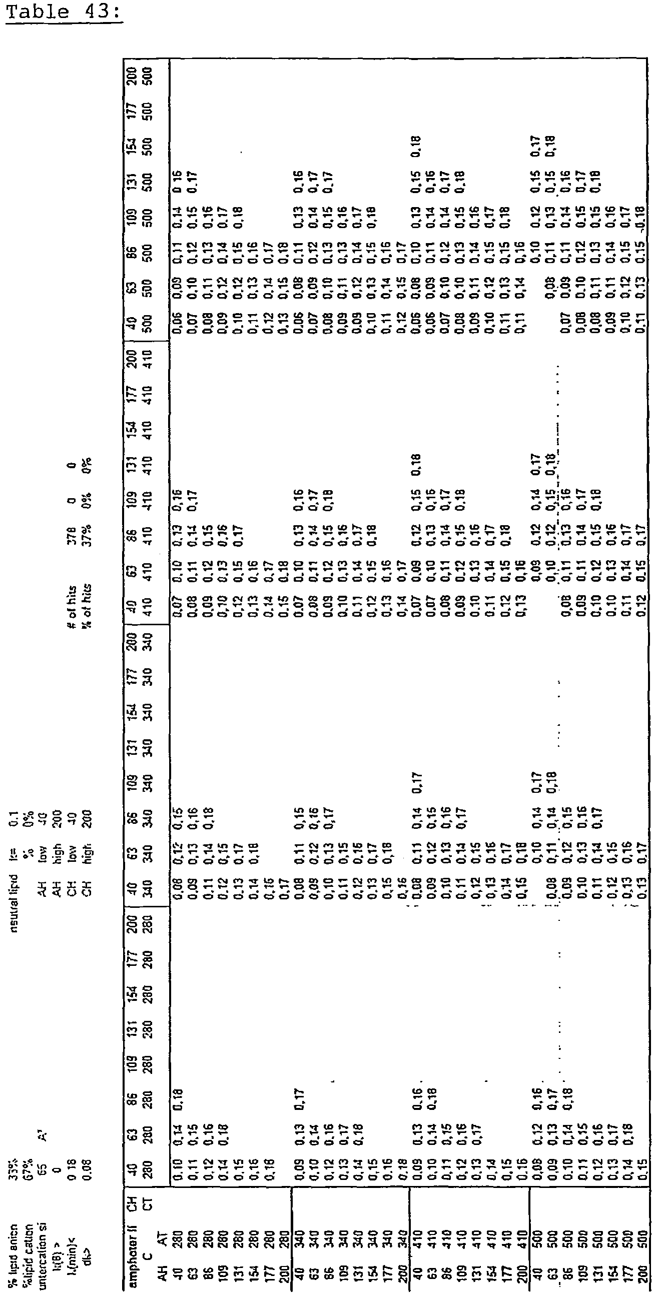

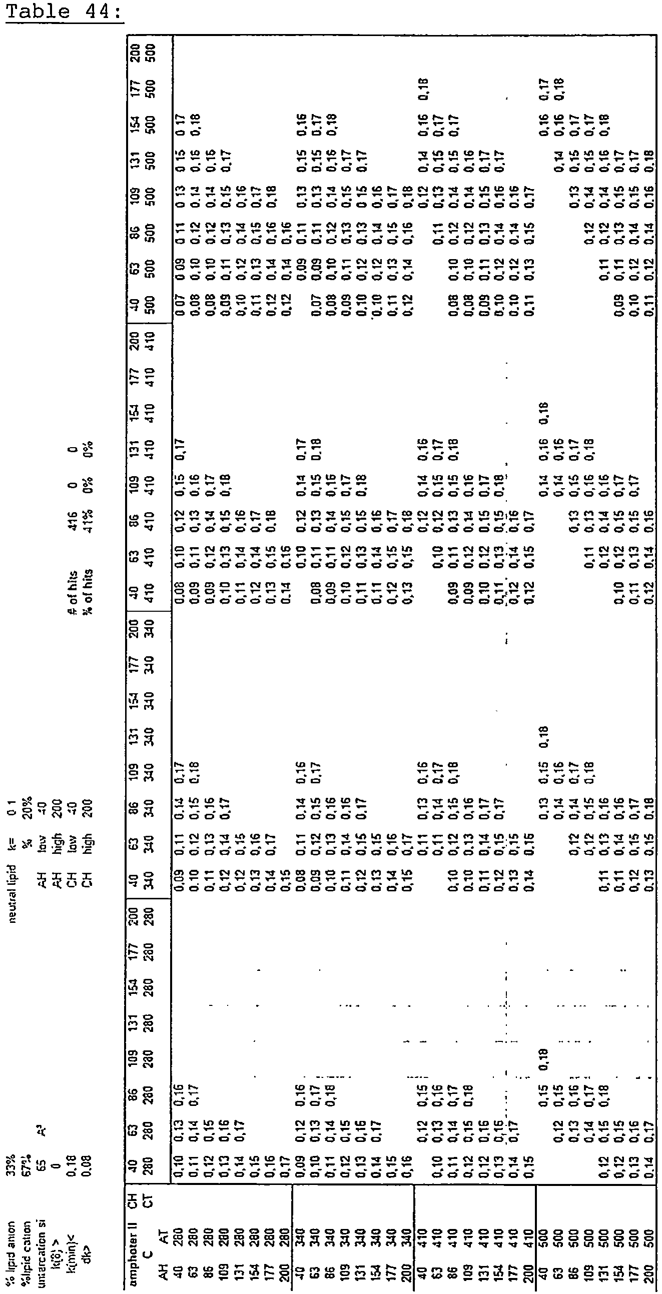

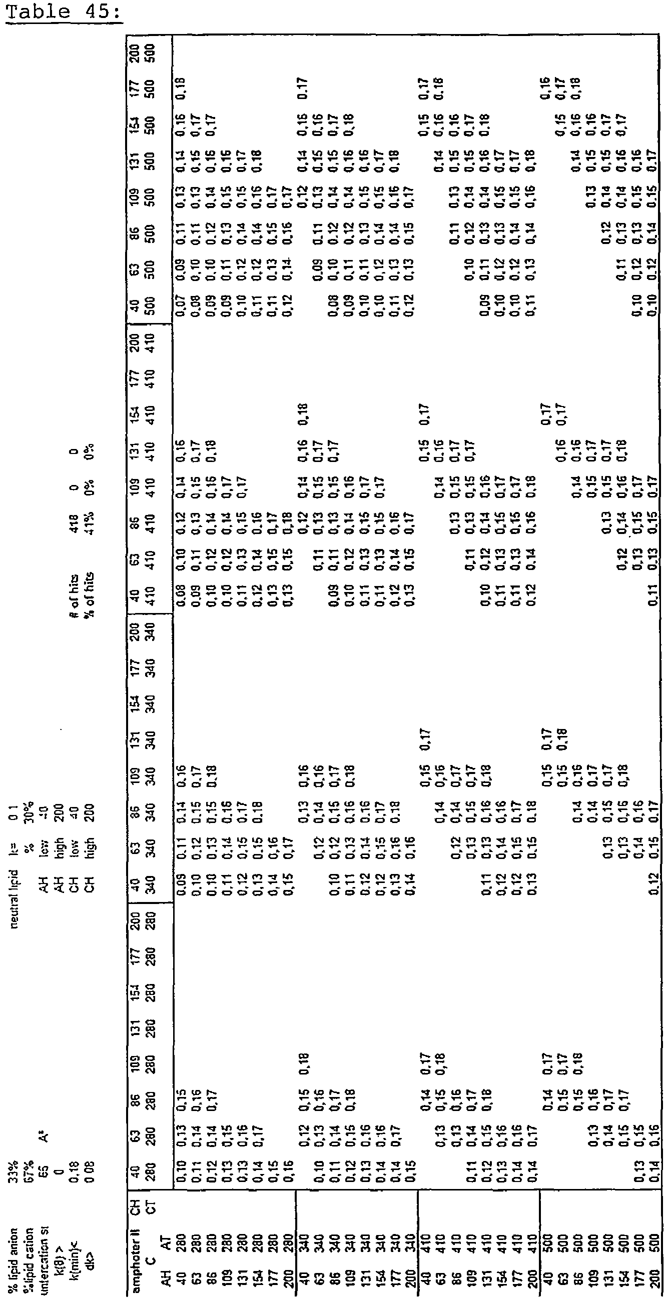

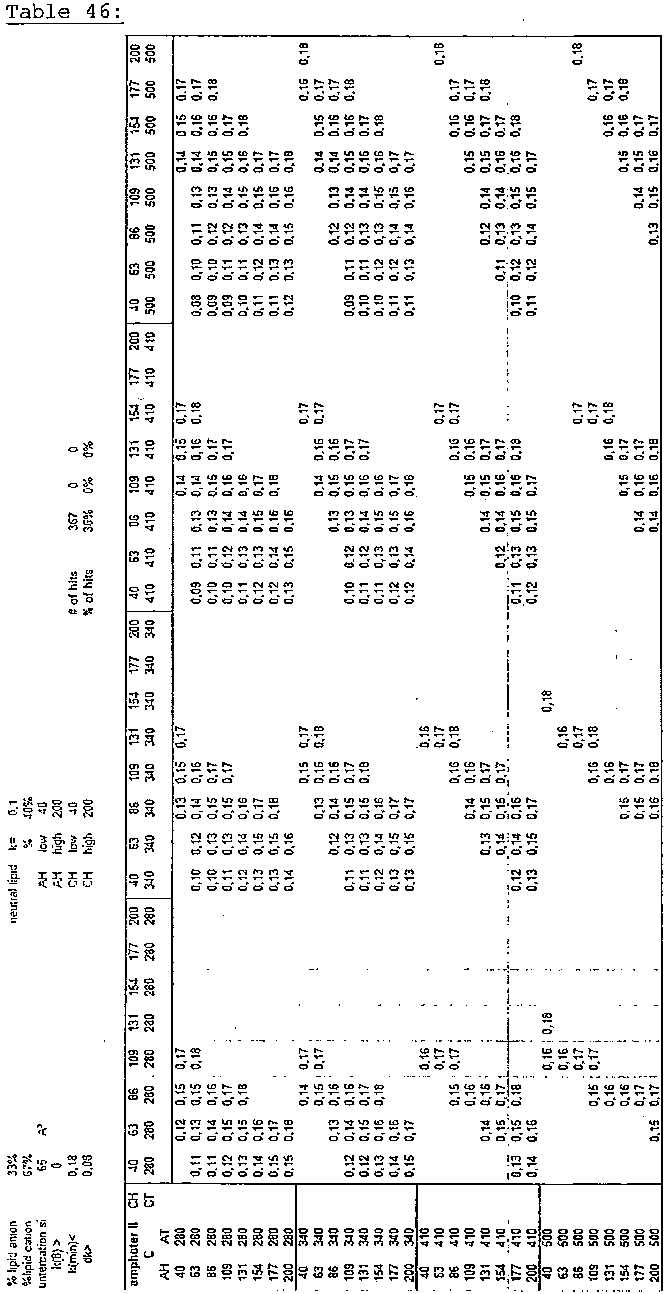

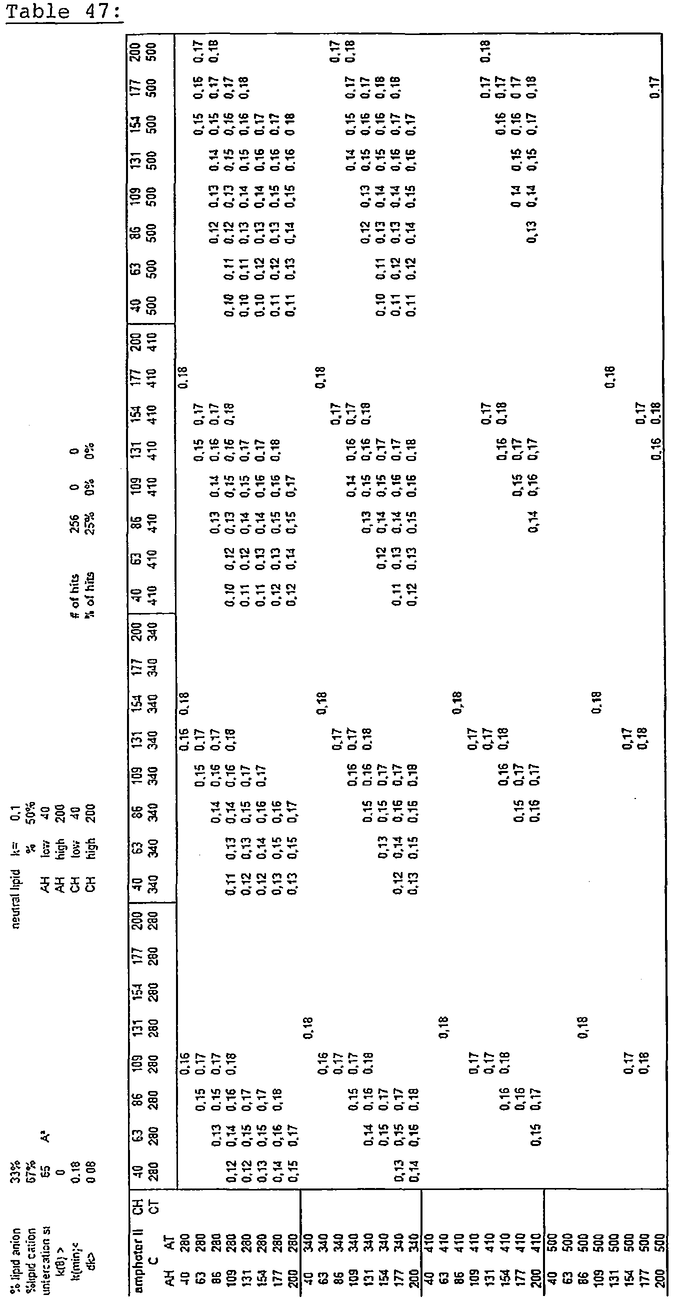

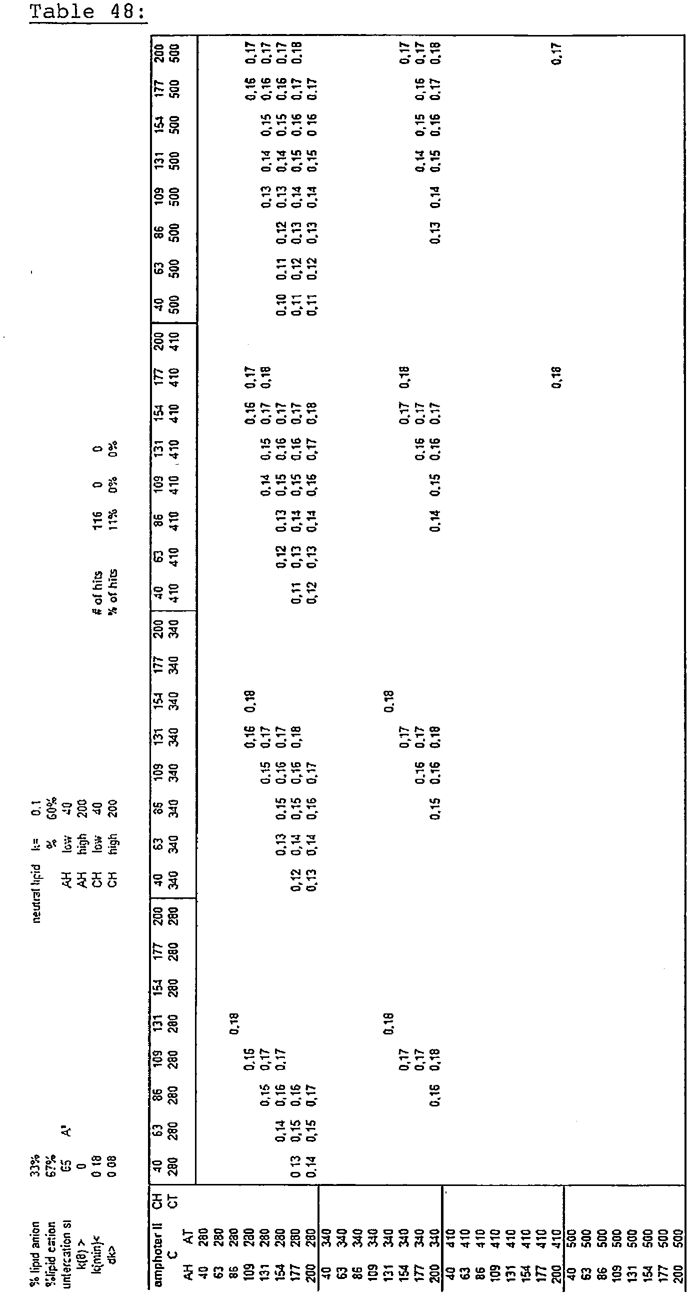

Use of neutral lipids with somewhat higher κ(neutral) is feasible and results for such mixtures comprising 30% of the neutral lipid component with κ(neutral)= 0.15, 0.2 or 0.25 are shown below in table 49-51:

### Tables 49-51:

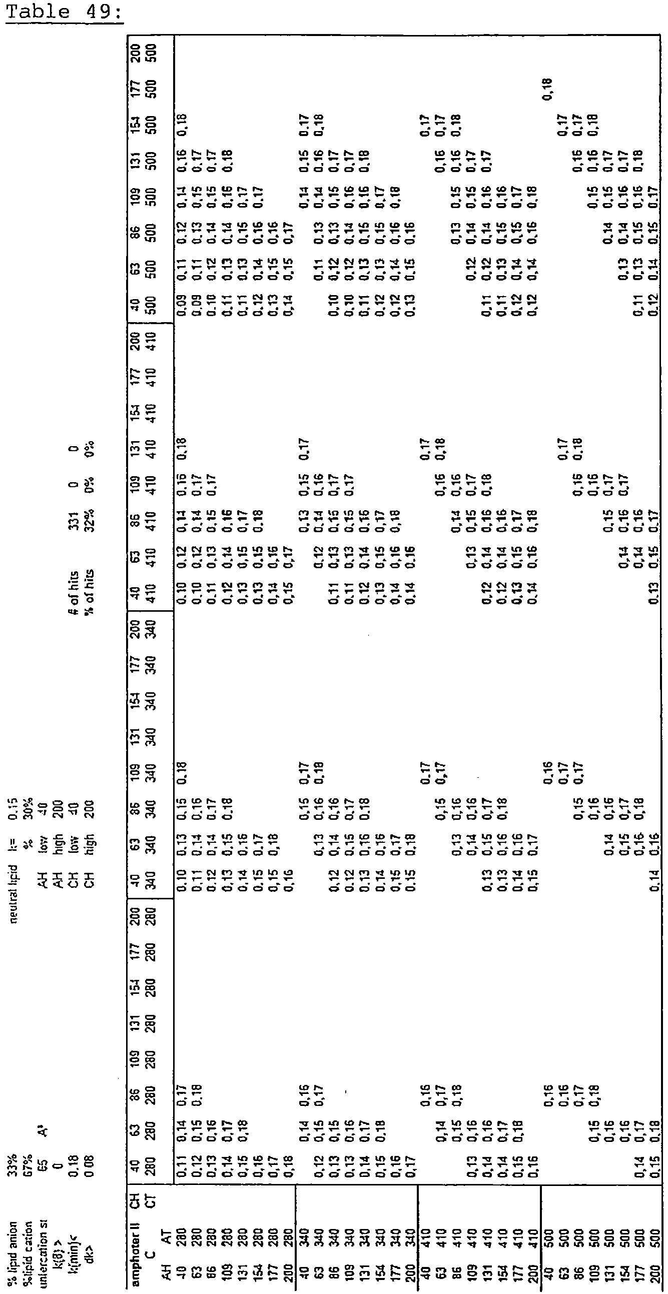

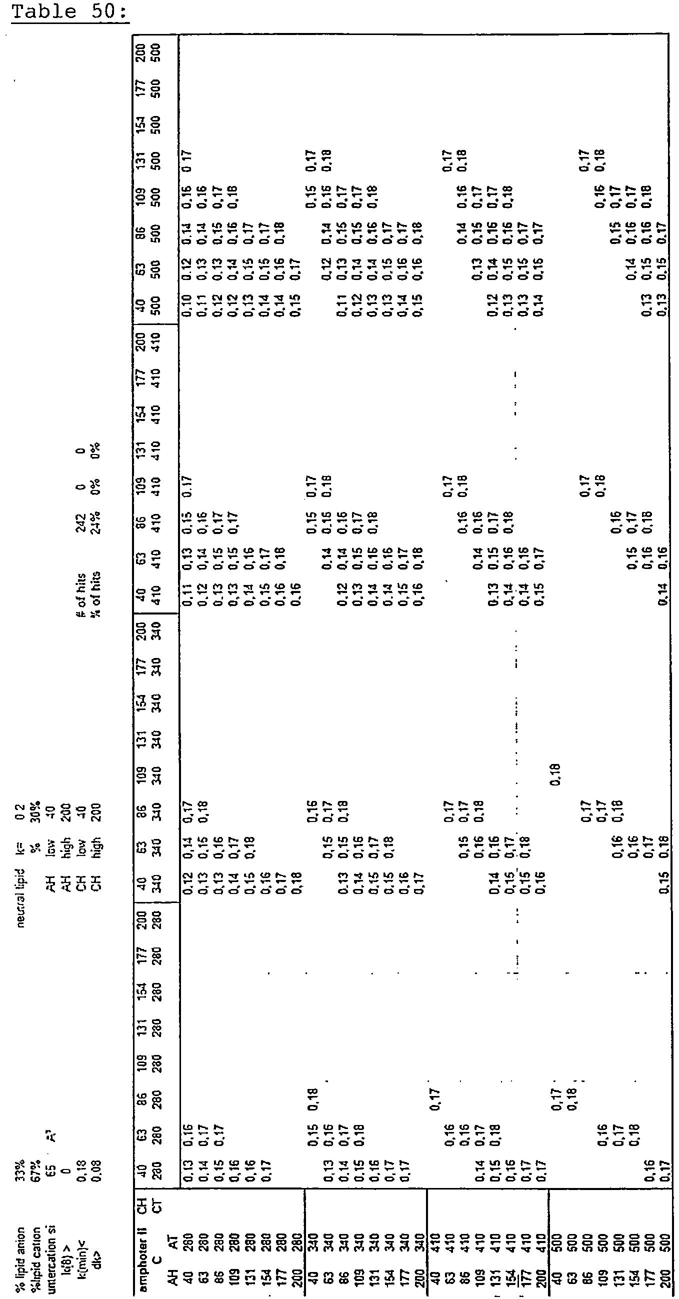

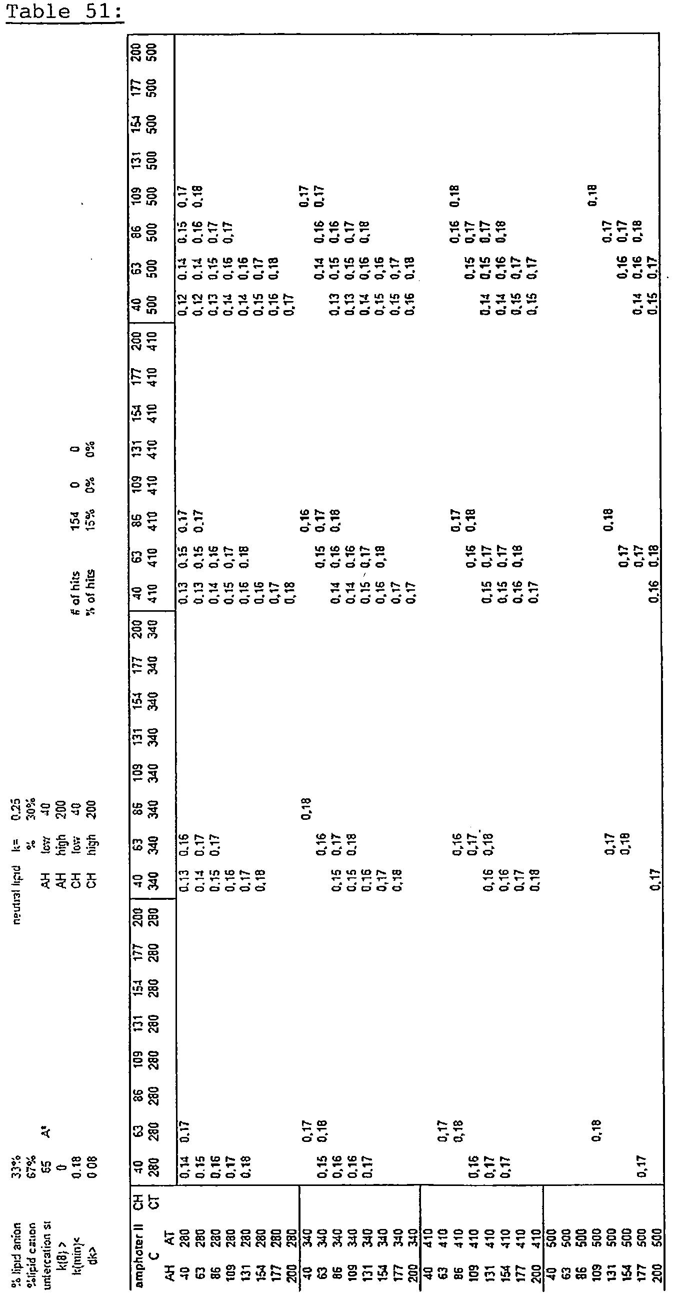

In contrast to amphoter I systems, a variation in the amount of the cationic lipid component does not challenge the system amplitude dκ(pH8), as no lipid salt formation occurs at neutral pH. The system becomes even more permissive and results in a higher frequency of positively screened species as shown below in table 52-53 for anion-rich amphoter II systems comprising 40 or 45% lipid anion and 30% cholesterol.

### Tables 52-53:

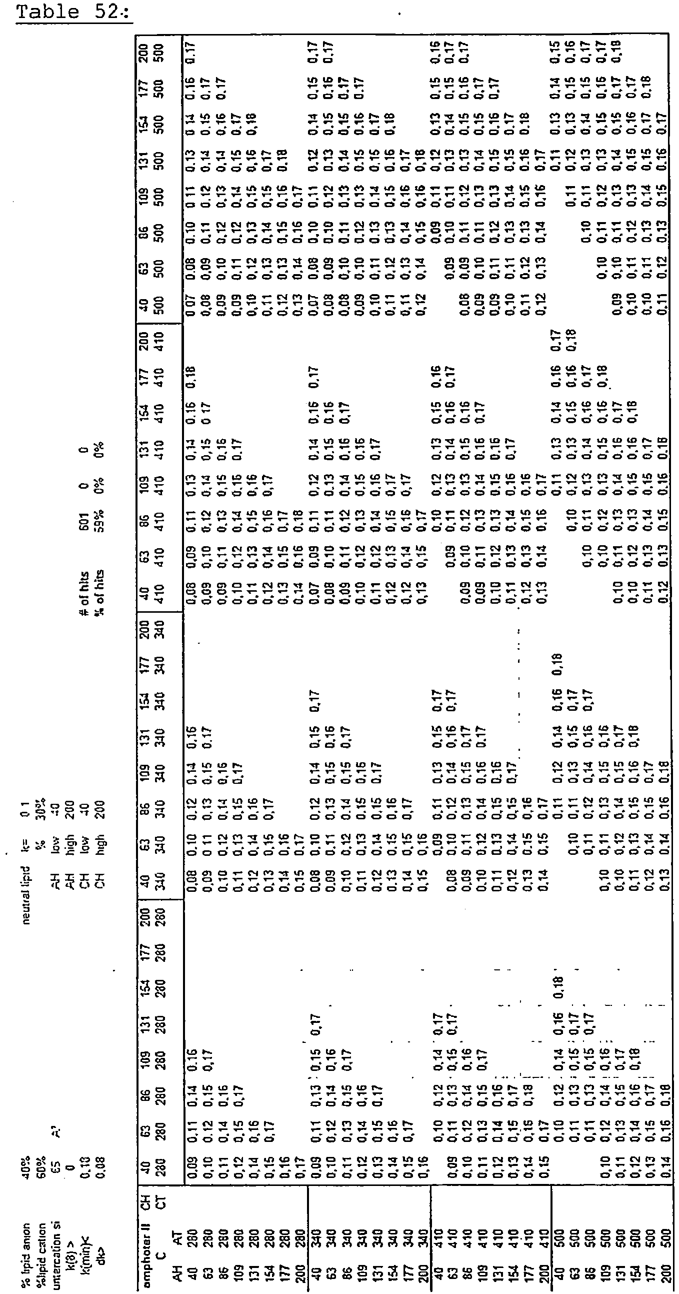

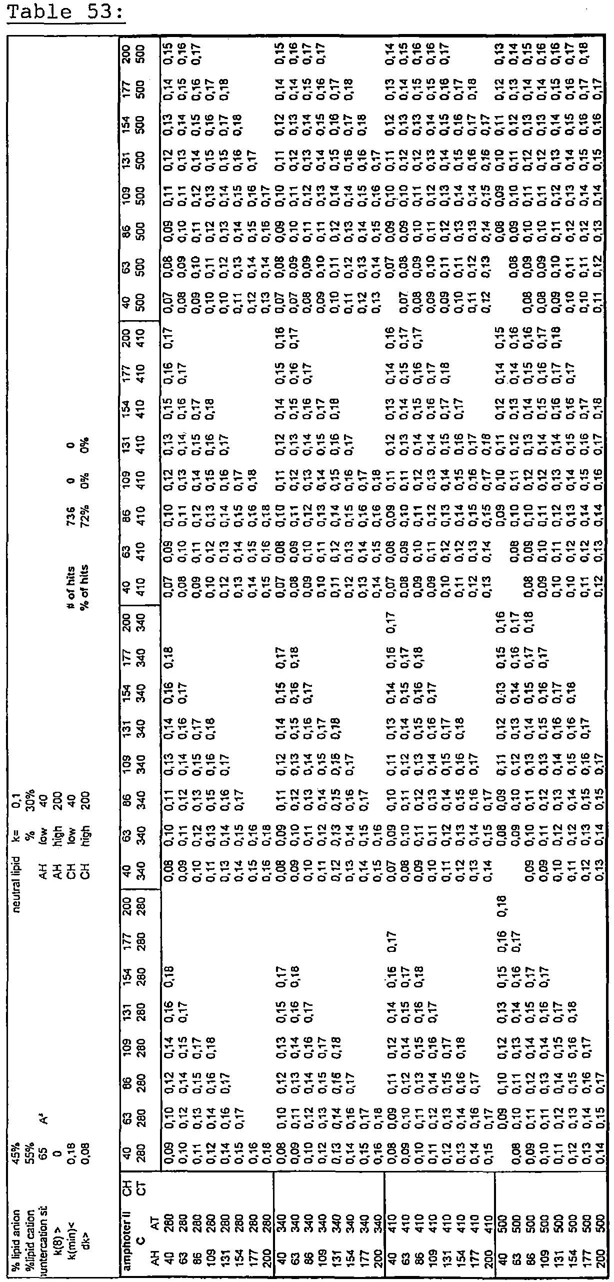

In another aspect of the present invention it was surprisingly found that particles having a certain isoelectric point (IP) between 5 and 6 were most efficacious in cellular transfection. While the fusion zone of amphoteric liposomes is localized around the isoelectric point of a given particle, the cellular response to these fusogenic carriers is limited towards those that become fusogenic in the abovementioned region. This is a cellular aspect that cannot be predicted using the algorithm of this invention.

This finding puts a limitation towards the molar ratios between the lipid anions and lipid cations that can be used to produce said preferred carriers with an IP between 5 and 6. The IP of a given mixture of electrolytes can be calculated as: $IP = - log \left({K}_{a}*\left(1-{x}_{an}/{x}_{cat}\right)/2-SQR\left({K}_{a}*{\left(1-{x}_{an}/{x}_{cat}\right)}^{2}+{K}_{c}*{K}_{a}*{x}_{an}/{x}_{cat}\right)\right) ,$ wherein Kₐ and K_{c} are the dissociation constants for the lipid anion and cation, respectively and xₐₙ and x_{cat} the respective molar fraction of the two; SQR stands for square root and log for the decadic logarithm.

Solutions of the equation for the preferred ranges of IP are listed below in table 54-58 for amphoter I, II and III systems with respect to the pK values of the lipids.

The pK of 15 stands for the high pK of many primary and secondary amines, but also for the non-existing pK of the ammonium groups in many lipid cations.

**Table 54:**

| | | | | | | | |
|---|---|---|---|---|---|---|---|
| amphoter I | with IP > | 5 | cation pK | | 15 | Kc | 1E-15 |
| | with IP< | 6 | | | | | |
| % anion | 55 | 60 | 65 | 70 | 75 | 80 | |
| xa/xc anion pK | 1,22 | 1,50 | 1,86 | 2,33 | 3,00 | 4,00 | Ka |
| 4,2 | | | | | | | 6,3096E-05 |
| 4,7 | 5,4 | 5,0 | | | | | 1,9953E-05 |
| 5,2 | 5,9 | 5,5 | 5,3 | 5,1 | | | 6,3096E-06 |
| 5,7 | | | 5,8 | 5,6 | 5,4 | 5,2 | 1,9953E-06 |

**Table 55-57:**

| | | | | | | | | | | | | | |
|---|---|---|---|---|---|---|---|---|---|---|---|---|---|
| amphoter II | with IP > | 5 | | cation pK | 7 | | | | | | | Kc | 0,0000001 |
| | with IP < | 6 | | | | | | | | | | | |
| % anion | 25 | 30 | 35 | 40 | 45 | 50 | 55 | 60 | 65 | 70 | 75 | 80 | |
| xa/xc anion pK | 0,33 | 0,43 | 0,54 | 0,67 | 0,82 | 1,00 | 1,22 | 1,50 | 1,86 | 2,33 | 3,00 | 4,00 | Ka |
| 4,2 | | | | | | 5,6 | | | | | | | 6,3096E-05 |
| 4,7 | | | | | | 5,9 | 5,3 | | | | | | 1,9953E-05 |
| 5,2 | | | | | | | 5,7 | 5,5 | 5,3 | 5,1 | | | 6,3096E-06 |
| 5,7 | | | | | | | | 5,9 | 5.7 | 5,5 | 5,4 | 5.2 | 1,9953E-06 |
| | | | | | | | | | | | | | |
| amphoter II | with IP > | 5 | | cation pK | 6 | | | | | | | Kc | 0,000001 |
| | with IP < | 6 | | | | | | | | | | | |
| % anion | 25 | 30 | 35 | 40 | 45 | 50 | 55 | 60 | 65 | 70 | 75 | 80 | |
| xa/xc anion pK | 0,33 | 0,43 | 0,54 | 0,67 | 0,82 | 1,00 | 1,22 | 1,50 | 1,86 | 2,33 | 3,00 | 4,00 | Ka |
| 4,2 | | | 5,9 | 5,7 | 5,5 | 5,1 | | | | | | | 6,3096E-05 |
| 4,7 | | | 6,0 | 5,8 | 5,6 | 5,4 | 5,1 | | | | | | 1,9953E-05 |
| 5,2 | | | | 5,9 | 5,8 | 5.6 | 5,4 | 5,3 | 5,2 | 5,0 | | | 6,3096E-06 |
| 5,7 | | | | | 6,0 | 5,9 | 5.7 | 5,6 | 5,5 | 5,4 | 5,3 | 5.1 | 1,9953E-06 |
| | | | | | | | | | | | | | |
| amphoter II | with IP > | 5 | | cation pK | 5 | | | | | | | Kc | 0,00001 |
| | with IP < | 6 | | | | | | | | | | | |
| % anion | 25 | 30 | 35 | 40 | 45 | 50 | 55 | 60 | 65 | 70 | 75 | 80 | |
| xa/xc anion pK | 0,33 | 0,43 | 0,54 | 0,67 | 0,82 | 1,00 | 1,22 | 1,50 | 1,86 | 2,33 | 3,00 | 4,00 | Ka |
| 4,2 | 5,3 | 5,2 | 5,0 | | | | | | | | | | 6,3096E-05 |
| 4,7 | 5,4 | 5,3 | 5,2 | 5,1 | | | | | | | | | 1,9953E-05 |
| 5,2 | 5,5 | 5,4 | 5,3 | 5,3 | 5.2 | 5,1 | 5,0 | | | | | | 6,3096E-06 |
| 5,7 | 5,7 | 5,6 | 5.5 | 5,5 | 5,4 | 5,4 | 5,3 | 5,2 | 5,2 | 5,1 | 5,0 | | 1,9953E-06 |

**Table 58:**

| | | | | | | | |
|---|---|---|---|---|---|---|---|
| amphoter III | with IP > | 5 | | anion pK | 3 | Ka | 0,001 |
| | with IP< | 6 | | | | | |
| % anion | 20 | 25 | 30 | 35 | 40 | 45 | |
| xa/xc cation pK | 0,25 | 0,33 | 0,43 | 0,54 | 0,67 | 0,82 | Kc |
| 7 | | | | | | | 0,0000001 |
| 6,5 | | | | | | 5,9 | 3,1623E-07 |
| 6 | | | | 5,9 | 5,7 | 5,4 | 0,000001 |
| 5,5 | 6,0 | 5,8 | 5,6 | 5,4 | 5,2 | | 3,1623E-06 |
| 5 | 5,5 | 5,3 | 5,1 | | | | 0,00001 |
| 4,5 | | | | | | | 3,1623E-05 |

It is now possible to further describe the preferred lipid species forming functional amphoteric liposomes. The in silico screening data give detailed description of useful combinations of charged and neutral lipids and include detailed information on their respective head and tail group sizes. The data also show how to identify and select the molar ratio between the lipid anion, lipid cation and one or more neutral lipid species in a membrane. A further specification was also made with respect to the pK of the lipid anions and lipid cations in question and the tables above provide a link between the pK of the charged species and the resulting molar ratios to achieve the preferred IP of the resulting mixture.

The state of the art provides methods and data how to determine the pK of a lipid, e.g. in Hafez et al. (2000) Biochim Biophys Acta 1463,107 -114 or Budker et al. (1996), Nature Biotechnology 14, 760-764) or Heyes et al. (2005), J. Control. Release 107(2), 276-287. Another way to determine the pK of a given structure includes the use of quantitative structure-activity relationships and the databases provided therein, e.g. as in ACD/pka DB (Version 7.06) (Advanced Chemistry Development Inc.), a software program that provides pK analysis and calculation.

The experimental pK values may differ from the calculated values to some extent, such difference can be attributed to different experimental methods being used or to the limited chemical activity of the charged groups when placed into the membrane context. In fact, the local concentrations for membrane-bound groups is much higher than in for the same material in free solution and reduced dissociation, hence reduced chemical activity is a known phenomenon for concentrated solutions of electrolytes. This results in a shift towards higher pratical pK values for the lipid anions and lower values for the lipid cations, such difference being +1 for the lipid anions and -0,5 for the lipid cations in many aspects of the present invention.

### Chemical representations of preferred lipid systems

This disclosure integrates experimental data from membrane mixings and cellular transfections with a mathematical description that transforms mechanistic insight based on lipid shape and lipid interaction into a system that allows a detailed description of preferred systems based molecular volumes, interaction types and pK values. While the mathematical description is continuous, any lipid gives a distinct representation within that continuum. The following tables give such distinct representations of some of the lipid head and tail groups that fall within the chemical space described by the experimental data and the in silico screens described above. All molecular volumes and all pK values from tables 59, 60 and 61 were calculated using DS Viewer Pro 5.0 (Accelrys Inc., San Diego, CA) and ACD/pka DB (Version 7.06) (Advanced Chemistry Development Inc.), respectively. pK values are given for a molecule in solution and the abovemade considerations for the pK shift in the membrane environment may apply accordingly on a case by case basis.

It is possible to use other tools well-known to those skilled in the art to calculate molecular volumes. The qualitative prediction would not even change if molecular cross-sections were used instead of the volumes. Of course, one would have to re-calibrate the results in such a case.

The molecular volume calculations disclosed herein are silent on chain saturation in the hydrophobic parts. Use of unsaturated lipids may have specific advantages, since lipid membranes comprising such lipids have higher fluidity at ambient temperature which may improve fusion behaviour. It is also known that unsaturated lipids exert lateral pressure in the membrane, thus a correction factor can be inserted to reflect the apparent volume of these components. Such correction factor is higher than 1.

### Lipid tail groups

List of the most frequently used lipid tail groups is given in table 1 of this disclosure.

### Lipid head groups: neutral head groups

Cholesterol and the zwitterionic phospholipids PC and PE are the most typical components in this category. The respective head group volumes are 30A³, 136A³ and 98A³, respectively. Cholesterol and PE are devoid of counterions, the first due to its neutral character, the second due to formation of a zwitterionic structure. The PC headgroup attracts both one counteranion and one counteraction and the respective molecular volumes are given in table 2 of this disclosure.

### Lipid head groups: anionic head groups

The standard charge element for lipid anions in amphoter I and II is the carboxyl group. Direct association with a membrane anchor yields the minimal head groups that are preferred in many formulations. A list of species is provided in the table 59 below.

**Table 59:**

| Structure | R= cholesterol | | | R=diacylglycerol | | |
|---|---|---|---|---|---|---|
| | Cpd.No. | head volume | pK | Cpd.No. | head volume | pK |
| R-COOH | 1 | 29,5 | 4,79 Chol-Cl | 12 | 29,4 | 1,9 for glyceric acid |
| R-O-CH₂-COOH | 2 | 49,4 | 3,45 | 13 | 61,4 | 3,21 |
| R-O-CH₂-CH₂- | 3 | 62,9 | 4,29 | 14 | 74,9 | 4,29 |
| COOH | | | | | | |
| R-O-CH₂-CH₂-CH₂-COOH | 4 | 75,1 | 4,63 | 15 | 87,1 | 4,6 |
| R-O-(CH₂)₄-COOH | 5 | 87,8 | 4,69 | 16 | 99,9 | 4,69 |
| R-O-C(O)-COOH | 6 | 52,4 | 1,44 | 17 | 64,5 | 1,28 |
| R-O-C(O)-CH₂-COOH | 7 | 66,3 | 2,74 Chol-C3 | 18 | 78,4 | 2,53 |
| R-O-C(O)-CH₂-CH₂-COOH | 8 | 78,2 | 4,41 | 19 | 90,2 | 4,33 |
| | | | CHEMS | | | DMGS |
| | | | | | | DOGS |
| R-O-C(O)-CH₂-CH₂-CH₂-COOH | 9 | 90,9 | 4,61 Chol-C5 | 20 | 102,9 | 4,6 |
| R-OOC-(CH₂)₄-COOH | 10 | 103,9 | 4,68 | 21 | 116,0 | 4,68 |
| R-OOC-(CH₂)₆-COOH | 11 | 130,1 | 4,76 | 22 | 142,1 | 4,76 |

In preferred embodiments of the invention, the diacylglycerols are dimyristoyl-, dipalmitoyl-, dioleoyl-, distearoyl- and palmitoyloleoylglycerols and R in the table above includes any selection from this group.

Besides the diacylglycerols and cholesterol compounds, long chain fatty acids can be used to construct amphoteric liposomes. While their tail volumes do vary, the head group is defined as the carbonyl atom and the C2. The volume of this fragment is 41,9Å³ and the pK for these acids is 4.78.

There are two relevant acidic head groups in phospholipids: phosphoglycerol, having a fragment volume of 115,9Å³ and phosphoserin, with a fragment size of 121,7Å³. The respective pK values are 1.34 for phosphoglycerol and 8.4 (amino function in phosphatidylserin), 1.96 (carboxyl function in phosphatidylserine) and 1.26 for its phosphate ester.

### Lipid head groups: cationic lipid head groups

The cationic lipids head groups are chemically more diverse compared to their anionic counterparts. While a pH-sensitive nitrogen functions as a charge centre, this element can be embedded into various aliphatic, heterocyclic or aromatic structures. The following list provides examples for small cationic head groups.

**Table 60:**

| **Structure** | **R= dialkyl** | | | **R=diacylglycerol** | | |
|---|---|---|---|---|---|---|
| | **Cpd.No.** | **head volume** | **pK** | **Cpd.No.** | **head volume** | **pK** |
| R-N (CH₃)₃ | | | | 59 | 66,3 DOTAP | ammonium salt |
| R-NH₂ | 23 | 34,1 | 10,84 | 60 | 22,5 | 6,22 |
| | | | Distearin | | | |
| R-NH-CH₃ | 24 | 45,7 | 9,81 | 61 | 34,1 | 8,07 |
| | | | | | | DOMAP |
| R-NH-CH₂-CH₃ | 25 | 56,8 | 9,89 | 62 | 45,1 | 8,07 |
| | | | | | | DOEAP |
| R-NH-CH₂-CH₂-CH₃ | 26 | 67,9 | 9,89 | 63 | 56,2 | 8,25 |
| | | | | | | DOPAP |
| R-N-(CH₃)₂ | 27 | 57,2 | ammonium salt | 64 | 45,7 | 8,02 |
| | | | DDAB | | | DODAP |
| 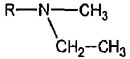 | 28 | 68,4 | ammonium salt | 65 | 56,8 | 8,10 |
| 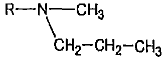 | 29 | 79,5 | ammonium salt | 66 | 67,8 | 8,10 |
| 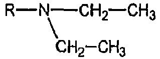 | 30 | 80,2 | ammonium salt | 67 | 68,5 | 9,03 |
| 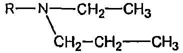 | 31 | 91,3 | ammonium salt | 68 | 79,6 | 8,18 |
| 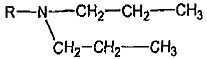 | 32 | 102,4 | ammonium salt | 69 | 90,2 | 8,18 |
| 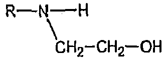 | 33 | 63,7 | 8,94 | 70 | 51,7 | 7,61 |
| 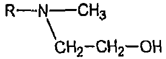 | 34 | 75,2 | ammonium salt | 71 | 63,7 | 7,15 |
| | | | | | | DOMHEAP |
| 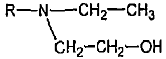 | 35 | 86,5 | ammonium salt | 72 | 74,8 | 7,23 |
| 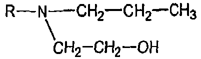 | 36 | 97,4 | ammonium salt | 73 | 85,8 | 7,23 |
| 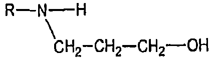 | 37 | 74,6 | 9,33 | 74 | 62,9 | 7,72 |
| 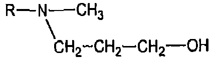 | 38 | 86,1 | ammonium salt | 75 | 74,4 | 7,54 |
| 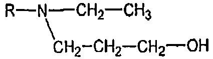 | 39 | 97,3 | ammonium salt | 76 | 85,7 | 7,62 |
| 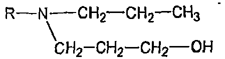 | 40 | 109,1 | ammonium salt | 77 | 97,3 | 7,62 |
| 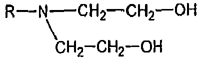 | 41 | 93,2 | ammonium salt | 78 | 81,4 | 6,75 |
| | | | | | | DODHEAP |
| 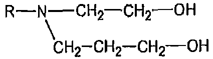 | 42 | 104,3 | ammonium salt | 79 | 92,7 | 6,67 |
| 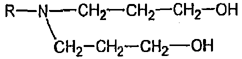 | 43 | 115,2 | ammonium salt | 80 | 103,4 | 7,07 |
| 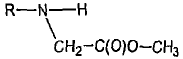 | 44 | 80,0 | 7,27 | 81 | 68,2 | 4,78 |
| | | | | | | DOGME |
| 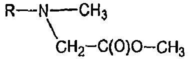 | 45 | 91,6 | ammonium salt | 82 | 79,5 | 5,48 |
| | | | | | | DOMGME |
| 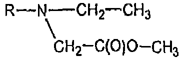 | 46 | 102,7 | ammonium salt | 83 | 90,9 | 5,56 |
| 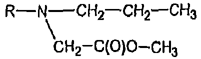 | 47 | 114,4 | ammonium salt | 84 | 102,0 | 5,56 |
| 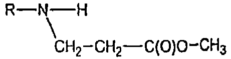 | 48 | 91,2 | 8,30 | 85 | 79,3 | 6,32 |
| 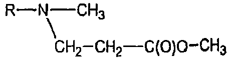 | 49 | 102,8 | ammonium salt | 86 | 91,4 | 6,51 |
| 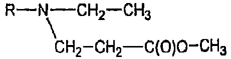 | 50 | 114,2 | ammonium salt | 87 | 102,6 | 6,59 |
| 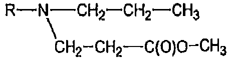 | 51 | 125,2 | ammonium salt | 88 | 113,7 | 6,59 |
| 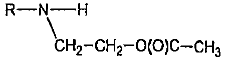 | 52 | 90,9 | 8,62 | 89 | 79,3 | 7,79 |
| 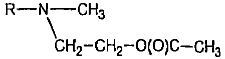 | 53 | 103,0 | ammonium salt | 90 | 91,3 | 6,83 |
| 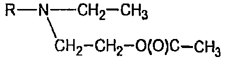 | 54 | 114,2 | ammonium salt | 91 | 102,3 | 6,91 |
| 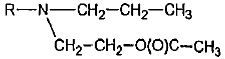 | 55 | 125,1 | ammonium salt | 92 | 113,2 | 6,91 |
| Phosphatidylserine methyl ester | 56 | | n.d. | 93 | 134,4 | 5,25 |
| R-morpholine | 57 | | n.d. | 94 | 71,4 | 6,18 |
| | | | | | | MODOG |
| R-imidazole | 58 | | n.d. | 95 | 56,9 | 6,50 |
| | | | | | | DPIM |
| | | | | | | DOIM |
| 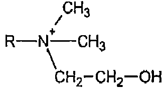 | | | | 96 | 88 | n.d. |
| 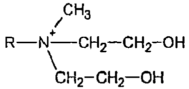 | | | | 97 | 108 | n.d. |

In preferred embodiments of the invention, the diacylglycerols are dimyristoyl-, dipalmitoyl-, dioleoyl-, distearoyl- and palmitoyloleoylglycerols and the dialkyls are dimyristyl-, dipalmityl-, dioleyl-, distearyl- and palmityloleyl and R in the table above includes any selection from this group.

A number of cationic lipid compounds use cholesterol as a membrane anchor. Typically, linker groups are inserted to mount the charged group onto this backbone and compounds in this group comprise HisChol, MoChol, CHIM and others. Fragment volumes and pK values are listed in the table 61 below.

**Table 61:**

| **Head group** | **Fragment volume** | **pK** |
|---|---|---|
| HisChol | 150,5 | 7,17 |
| MoChol (C4Mo2) | 168,2 | 7,01 |
| DmC3Mo2 | 181,2 | 6,95 |
| C4Mo4 | 193,9 | 7,71 |
| DmC4Mo2 | 195,3 | 7,01 |
| C3Mo3 | 168,5 | 7,51 |
| C3Mo2 | 155,2 | 6,96 |
| C5Mo2 | 180,8 | 7,04 |
| C6Mo2 | 193,8 | 7,05 |
| C8Mo2 | 219,5 | 7,05 |
| CHIM | 119,2 | 7,00 |
| DC-CHol | 87,2 | 8,12 |
| TC-Chol | 98,9 | Ammonium salt |
| MoC3Chol | 123,8 | 7,61 |
| N-methyl-PipChol | 103,1 | 6,99 |
| DOEPC+ | 161,4 | Ammonium salt |

The documentation provided above allows the identification of useful lipid species with respect to all necessary parameters such as lipid head group size and pK as well as lipid tail group sizes.

The following disclosure combines the abovementioned findings for specific embodiments of the invention. In there, the limitations towards κ(min), dκ(pH8) and IP are applied towards specific lipid chemistries and specific formulations are described.

In some preferred aspects of such embodiment, CHEMS, DMGS, DOGS or Chol-Cl are used as the anionic lipid species. The following table 62 provide an analysis for these lipids in amphoter I systems, wherein the lipid cation is a strong cation with a pK greater then 8.5 and said lipid cation has V_{CH}=50Å³ or 100Å³, respectively and V_{CT}=500Å³, wherein the neutral lipid is cholesterol, κ(min)<0,13 and >0,09; dκ(pH8)>0,04 and the IP between 5 and 6:

**Table 62:**

| Chol C1 - no hits, equation has no solution | | | | | | | | |
|---|---|---|---|---|---|---|---|---|
| **screening parameter** | | | **system** | | | | | |
| k(min) < | 0,13 | | k(neutral) | Chol | | | | |
| k(min)> | 0,09 | | anion | CHEMS | | | | |
| dk8> | 0,04 | | cation | 50/500 strong | | | | |
| IP> | 5 | | counteranion | PO4 | | | | |
| IP< | 6 | | countercation | Na | | | | |
| Isoelectric point (IP) | | 15,3 | 14,9 | 14,7 | 10,3 | 5,8 | 5,6 | 5,2 |
| | % anion | 25 | 35 | 40 | 50 | 60 | 65 | 75 |
| % neutral lipid | 0 | | | | | | | |
| | 10 | | | | | | 0,13 | |
| | 20 | | | | | 0,11 | 0,12 | |
| | 30 | | | | | 0,11 | 0,12 | |
| | 40 | | | | | 0,11 | 0,12 | |
| | 50 | | | | | 0,11 | 0,11 | 0,13 |
| | 60 | | | | | 0,10 | 0,11 | 0,12 |
| | 70 | | | | | 0,10 | 0,10 | 0,11 |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | DMGS | | | | |
| dk8> | 0,04 | | cation | 50/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,2 | 5,6 | 5,4 | 5,0 |
| | % anion | 25 | 35 | 40 | 50 | 60 | 65 | 75 |
| % neutral lipid | 0 | | | | | | | |
| | 10 | | | | | | 0,13 | |
| | 20 | | | | | 0,11 | 0,12 | |
| | 30 | | | | | 0,11 | 0,12 | |
| | 40 | | | | | 0,10 | 0,12 | 0,13 |
| | 50 | | | | | 0,10 | 0,11 | 0,12 |
| | 60 | | | | | 0,10 | 0,11 | 0,12 |
| | 70 | | | | | 0,10 | 0,10 | 0,11 |
| | | | | | | | | |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | DOGS | | | | |
| dk8> | 0,04 | | cation | 50/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,2 | 5,6 | 5,4 | 5,0 |
| | % anion | 25 | 35 | 40 | 50 | 60 | 65 | 75 |
| % neutral lipid | 0 | | | | | | | 0,13 |
| | 10 | | | | | | | 0,12 |
| | 20 | | | | | | 0,11 | 0,12 |
| | 30 | | | | | 0,09 | 0,11 | 0,12 |
| | 40 | | | | | 0,09 | 0,10 | 0,11 |
| | 50 | | | | | 0,09 | 0,10 | 0,11 |
| | 60 | | | | | 0,09 | 0,10 | 0,11 |
| | 70 | | | | | 0,09 | 0,10 | 0,10 |
| | | | | | | | | |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | Chol C1 | | | | |
| dk8> | 0,04 | | cation | 100/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,4 | 6,1 | 5,9 | 5,5 |
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | | | |
| | 40 | | | | | | | |
| | 50 | | | | | | | 0,09 |
| | 60 | | | | | | | 0,09 |
| | 70 | | | | | | | 0,09 |
| k(min) < | 0,13 | | k(neutral) | Chol | | | | |
| k(min)> | 0,09 | | anion | CHEMS | | | | |
| dk8> | 0,04 | | cation | 100/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,3 | 5,8 | 5,6 | 5,2 |
| | % anion | 25 | 35 | 40 | 50 | 60 | 65 | 75 |
| % neutral lipid | 0 | | | | | | | |
| | 10 | | | | | | | |
| | 20 | | | | | 0,13 | | |
| | 30 | | | | | 0,13 | | |
| | 40 | | | | | 0,12 | 0,13 | |
| | 50 | | | | | 0,12 | 0,12 | |
| | 60 | | | | | 0,11 | 0,11 | 0,13 |
| | 70 | | | | | 0,11 | 0,11 | 0,12 |
| | | | | | | | | |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | DMGS | | | | |
| dk8> | 0,04 | | cation | 100/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,2 | 5,6 | 5,4 | 5,0 |
| | % anion | 25 | 35 | 40 | 50 | 60 | 65 | 75 |
| % neutral lipid | 0 | | | | | | | |
| | 10 | | | | | | | |
| | 20 | | | | | 0,12 | | |
| | 30 | | | | | 0,12 | | |
| | 40 | | | | | 0,12 | 0,13 | |
| | 50 | | | | | 0,11 | 0,12 | 0,13 |
| | 60 | | | | | 0,11 | 0,12 | 0,12 |
| | 70 | | | | | 0,10 | 0,11 | 0,11 |
| | | | | | | | | |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | DOGS | | | | |
| dk8> | 0,04 | | cation | 100/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,2 | 5,6 | 5,4 | 5,0 |
| | % anion | 25 | 35 | 40 | 50 | 60 | 65 | 75 |
| % neutral lipid | 0 | | | | | | | |
| | 10 | | | | | | | |
| | 20 | | | | | | 0,12 | |
| | 30 | | | | | 0,11 | 0,12 | 0,13 |
| | 40 | | | | | 0,11 | 0,11 | 0,12 |
| | 50 | | | | | 0,10 | 0,11 | 0,12 |
| | 60 | | | | | 0,10 | 0,11 | 0,11 |
| | 70 | | | | | 0,10 | 0,10 | 0,11 |

Table 63 provides such analysis for neutral lipids having a κ(neutral)=0,2.

**Table 63:**

| **screening parameter** | | | **system** | | | | | |
|---|---|---|---|---|---|---|---|---|
| | | | | | | | | |
| k(min) < | 0,13 | | k(neutral) | 0.2 | | | | |
| k(min)> | 0,09 | | anion | Chol C1 | | | | |
| dk8> | 0,04 | | cation | 50/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,4 | 6,1 | 5,9 | 5,5 |
| | % anion | 25 | 35 | 40 | 50 | 60 | 65 | 75 |
| % neutral lipid | 0 | | | | | | | |
| | 10 | | | | | | | |
| | 20 | | | | | | 0,09 | 0,10 |
| | 30 | | | | | | 0,10 | 0,11 |
| | 40 | | | | | | 0,12 | 0,13 |
| | 50 | | | | | | | |
| | 60 | | | | | | | |
| | 70 | | | | | | | |
| | | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | CHEMS | | | | |
| dk8> | 0,04 | | cation | 50/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,3 | 5,8 | 5,6 | 5,2 |
| | % anion | 25 | 35 | 40 | 50 | 60 | 65 | 75 |
| % neutral lipid | 0 | | | | | | | |
| | 10 | | | | | 0,13 | | |
| | 20 | | | | | | | |
| | 30 | | | | | | | |
| | 40 | | | | | | | |
| | 50 | | | | | | | |
| | 60 | | | | | | | |
| | 70 | | | | | | | |
| k(min) < | 0,13 | | k(neutral) | 0.2 | | | | |
| k(min)> | 0,09 | | anion | DMGS | | | | |
| dk8> | 0,04 | | cation | 50/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,2 | 5,6 | 5,4 | 5,0 |
| | % anion | 25 | 35 | 40 | 50 | 60 | 65 | 75 |
| % neutral lipid | 0 | | | | | | | |
| | 10 | | | | | 0,12 | | |
| | 20 | | | | | 0,13 | | |
| | 30 | | | | | | | |
| | 40 | | | | | | | |
| | 50 | | | | | | | |
| | 60 | | | | | | | |
| | 70 | | | | | | | |
| | | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | DOGS | | | | |
| dk8> | 0,04 | | cation | 50/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,2 | 5,6 | 5,4 | 5,0 |
| | % anion | 25 | 35 | 40 | 50 | 60 | 65 | 75 |
| % neutral lipid | 0 | | | | | | | 0,13 |
| | 10 | | | | | 0,10 | 0,12 | |
| | 20 | | | | | 0,12 | 0,13 | |
| | 30 | | | | | 0,13 | | |
| | 40 | | | | | | | |
| | 50 | | | | | | | |
| | 60 | | | | | | | |
| | 70 | | | | | | | |
| | | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | Chol C1 | | | | |
| dk8> | 0,04 | | cation | 100/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,4 | 6,1 | 5,9 | 5,5 |
| | % anion | 25 | 35 | 40 | 50 | 60 | 65 | 75 |
| % neutral lipid | 0 | | | | | | | |
| | 10 | | | | | | 0,09 | 0,10 |
| | 20 | | | | | | 0,10 | 0,11 |
| | 30 | | | | | | 0,12 | 0,12 |
| | 40 | | | | | | 0,13 | |
| | 50 | | | | | | | |
| | 60 | | | | | | | |
| | 70 | | | | | | | |
| k(min) < | 0,13 | | k(neutral) | 0.2 | | | | |
| k(min)> | 0,09 | | anion | CHEMS | | | | |
| dk8> | 0,04 | | cation | 100/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,3 | 5,8 | 5,6 | 5,2 |
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | | | |
| | 40 | | | | | | | |
| | 50 | | | | | | | |
| | 60 | | | | | | | |
| | 70 | | | | | | | |
| | | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | DMGS | | | | |
| dk8> | 0,04 | | cation | 100/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,2 | 5,6 | 5,4 | 5,0 |
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | | | |
| | 40 | | | | | | | |
| | 50 | | | | | | | |
| | 60 | | | | | | | |
| | 70 | | | | | | | |
| | | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | DOGS | | | | |
| dk8> | 0,04 | | cation | 100/500 strong | | | | |
| IP> | 5 | | counteranion | PO4 | | | | |
| IP< | 6 | | countercation | Na | | | | |
| | | | | | | | | |
| Isoelectric point (IP) | | 15,3 | 14,9 | 14,7 | 10,2 | 5,6 | 5,4 | 5,0 |
| | % anion | 25 | 35 | 40 | 50 | 60 | 65 | 75 |
| % neutral lipid | 0 | | | | | | | |
| | 10 | | | | | 0,12 | | |
| | 20 | | | | | | | |
| | 30 | | | | | | | |
| | 40 | | | | | | | |
| | 50 | | | | | | | |
| | 60 | | | | | | | |
| | 70 | | | | | | | |

Lowering the pK of the lipid cation towards 7.5 or 8 results in a first improvement of the system amplitude, as less lipid anion is sequestered into the lipid salt at pH8. The following table 64 provides an analysis for such systems wherein CHEMS, DMGS, DOGS or Chol-Cl are used as the anionic lipid species; the lipid cation has a pK of 7.7 and the lipid cation has V_{CH}=50Å³ or 100Å³, respectively and V_{CT}=500Å³, wherein the neutral lipid is cholesterol, κ(min)<0,13 and >0,09; dκ(pH8)>0,04 and the IP between 5 and 6:

**Table 64:**

| **screening parameter** | | | **system** | | | | | |
|---|---|---|---|---|---|---|---|---|
| k(min) < | 0,13 | | k(neutral) | Chol | | | | |
| k(min)> | 0,09 | | anion | Chol C1 | | | | |
| dk8> | 0,04 | | cation | 50/500 pK7.7 | | | | |
| IP> | 5 | | counteranion | PO4 | | | | |
| IP< | 6 | | countercation | Na | | | | |
| | | | | | | | | |
| Isoelectric point (IP) | | 8,0 | 7,6 | 7,4 | 6,7 | 6,1 | 5,8 | 5,5 |
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | | | |
| | 40 | | | | | | | |
| | 50 | | | | | | | |
| | 60 | | | | | | | |
| | 70 | | | | | | | |
| | | | | | | | | |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | CHEMS | | | | |
| dk8> | 0,04 | | cation | 50/500 pK7.7 | | | | |
| IP> | 5 | | counteranion | PO4 | | | | |
| IP< | 6 | | countercation | Na | | | | |
| | | | | | | | | |
| Isoelectric point (IP) | | 8,0 | 7,6 | 7,4 | 6,6 | 5,8 | 5,6 | 5,2 |
| | % anion | 25 | 35 | 40 | 50 | 60 | 65 | 75 |
| % neutral lipid | 0 | | | | | 0,12 | | |
| | 10 | | | | | 0,12 | 0,13 | |
| | 20 | | | | | 0,12 | 0,12 | |
| | 30 | | | | | 0,11 | 0,12 | |
| | 40 | | | | | 0,11 | 0,12 | |
| | 50 | | | | | 0,11 | 0,11 | 0,13 |
| | 60 | | | | | 0,10 | 0,11 | 0,12 |
| | 70 | | | | | 0,10 | 0,10 | 0,11 |
| k(min) < | 0,13 | | k(neutral) | Chol | | | | |
| k(min)> | 0,09 | | anion | DMGS | | | | |
| dk8> | 0,04 | | cation | 50/500 pK7.7 | | | | |
| IP> | 5 | | counteranion | PO4 | | | | |
| IP< | 6 | | countercation | Na | | | | |
| | | | | | | | | |
| Isoelectric point (IP) | | 8,0 | 7,6 | 7,4 | 6,5 | 5,6 | 5,4 | 5,0 |
| | % anion | 25 | 35 | 40 | 50 | 60 | 65 | 75 |
| % neutral lipid | 0 | | | | | 0,11 | | |
| | 10 | | | | | 0,11 | 0,13 | |
| | 20 | | | | | 0,11 | 0,12 | |
| | 30 | | | | | 0,11 | 0,12 | |
| | 40 | | | | | 0,10 | 0,12 | 0,13 |
| | 50 | | | | | 0,10 | 0,11 | 0,12 |
| | 60 | | | | | 0,10 | 0,11 | 0,12 |
| | 70 | | | | | 0,10 | 0,10 | 0,11 |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | DOGS | | | | |
| dk8> | 0,04 | | cation | 50/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,5 | 5,6 | 5,4 | 5,0 |
| | % anion | 25 | 35 | 40 | 50 | 60 | 65 | 75 |
| % neutral lipid | 0 | | | | | 0,09 | 0,11 | 0,13 |
| | 10 | | | | | 0,09 | 0,11 | 0,12 |
| | 20 | | | | | 0,09 | 0,11 | 0,12 |
| | 30 | | | | | 0,09 | 0,11 | 0,12 |
| | 40 | | | | | 0,09 | 0,10 | 0,11 |
| | 50 | | | | | 0,09 | 0,10 | 0,11 |
| | 60 | | | | | 0,09 | 0,10 | 0,11 |
| | 70 | | | | | 0,09 | 0,10 | 0,10 |
| | | | | | | | | |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | Chol C1 | | | | |
| dk8> | 0,04 | | cation | 100/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,7 | 6,1 | 5,8 | 5,5 |
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | | | 0,09 |
| | 40 | | | | | | | 0,09 |
| | 50 | | | | | | | 0,09 |
| | 60 | | | | | | | 0,09 |
| | 70 | | | | | | | 0,09 |
| k(min) < | 0,13 | | k(neutral) | Chol | | | | |
| k(min)> | 0,09 | | anion | CHEMS | | | | |
| dk8> | 0,04 | | cation | 100/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,6 | 5,8 | 5,6 | 5,2 |
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | 0,13 | | |
| | 40 | | | | | 0,12 | 0,13 | |
| | 50 | | | | | 0,12 | 0,12 | |
| | 60 | | | | | 0,11 | 0,11 | 0,13 |
| | 70 | | | | | 0,11 | 0,11 | 0,12 |
| | | | | | | | | |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | DMGS | | | | |
| dk8> | 0,04 | | cation | 100/500 pK7.7 | | | | |
| IP> | 5 | | counteranion | PO4 | | | | |
| IP< | 6 | | countercation | Na | | | | |
| | | | | | | | | |
| Isoelectric point (IP) | | 8,0 | 7,6 | 7,4 | 6,5 | 5,6 | 5,4 | 5,0 |
| | % anion | 25 | 35 | 40 | 50 | 60 | 65 | 75 |
| % neutral lipid | 0 | | | | | | | |
| | 10 | | | | | 0,13 | | |
| | 20 | | | | | 0,12 | | |
| | 30 | | | | | 0,12 | | |
| | 40 | | | | | 0,12 | 0,13 | |
| | 50 | | | | | 0,11 | 0,12 | 0,13 |
| | 60 | | | | | 0,11 | 0,12 | 0,12 |
| | 70 | | | | | 0,10 | 0,11 | 0,11 |
| | | | | | | | | |

| **screening parameter** | | | **system** | | | | | |
|---|---|---|---|---|---|---|---|---|
| k(min) < | 0,13 | | k(neutral) | Chol | | | | |
| k(min)> | 0,09 | | anion | DOGS | | | | |
| dk8> | 0,04 | | cation | 100/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,5 | 5,6 | 5,4 | 5,0 |
| | % anion | 25 | 35 | 40 | 50 | 60 | 65 | 75 |
| % neutral lipid | 0 | | | | | 0,11 | | |
| | 10 | | | | | 0,11 | 0,13 | |
| | 20 | | | | | 0,11 | 0,12 | |
| | 30 | | | | | 0,11 | 0,12 | 0,13 |
| | 40 | | | | | 0,11 | 0,12 | 0,12 |
| | 50 | | | | | 0,10 | 0,11 | 0,12 |
| | 60 | | | | | 0,10 | 0,11 | 0,11 |
| | 70 | | | | | 0,10 | 0,10 | 0,11 |

The following table 65 provides an analysis for the systems described for 64, but with κ(neutral)=0.2.

**Table 65:**

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | Chol C1 | | | | |
| dk8> | 0,04 | | cation | 50/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,7 | 6,1 | 5,8 | 5,5 |
| | % anion | 25 | 35 | 40 | 50 | 60 | 65 | 75 |
| % neutral lipid | 0 | | | | | | | |
| | 10 | | | | | | | |
| | 20 | | | | | | 0,09 | 0,10 |
| | 30 | | | | | | 0,10 | 0,11 |
| | 40 | | | | | | 0,12 | 0,13 |
| | 50 | | | | | | | |
| | 60 | | | | | | | |
| | 70 | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | CHEMS | | | | |
| dk8> | 0,04 | | cation | 50/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,6 | 5,8 | 5,6 | 5,2 |
| | % anion | 25 | 35 | 40 | 50 | 60 | 65 | 75 |
| % neutral lipid | 0 | | | | | 0,12 | | |
| | 10 | | | | | 0,13 | | |
| | 20 | | | | | | | |
| | 30 | | | | | | | |
| | 40 | | | | | | | |
| | 50 | | | | | | | |
| | 60 | | | | | | | |
| | 70 | | | | | | | |
| k(min) < | 0,13 | | k(neutral) | 0.2 | | | | |
| k(min)> | 0,09 | | anion | DMGS | | | | |
| dk8> | 0,04 | | cation | 50/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,5 | 5,6 | 5,4 | 5,0 |
| | % anion | 25 | 35 | 40 | 50 | 60 | 65 | 75 |
| % neutral lipid | 0 | | | | | 0,11 | | |
| | 10 | | | | | 0,12 | | |
| | 20 | | | | | 0,13 | | |
| | 30 | | | | | | | |
| | 40 | | | | | | | |
| | 50 | | | | | | | |
| | 60 | | | | | | | |
| | 70 | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | DOGS | | | | |
| dk8> | 0,04 | | cation | 50/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,5 | 5,6 | 5,4 | 5,0 |
| | % anion | 25 | 35 | 40 | 50 | 60 | 65 | 75 |
| % neutral lipid | 0 | | | | | 0,09 | 0,11 | 0,13 |
| | 10 | | | | | 0,10 | 0,12 | |
| | 20 | | | | | 0,12 | 0,13 | |
| | 30 | | | | | 0,13 | | |
| | 40 | | | | | | | |
| | 50 | | | | | | | |
| | 60 | | | | | | | |
| | 70 | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | Chol C1 | | | | |
| dk8> | 0,04 | | cation | 100/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,7 | 6,1 | 5,8 | 5,5 |
| | % anion | 25 | 35 | 40 | 50 | 60 | 65 | 75 |
| % neutral lipid | 0 | | | | | | | |
| | 10 | | | | | | 0,09 | 0,10 |
| | 20 | | | | | | 0,11 | 0,11 |
| | 30 | | | | | | 0,12 | 0,12 |
| | 40 | | | | | | 0,13 | |
| | 50 | | | | | | | |
| | 60 | | | | | | | |
| | 70 | | | | | | | |
| k(min]< | 0,13 | | k(neutral) | 0.2 | | | | |
| k(min)> | 0,09 | | anion | CHEMS | | | | |
| dk8> | 0,04 | | cation | 100/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,6 | 5,8 | 5,6 | 5,2 |
| | % anion | 25 | 35 | 40 | 50 | 60 | 65 | 75 |
| | 0 | | | | | | | |
| | 10 | | | | | | | |
| | 20 | | | | | | | |
| % neutral | 30 | | | | | | | |
| lipid | 40 | | | | | | | |
| | 50 | | | | | | | |
| | 60 | | | | | | | |
| | 70 | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | DMGS | | | | |
| dk8> | 0,04 | | cation | 100/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,5 | 5,6 | 5,4 | 5,0 |
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | | | |
| | 40 | | | | | | | |
| | 50 | | | | | | | |
| | 60 | | | | | | | |
| | 70 | | | | | | | |

| **screening parameter** | | | **system** | | | | | |
|---|---|---|---|---|---|---|---|---|
| k(min) < | 0,13 | | k(neutral) | 0.2 | | | | |
| k(min)> | 0,09 | | anion | DOGS | | | | |
| dk8> | 0,04 | | cation | 100/500 pK7.7 | | | | |
| IP> | 5 | | counteranion | PO4 | | | | |
| IP< | 6 | | countercation | Na | | | | |
| Isoelectric point (IP) | | 8,0 | 7,6 | 7,4 | 6,5 | 5,6 | 5,4 | 5,0 |
| | % anion | 25 | 35 | 40 | 50 | 60 | 65 | 75 |
| % neutral lipid | 0 | | | | | 0,11 | | |
| | 10 | | | | | 0,12 | | |
| | 20 | | | | | | | |
| | 30 | | | | | | | |
| | 40 | | | | | | | |
| | 50 | | | | | | | |
| | 60 | | | | | | | |
| | 70 | | | | | | | |

A further lowering the pK of the lipid cation towards 7 releases the selection pressure from dκ(pH8) as no substantial lipid salt formation occurs at neutral pH anymore. The following table 66 provides an analysis for such amphoter II systems wherein CHEMS, DMGS, DOGS or Chol-Cl are used as the anionic lipid species; the lipid cation has a pK of 7.0 and the lipid cation has V_{CH}=50Å³ or 100Å³, respectively and V_{CT}=500Å³, wherein the neutral lipid is cholesterol, κ(min)<0,18 and >0,09; dκ(pH8)>0,08 and the IP between 5 and 6:

**Table 66:**

| **screening parameter** | | | **system** | | | | | |
|---|---|---|---|---|---|---|---|---|
| k(min) < | 0,18 | | k(neutral) | Chol | | | | |
| k(min)> | 0,09 | | anion | Chol C1 | | | | |
| dk8> | 0,08 | | cation | 50/500 pK7 | | | | |
| IP> | 5 | | counteranion | PO4 | | | | |
| IP< | 6 | | countercation | Na | | | | |
| Isoelectric point (IP) | | 7,3 | 7,0 | 6,8 | 6,4 | 6,0 | 5,8 | 5,5 |
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | | | |
| | 40 | | | | | | | |
| | 50 | | | | | | | |
| | 60 | | | | | | | |
| | 70 | | | | | | | |

| **screening parameter** | | | **system** | | | | | |
|---|---|---|---|---|---|---|---|---|
| k(min) < | 0,18 | | k(neutral) | Chol | | | | |
| k(min)> | 0,09 | | anion | CHEMS | | | | |
| dk8> | 0,08 | | cation | 50/500 pK7 | | | | |
| IP> | 5 | | counteranion | PO4 | | | | |
| IP< | 6 | | countercation | Na | | | | |
| Isoelectric point (IP) | | 7,3 | 7,0 | 6,8 | 6,3 | 5,7 | 5,5 | 5,2 |
| | % anion | 25 | 35 | 40 | 50 | 60 | 65 | 75 |
| % neutral lipid | 0 | | | | | 0,13 | 0,14 | 0,17 |
| | 10 | | | | | 0,13 | 0,13 | 0,16 |
| | 20 | | | | | 0,12 | 0,13 | 0,15 |
| | 30 | | | | | 0,12 | 0,12 | 0,15 |
| | 40 | | | | | 0,11 | 0,12 | 0,14 |
| | 50 | | | | | 0,11 | 0,11 | 0,13 |
| | 60 | | | | | 0,11 | 0,11 | 0,12 |
| | 70 | | | | | 0,10 | 0,11 | 0,11 |
| k(min) < | 0,18 | | k(neutral) | Chol | | | | |
| k(min)> | 0,09 | | anion | DMGS | | | | |
| dk8> | 0,08 | | cation | 50/500 pK7 | | | | |
| IP> | 5 | | counteranion | PO4 | | | | |
| IP< | 6 | | countercation | Na | | | | |
| Isoelectric point (IP) | | 7,3 | 7,0 | 6,7 | 6,2 | 5,6 | 5,4 | 5,0 |
| | % anion | 25 | 35 | 40 | 50 | 60 | 65 | 75 |
| % neutral lipid | 0 | | | | | 0,11 | 0,13 | 0,15 |
| | 10 | | | | | 0,11 | 0,13 | 0,15 |
| | 20 | | | | | 0,11 | 0,12 | 0,14 |
| | 30 | | | | | 0,11 | 0,12 | 0,13 |
| | 40 | | | | | 0,10 | 0,12 | 0,13 |
| | 50 | | | | | 0,10 | 0,11 | 0,12 |
| | 60 | | | | | 0,10 | 0,11 | 0,12 |
| | 70 | | | | | 0,10 | 0,10 | 0,11 |

| **screening parameter** | | | **system** | | | | | |
|---|---|---|---|---|---|---|---|---|
| k(min) < | 0,18 | | k(neutral) | Chol | | | | |
| k(min)> | 0,09 | | anion | DOGS | | | | |
| dk8> | 0,08 | | cation | 50/500 pK7 | | | | |
| IP> | 5 | | counteranlon | PO4 | | | | |
| IP< | 6 | | countercation | Na | | | | |
| Isoelectric point (IP) | | 7,3 | 7,0 | 6,7 | 6,2 | 5,6 | 5,4 | 5,0 |
| | % anion | 25 | 35 | 40 | 50 | 60 | 65 | 75 |
| % neutral lipid | 0 | | | | | 0,09 | 0,11 | 0,13 |
| | 10 | | | | | 0,09 | 0,11 | 0,12 |
| | 20 | | | | | 0,09 | 0,11 | 0,12 |
| | 30 | | | | | 0,09 | 0,11 | 0,12 |
| | 40 | | | | | 0,09 | 0,10 | 0,11 |
| | 50 | | | | | 0,09 | 0,10 | 0,11 |
| | 60 | | | | | 0,09 | 0,10 | 0,11 |
| | 70 | | | | | 0,09 | 0,10 | 0,10 |

| **screening parameter** | | | **system** | | | | | |
|---|---|---|---|---|---|---|---|---|
| k(min) < | 0,18 | | k(neutral) | Chol | | | | |
| k(min)> | 0,09 | | anion | Chol C1 | | | | |
| dk8> | 0,08 | | cation | 100/500 pK7 | | | | |
| IP> | 5 | | counteranion | PO4 | | | | |
| IP< | 6 | | countercation | Na | | | | |
| Isoelectric point (IP) | | 7,3 | 7,0 | 6,8 | 6,4 | 6,0 | 5,8 | 5,5 |
| | % anion | 25 | 35 | 40 | 50 | 60 | 65 | 75 |
| % neutral lipid | 0 | | | | | | | 0,09 |
| | 10 | | | | | | | 0,09 |
| | 20 | | | | | | | 0,09 |
| | 30 | | | | | | | 0,09 |
| | 40 | | | | | | | 0,09 |
| | 50 | | | | | | 0,09 | 0,09 |
| | 60 | | | | | 0,09 | 0,09 | 0,09 |
| | 70 | | | | | 0,09 | 0,09 | 0,09 |
| k(min) < | 0,18 | | k(neutral) | Chol | | | | |
| k(min)> | 0,09 | | anion | CHEMS | | | | |
| dk8> | 0,08 | | cation | 100/500 pK7 | | | | |
| IP> | 5 | | counteranion | PO4 | | | | |
| IP< | 6 | | countercation | Na | | | | |
| Isoelectric point (IP) | | 7,3 | 7,0 | 6,8 | 6,3 | 5,7 | 5,5 | 5,2 |
| | % anion | 25 | 35 | 40 | 50 | 60 | 65 | 75 |
| % neutral lipid | 0 | | | | | 0,15 | 0,15 | |
| | 10 | | | | | 0,15 | 0,15 | 0,17 |
| | 20 | | | | | 0,14 | 0,14 | 0,16 |
| | 30 | | | | | 0,13 | 0,14 | 0,15 |
| | 40 | | | | | 0,13 | 0,13 | 0,15 |
| | 50 | | | | | 0,12 | 0,12 | 0,14 |
| | 60 | | | | | 0,12 | 0,12 | 0,13 |
| | 70 | | | | | 0,11 | 0,11 | 0,12 |

| **screening parameter** | | | **system** | | | | | |
|---|---|---|---|---|---|---|---|---|
| k(min) < | 0,18 | | k(neutral) | Chol | | | | |
| k(min)> | 0,09 | | anion | DMGS | | | | |
| dk8> | 0,08 | | cation | 100/500 pK7 | | | | |
| IP> | 5 | | counteranion | PO4 | | | | |
| IP< | 6 | | countercation | Na | | | | |
| Isoelectric point (IP) | | 7,3 | 7,0 | 6,7 | 6,2 | 5,6 | 5,4 | 5,0 |
| | % anion | 25 | 35 | 40 | 50 | 60 | 65 | 75 |
| % neutral lipid | 0 | | | | | 0,13 | 0,15 | 0,17 |
| | 10 | | | | | 0,13 | 0,14 | 0,16 |
| | 20 | | | | | 0,12 | 0,14 | 0,15 |
| | 30 | | | | | 0,12 | 0,13 | 0,14 |
| | 40 | | | | | 0,12 | 0,13 | 0,14 |
| | 50 | | | | | 0,11 | 0,12 | 0,13 |
| | 60 | | | | | 0,11 | 0,12 | 0,12 |
| | 70 | | | | | 0,10 | 0,11 | 0,11 |

| **screening parameter** | | | **system** | | | | | |
|---|---|---|---|---|---|---|---|---|
| k(min) < | 0,18 | | k(neutral) | Chol | | | | |
| k(min)> | 0,09 | | anion | DOGS | | | | |
| dk8> | 0,08 | | cation | 100/500 pK7 | | | | |
| IP> | 5 | | counteranion | PO4 | | | | |
| IP< | 6 | | countercation | Na | | | | |
| Isoelectric point (IP) | | 7,3 | 7,0 | 6,7 | 6,2 | 5,6 | 5,4 | 5,0 |
| | % anion | 25 | 35 | 40 | 50 | 60 | 65 | 75 |
| % neutral lipid | 0 | | | | | 0,12 | 0,13 | 0,14 |
| | 10 | | | | | 0,11 | 0,13 | 0,14 |
| | 20 | | | | | 0,11 | 0,12 | 0,13 |
| | 30 | | | | | 0,11 | 0,12 | 0,13 |
| | 40 | | | | | 0,11 | 0,11 | 0,12 |
| | 50 | | | | | 0,10 | 0,11 | 0,12 |
| | 60 | | | | | 0,10 | 0,11 | 0,11 |
| | 70 | | | | | 0,10 | 0,10 | 0,11 |

Substitution of the neutral lipid used in table 66 towards a species with larger κ(neutral) of 0.2 results in the following picture of table 67:

**Table 67:**

| **screening parameter** | | | **system** | | | | | |
|---|---|---|---|---|---|---|---|---|
| k(min) < | 0,18 | | k(neutral) | 0.2 | | | | |
| k(min)> | 0,09 | | anion | Chol C1 | | | | |
| dk8> | 0,08 | | cation | 50/500 pK7 | | | | |
| IP> | 5 | | counteranlon | PO4 | | | | |
| IP< | 6 | | countercation | Na | | | | |
| Isoelectric point (IP) | | 7,3 | 7,0 | 6,8 | 6,4 | 6,0 | 5,8 | 5,5 |
| | % anion | 25 | 35 | 40 | 50 | 60 | 65 | 75 |
| % neutral lipid | 0 | | | | | | | |
| | 10 | | | | | | | 0,09 |
| | 20 | | | | | 0,09 | 0,10 | 0,10 |
| | 30 | | | | | 0,11 | 0,11 | 0,12 |
| | 40 | | | | | 0,12 | 0,12 | 0,13 |
| | 50 | | | | | 0,13 | 0,13 | 0,14 |
| | 60 | | | | | 0,15 | 0,15 | 0,15 |
| | 70 | | | | | 0,16 | 0,16 | 0,16 |

| **screening parameter** | | | **system** | | | | | |
|---|---|---|---|---|---|---|---|---|
| k(min) < | 0,18 | | k(neutral) | 0.2 | | | | |
| k(min)> | 0,09 | | anion | CHEMS | | | | |
| dk8> | 0,08 | | cation | 50/500 pK7 | | | | |
| IP> | 5 | | counteranion | PO4 | | | | |
| IP< | 6 | | countercation | Na | | | | |
| Isoelectric point (IP) | | 7,3 | 7,0 | 6,8 | 6,3 | 5,7 | 5,5 | 5,2 |
| | % anion | 25 | 35 | 40 | 50 | 60 | 65 | 75 |
| % neutral lipid | 0 | | | | | 0,13 | 0,14 | 0,17 |
| | 10 | | | | | 0,14 | 0,14 | 0,17 |
| | 20 | | | | | 0,14 | 0,15 | 0,17 |
| | 30 | | | | | 0,15 | 0,16 | 0,18 |
| | 40 | | | | | 0,16 | 0,16 | |
| | 50 | | | | | 0,16 | 0,17 | |
| | 60 | | | | | 0,17 | 0,17 | |
| | 70 | | | | | 0,18 | | |
| k(min) < | 0,18 | | k(neutral) | 0.2 | | | | |
| k(min)> | 0,09 | | anion | DMGS | | | | |
| dk8> | 0,08 | | cation | 50/500 pK7 | | | | |
| IP> | 5 | | counteranion | PO4 | | | | |
| IP< | 6 | | countercation | Na | | | | |
| Isoelectric point (IP) | | 7,3 | 7,0 | 6,7 | 6,2 | 5,6 | 5,4 | 5,0 |
| | % anion | 25 | 35 | 40 | 50 | 60 | 65 | 75 |
| % neutral lipid | 0 | | | | | 0,11 | 0,13 | 0,15 |
| | 10 | | | | | 0,12 | 0,14 | 0,16 |
| | 20 | | | | | 0,13 | 0,15 | 0,16 |
| | 30 | | | | | 0,14 | 0,15 | 0,17 |
| | 40 | | | | | 0,15 | 0,16 | 0,17 |
| | 50 | | | | | 0,16 | 0,17 | 0,18 |
| | 60 | | | | | 0,16 | 0,17 | |
| | 70 | | | | | 0,17 | 0,18 | |

| **screening para meter** | | | **system** | | | | | |
|---|---|---|---|---|---|---|---|---|
| k(min) < | 0,18 | | k(neutral) | 0.2 | | | | |
| k(min)> | 0,09 | | anion | DOGS | | | | |
| dk8> | 0,08 | | cation | 50/500 pK7 | | | | |
| IP> | 5 | | counteranion | PO4 | | | | |
| IP< | 6 | | countercation | Na | | | | |
| Isoelectric point (IP) | | 7,3 | 7,0 | 6,7 | 6,2 | 5,6 | 5,4 | 5,0 |
| | % anion | 25 | 35 | 40 | 50 | 60 | 65 | 75 |
| % neutral lipid | 0 | | | | | 0,09 | 0,11 | 0,13 |
| | 10 | | | | | 0,11 | 0,12 | 0,13 |
| | 20 | | | | | 0,12 | 0,13 | 0,14 |
| | 30 | | | | | 0,13 | 0,14 | 0,15 |
| | 40 | | | | | 0,14 | 0,15 | 0,16 |
| | 50 | | | | | 0,15 | 0,16 | 0,16 |
| | 60 | | | | | 0,16 | 0,16 | 0,17 |
| | 70 | | | | | 0,17 | 0,17 | 0,18 |

| **screening parameter** | | | **system** | | | | | |
|---|---|---|---|---|---|---|---|---|
| k(min) < | 0,18 | | k(neutral) | 0.2 | | | | |
| k(min)> | 0,09 | | anion | Chol C1 | | | | |
| dk8> | 0,08 | | cation | 100/500 pK7 | | | | |
| IP> | 5 | | counteranlon | PO4 | | | | |
| IP< | 6 | | countercation | Na | | | | |
| Isoelectric point (IP) | | 7,3 | 7,0 | 6,8 | 6,4 | 6,0 | 5,8 | 5,5 |
| | % anion | 25 | 35 | 40 | 50 | 60 | 65 | 75 |
| % neutral lipid | 0 | | | | | | | 0,09 |
| | 10 | | | | | 0,10 | 0,10 | 0,10 |
| | 20 | | | | | 0,11 | 0,11 | 0,11 |
| | 30 | | | | | 0,12 | 0,12 | 0,12 |
| | 40 | | | | | 0,13 | 0,13 | 0,14 |
| | 50 | | | | | 0,14 | 0,14 | 0,15 |
| | 60 | | | | | 0,15 | 0,16 | 0,16 |
| | 70 | | | | | 0,17 | 0,17 | 0,17 |
| k(min) < | 0,18 | | k(neutral) | 0.2 | | | | |
| k(min)> | 0,09 | | anion | CHEMS | | | | |
| dk8> | 0,08 | | cation | 100/500 pK7 | | | | |
| IP> | 5 | | counteranion | PO4 | | | | |
| IP< | 6 | | countercation | Na | | | | |
| Isoelectric point (IP) | | 7,3 | 7,0 | 6,8 | 6,3 | 5,7 | 5,5 | 5,2 |
| | % anion | 25 | 35 | 40 | 50 | 60 | 65 | 75 |
| % neutral lipid | 0 | | | | | 0,15 | 0,15 | |
| | 10 | | | | | 0,16 | 0,16 | |
| | 20 | | | | | 0,16 | 0,16 | |
| | 30 | | | | | 0,17 | 0,17 | |
| | 40 | | | | | 0,17 | 0,17 | |
| | 50 | | | | | 0,18 | 0,18 | |
| | 60 | | | | | | | |
| | 70 | | | | | | | |

| **screening parameter** | | | **system** | | | | | |
|---|---|---|---|---|---|---|---|---|
| k(min) < | 0,18 | | k(neutral) | 0.2 | | | | |
| k(min)> | 0,09 | | anion | DMGS | | | | |
| dk8> | 0,08 | | cation | 100/500 pK7 | | | | |
| IP> | 5 | | counteranion | PO4 | | | | |
| IP< | 6 | | countercation | Na | | | | |
| Isoelectric point (IP) | | 7,3 | 7,0 | 6,7 | 6,2 | 5,6 | 5,4 | 5,0 |
| | % anion | 25 | 35 | 40 | 50 | 60 | 65 | 75 |
| % neutral lipid | 0 | | | | | 0,13 | 0,15 | 0,17 |
| | 10 | | | | | 0,14 | 0,16 | 0,17 |
| | 20 | | | | | 0,15 | 0,16 | 0,17 |
| | 30 | | | | | 0,15 | 0,17 | 0,18 |
| | 40 | | | | | 0,16 | 0,17 | 0,18 |
| | 50 | | | | | 0,17 | 0,18 | |
| | 60 | | | | | 0,17 | | |
| | 70 | | | | | 0,18 | | |

| **screening parameter** | | | **system** | | | | | |
|---|---|---|---|---|---|---|---|---|
| k(min) < | 0,18 | | k(neutral) | 0.2 | | | | |
| k(min)> | 0,09 | | anion | DOGS | | | | |
| dk8> | 0,08 | | cation | 100/500 pK7 | | | | |
| IP> | 5 | | counteranion | PO4 | | | | |
| IP< | 6 | | countercation | Na | | | | |
| Isoelectric point (IP) | | 7,3 | 7,0 | 6,7 | 6,2 | 5,6 | 5,4 | 5,0 |
| | % anion | 25 | 35 | 40 | 50 | 60 | 65 | 75 |
| % neutral lipid | 0 | | | | | 0,12 | 0,13 | 0,14 |
| | 10 | | | | | 0,12 | 0,14 | 0,15 |
| | 20 | | | | | 0,13 | 0,14 | 0,15 |
| | 30 | | | | | 0,14 | 0,15 | 0,16 |
| | 40 | | | | | 0,15 | 0,16 | 0,16 |
| | 50 | | | | | 0,16 | 0,17 | 0,17 |
| | 60 | | | | | 0,17 | 0,17 | 0,18 |
| | 70 | | | | | 0,17 | 0,18 | |

As discussed before, a further lowering the pK of the lipid cation towards 6.3 creates a limitation for the ability of the lipid anion and lipid cation to maximize the lipid salt formation at the isoelectric point of the mixture, said limitation raises κ(min) and reduces dκ(pH8) at the same time. The following table 68 provides an analysis for such amphoter II systems wherein CHEMS, DMGS, DOGS or Chol-Cl are used as the anionic lipid species; the lipid cation has a pK of 6.3 and the lipid cation has V_{CH}=50Å³ or 100Å³, respectively and V_{CT}=500Å³, wherein the neutral lipid is cholesterol, κ(min)<0,18 and >0,09; dκ(pH8)>0,08 and the IP between 5 and 6:

**Table 68:**

| screening parameter | | | system | | | | | |
|---|---|---|---|---|---|---|---|---|
| k(min) < | 0,18 | | k(neutral) | Chol | | | | |
| k(min)> | 0,09 | | anion | Chol C1 | | | | |
| dk8> | 0,08 | | cation | 50/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,7 | 6,4 | 6,3 | 6,0 | 5,8 | 5,7 | 5,4 |
|---|---|---|---|---|---|---|---|---|
| | % anion | 25 | 35 | 40 | 50 | 60 | 65 | 75 |
| % neutral lipid | 0 | | | | | | | |
| | 10 | | | | | | | |
| | 20 | | | | | | | |
| | 30 | | | | | | | |
| | 40 | | | | | | | |
| | 50 | | | | | | | |
| | 60 | | | | | | | |
| | 70 | | | | | | | |
| | | | | | | | | |

| screening parameter | | | system | | | | | |
|---|---|---|---|---|---|---|---|---|
| k(min) < | 0,18 | | k(neutral) | Chol | | | | |
| k(min)> | 0,09 | | anion | CHEMS | | | | |
| dk8> | 0,08 | | cation | 50/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,9 | 5,6 | 5,5 | 5,2 |
|---|---|---|---|---|---|---|---|---|
| | % anion | 25 | 35 | 40 | 50 | 60 | 65 | 75 |
| % neutral lipid | 0 | | | | 0,11 | 0,12 | 0,15 | 0,17 |
| | 10 | | | | 0,11 | 0,12 | 0,15 | 0,17 |
| | 20 | | | | 0,10 | 0,12 | 0,14 | 0,16 |
| | 30 | | | | 0,10 | 0,12 | 0,13 | 0,15 |
| | 40 | | | | 0,10 | 0,11 | 0,13 | 0,14 |
| | 50 | | | | 0,10 | 0,11 | 0,12 | 0,13 |
| | 60 | | | | 0,10 | 0,11 | 0,12 | 0,13 |
| | 70 | | | | 0,10 | 0,10 | 0,11 | 0,12 |

| screening parameter | | | system | | | | | |
|---|---|---|---|---|---|---|---|---|
| k(min) < | 0,18 | | k(neutral) | Chol | | | | |
| k(min)> | 0,09 | | anion | DMGS | | | | |
| dk8> | 0,08 | | cation | 50/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,8 | 5,5 | 5,3 | 5,0 |
|---|---|---|---|---|---|---|---|---|
| | % anion | 25 | 35 | 40 | 50 | 60 | 65 | 75 |
| % neutral lipid | 0 | | | | 0,11 | 0,13 | 0,13 | |
| | 10 | | | | 0,11 | 0,12 | 0,13 | |
| | 20 | | | | 0,11 | 0,12 | 0,12 | |
| | 30 | | | | 0,10 | 0,12 | 0,12 | |
| | 40 | | | | 0,10 | 0,11 | 0,11 | |
| | 50 | | | | 0,10 | 0,11 | 0,11 | |
| | 60 | | | | 0,10 | 0,11 | 0,11 | |
| | 70 | | | | 0,10 | 0,10 | 0,10 | |
| | | | | | | | | |

| screening parameter | | | system | | | | | |
|---|---|---|---|---|---|---|---|---|
| k(min) < | 0,18 | | k(neutral) | Chol | | | | |
| k(min)> | 0,09 | | anion | DOGS | | | | |
| dk8> | 0,08 | | cation | 50/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,8 | 5,5 | 5,3 | 5,0 |
|---|---|---|---|---|---|---|---|---|
| | % anion | 25 | 35 | 40 | 50 | 60 | 65 | 75 |
| % neutral lipid | 0 | | | | 0,09 | 0,11 | 0,11 | |
| | 10 | | | | 0,09 | 0,11 | 0,11 | |
| | 20 | | | | 0,09 | 0,11 | 0,11 | |
| | 30 | | | | 0,09 | 0,10 | 0,10 | |
| | 40 | | | | 0,09 | 0,10 | 0,10 | |
| | 50 | | | | 0,09 | 0,10 | 0,10 | |
| | 60 | | | | 0,09 | 0,10 | 0,10 | |
| | 70 | | | | 0,09 | 0,10 | 0,10 | |
| | | | | | | | | |

| screening parameter | | | system | | | | | |
|---|---|---|---|---|---|---|---|---|
| k(min) < | 0,18 | | k(neutral) | Chol | | | | |
| k(min)> | 0,09 | | anion | Chol C1 | | | | |
| dk8> | 0,08 | | cation | 100/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,7 | 6,4 | 6,3 | 6,0 | 5,8 | 5,7 | 5,4 |
|---|---|---|---|---|---|---|---|---|
| | % anion | 25 | 35 | 40 | 50 | 60 | 65 | 75 |
| % neutral lipid | 0 | | | | | 0,10 | 0,09 | 0,09 |
| | 10 | | | | | 0,10 | 0,09 | 0,09 |
| | 20 | | | | | 0,10 | 0,09 | 0,09 |
| | 30 | | | | | 0,10 | 0,09 | 0,09 |
| | 40 | | | | | 0,10 | 0,09 | 0,09 |
| | 50 | | | | | 0,10 | 0,09 | 0,09 |
| | 60 | | | | | 0,10 | 0,09 | 0,09 |
| | 70 | | | | | 0,09 | 0,09 | 0,09 |

| screening parameter | | | system | | | | | |
|---|---|---|---|---|---|---|---|---|
| k(min) < | 0,18 | | k(neutral) | Chol | | | | |
| k(min)> | 0,09 | | anion | CHEMS | | | | |
| dk8> | 0,08 | | cation | 100/500 pK6.3 | | | | |
| IP> | 5 | | counteranlon | PO4 | | | | |
| IP< | 6 | | countercation | Na | | | | |
| | | | | | | | | |

| Isoelectric point (IP) | | 6,6 | 6,3 | 6,2 | 5,9 | 5,6 | 5,5 | 5,2 |
|---|---|---|---|---|---|---|---|---|
| | % anion | 25 | 35 | 40 | 50 | 60 | 65 | 75 |
| % neutral lipid | 0 | | | | 0,14 | 0,15 | 0,17 | |
| | 10 | | | | 0,13 | 0,14 | 0,16 | 0,18 |
| | 20 | | | | 0,13 | 0,14 | 0,16 | 0,17 |
| | 30 | | | | 0,13 | 0,13 | 0,15 | 0,16 |
| | 40 | | | | 0,12 | 0,13 | 0,14 | 0,15 |
| | 50 | | | | 0,12 | 0,12 | 0,13 | 0,14 |
| | 60 | | | | 0,11 | 0,11 | 0,12 | 0,13 |
| | 70 | | | | 0,11 | 0,11 | 0,12 | 0,12 |
| | | | | | | | | |

| screening parameter | | | system | | | | | |
|---|---|---|---|---|---|---|---|---|
| k(min) < | 0,18 | | k(neutral) | Chol | | | | |
| k(min)> | 0,09 | | anion | DMGS | | | | |
| dk8> | 0,08 | | cation | 100/500 pK6.3 | | | | |
| IP> | 5 | | counteranlon | PO4 | | | | |
| IP< | 6 | | countercation | Na | | | | |
| | | | | | | | | |

| Isoelectric point (IP) | | 6,6 | 6,3 | 6,2 | 5,8 | 5,5 | 5,3 | 5,0 |
|---|---|---|---|---|---|---|---|---|
| | % anion | 25 | 35 | 40 | 50 | 60 | 65 | 75 |
| % neutral lipid | 0 | | | | 0,14 | 0,15 | 0,15 | |
| | 10 | | | | 0,14 | 0,15 | 0,14 | |
| | 20 | | | | 0,13 | 0,14 | 0,14 | |
| | 30 | | | | 0,13 | 0,13 | 0,13 | |
| | 40 | | | | 0,12 | 0,13 | 0,13 | |
| | 50 | | | | 0,12 | 0,12 | 0,12 | |
| | 60 | | | | 0,11 | 0,12 | 0,11 | |
| | 70 | | | | 0,11 | 0,11 | 0,11 | |
| | | | | | | | | |

| screening parameter | | | system | | | | | |
|---|---|---|---|---|---|---|---|---|
| k(min) < | 0,18 | | k(neutral) | Chol | | | | |
| k(min)> | 0,09 | | anion | DOGS | | | | |
| dk8> | 0,08 | | cation | 100/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,8 | 5,5 | 5,3 | 5,0 |
|---|---|---|---|---|---|---|---|---|
| | % anion | 25 | 35 | 40 | 50 | 60 | 65 | 75 |
| % neutral lipid | 0 | | | | 0,13 | 0,13 | 0,13 | |
| | 10 | | | | 0,12 | 0,13 | 0,12 | |
| | 20 | | | | 0,12 | 0,12 | 0,12 | |
| | 30 | | | | 0,12 | 0,12 | 0,12 | |
| | 40 | | | | 0,11 | 0,12 | 0,11 | |
| | 50 | | | | 0,11 | 0,11 | 0,11 | |
| | 60 | | | | 0,11 | 0,11 | 0,11 | |
| | 70 | | | | 0,10 | 0,10 | 0,10 | |

Eventually, the lipid systems described in table 68 are also analyzed in presence of a neutral lipid system having a κ(neutral) of 0.2, results are provided in table 69 below:

**Table 69:**

| screening parameter | | | system | | | | | |
|---|---|---|---|---|---|---|---|---|
| k(min)< | 0,18 | | k(neutral) | 0.2 | | | | |
| k(min)> | 0,09 | | anion | Chol C1 | | | | |
| dk8> | 0,08 | | cation | 50/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,7 | 6,4 | 6,3 | 6,0 | 5,8 | 5,7 | 5,4 |
|---|---|---|---|---|---|---|---|---|
| | % anion | 25 | 35 | 40 | 50 | 60 | 65 | 75 |
| % neutral lipid | 0 | | | | | | | |
| | 10 | | | | | | | 0,09 |
| | 20 | | | | | 0,10 | 0,10 | 0,10 |
| | 30 | | | | | 0,11 | 0,11 | 0,11 |
| | 40 | | | | | 0,13 | 0,12 | 0,13 |
| | 50 | | | | | 0,14 | 0,14 | 0,14 |
| | 60 | | | | | 0,15 | 0,15 | 0,15 |
| | 70 | | | | | 0,16 | 0,16 | 0,16 |
| | | | | | | | | |

| screening parameter | | | system | | | | | |
|---|---|---|---|---|---|---|---|---|
| k(min) < | 0,18 | | k(neutral) | 0.2 | | | | |
| k(min)> | 0,09 | | anion | CHEMS | | | | |
| dk8> | 0,08 | | cation | 50/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,9 | 5,6 | 5,5 | 5,2 |
|---|---|---|---|---|---|---|---|---|
| | % anion | 25 | 35 | 40 | 50 | 60 | 65 | 75 |
| % neutral lipid | 0 | | | | 0,11 | 0,12 | 0,15 | 0,17 |
| | 10 | | | | 0,12 | 0,13 | 0,16 | 0,18 |
| | 20 | | | | 0,13 | 0,14 | 0,16 | 0,18 |
| | 30 | | | | 0,14 | 0,15 | 0,17 | |
| | 40 | | | | 0,14 | 0,15 | 0,17 | |
| | 50 | | | | 0,15 | 0,16 | 0,18 | |
| | 60 | | | | 0,16 | 0,17 | | |
| | 70 | | | | 0,17 | 0,18 | | |

| screening parameter | | | system | | | | | |
|---|---|---|---|---|---|---|---|---|
| k(min) < | 0,18 | | k(neutral) | 0.2 | | | | |
| k(min)> | 0,09 | | anion | DMGS | | | | |
| dk8> | 0,08 | | cation | 50/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,8 | 5,5 | 5,3 | 5,0 |
|---|---|---|---|---|---|---|---|---|
| | % anion | 25 | 35 | 40 | 50 | 60 | 65 | 75 |
| % neutral lipid | 0 | | | | 0,11 | 0,13 | 0,13 | |
| | 10 | | | | 0,12 | 0,14 | 0,14 | |
| | 20 | | | | 0,13 | 0,14 | 0,14 | |
| | 30 | | | | 0,14 | 0,15 | 0,15 | |
| | 40 | | | | 0,15 | 0,16 | 0,16 | |
| | 50 | | | | 0,15 | 0,16 | 0,16 | |
| | 60 | | | | 0,16 | 0,17 | 0,17 | |
| | 70 | | | | 0,17 | 0,18 | 0,18 | |
| | | | | | | | | |

| screening parameter | | | system | | | | | |
|---|---|---|---|---|---|---|---|---|
| k(min) < | 0,18 | | k(neutral) | 0.2 | | | | |
| k(min)> | 0,09 | | anion | DOGS | | | | |
| dk8> | 0,08 | | cation | 50/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,8 | 5,5 | 5,3 | 5,0 |
|---|---|---|---|---|---|---|---|---|
| | % anion | 25 | 35 | 40 | 50 | 60 | 65 | 75 |
| % neutral lipid | 0 | | | | 0,09 | 0,11 | 0,11 | |
| | 10 | | | | 0,11 | 0,12 | 0,12 | |
| | 20 | | | | 0,12 | 0,13 | 0,13 | |
| | 30 | | | | 0,13 | 0,14 | 0,14 | |
| | 40 | | | | 0,14 | 0,15 | 0,15 | |
| | 50 | | | | 0,15 | 0,15 | 0,15 | |
| | 60 | | | | 0,16 | 0,16 | 0,16 | |
| | 70 | | | | 0,17 | 0,17 | 0,17 | |
| | | | | | | | | |

| screening parameter | | | system | | | | | |
|---|---|---|---|---|---|---|---|---|
| k(min) < | 0,18 | | k(neutral) | 0.2 | | | | |
| k(min)> | 0,09 | | anion | Chol C1 | | | | |
| dk8> | 0,08 | | cation | 100/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,7 | 6,4 | 6,3 | 6,0 | 5,8 | 5,7 | 5,4 |
|---|---|---|---|---|---|---|---|---|
| | % anion | 25 | 35 | 40 | 50 | 60 | 65 | 75 |
| % neutral lipid | 0 | | | | | 0,10 | 0,09 | 0,09 |
| | 10 | | | | | 0,11 | 0,10 | 0,10 |
| | 20 | | | | | 0,12 | 0,12 | 0,12 |
| | 30 | | | | | 0,13 | 0,13 | 0,13 |
| | 40 | | | | | 0,14 | 0,14 | 0,14 |
| | 50 | | | | | 0,15 | 0,15 | 0,15 |
| | 60 | | | | | 0,16 | 0,16 | 0,16 |
| | 70 | | | | | 0,17 | 0,17 | 0,17 |

| screening parameter | | | system | | | | | |
|---|---|---|---|---|---|---|---|---|
| k(min) < | 0,18 | | k(neutral) | 0.2 | | | | |
| k(min)> | 0,09 | | anion | CHEMS | | | | |
| dk8> | 0,08 | | cation | 100/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,9 | 5,6 | 5,5 | 5,2 |
|---|---|---|---|---|---|---|---|---|
| | % anion | 25 | 35 | 40 | 50 | 60 | 65 | 75 |
| % neutral lipid | 0 | | | | 0,14 | 0,15 | 0,17 | |
| | 10 | | | | 0,15 | 0,15 | 0,18 | |
| | 20 | | | | 0,15 | 0,16 | 0,18 | |
| | 30 | | | | 0,16 | 0,16 | | |
| | 40 | | | | 0,16 | 0,17 | | |
| | 50 | | | | 0,17 | 0,17 | | |
| | 60 | | | | 0,18 | 0,18 | | |
| | 70 | | | | | | | |
| | | | | | | | | |

| screening parameter | | | system | | | | | |
|---|---|---|---|---|---|---|---|---|
| k(min) < | 0,18 | | k(neutral) | 0.2 | | | | |
| k(min)> | 0,09 | | anion | DMGS | | | | |
| dk8> | 0,08 | | cation | 100/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,8 | 5,5 | 5,3 | 5,0 |
|---|---|---|---|---|---|---|---|---|
| | % anion | 25 | 35 | 40 | 50 | 60 | 65 | 75 |
| % neutral lipid | 0 | | | | 0,14 | 0,15 | 0,15 | |
| | 10 | | | | 0,15 | 0,16 | 0,15 | |
| | 20 | | | | 0,15 | 0,16 | 0,16 | |
| | 30 | | | | 0,16 | 0,17 | 0,16 | |
| | 40 | | | | 0,16 | 0,17 | 0,17 | |
| | 50 | | | | 0,17 | 0,18 | 0,17 | |
| | 60 | | | | 0,18 | | 0,18 | |
| | 70 | | | | | | | |
| | | | | | | | | |

| screening parameter | | | system | | | | | |
|---|---|---|---|---|---|---|---|---|
| k(min) < | 0,18 | | k(neutral) | 0.2 | | | | |
| k(min)> | 0,09 | | anion | DOGS | | | | |
| dk8> | 0,08 | | cation | 100/500 pK6.3 | | | | |
| IP> | 5 | | counteranion | PO4 | | | | |
| IP< | 6 | | countercation | Na | | | | |
| | | | | | | | | |

| Isoelectric point (IP) | | 6,6 | 6,3 | 6,2 | 5,8 | 5,5 | 5,3 | 5,0 |
|---|---|---|---|---|---|---|---|---|
| | % anion | 25 | 35 | 40 | 50 | 60 | 65 | 75 |
| % neutral lipid | 0 | | | | 0,13 | 0,13 | 0,13 | |
| | 10 | | | | 0,13 | 0,14 | 0,14 | |
| | 20 | | | | 0,14 | 0,15 | 0,14 | |
| | 30 | | | | 0,15 | 0,15 | 0,15 | |
| | 40 | | | | 0,16 | 0,16 | 0,16 | |
| | 50 | | | | 0,16 | 0,17 | 0,16 | |
| | 60 | | | | 0,17 | 0,17 | 0,17 | |
| | 70 | | | | 0,18 | 0,18 | 0,18 | |

In summary, the amphoteric liposomes of the present invention comprise neutral lipids selected from cholesterol or mixtures of cholesterol with one or more neutral or zwitterionic lipids, such as phosphatidylethanolamine or phosphatidylcholine and are well suited for the delivery of active agents into cells or tissues. Numerous specific examples for active formulations are disclosed in the description and in the examples of this invention. The chemical space providing a high frequency of successful compositions has been described using an algorithm and the parameters κ(min) and dκ(pH8) described therein; particularly preferred formulations have
- an amphoter I interaction type; a κ(min) between 0,09 and 0,15 and a dκ(pH8)>0,04 and an isoelectric point between 5 and 6.
- an amphoter II interaction type; a κ(min)<0,18 and a dκ(pH8)>0,08 and an isoelectric point between 5 and 6;
all of the above amphoteric formulations further comprise neutral lipids selected from the group comprising cholesterol or mixtures of cholesterol with one or more neutral or zwitterionic lipids which are phosphatidylethanolamine and/or phosphatidylcholine and wherein κ(neutral) of said mixture is 0.25 or less.

Examples are given with the understanding of further detailing certain aspects of practising the current invention. The examples by no means limit the scope of this disclosure.

### Example 1 - Preparation of liposomes and pH -dependent fusion experiment

### Buffer system

100 mM sodium citrate and 200 mM sodium hydrogen phosphate were prepared as stock solutions and variable amounts of both solutions were mixed to adjust for the pH needed. CiP 7.0 as an example specifies a buffer from that series having a pH of 7.0 and is made from citrate and phosphate.

### Liposome production

Liposomes were formed from a dried lipid film. In brief, 20 *µ*mol of the respective lipid composition was dissolved in 1mL chloroform/methanol 3:1 and dried in vacuum using a rotary evaporator. The resulting film was hydrated for 45 min in 1 mL of CiP 8.0 with gentle agitation. The resulting liposome suspension was frozen, sonicated after thawing and eventually extruded through 200 nm polycarbonate filters.

### pH -jump experiment

10 *µ*l liposomes in CiP 8.0 were placed into a glass tube and mixed rapidly with 1 mL of CiP buffer of the pH needed. Samples were allowed to stand for 1 h at room temperature and 3 mL of 200 mM sodium hydrogen phosphate were rapidly mixed with the sample. Liposomes were analyzed for size using a MALVERN Zetasizer 3000HS and sizes were recorded as Z-average.

### Example 2 - Fusion of amphoter I lipid mixtures

Liposomes were prepared from DOTAP and CHEMS in sodium citrate/ sodium phosphate pH 8.0 (CiP 8.0) and small amounts were injected into a CiP buffer with a lower pH (see Example 1 for details). Any larger structures observed at the lower pH might be either due to aggregate formations and generation of multicentric honeycomb structures or such structures might result from genuine fusion. To separate between these two outcomes we readjusted the pH to neutrality using 200mM sodium hydrogen phosphate. Electrostatic repulsion dissociates multicentric vesicles but not fusion products. The results are illustrated in Fig. 7.

As predicted in the mathematical salt bridge model, a valley of instability exists at slightly acidic conditions and fusion to larger particles was observed starting from pH 6.5. However, fast addition of the liposomes into low pH resulted in stabilisation of the particles as long as some DOTAP was present in the mixture. Liposomes from 100 mol.% CHEMS enter a fusogenic state below pH 4.5 and do not get stabilised at the lower pH.

Noteworthy, a 1:1 mixture of DOTAP/CHEMS cannot form liposomes in CiP 8.0 which is in good agreement with the mathematical model that predicts a non-lamellar phase for these parameters.

### Example 3 - fusion of amphoter II systems

Liposomes were prepared from MoChol and CHEMS in sodium citrate/ sodium phosphate pH 8.0 (CiP 8.0) and small amounts were injected into a CiP buffer with a lower pH (see Example 1 for details). Any larger structures observed at the lower pH might be either due to aggregate formations and generation of multicentric honeycomb structures or such structures might result from genuine fusion. To separate between these two outcomes we readjusted the pH to neutrality using 200 mM sodium hydrogen phosphate. Electrostatic repulsion dissociates multicentric vesicles but not fusion products.

Experimental evidence supports the salt bridge model. (See Fig. 8). The fusion zone is inclined towards high anion content due to the large head-group size of MoChol.Consequently, no fusion occurs with 33 mol.% or 50 mol.% CHEMS in the mixture, whereas mixtures containing 66 mol.% or 75 mol.% CHEMS undergo fusion when exposed to a pH between 4 and 6. As predicted, the onset of fusion is shifted to lower pH values with higher amounts of CHEMS. Again, 100 mol.% CHEMS is fusogenic with low pH but has no stable state at low pH.

The parameters used for the calculation are given in Table 70 below; CHEMS and MoChol in Na/ H₂PO₄ were used as model compounds; all volumes in Å³.

**Table 70**

| | |
|---|---|
| Anion head volume | 76 |
| Anion tail volume | 334 |
| Anion pK | 5.8 |
| Cation head volume | 166 |
| Cation tail volume | 371 |
| Cation pK | 6.5 |
| Counterion+ volume | 65 |
| Counterion-volume | 49 |

### Example 4 - lipid salt formation with monoalkyl lipids

Oleic acid was chosen as a known and popular pH -sensitive membrane component. As the lipid tail is relatively small in volume, any change in the head-group has more pronounced consequences for the membrane stability. As shown in Fig. 9, modelling predicts oleic acid to be a strong driver for fusion in an amphoter II system with MoChol. This is confirmed experimentally. Mixtures of oleic acid do form liposomes with Mo-Chol and particles rapidly undergo fusion when exposed to different conditions. As expected from the algorithm, the extent of fusion is limited for smaller amounts of OA in the mixture, but 50 mol.% of the anion results in the classic valley type fusion pattern. Since the fusion tendency is much stronger with OA, a bigger portion of that anion in the mix results in extensive fusion over a wide range of pH values. Still, mixtures can always be stabilised at low pH. Details as per Example 1.

**Table 71**

| | |
|---|---|
| MoChol head-group volume | 166 |
| MoChol tail volume | 371 |
| MoChol pK | 6.5 |
| Oleic acid head volume | 42 |
| Oleic acid tail volume | 208 |
| Oleic acid pK | 4.5 |
| Counterion citrate volume | 121 |
| Counterion sodium volume | 65 |

### Example 5 - fusion assay based on fluorescence resonance energy transfer (FRET)

To investigate the fusability of different amphoteric lipid mixtures a lipid mixing assay, based on FRET was used. Liposomes, single labelled with 0.6 mol% NBD-PE (N-(7-nitrobenz-2-oxa-1,3-diazol-4-yl)-1,2-dihexadecanoyl-sn-glycero-3-phosphoethanol-amine, triethylammonium salt) or Rhodamine-PE (Lissamine™ rhodamine B 1,2-dihexadecanoyl-sn-glycero-3-phosphoethanolamine, triethylammonium salt), respectively, were prepared to monitor lipid fusion through the appearance of a FRET signal.

Lipids were dissolved in isopropanol (final lipid concentration 16 mM) and mixed. Liposomes were produced by adding buffer (acetic acid 10 mM, phosphoric acid 10 mM, NaOH, pH 7.5) to the alcoholic lipid mix, resulting in a final lipid concentration of 1.95 mM and a final isopropanol concentration of 12.2%. For the preparation of the liposomes a liquid handling robot (Perkin Elmer, Multiprobe II Ex) was used. The NBD-labelled and Rh-labelled amphoteric liposomes were combined in a ratio 1:1 and subsequent diluted 1:1 with the buffer mentioned above. Finally small aliquots of this mixed sample were brought to decreasing specific pH (HAc 50 mM, Phosphoric acid 50 mM, NaOH, pH 7,5 - 2,5) and incubated at 37 °C for 2 h. Liposomes were diluted again 1:1 in this step.

Samples were measured for fluorescence using two sets of filters: NBD/Rhodamine: 460/590nm and NBD/NBD: 460/530nm. FRET as a signal for membrane fusion was expressed as the ratio of emission(590nm) / emission (530nm). A background of 0.4 indicates background fluorescence and was therefore subtracted from the FRET signals.

To discriminate between fusion and mere aggregation the suspension was neutralized to pH 7.5 and FRET signals were measured again. A possible interference of the remaining alcohol content of 3 % on the fusion of the liposomes was excluded by pre-experiments.

### Example 6: Impact of neutral or zwitterionic lipids on the fusion of amphoteric lipid mixtures

Amphoteric liposomes with increasing amounts of neutral or zwitterionic lipids were prepared as described in Example 5. Initially, an amphoter I system (DOTAP/DMGS) and an amphoter II (MoChol/DOGS) were prepared with the addition of 10-50 % different neutral or zwitterionic lipids or mixtures thereof. Fusion was measured for a series of liposomes having different C/A ratios. Systems can be characterized using the sum of all such measurements in the entire matrix. The effect of the neutral or zwitterionic lipids was then analyzed using this global parameter (∑ FRET).

Fig. 14 shows the influence of different neutral or zwitterionic lipids on the fusogenicity of the amphoteric lipid mixture MoChol/DOGS. It is apparent that neutral lipids having a high κ, such as POPC or DOPC, decrease the fusogenicity of the amphoteric liposomes, whereas the lipids having a lower κ, such as DOPE or cholesterol, have little impact on the fusogenicity or may even improve the fusion. Mixtures of POPC and DOPE and mixtures of POPC or DOPC and cholesterol may have little impact or decrease the fusion ability, depending of the ratio of the two lipids. This is further illustrated in Fig.15. The higher the molar ratio PC/Chol the lower the fusogenicity of the amphoteric liposomes. These findings correlate very well with the model as shown in Figs. 10-13 for the neutral lipids POPC, DOPE, Cholesterol and mixtures of POPC/Chol=1. In the figures ∑ FRET of liposomes from DOTAP/DMGS (C/A=0.17-0.75) or MoCHol/DOGS (C/A 0.33-3) was plotted against k(min) for mixtures with 0% - 50% neutral lipid. The reference K(min) was modelled for C/A=0.66 (DOTAP/DMGS) or C/A=1(MoChol/DOGS).

In a further experiment the effect of POPC or cholesterol on the fusogenicity of other amphoteric lipid systems were determined. Tables 72 and 73 summarize these data and confirm the results of the first part of the experiment. Tables 72 and 73 show the ∑ Fret and range of C/A ratios for which the amphoteric liposomes are stable at pH 7 to pH 8 and fuse between pH 3 to pH 6, preferably between pH 4 to pH 6.

It becomes apparent that amphoteric lipid systems having low fusogenicity can be clearly improved by the addition of cholesterol. Furthermore the results indicate that cholesterol may have also an impact on the range of fusogenicity. This means that the range of C/A ratios can be broadened.

**Table 72:**

| Cation | Anion | K(salt) | Σ Fret 0% neutral lipid | Σ Fret 20 % POPC | Σ Fret 40% POPC | Σ Fret 20 % Chol | Σ Fret **40** % Chol |
|---|---|---|---|---|---|---|---|
| | | | C/A ratio | C/A ratio | C/A ratio | C/A ratio | C/A ratio |
| DODAP | DMGS | 0.157 | 42 | 31 | 21 | 40 | 42 |
| DODAP | DMGS | | >0 - <1 | >0 - <1 | >0 - 0.67 | >0 - <1 | >0 - <1 |
| | | | | | | | |
| N-methyl-PipChol | DMGS | 0.271 | 38 | 16 | 3 | 40 | 44 |
| N-methyl-PipChol | DMGS | | >0 - <1 | >0 - 0.5 | - | >0 - <1 | >0 - <1 |
| | | | | | | | |
| DDAB | DMGS | 0.182 | 35 | 7 | 4 | 16 | 21 |
| DDAB | DMGS | | >0 - 0.5 | >0 - | - | >0 - 0.5 | >0 - 0.5 |
| | | | | | | | |
| DC-Chol | DOGS | 0.225 | 35 | 20 | 10 | 25 | 24 |
| DC-Chol | DOGS | | >0 - <1 | >0 - 0.67 | >0 - 0.4 | >0 - 0.67 | >0 - <1 |
| | | | | | | | |
| DOTAP | DMGS | 0.169 | 33 | 28 | 24 | 31 | 32 |
| DOTAP | DMGS | | >0 - 0.5 | >0 - 0.67 | >0 - 0.67 | >0 - 0.5 | >0 - 0.67 |
| | | | | | | | |
| DC-Chol | DMGS | 0.254 | 32 | 22 | 13 | 35 | 42 |
| DC-Chol | DMGS | | >0 - <1 | >0 - 0.4 | >0 - 0.4 | >0 - <1 | >0 - <1 |
| | | | | | | | |
| DC-Chol | Chems | 0.265 | 31 | 8 | 1 | 33 | 33 |
| DC-Chol | Chems | | >0 - <1 | >0 - 0.17 | - | >0 - <1 | >0 - <1 |
| | | | | | | | |
| DOTAP | Chems | 0.171 | 17 | 11 | 3 | 21 | 25 |
| DOTAP | Chems | | >0 - 0.4 | >0 - 0.4 | >0 - 0.17 | >0 - 0.5 | >0 - 0.67 |
| | | | | | | | |
| DOTAP | DOGS | 0.153 | 17 | 17 | 17 | 36 | 37 |
| DOTAP | DOGS | | >0 - 0.4 | >0 - 0.4 | >0 - 0.4 | >0 - 0.4 | >0 - 0.67 |
| | | | | | | | |
| DDAB | Chems | 0.186 | 6 | 7 | 0 | 33 | 52 |
| DDAB | Chems | | >0 - 0.17 | >0 - 0.17 | - | >0 - 0.67 | >0 - <1 |

**Table 73:**

| Cation | Anion | K(salt) | ∑ Fret 0% neutral lipid | ∑ Fret 20 % POPC | ∑ Fret 40% POPC | ∑ Fret 20 % Chol | ∑ Fret 40 % Chol |
|---|---|---|---|---|---|---|---|
| | | | C/A ratio | C/A ratio | C/A ratio | C/A ratio | C/A ratio |
| HisChol | DOGS | 0.282 | 37 | 19 | 9 | 33 | 50 |
| HisChol | DOGS | | >0 - 0.7 | >0 - 0.7 | >0 - 0.7 | >0 - 0.7 | <0 - 0.33 |
| | | | | | | | |
| Chim | DMGS | 0.278 | 36 | 12 | 5 | 37 | 42 |
| Chim | DMGS | | >0 - 2 | >0 - 0.7 | >0 - 0.3 | >0 - 1.5 | >0 - 1 |
| | | | | | | | |
| DmC4Mo2 | Chems | 0.403 | 33 | 10 | 0 | 32 | 23 |
| DmC4Mo2 | Chems | | >0 | >0 - 0.7 | - | >0 | ≥1 |
| | | | | | | | |
| Chim | Chems | 0.292 | 30 | ND | 0 | 33 | 27 |
| Chim | Chems | | >0 - 1.5 | ND | - | >0 - 0.7 | >0 - 0.7 |
| | | | | | | | |
| Chim | DOGS | 0.247 | 30 | 16 | 14 | 29 | 29 |
| Chim | DOGS | | >0 - 1 | >0 - 0.7 | >0 - 0.7 | >0 - 1 | >0 - 1.5 |
| | | | | | | | |
| DmC4Mo2 | DMGS | 0.378 | 28 | 22 | 9 | 33 | 41 |
| DmC4Mo2 | DMGS | | >0 | >0- 0.7 | >0 - 0.7 | >0 | >0 |
| | | | | | | | |
| MoC3Chol | DOGS | 0.269 | 26 | 15 | 9 | 30 | 34 |
| MoC3Chol | DOGS | | >0 - 0.7 | >0 - 0.7 | >0 - 0.5 | >0 - 0.7 | >0 - 0.7 |
| | | | | | | | |
| MoChol | DOGS | 0.303 | 25 | 17 | 11 | 24 | ND |
| MoChol | DOGS | | >0 - 1 | >0 - 0.7 | >0 - 0.7 | >0 - 1 | ND |
| | | | | | | | |
| HisChol | Chems | 0.336 | 22. | 9 | 1 | 22 | 25 |
| HisChol | Chems | | >0 - 0.7 | >0 - 0.5 | - | >0 - 1 | >0 - 0.7 |
| | | | | | | | |
| MoChol | Chems | 0.363 | 19 | 3 | 0 | 20 | 24 |
| MoChol | Chems | | >0 - 0.7 | >0 - 0.33 | - | >0 - 1 | >0 - 1 |
| | | | | | | | |
| MoChol | DMGS | 0.342 | 15 | 8 | 4 | 18 | 23 |
| MoChol | DMGS | | >0 - 0.7 | >0 - 0.7 | - | >0 - 1 | >0 - 1 |
| | | | | | | | |
| DOIM | DOGS | 0.145 | 14 | 9 | 10 | 13 | 26 |
| DOIM | DOGS | | >0 - 0.7 | >0 - 0.7 | >0 - 0.7 | >0 - 0.7 | >0 - 0.7 |

### Example 7: Colloidal stabilization of amphoteric liposomes by neutral lipids

Fusion assays were performed as described in Example 5. DOTAP/Oleic Acid formulations with 0 or 20 mol% cholesterol were tested for fusion in cation/anion molar ratios (C/A ratio) of 0.17, 0.33, 0.40, 0.50, 0.67, 0.75 and pure anionic liposomes were prepared as controls.

The following Tables 74 and 75 show the fusion profiles for the two DOTAP/Oleic acid amphoter systems as matrix C/A vs. pH. In addition the fusion of liposomes of pure anionic lipid is shown (C/A=0).

The tables indicate that the addition of cholesterol leads to a colloidal stabilization of amphoteric liposomes at pH 7.5 and C/A ratios of 0.67 and 0.75.

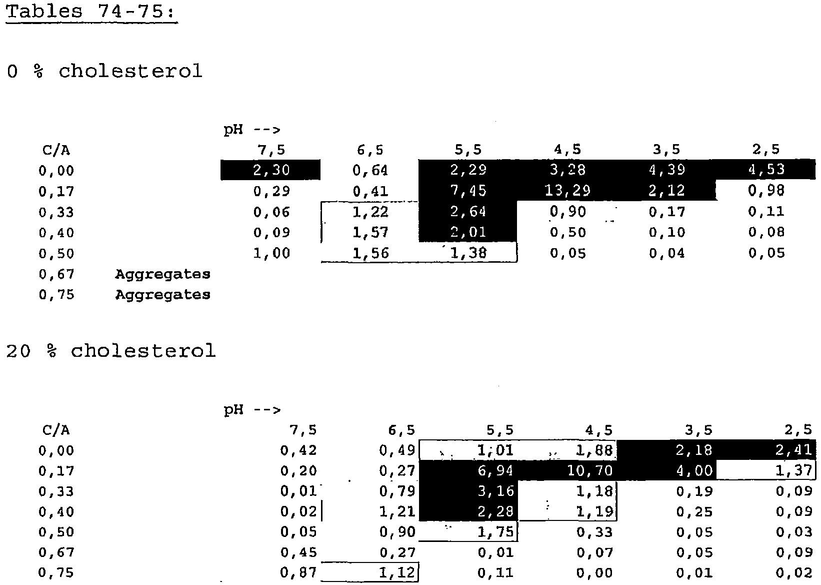

### Example 8: In vitro transfection of Hela cells with amphoteric liposomes encapsulating siRNA targeting Plk-1 or non-targeting scrambled (scr) siRNA

### Preparation of liposomes:

Liposomes were manufactured by an isopropanol-injection method. Lipids were dissolved in isopropanol (30 mM lipid concentration) and mixed. Liposomes were produced by adding siRNA solution in NaAc 20mM, Sucrose 300 mM, pH 4.0 (pH adjusted with HAc) to the alcoholic lipid mix, resulting in a final alcohol concentration of 30%. The formed liposomal suspensions were shifted to pH 7.5 with twice the volume of Na₂HPO₄ 136mM, NaCl 100mM (pH 9), resulting in a final lipid concentration of 3 mM and a final isopropanol concentration of 10%.

Some formulations were prepared with 20 mM lipid as starting concentration and 2 mM as final lipid concentration. Such formulations were marked with an asterisk in table 76 and table 77.

N/P = the ratio cationic charges from the lipids to anionic charges from the siRNA during manufacturing.

Size of the liposomal formulations was characterized using dynamic light scattering (Zetasizer 3000, Malvern).

Following liposomal amphoter I formulations encapsulating siRNA targeting PLK-1 or non-targeting scrambled siRNA were produced:
PLK-1 siRNA as in Haupenthal et al., Int J Cancer, 121, 206-210 (2007).

**Table 76:**

| **C/A ratio** | **Amphoter I system** | **Molar Amount of Chol (%)** | **Molar Amount of DOPE/Chol (molar ratio 0.5)** | **Molar Amount of POPC/Chol (molar ratio 0.5)** | **N/P** |
|---|---|---|---|---|---|
| 0.33 | DOTAP/DMGS | 0; 20; 30; 40; 50; 60 | 20; 40; 60 | 20; 40; 60 | 5 |
| 0.5 | DOTAP/DMGS | 0; 20; 30; 40; 50; 60 | 20; 40; 60 | 20; 40; 60 | 5 |
| 0.67 | DOTAP/DMGS | 0; 20; 30; 40; 50; 60 | 20; 40; 60 | 20; 40; 60 | 5 |
| 0.82 | DOTAP/DMGS | 0; 20; 30; 40; 50; 60 | 20; 40; 60 | 20; 40; 60 | 5 |
| | | | | | |
| 0.33 | DOTAP/DOGS | 0; 20; 30; 40; 50; 60 | 20; 40; 60 | 20; 40; 60 | 5 |
| 0.5 | DOTAP/DOGS | 0; 20; 30; 40; 50; 60 | 20; 40; 60 | 20; 40; 60 | 5 |
| 0.67 | DOTAP/DOGS | 0; 20; 30; 40; 50; 60 | 20; 40; 60 | 20; 40; 60 | 5 |
| 0.82 | DOTAP/DOGS | 0; 20; 30; 40; 50; 60 | 20; 40; 60 | 20; 40; 60 | 5 |
| | | | | | |
| 0.33 | DOTAP/CHEMS | 0; 20; 30; 40; 50; 60 | 20; 40; 60 | 20; 40; 60 | 5 |
| 0.5 | DOTAP/CHEMS | 0; 20; 30; 40; 50; 60 | 20; 40; 60 | 20; 40; 60 | 5 |
| 0.67 | DOTAP/CHEMS | 0; 20; 30; 40; 50; 60 | 20; 40; 60 | 20; 40; 60 | 5 |
| 0.82 | DOTAP/CHEMS | 0; 20; 30; 40; 50; 60 | 20; 40; 60 | 20; 40; 60 | 5 |
| | | | | | |
| 0.33 | DOTAP/Chol-C12 | 0; 20; 40 | - | - | 3 |
| 0.5 | DOTAP/Chol-C12 | 0; 20; 40 | - | - | 3 |
| 0.67 | DOTAP/Chol-C12 | 0; 20; 40 | - | - | 3 |
| 0.82 | DOTAP/Chol-C12 | 0; 20; 40 | - | - | 3 |
| | | | | | |
| 0.33 | DOTAP/Chol-C13N | 0; 20; 40 | - | - | 3 |
| 0.5 | DOTAP/Chol-C13N | 0; 20; 40 | - | - | 3 |
| 0.67 | DOTAP/Chol-C13N | 0; 20; 40 | - | - | 3 |
| 0.82 | DOTAP/Chol-C13N | 0; 20; 40 | - | - | 3 |
| | | | | | |
| 0.33 | DODAP/DMGS | 0; 20; 30; 40; 50; 60 | 20; 40; 60 | 20; 40; 60 | 5 |
| 0.5 | DODAP/DMGS | 0; 20; 30; 40; 50; 60 | 20; 40; 60 | 20; 40; 60 | 5 |
| 0.67 | DODAP/DMGS | 0; 20; 30; 40; 50; 60 | 20; 40; 60 | 20; 40; 60 | 5 |
| 0.82 | DODAP/DMGS | 0; 20; 30; 40; 50; 60 | 20; 40; 60 | 20; 40; 60 | 5 |
| | | | | | |
| 0.33 | DODAP/DOGS | 0; 20; 30; 40; 50; 60 | 20; 40; 60 | 20; 40; 60 | 5 |
| 0.5 | DODAP/DOGS | 0; 20; 30; 40; 50; 60 | 20; 40; 60 | 20; 40; 60 | 5 |
| 0.67 | DODAP/DOGS | 0; 20; 30; 40; 50; 60 | 20; 40; 60 | 20; 40; 60 | 5 |
| 0.82 | DODAP/DOGS | 0; 20; 30; 40; 50; 60 | 20; 40; 60 | 20; 40; 60 | 5 |
| | | | | | |
| 0.33 | DODAP/CHEMS | 0; 20; 30; | 20; 40; 60 | 20; 40; 60 | 5 |
| | | 40; 50; 60 | | | |
| 0.5 | DODAP/CHEMS | 0; 20; 30; 40; 50; 60 | 20; 40; 60 | 20; 40; 60 | 5 |
| 0.67 | DODAP/CHEMS | 0; 20; 30; 40; 50; 60 | 20; 40; 60 | 20; 40; 60 | 5 |
| 0.82 | DODAP/CHEMS | 0; 20; 30; 40; 50; 60 | 20; 40; 60 | 20; 40; 60 | 5 |
| | | | | | |
| 0.33 | DODAP/Chol-C6 | 0; 20; 30; 40; 50; 60 | - | - | 3 |
| 0.5 | DODAP/Chol-C6 | 0; 20; 30; 40; 50; 60 | - | - | 3 |
| 0.67 | DODAP/Chol-C6 | 0; 20; 30; 40; 50; 60 | - | - | 3 |
| 0.82 | DODAP/Chol-C6 | 0; 20; 30; 40; 50; 60 | - | - | 3 |
| | | | | | |
| 0.33 | DC-Chol/DMGS | 0; 20; 30; 40; 50; 60 | 20; 40; 60 | 20; 40; 60 | 5 |
| 0.5 | DC-Chol/DMGS | 0; 20; 30; 40; 50; 60 | 20; 40; 60 | 20; 40; 60 | 5 |
| 0.67 | DC-Chol/DMGS | 0; 20; 30; 40; 50; 60 | 20; 40; 60 | 20; 40; 60 | 5 |
| 0.82 | DC-Chol/DMGS | 0; 20; 30; 40; 50; 60 | 20; 40; 60 | 20; 40; 60 | 5 |
| | | | | | |
| 0.33 | DORI/Chems | 0; 20; 30; 40; 50; 60 | - | - | 3 |
| 0.5 | DORI/Chems | 0; 20; 30; 40; 50; 60 | - | - | 3 |
| 0.67 | DORI/Chems | 0; 20; 30; | - | - | 3 |
| | | 40; 50; 60 | | | |
| 0.82 | DORI/Chems | 0; 20; 30; 40; 50; 60 | - | - | 3 |
| | | | | | |
| 0.33 | DORI/DMGS | 0; 20; 40 | - | - | 3 |
| 0.5 | DORI/DMGS | 0; 20; 40 | - | - | 3 |
| 0.67 | DORI/DMGS | 0; 20; 40 | - | - | 3 |
| 0.82 | DORI/DMGS | 0; 20; 40 | - | - | 3 |
| | | | | | |
| 0.33 | DORI/DOGS | 0; 20; 40 | - | - | 3 |
| 0.5 | DORI/DOGS | 0; 20; 40 | - | - | 3 |
| 0.67 | DORI/DOGS | 0; 20; 40 | - | - | 3 |
| 0.82 | DORI/DOGS | 0; 20; 40 | - | - | 3 |
| | | | | | |
| 0.33 | DOP5P/DMGS | 0; 20; 40 | - | - | 3 |
| 0.5 | DOP5P/DMGS | 0; 20; 40 | - | - | 3 |
| 0.67 | DOP5P/DMGS | 0; 20; 40 | - | - | 3 |
| 0.82 | DOP5P/DMGS | 0; 20; 40 | - | - | 3 |
| | | | | | |
| 0.33 | DOP5P/Chems | 0; 20; 30; 40; 50; 60 | - | - | 3 |
| 0.5 | DOP5P/Chems | 0; 20; 30; 40; 50; 60 | - | - | 3 |
| 0.67 | DOP5P/Chems | 0; 20; 30; 40; 50; 60 | - | - | 3 |
| 0.82 | DOP5P/Chems | 0; 20; 30; 40; 50; 60 | - | - | 3 |
| | | | | | |
| 0.33 | DOP6P/DMGS | 0; 20; 40 | - | - | 3 |
| 0.5 | DOP6P/DMGS | 0; 20; 40 | - | - | 3 |
| 0.67 | DOP6P/DMGS | 0; 20; 40 | - | - | 3 |
| 0.82 | DOP6P/DMGS | 0; 20; 40 | - | - | 3 |
| | | | | | |
| 0.33 | DOP6P/Chems | 0; 20; 30; 40; 50; 60 | - | - | 3 |
| 0.5 | DOP6P/Chems | 0; 20; 30; 40; 50; 60 | - | - | 3 |
| 0.67 | DOP6P/Chems | 0; 20; 30; 40; 50; 60 | - | - | 3 |
| 0.82 | DOP6P/Chems | 0; 20; 30; 40; 50; 60 | - | - | 3 |
| | | | | | |
| 0.33 | DOTAP/DMGS | 0; 20; 30; 40; 50; 60 | - | - | 3 |
| | | | | | |
| 0.33 | DOTAP/DMGS* | 40 | - | - | 1.5 |
| 0.4 | DOTAP/DMGS* | 40 | - | - | 1.5 |
| 0.5 | DOTAP/DMGS* | 40 | - | - | 1.5 |
| 0.33 | DOTAP/DMGS* | 40 | - | - | 3 |
| 0.4 | DOTAP/DMGS* | 40 | - | - | 3 |
| 0.5 | DOTAP/DMGS* | 40 | - | - | 3 |
| 0.33 | DOTAP/DMGS* | 40 | - | - | 6 |
| 0.4 | DOTAP/DMGS* | 40 | - | - | 6 |
| 0.5 | DOTAP/DMGS* | 40 | - | - | 6 |
| | | | | | |
| 0.33 | DOTAP/DOGS* | 40 | - | - | 1.5 |
| 0.4 | DOTAP/DOGS* | 40 | - | - | 1.5 |
| 0.5 | DOTAP/DOGS* | 40 | - | - | 1.5 |
| 0.33 | DOTAP/DOGS* | 40 | - | - | 3 |
| 0.4 | DOTAP/DOGS* | 40 | - | - | 3 |
| 0.5 | DOTAP/DOGS* | 40 | - | - | 3 |
| 0.33 | DOTAP/DOGS* | 40 | - | | 6 |
| 0.4 | DOTAP/DOGS* | 40 | - | - | 6 |
| 0.5 | DOTAP/DOGS* | 40 | - | - | 6 |
| | | | | | |
| 0.33 | DOTAP/OA* | 40 | - | - | 1.5 |
| 0.4 | DOTAP/OA* | 40 | - | - | 1.5 |
| 0.5 | DOTAP/OA* | 40 | - | - | 1.5 |
| 0.33 | DOTAP/OA* | 40 | - | - | 3 |
| 0.4 | DOTAP/OA* | 40 | - | - | 3 |
| 0.5 | DOTAP/OA* | 40 | - | - | 3 |
| 0.33 | DOTAP/OA* | 40 | - | - | 6 |
| 0.4 | DOTAP/OA* | 40 | - | - | 6 |
| 0.5 | DOTAP/OA* | 40 | - | - | 6 |

Following liposomal amphoter II formulations encapsulating siRNA targeting PLK-1 or non-targeting scrambled siRNA were produced:

**Table 77:**

| **C/A ratio** | **Amphoter II system** | **Molar Amount of Chol (%)** | **Molar Amount of DOPE/Chol (molar ratio 0.5)** | **Molar Amount of POPC/Chol (molar ratio 0.5)** | **N/P** |
|---|---|---|---|---|---|
| 0.33 | HisChol/DMGS | 0; 20; 30; 40; 50; 60 | 20; 40; 60 | 20; 40; 60 | 5 |
| 0.5 | HisChol/DMGS | 0; 20; 30; 40; 50; 60 | 20; 40; 60 | 20; 40; 60 | 5 |
| 1 | HisChol/DMGS | 0; 20; 30; 40; 50; 60 | 20; 40; 60 | 20; 40; 60 | 5 |
| 2 | HisChol/DMGS | 0; 20; 30; 40; 50; 60 | 20; 40; 60 | 20; 40; 60 | 5 |
| | | | | | |
| 0.33 | MoChol/DMGS | 0; 20; 30; 40; 50; 60 | 20; 40; 60 | 20; 40; 60 | 5 |
| 0.5 | MoChol/DMGS | 0; 20; 30; 40; 50; 60 | 20; 40; 60 | 20; 40; 60 | 5 |
| 1 | MoChol/DMGS | 0; 20; 30; 40; 50; 60 | 20; 40; 60 | 20; 40; 60 | 5 |
| 2 | MoChol/DMGS | 0; 20; 30; 40; 50; 60 | 20; 40; 60 | 20; 40; 60 | 5 |
| | | | | | |
| 0.33 | Chim/DMGS | 0; 20; 30; 40; 50; 60 | 20; 40; 60 | 20; 40; 60 | 5 |
| 0.5 | Chim/DMGS | 0; 20; 30; 40; 50; 60 | 20; 40; 60 | 20; 40; 60 | 5 |
| 1 | Chim/DMGS | 0; 20; 30; 40; 50; 60 | 20; 40; 60 | 20; 40; 60 | 5 |
| 2 | Chim/DMGS | 0; 20; 30; 40; 50; 60 | 20; 40; 60 | 20; 40; 60 | 5 |
| | | | | | |
| 0.33 | CholC4N-Mo2/DMGS | 0; 20; 30; 40; 50; 60 | 20; 40; 60 | 20; 40; 60 | 5 |
| 0.5 | CholC4N-Mo2/DMGS | 0; 20; 30; 40; 50; 60 | 20; 40; 60 | 20; 40; 60 | 5 |
| 1 | CholC4N-Mo2/DMGS | 0; 20; 30; 40; 50; 60 | 20; 40; 60 | 20; 40; 60 | 5 |
| 2 | CholC4N-Mo2/DMGS | 0; 20; 30; 40; 50; 60 | 20; 40; 60 | 20; 40; 60 | 5 |
| | | | | | |
| 0.33 | CholC3N-Mo2/DMGS | 0; 20; 30; 40; 50; 60 | 20; 40; 60 | 20; 40; 60 | 5 |
| 0.5 | CholC3N-Mo2/DMGS | 0; 20; 30; 40; 50; 60 | 20; 40; 60 | 20; 40; 60 | 5 |
| 1 | CholC3N-Mo2/DMGS | 0; 20; 30; 40; 50; 60 | 20; 40; 60 | 20; 40; 60 | 5 |
| 2 | CholC3N-Mo2/DMGS | 0; 20; 30; 40; 50; 60 | 20; 40; 60 | 20; 40; 60 | 5 |
| | | | | | |
| 0.33 | HisChol/DOGS | 0; 20; 30; 40; 50; 60 | 20; 40; 60 | 20; 40; 60 | 5 |
| 0.5 | HisChol/DOGS | 0; 20; 30; 40; 50; 60 | 20; 40; 60 | 20; 40; 60 | 5 |
| 1 | HisChol/DOGS | 0; 20; 30; 40; 50; 60 | 20; 40; 60 | 20; 40; 60 | 5 |
| 2 | HisChol/DOGS | 0; 20; 30; 40; 50; 60 | 20; 40; 60 | 20; 40; 60 | 5 |
| | | | | | |
| 0.66 | DOMCAP/Chol-C1 | | 30 (molar ratio 1.5) | - | 5 |
| | | | | | |
| 1 | HisChol/DMGS* | 40 | - | - | 1.5 |
| 2 | HisChol/DMGS* | 40 | - | - | 1.5 |
| 3 | HisChol/DMGS* | 40 | - | - | 1.5 |
| 1 | HisChol/DMGS* | 40 | - | - | 3 |
| 2 | HisChol/DMGS* | 40 | - | - | 3 |
| 3 | HisChol/DMGS* | 40 | - | - | 3 |
| | | | | | |
| 1 | HisChol/DOGS* | 40 | - | - | 1.5 |
| 2 | HisChol/DOGS* | 40 | - | - | 1.5 |
| 3 | HisChol/DOGS* | 40 | - | - | 1.5 |
| 1 | HisChol/DOGS* | 40 | - | - | 3 |
| 2 | HisChol/DOGS* | 40 | - | - | 3 |
| 3 | HisChol/DOGS* | 40 | - | - | 3 |
| | | | | | |
| 1 | CHIM/DMGS* | 40 | - | - | 1.5 |
| 2 | CHIM/DMGS* | 40 | - | - | 1.5 |
| 3 | CHIM/DMGS* | 40 | - | - | 1.5 |
| 1 | CHIM/DMGS* | 40 | - | - | 3 |
| 2 | CHIM/DMGS* | 40 | - | - | 3 |
| 3 | CHIM/DMGS* | 40 | - | - | 3 |
| 1 | CHIM/DMGS* | 40 | - | - | 6 |
| 2 | CHIM/DMGS* | 40 | - | - | 6 |
| 3 | CHIM/DMGS* | 40 | - | - | 6 |
| | | | | | |
| 1 | CHIM/CHEMS* | 40 | - | - | 1.5 |
| 2 | CHIM/CHEMS* | 40 | - | - | 1.5 |
| 3 | CHIM/CHEMS* | 40 | - | - | 1.5 |
| 1 | CHIM/CHEMS* | 40 | - | - | 3 |
| 2 | CHIM/CHEMS* | 40 | - | - | 3 |
| 3 | CHIM/CHEMS* | 40 | - | - | 3 |
| 1 | CHIM/CHEMS* | 40 | - | - | 6 |
| 2 | CHIM/CHEMS* | 40 | - | - | 6 |
| 3 | CHIM/CHEMS* | 40 | - | - | 6 |

### Transfection protocol:

HeLa cells were obtained from DSMZ (German Collection of Micro Organism and Cell Cultures) and maintained in DMEM. Media were purchased from Gibco-Invitrogen and supplemented with 10% FCS. The cells were plated at a density of 2.5^{∗}10⁴ cells/ml and cultivated in 100 *µ*l medium at 37 °C under 5% CO₂. After 16 h the liposomes containing siRNA were diluted in the manufacturing buffer system (see above) or in PBS or in OptimemI (Gibco-Invitrogen), optionally after a preincubation in serum. Then 10 *µ*l were added to the cells (110*µ*l final Volume and 9.1% FCS per well) (doses varied of between 0,4 to 150 nM Plkl or scrambled siRNA and maximum tested doses varied for Amphoter I formulations of between 12.5 and 150 nM and for Amphoter II of between 40 and 150 nM). 10*µ*l dilution buffer were also added to untreated cells and into wells without cells. In addition, as control, free siRNA was added to the cells (10 to 80 nM Plk-1 or scrambled siRNA). Cell culture dishes were incubated for 72 h hours at 37 °C under 5% CO₂. Transfection efficiency was analyzed using a cell proliferation/viability assay.

### Cell proliferation/viability assay:

Cell proliferation/viability was determined by using the CellTiter-Blue Cell Viability assay (Promega, US). In brief, 72 hours after transfection, 100 *µ*l Medium/CellTiter-Blue reagent (Pre-mix of 80*µ*l Medium and 20*µ*l CellTiter-Blue reagent) were added to the wells. Following incubation at 37 °C for 2.5 hours, 80 *µ*l of the medium were transferred into the wells of a black microtiter plate (NUNC, Denmark). Fluorescence was recorded using a fluorescence plate reader (Ex. 550 nm/Em. 590 nm). On each plate the following controls were included: i) wells without cells but with medium (control for culture medium background fluorescence) and ii) wells with cells (untreated cells = mock-transfected cells). For calculation, the mean fluorescence value of the culture medium background was subtracted from all mean (triplicates) values of experimental wells (transfected and mock-transfected cells). The fluorescence values from each transfection were normalized to the mean fluorescence value from mock-transfected cells, which was set as being 100%.

### Results:

Figure 17 shows a plot IC50 values vs. κ(min) values of all amphoter I liposomes including neutral liposomes of table 76. Low IC50 values indicate a high transfection efficiency of the liposomal delivery system. As the formulations are tested in different dose ranges some formulations in the plot marked as "IC50 > 12.5 nM", indicating that these formulations are not efficient in the appropriate tested dose range. The plot clearly shows an optimum of the transfection efficiency of the amphoter I liposomes at a specific range of κ(min) values.

Similarly, in figure 18 the IC50 values vs. κ(min) values of all amphoter II liposomes including neutral lipids of table 77 are shown. As the formulations are tested only in different dose ranges some formulations in the plot marked as "IC50 > 40 nM", indicating that these formulations are not efficient in the appropriate tested dose range.
It becomes apparent from the figure that the amphoteric liposomes have to reach a certain minimum of κ(min) to transfect the cells.

The plot in figure 19 shows the size of all liposomes comprising neutral lipids from table 76 and 77 vs. dK(pH 8) of the formulations and indicates that very small particles preferably are obtained with dκ (pH8) > 0.04. dK (pH8) is the difference of κ(pH8) and κ(min).

The influence of the addition of the neutral lipids on the transfection efficiency of two selected amphoteric lipid mixtures is demonstrated in figure 20 and figure 21. In both cases the addition of the neutral lipids improve the transfection efficieny of the amphoteric liposomes clearly and in both cases a dose response is seen, whereas the control scrambled siRNA encapsulated in the appropriate amphoteric liposomes do not show an effect.

Figure 22 and 23 show for an amphoter I (DC-CHOL/DMGS) and for an amphoter II (Chim/DMGS) system that the addition of 60 mol% of a POPC/Chol mixture (molar ratio 0.5) inhibits the transfection of the cells almost completely. In contrast, the addition of 20 mol% or 40 mol% of the POPC/Chol mixture does not inhibit the transfection of the cells.

Furthermore, the influence of the isoelectric point (IP) of the amphoteric lipid mixtures on the transfection efficieny of the inventive amphoteric liposomes is shown in figure 24 for different amphoteric liposomes according to the invention.

### Example 9: In vitro transfection of primary hepatocytes with amphoteric liposomes encapsulating siRNA targeting ApoB 100 or non-targeting scrambled (scr) siRNA

### Preparation of liposomes encapsulating siRNA targeting ApoB 100 or non-targeting scrambled (scr) siRNA:

Liposomes were manufactured by an isopropanol-injection method. Lipid mixtures were dissolved in isopropanol. A stock solution of the active ApoB 100 or non-active scrambled siRNA was diluted in buffer to the appropriate concentration and was transferred to a round-bottom flask. Both solutions were mixed at pH 4 using an injection device with pumps in a ratio 10:23.3 (lipids in solvent: siRNA in aqueous buffer) to an isopropanol concentration of 30%. The resulting liposomal suspensions were shifted to pH 7.5 and a final alcohol concentration of 10 %. The final liposomes were dialyzed to remove non-encapsulated siRNA and the alcohol. Subsequently, the liposomal suspensions were concentrated to the desired siRNA-concentration.

ApoB 100 siRNA as in Soutschek et al., Nature, 432, 173-178 (2004), further comprising a 5'phosphorylation on the guide strand.

**Table 78:**

| | **DOTAP/DOGS/Chol 15:45:40 (mol%)** | **DODAP/DMGS/Chol 24:36:40 (mol%)** |
|---|---|---|
| **Size** | 185 nm | 99 nm |
| **PI** | 0,08 | 0,12 |

| | | |
|---|---|---|
| PI = Polydispersity index | | |

### Transfection protocol:

Primary mouse hepatocytes were isolated according to the protocol of Seglen (Seglen, P.O. Preparation of isolated rat liver cells. Methods Cell Biol. 13:29-83; 1976) and modified for mouse cell preparation. The mouse hepatocytes were resuspended finally in DME-Media (Gibco-Invitrogen). The cells were plated onto 6-well-plates at a density of 4 x 10⁵ cells/ well and cultivated in 2000 *µ*l of DME-Media with 10% FCS at 37 °C under 5% CO₂.

For transfection the liposomes containing siRNA were diluted in Optimem I (Gibco-Invitrogen, Karlsruhe, Germany) to the desired dose. A volume of 200 *µ*l were added to the cells (2200*µ*l final volume and 9.1% FCS per well). Cells treated with Optimem I or dialysis buffer served as untreated control. Cell culture dishes were incubated for 70 h hours at 37 °C under 5% CO₂.

Reduction of the target mRNA (ApoB) was quantified using the Quantigene Assay (Panomics, Fremint, CA, USA).

### Results:

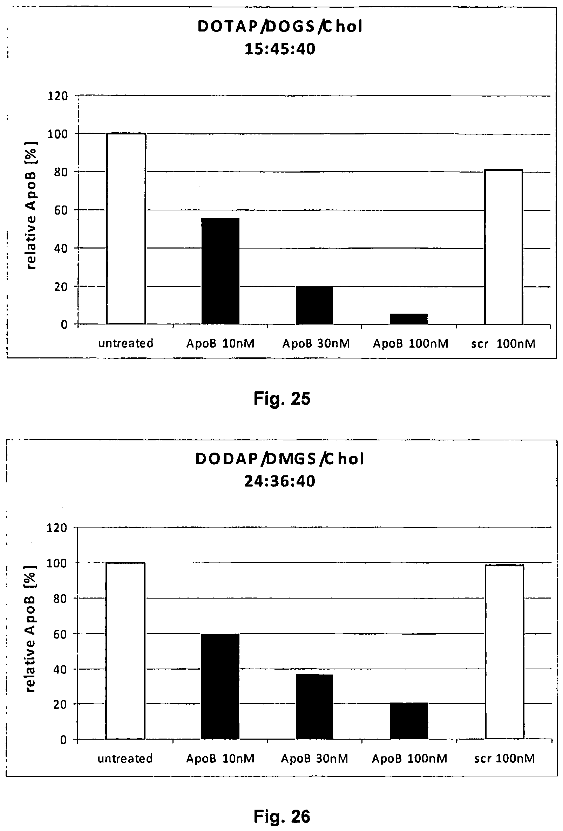

Both amphoteric liposome formulations show a knockdown of the target ApoB mRNA in a dose dependent manner down to 5 or 20 % compared to the untreated cells (see Figs. 25 and 26). In contrast, the formulations encapsulating non targeting scrambled siRNA have almost no effect on the ApoB mRNA level of the cells indicating that the liposomal formulations show no toxicity.

### Example 10: In vitro transfection of RAW 264.7 cells (mouse leukaemic monocyte macrophage cell line) with amphoteric liposomes encapsulating siRNA targeting PLK-1 or non-targeting scrambled (scr) siRNA

### Preparation of liposomes encapsulating siRNA targeting PLK-1 or non-targeting scrambled (scr) siRNA:

Liposomes F1-F4 were manufactured by an isopropanol-injection method. Lipid mixtures were dissolved in isopropanol. A stock solution of the active PLK-1 or non-active scrambled siRNA was buffer to the appropriate concentration and was transferred to a round-bottom flask. Both solutions were mixed at pH 4 using an injection device with pumps in a ratio 10:23.3 (lipids in solvent: siRNA in aqueous buffer) to an isopropanol concentration of 30%. The resulting liposomal suspensions were shifted to pH 7.5 and a final alcohol concentration of 10 %. Subsequently, the liposomal suspensions were concentrated to the desired siRNA-concentration.

PLK-1 siRNA as in Haupenthal et al., Int J Cancer, 121, 206-210 (2007).

| | | |
|---|---|---|
| F1: | DOTAP/Chems/Chol | 24:26:40 (mol%) |
| F2: | DOTAP/DOGS/Chol | 15:45:40 (mol%) |
| F3: | DOTAP/DMGS/Chol | 15:45:40 (mol%) |
| F4: | DOTAP/DMGS/Chol | 17:53:30 (mol%) |

**Table 79:**

| | **F1** | **F2** | **F3** | **F4** |
|---|---|---|---|---|
| **Size** | 105 nm | 150 nm | 203 nm | 198 nm |
| **PI** | 0.21 | 0.16 | 0.15 | 0.25 |

### Transfection protocol:

RAW 264.7 cells were obtained from ATCC and maintained in DMEM. Media were purchased from Gibco-Invitrogen and supplemented with 10% FCS. The cells were plated at a density of 4^{∗}10⁴ cells/ml and cultivated in 100 *µ*l medium at 37 °C under 5% CO₂. Liposomes containing siRNA were diluted in the manufacturing buffer system. Then 10 *µ*l were added to the cells (110*µ*l final Volume and 9.1% FCS per well) (19 to 600 nM Plk1 or scrambled siRNA). 10*µ*l dilution buffer were also added to untreated cells and into wells without cells. Cell culture dishes were incubated for 72 h hours at 37 °C under 5% CO₂. Transfection efficiency was analyzed using a cell proliferation/viability assay as described in example 8.

### Results:

The amphoteric liposome formulations F1-F4 encapsulating PLK-1 siRNA are effective in transfecting RAW 264.7 cells, a mouse leukaemic monocyte macrophage cell line. IC 50 values are shown in table 80 below:

**Table 80:**

| ***Formulation*** | ***IC 50 [nM]*** |
|---|---|
| F1 | 75 |
| F2 | 38 |
| F3 | 50 |
| F4 | 25 |

### Example 11: Serum stability of amphoteric liposomes

### Prepararation of siRNA encapsulating liposomes:

Amphoteric liposomes encapsulating a mixture of Plk-1 siRNA /scr siRNA-Cy 5.5 labelled (9:1 w/w) were prepared as described in example 10. After the manufacturing process the liposomes are concentrated and dialyzed to remove non-encapsulated siRNA and the alcohol.

| | | |
|---|---|---|
| F5: | DOTAP/Chems/Chol | 31:39:30 (mol%) |
| F6: | DOTAP/DMGS/Chol | 15:45:40 (mol%) |
| F7: | CholC4N-Mo2/DMGS/Chol | 23:47:30 (mol%) |
| F8: | POPC/DOPE/HisChol/DMGS/Chol | 7:28:25:30:10 (mol%) |

**Table 81:**

| | **F5** | **F6** | **F7** | **F8** |
|---|---|---|---|---|
| **Size** | 124 nm | 115 nm | 125 | 119 nm |
| **PI** | 0.11 | 0.15 | 0.13 | 0.14 |
| **Encapsulation efficiency** | 79 % | 87% | 95 % | 93% |

For determination of the serum stability the liposomes were diluted to a lipid concentration of 2 mM and then incubated in 75 % mouse serum at 37 °C for 2 h (final lipid concentration 0,5 mM). Release of siRNA during serum incubation was monitored by gel electrophoresis on a 15 % polyacrylamide gel (Biorad) in TBE buffer. As only free siRNA enters the gel the siRNA released from the liposomes during serum incubation can be detected on the gel using an ODYSSEY Infrared Imaging System (LI-COR Biosciences) which detects the Cy 5.5 labelled siRNA.

### Results:

**Table 82:**

| | **F5** | **F6** | **F7** | **F8** |
|---|---|---|---|---|
| **siRNA release after 30 min** | 29% | 16% | 0% | 15% |
| **siRNA release after 2h** | 40% | 33% | 0% | 15% |

### Example 12: Biodistribution and tolerability of amphoteric liposomes in mice

Amphoteric liposomes F5, F7 and F8 of example 11 were injected intravenously into the tail vein of female BALB/c mice in a dose of 8 mg/kg siRNA. Mice were sacrified after 2h and cryosections of liver and spleen were prepared and analyzed using an ODYSSEY Infrared Imaging System (LI-COR Biosciences) which detects the Cy 5.5 labelled siRNA. Average intensities of the cryosections are calculated by total intensity/area.

### Results:

Mice showed no signs of side effects, such as scrubby fur, dyspnea or apathy.

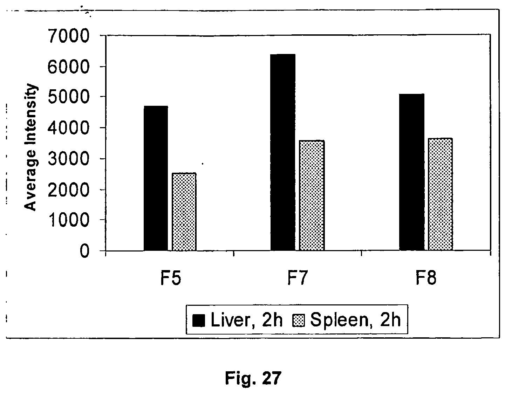

The biodistribution of the amphoteric liposomes after two hours in liver and spleen is shown in Fig.27. All amphoteric liposome formulations can be found in the liver and in a somewhat lower concentration in the spleen.

### Example 13: Amphoteric liposomes encapsulating siRNA

siRNA-loaded amphoteric liposomes were manufactured using non-targeting scrambled siRNA. The lipid mixtures A (DC-Chol:DMGS :Chol, 26:39:35 mol%) or B (DC-Chol:DMGS:Chol, 20:40:40 mol%) were dissolved at a concentration of 30 mM or 60 mM (final lipid concentration) for both mixtures in ethanol. Appropriate volumes of siRNA stock were diluted in 20 mM NaAc, 300 mM Sucrose/NaOH pH 4.0. The organic and the aqueous solution were mixed in a 3:7 ratio and the liposomal suspension was immediately shifted to pH > 7.5 with 136 mM Na₂HPO₄, 100 mM NaCl.

The amount of unencapsulated siRNA was determined by using ultrafiltration with Centrisart (Molecular Weight Cut off 300 kD (Sartorius, Göttingen, Germany)). The siRNA concentration of the filtrate was measured spectroscopically (OD260nm). The amount of encapsulated oligonucleotide was determined by subtraction of unencapsulated amount of siRNA from the total amount of siRNA.

Particle characteristics after manufacturing:

**Table 83:**

| Formulation | Initial lipid concentration | Size // Polydispersity | Encapsulation efficacy |
|---|---|---|---|
| | | index | |
| A | 30 mM | 256 nm // 0.319 | 63% |
| A | 60 mM | 313 nm // 0.490 | 64% |
| B | 30 mM | 184 nm // 0.055 | 69% |
| B | 60 mM | 206 nm // 0.135 | 77% |

### Example 14 - Synthesis of 1,2-Dioleoyl-3-methyl-(methoxycarbonyl-ethyl)ammonium-Propane (DOMCAP)

### Step A: Synthesis of 1,2-Dihydroxy-3-methyl-(methoxycarbonylethyl)ammonium-Propane

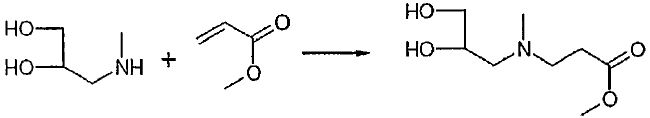

The compound was synthesized according to Xu et al., Synlett 2003, 2425-2427. Briefly, 5.26 g 3-Methylamino-1,2-propanediol was added to 80 ml acetonitrile and the mixture was stirred for 2.5 h. Then 4.31 g acrylic acid methylester and 0.5 g copper(II) acetate monohydrate were added and the reaction was allowed to stir overnight at room temperature. The solvent was removed by rotary evaporation and the crude product, a blue oil, was purified by a flash column chromatography on silica gel (eluent: acetic acid ethylester). The product, a colourless oil, was characterized by ¹H-NMR.

### Step B: Synthesis of 1,2-Dioleoyl-3-methyl-(methoxycarbonylethyl)ammonium-Propane

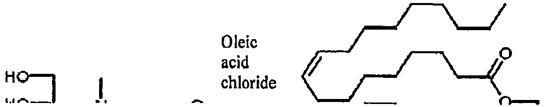

1.9 g 1,2-Dihydroxy-3-methyl-(methoxycarbonyl-ethyl)ammonium-propane were dissolved in 40 ml dry dichloromethane. Subsequently, 2.23 g triethylamine and 0.318 mg 4-dimethylaminopyridine were added and the mixture was cooled with an ice bath down to 5-10 °C. Then a solution of 6.62 g oleic acid chloride in 10 ml dichloromethane was added dropwise to the reaction mixture whereas the temperature was controlled to be lower than 15 °C. After the addition the ice bath was removed and the mixture allowed to stir at 20 °C for two hours. Finally, the reaction mixture was filtered and the residue washed with 50 ml dichloromethane. The solvent of the filtrate was removed by rotary evaporation and the crude product, a yellow oil, was purified by flash column chromatography on silica gel (eluent: acetic acid ethylester:petrolether 1:9). The product, a yellow oil, was characterized by ¹H-NMR and LC-MS.

### Example 15: Synthesis of 1,2-Dioleoyl-3-N-pyrrolidine-propane (DOP5P)

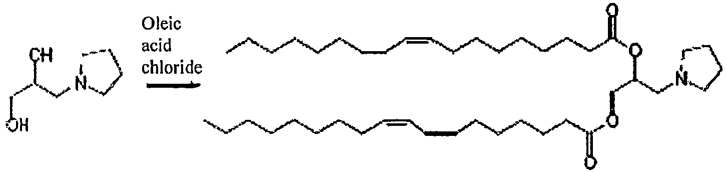

Under N₂ atmosphere 2 g pyrrolidino-1,2-propandiol were combined with 25 ml dichloromethane. Then 2.79 g triethylamine and 0.01 g 4-dimethylaminopyridine were added and the reaction mixture was stirred and cooled in an ice bath. Subsequently, 8.29 g oleic acid chloride in 25 ml dichloromethane were added dropwise over 45 min. The reaction mixture was allowed to stir for 2 days at room temperature. The crude product was purified by column chromatography on silica gel (eluent: acetic acid ethyl ester : petrol ether 1:1). The product, a yellow oil, was characterized by ¹H-NMR, ¹³C-NMR and LC-MS.

### Example 16: Synthesis of 1,2-Dioleoyl-3-N-pyrridinium-propane, bromide salt (DOP6P)

### Step A: Synthesis of 1,2-Dioleoyl-3-bromo-propane

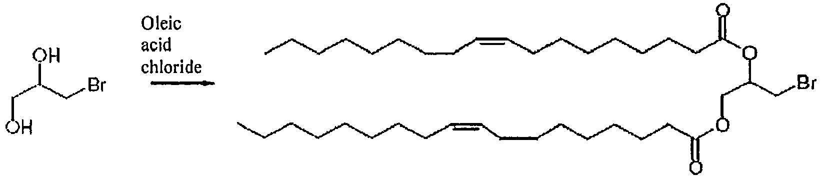

Under N₂ atmosphere 7.75 g 3-bromo-1,2-propandiol were dissolved in 300 ml dichloromethane. The reaction mixture was cooled with an ice bath and subsequently 19.39 g N,N-diisopropyl ethylamine and 36.11 g oleic acid chloride were added. The reaction was allowed to stir over night. Then the solvent was removed by rotary evaporation. After the addition of 300 ml petrol ether a white solid precipitated which was removed. The crude product was purified by flash column chromatorgraphy on silica gel (eluent: petrol ether). The product, a yellow oil was characterized by ¹H-NMR.

### Step B: Synthesis of 1,2-Dioleoyl-3-N-pyrridinium-propane, bromide salt

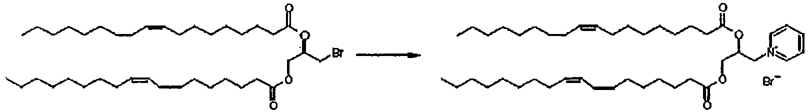

5.56 g 1,2-Dioleoyl-3-bromo-propane were dissloved in 80 ml pyridine and the reaction mixture was allowed to stir over night at 85 °C. The solvent was removed by rotary evaporation and the crude product was purified by column chromatography on silica gel (eluents: chloroform; chloroform : methanol 4:1). The product, a brown oil, was characterized by ¹H-NMR.

## Claims

1. An amphoteric liposome comprising:
(a) neutral lipids selected from cholesterol or a mixture of cholesterol and at least one neutral or zwitterionic lipid, wherein κ(neutral) of said mixture is 0.25 or less and wherein κ(neutral) is calculated by the following formula: $κ \left(neutral\right) = κ \left(Lipid 1\right) ∗ c \left(Lipid 1\right) + κ \left(Lipid 2\right) ∗ c \left(Lipid 2\right) + … κ \left(Lipid i\right) ∗ c \left(Lipid i\right)$
wherein κ(Lipid) is the κ value of the appropriate neutral or zwitterionic lipid, c(Lipid) is the concentration of said lipid and i is the running variable,
wherein κ is the volume ratio between the polar and apolar section of a lipid: $κ = molecular volume \left(head\right) / molecular volume \left(tail\right) ;$ wherein the mixture of cholesterol and at least one neutral or zwitterionic lipid is selected from the group consisting of:
(i) cholesterol/phosphatidylcholine;
(ii) cholesterol/phosphatidylethanolamine; and
(iii) cholesterol/phosphatidylethanolamine/phosphatidylcholine; and
(b) a mixture of lipid components comprising:
(i) a stable cationic lipid and a chargeable anionic lipid, referred to as amphoter I mixture; or
(ii) a chargeable cationic lipid and a chargeable anionic lipid, referred to as amphoter II mixture; or
(iii) a chargeable cationic lipid and a stable anionic lipid, referred to as amphoter III mixture;
further wherein the mixture of lipid components comprises at least one lipid ion which is a pH-sensitive, weak acid or base which is chargeable; and
further wherein the minimum value of κ(total) of the mixture is smaller than 0.25.

2. The amphoteric liposome as claimed in claim 1, wherein the neutral lipids (a) are a mixture of cholesterol and at least one neutral or zwitterionic lipid.

3. The amphoteric liposome as claimed in claim 1 or claim 2, wherein κ(neutral) of said mixture (a) is less than 0.2 and preferably less than 0.15.

4. The amphoteric liposome as claimed in any one of the preceding claims, wherein the phosphatidylethanolamine is DOPE.

5. The amphoteric liposome as claimed in any one of the preceding claims, wherein the phosphatidylcholine is selected from the group consisting of DMPC, DPPC, DSPC, POPC, DOPC, soy bean PC and egg PC.

6. The amphoteric liposome as claimed in any one of the preceding claims, wherein the phosphatidylethanolamine is DOPE and the phosphatidylcholine is either POPC or DOPC.

7. The amphoteric liposome as claimed in any preceding claim, wherein the cholesterol and the at least one neutral or zwitterionic lipid are present in said mixture (a) at a molar ratio of between 4 and 0.25.

8. The amphoteric liposome as claimed in any preceding claim, wherein:
(I) said anionic lipid is selected from the group consisting of:
diacylglycerolhemisuccinates, such as DOGS, DMGS, POGS, DPGS, DSGS;
diacylglycerolhemimalonates, such as DOGM or DMGM;
diacylglycerolhemiglutarates, such as DOGG or DMGG;
diacylglycerolhemiadipates, such as DOGS or DMGA;
diacylglycerolhemicyclohexane-1,4-dicarboxylic acids, such as DO-cHA, DM-cHA:
(2,3-Diacyl-propyl)amino}-oxoalkanoic acids, such a DOAS, DOAM, DOAG, DOAA, DMAS, DMAM, DMAG, DMAA;
Diacyl-alkanoic acids, such as DOP, DOB, DOS, DOM, DOG, DOA, DMP, DOB, DMS, DMM, DMG, DMA;
CHEMS and derivatives thereof, such as Chol-C2, Chol-C3, Chol-C5, Chol-C6, Chol-C7 or Chol-C8;
Chol-C1, CholC3N or Cholesterolhemidicarboxylic acids and Cholesteryloxycarbonylaminocarboxylic acids, such as Chol-C12 or Chol-C13N;
fatty acids, such as Oleic acid, Myristic Acid, Palmitic acid, Stearic acid, Nervonic Acid, Behenic Acid;
DOPA, DMPA, DPPA, POPA, DSPA, Chol-SO4, DOPG, DMPG, DPPG, POPG, DSPG or DOPS, DMPS, DPPS, POPS, DSPS or Cetyl-phosphate; and/or
(II) said cationic lipid is selected from the group consisting of:
DOTAP, DMTAP, DPTAP, DSTAP, POTAP, DODAP, PODAP, DMDAP, DPDAP, DSDAP, DODMHEAP or DORI, PODMHEAP or PORI, DMDMHEAP or DMRI, DPDMHEAP or DPRI, DSDMHEAP or DSRI, DOMDHEAP, POMDHEAP, DMMDHEAP, DPMDHEAP, DSMDHEAP, DOMHEAP, POMHEAP, DMMHEAP, DPMHEAP, DSMHEAP, DODHEAP, PODHEAP, DMDHEAP, DPDHEAP, DSDHEAP, DDAB, DODAC, DOEPC, DMEPC, DPEPC, DSEPC, POEPC, DORIE, DMRIE, DOMCAP, DOMGME, DOP5P, DOP6P, DC-Chol, TC-Chol, DAC-Chol, Chol-Betaine, N-methyl-PipChol, CTAB, DOTMA, MoChol, HisChol, Chim, MoC3Chol, Chol-C3N-Mo3, Chol-C3N-Mo2, Chol-C4N-Mo2, Chol-DMC3N-Mo2, CholC4Hex-Mo2, DmC4Mo2, DmC3Mo2, C3Mo2, C3Mo3, C5Mo2, C6Mo2, C8Mo2, C4Mo4, PipC2-Chol, MoC2Chol, PyrroC2Chol, ImC3Chol, PyC2Chol, MoDO, MoDP, DOIM or DPIM.

9. The amphoteric liposome as claimed in any preceding claim, wherein said amphoteric liposome has an isoelectric point between 4.5 and 6.5, or has a size in the range of 50 to 1000 nm.

10. The amphoteric liposome as claimed in any preceding claim, wherein said liposome comprises:
cell targeting ligands selected from the group consisting of antibodies or their fragments, sugars, hormones, vitamins, peptides, growth factors, billirubin, transferrin and folate; and/or
membrane forming or membrane situated molecules selected from the group consisting of amphipathic dextranes, polysialic acids, hydroxyethyl starches, hyaluronic acids, polyethylenglycols, Tween 80 or GM1 gangliosides.

11. The amphoteric liposome as claimed in any preceding claim, wherein said liposomes encapsulate at least one active agent, wherein preferably said active agent:
(i) comprises a nucleic acid that is capable of being transcribed in a vertebrate cell into one or more RNAs, said RNAs being mRNAs, shRNAs, miRNAs or ribozymes, said mRNAs coding for one or more proteins or polypeptides, preferably wherein said nucleic acid is a circular DNA plasmid, a linear DNA construct or an mRNA; or
(ii) is an oligonucleotide, preferably a decoy oligonucleotide, an antisense oligonucleotide, a siRNA, an agent influencing transcription, an agent influencing splicing, Ribozymes, DNAzymes or Aptamers, optionally wherein said oligonucleotide comprises modified nucleosides such as DNA, RNA, locked nucleic acids (LNA), peptide nucleic acids (PNA), 2'O-methyl RNA (2'Ome), 2' O-methoxyethyl RNA (2'MOE) in their phosphate or phosphothioate forms.

12. An amphoteric liposome as claimed in claim 11, wherein at least 80 wt.% of said active agent is disposed inside said liposomes.

13. A pharmaceutical composition comprising active agent-loaded amphoteric liposomes as claimed in claim 11 or claim 12 and a pharmaceutically acceptable vehicle therefor.

14. Amphoteric liposomes as claimed in any of claims 1 to 12 for use in the *in vitro, in vivo* or *ex-vivo* transfection of cells.

## Patentansprüche

1. Amphoteres Liposom, umfassend:
(a) neutrale Lipide, ausgewählt aus Cholesterin oder einem Gemisch aus Cholesterin und mindestens einem neutralen oder zwitterionischen Lipid, wobei κ(neutral) des Gemischs 0,25 oder weniger ist und wobei κ(neutral) durch die folgende Formel berechnet wird: $κ \left(neutral\right) = κ \left(Lipid 1\right) ∗ c \left(Lipid 1\right) + κ \left(Lipid 2\right) ∗ c \left(Lipid 2\right) + … κ \left(Lipid i\right) ∗ c \left(Lipid i\right)$
wobei κ(Lipid) der κ-Wert des entsprechenden neutralen oder zwitterionischen Lipids ist, c(Lipid) die Konzentration des Lipids ist und i die Zuteilungsvariable ist,
wobei κ das Volumenverhältnis zwischen dem polaren und apolaren Abschnitt eines Lipids ist: $κ = molekulares Volumen \left(Kopf\right) / molekulares Volumen \left(Schwanz\right) ;$ wobei das Gemisch aus Cholesterin und mindestens einem neutralen oder zwitterionischen Lipid ausgewählt ist aus der Gruppe bestehend aus:
(i) Cholesterin/Phosphatidylcholin;
(ii) Cholesterin/Phosphatidylethanolamin; und
(iii) Cholesterin/Phosphatidylethanolamin/Phosphatidylcholin; und
(b) einem Gemisch aus Lipidkomponenten, umfassend:
(i) ein stabiles kationisches Lipid und ein aufladbares anionisches Lipid, bezeichnet als Amphoter-I-Gemisch; oder
(ii) ein aufladbares kationisches Lipid und ein aufladbares anionisches Lipid, bezeichnet als Amphoter-II-Gemisch; oder
(iii) ein aufladbares kationisches Lipid und ein stabiles anionisches Lipid, bezeichnet als Amphoter-III-Gemisch;
ferner wobei das Gemisch aus Lipidkomponenten mindestens ein Lipidion umfasst, das eine pH-empfindliche, schwache Säure oder Base ist, die aufladbar ist; und
ferner wobei der Minimalwert von κ(total) des Gemischs kleiner als 0,25 ist.

2. Amphoteres Liposom nach Anspruch 1, wobei die neutralen Lipide (a) ein Gemisch aus Cholesterin und mindestens einem neutralen oder zwitterionischen Lipid sind.

3. Amphoteres Liposom nach Anspruch 1 oder Anspruch 2, wobei κ(neutral) des Gemischs (a) weniger als 0,2 und bevorzugt weniger als 0,15 ist.

4. Amphoteres Liposom nach einem der vorhergehenden Ansprüche, wobei das Phosphatidylethanolamin DOPE ist.

5. Amphoteres Liposom nach einem der vorhergehenden Ansprüche, wobei
das Phosphatidylcholin ausgewählt ist aus der Gruppe bestehend aus DMPC, DPPC, DSPC, POPC, DOPC, Sojabohne-PC und Ei-PC.

6. Amphoteres Liposom nach einem der vorhergehenden Ansprüche, wobei
das Phosphatidylethanolamin DOPE ist und das Phosphatidylcholin entweder POPC oder DOPC ist.

7. Amphoteres Liposom nach einem vorhergehenden Anspruch, wobei das
Cholesterin und das mindestens eine neutrale oder zwitterionische Lipid in dem Gemisch (a) in einem Molverhältnis von zwischen 4 und 0,25 vorhanden sind.

8. Amphoteres Liposom nach einem vorhergehenden Anspruch, wobei:
(I) das anionische Lipid ausgewählt ist aus der Gruppe bestehend aus:
Diacylglycerolhemisuccinaten, wie z. B. DOGS, DMGS, POGS, DPGS, DSGS;
Diacylglycerolhemimalonaten, wie z. B. DOGM oder DMGM;
Diacylglycerolhemiglutaraten, wie z. B. DOGG oder DMGG; Diacylglycerolhemiadipaten, wie z. B. DOGS oder DMGA;
Diacylglycerolhemicyclohexan-1,4-dicarbonsäuren, wie z.B. DO-cHA, DM-cHA:
(2,3-Diacyl-propyl)amino}-oxoalkansäuren, wie z. B. DOAS, DOAM, DOAG, DOAA, DMAS, DM AM, DMAG, DMAA;
Diacyl-alkansäuren, wie z. B. DOP, DOB, DOS, DOM, DOG, DOA, DMP, DOB, DMS, DMM, DMG, DMA;
CHEMS und Derivaten davon, wie z. B. Chol-C2, Chol-C3, Chol-C5, Chol-C6, Chol-C7 oder Chol-C8;
Chol-C1, CholC3N oder Cholesterinhemidicarbonsäuren und Cholesteryloxycarbonylaminocarbonsäuren, wie z. B. Chol-C12 oder Chol-Cl3N; Fettsäuren, wie z. B. Ölsäure, Myristinsäure, Palmitinsäure, Stearinsäure, Nervonsäure, Behensäure;
DOPA, DMPA, DPPA, POPA, DSPA, Chol-SO4, DOPG, DMPG, DPPG, POPG, DSPG oder DOPS, DMPS, DPPS, POPS, DSPS oder Cetyl-phosphat; und/oder
(II) das kationische Lipid ausgewählt ist aus der Gruppe bestehend aus:
DOTAP, DMTAP, DPTAP, DSTAP, POTAP, DODAP, PODAP, DMDAP, DPDAP, DSDAP, DODMHEAP oder DORI, PODMHEAP oder PORI, DMDMHEAP oder DMRI, DPDMHEAP oder DPRI, DSDMHEAP oder DSRI, DOMDHEAP, POMDHEAP, DMMDHEAP, DPMDHEAP, DSMDHEAP, DOMHEAP, POMHEAP, DMMHEAP, DPMHEAP, DSMHEAP, DODHEAP, PODHEAP, DMDHEAP, DPDHEAP, DSDHEAP, DDAB, DODAC, DOEPC, DMEPC, DPEPC, DSEPC, POEPC, DORIE, DMRIE, DOMCAP, DOMGME, DOP5P, DOP6P, DC-Chol, TC-Chol, DAC-Chol, Chol-Betain, N-Methyl-PipChol, CTAB, DOTMA, MoChol, HisChol, Chim, MoC3Chol, Chol-C3N-Mo3, Chol-C3N-Mo2, Chol-C4N-Mo2, Chol-DMC3N-Mo2, CholC4Hex-Mo2, DmC4Mo2, DmC3Mo2, C3Mo2, C3Mo3, C5Mo2, C6Mo2, C8Mo2, C4Mo4, PipC2-Chol, MoC2Chol, PyrroC2Chol, ImC3Chol, PyC2Chol, MoDO, MoDP, DOIM oder DPIM.

9. Amphoteres Liposom nach einem vorhergehenden Anspruch, wobei das amphotere Liposom einen isoelektrischen Punkt zwischen 4,5 und 6,5 hat oder eine Größe im Bereich von 50 bis 1000 nm hat.

10. Amphoteres Liposom nach einem vorhergehenden Anspruch, wobei das Liposom Folgendes umfasst:
Zelltargeting-Liganden, ausgewählt aus der Gruppe bestehend aus Antikörpern oder ihren Fragmenten, Zuckern, Hormonen, Vitaminen, Peptiden, Wachstumsfaktoren, Bilirubin, Transferrin und Folat; und/oder
membranbildende oder membranständige Moleküle, ausgewählt aus der Gruppe bestehend aus amphipathischen Dextranen, Polysialinsäuren, Hydroxyethylstärken, Hyaluronsäuren, Polyethylenglykolen, Tween 80 oder GM1-Gangliosiden.

11. Amphoteres Liposom nach einem vorhergehenden Anspruch, wobei die Liposome mindestens einen Wirkstoff einkapseln, wobei der Wirkstoff bevorzugt:
(i) eine Nukleinsäure umfasst, die in der Lage ist, in einer Wirbeltierzelle in eine oder mehrere RNAs transkribiert zu sein, wobei die RNAs mRNAs, shRNAs, miRNAs oder Ribozyme sind, wobei die mRNAs für ein oder mehrere Proteine oder Polypeptide kodieren, bevorzugt wobei die Nukleinsäure ein zirkuläres DNA-Plasmid, ein lineares DNA-Konstrukt oder eine mRNA ist; oder
(ii) ein Oligonukleotid, bevorzugt ein Decoy-Oligonukleotid, ein Antisense-Oligonukleotid, eine siRNA, ein Mittel, das die Transkription beeinflusst, ein Mittel, das Spleißen beeinflusst, Ribozyme, DNAzyme oder Aptamere ist, optional wobei das Oligonukleotid modifizierte Nukleoside umfasst, wie z. B. DNA, RNA, verschlossene Nukleinsäuren (LNA), Peptidnukleinsäuren (PNA), 2'O-methyl-RNA (2'Ome), 2'O-methoxyethyl-RNA (2'MOE) in ihren Phosphat- oder Phosphothioatformen.

12. Amphoteres Liposom nach Anspruch 11, wobei mindestens 80 Gew.-% des Wirkstoffs in den Liposomen angeordnet ist.

13. Pharmazeutische Zusammensetzung, umfassend wirkstoffbeladene amphotere Liposome nach Anspruch 11 oder Anspruch 12 und eine pharmazeutisch annehmbare Trägersubstanz dafür.

14. Amphotere Liposome nach einem der Ansprüche 1 bis 12 zur Verwendung in der *in-vitro-*, *in-vivo-* oder *ex-vivo*-Transfektion von Zellen.

## Revendications

1. Liposome amphotère, comprenant :
(a) des lipides neutres sélectionnés à partir de cholestérol ou d'un mélange de cholestérol et d'au moins un lipide neutre ou zwittérionique, dans lequel κ(neutre) dudit mélange est 0,25 ou moins et dans lequel κ(neutre) est calculé par la formule suivante : $κ \left(neutre\right) = κ \left(Lipide 1\right) ∗ c \left(Lipide 1\right) + κ \left(Lipide 2\right) ∗ c \left(Lipide 2\right) + … κ \left(Lipide i\right) ∗ c \left(Lipide i\right)$
dans lequel κ(Lipide) est la valeur κ du lipide neutre ou zwittérionique approprié, c(Lipide) est la concentration dudit lipide et i est la variable courante,
dans lequel κ est le rapport volumique entre la section polaire et apolaire d'un lipide : $κ = volume moléculaire \left(tête\right) / volume moléculaire \left(queue\right) ;$ dans lequel le mélange de cholestérol et d'au moins un lipide neutre ou zwittérionique est sélectionné à partir du groupe constitué de :
(i) cholestérol/phosphatidylcholine ;
(ii) cholestérol/phosphatidyléthanolamine ; et
(iii) cholestérol/phosphatidyléthanolamine/phosphatidylcholine ; et
(b) un mélange de composants lipidiques comprenant :
(i) un lipide cationique stable et un lipide cationique chargeable, appelé mélange I amphotère ; ou
(ii) un lipide cationique chargeable et un lipide cationique chargeable, appelé mélange II amphotère ; ou
(iii) un lipide cationique chargeable et un lipide anionique stable, appelé mélange III amphotère ;
en outre dans lequel le mélange de composants lipidiques comprend au moins un ion lipidique qui est un acide ou base faible sensible au pH qui est chargeable ; et en outre dans lequel la valeur minimum de κ(total) du mélange est inférieure à 0,25.

2. Liposome amphotère selon la revendication 1, dans lequel les lipides neutres (a) sont un mélange de cholestérol et d'au moins un lipide neutre ou zwittérionique.

3. Liposome amphotère selon la revendication 1 ou la revendication 2, dans lequel κ(neutre) dudit mélange (a) est moins de 0,2 et de préférence moins de 0,15.

4. Liposome amphotère selon l'une quelconque des revendications précédentes, dans lequel la phosphatidyléthanolamine est DOPE.

5. Liposome amphotère selon l'une quelconque des revendications précédentes, dans lequel la phosphatidylcholine est sélectionné à partir du groupe constitué de : DMPC, DPPC, DSPC, POPC, DOPC, PC de soja et PC d'œuf.

6. Liposome amphotère selon l'une quelconque des revendications précédentes, dans lequel la phosphatidyléthanolamine est DOPE et la phosphatidylcholine est POPC ou DOPC.

7. Liposome amphotère selon une quelconque revendication précédente, dans lequel le cholestérol et l'au moins un lipide neutre ou zwittérionique sont présents dans ledit mélange (a) à un rapport molaire d'entre 4 et 0,25.

8. Liposome amphotère selon une quelconque revendication précédente, dans lequel :
(I) ledit lipide anionique est sélectionné à partir du groupe constitué de :
diacylglycérolhemisuccinates, tels que DOGS, DMGS, POGS, DPGS, DSGS;
diacylglycérolhemimalonates, tels que DOGM ou DMGM ;
diacylglycérolhemiglutarates, tels que DOGG ou DMGG ;
diacylglycérolhemiadipates, tels que DOGS ou DMGA ;
acides diacylglycérolhemicyclohexane-1,4-dicarboxyliques, tels que DO-cHA, DM-cHA ;
acides (2,3-diacyl-propyl)amino}-oxoalkanoïques, tels que DOAS, DOAM, DOAG, DOAA, DMAS, DMAM, DMAG, DMAA ;
acides diacyl-alkanoïques tels que DOP, DOB, DOS, DOM, DOG, DOA, DMP, DOB, DMS, DMM, DMG, DMA ;
CHEMS et des dérivés de ceux-ci, tels que Chol-C2, Chol-C3, Chol-C5, Chol-C6, Chol-C7 ou Chol-C8 ;
Chol-C1, CholC3N ou acides cholestérolhémidicarboxyliques et acides cholestéryloxycarbonylaminocarboxyliques, tels que Chol-C12 ou Chol-C13N ; acides gras, tels qu'acide oléïque, acide myristique, acide palmitique, acide stéarique, acide nervonique, acides béhénique ;
DOPA, DMPA, DPPA, POPA, DSPA, Chol-SO4, DOPG, DMPG, DPPG, POPG, DSPG ou DOPS, DMPS, DPPS, POPS, DSPS ou cétyl-phosphate ; et/ou
(II) ledit lipide cationique est sélectionné à partir du groupe constitué de :
DOTAP, DMTAP, DPTAP, DSTAP, POTAP, DODAP, PODAP, DMDAP, DPDAP, DSDAP, DODMHEAP ou DORI, PODMHEAP ou PORI, DMDMHEAP ou DMRI, DPDMHEAP ou DPRI, DSDMHEAP ou DSRI, DOMDHEAP, POMDHEAP, DMMDHEAP, DPMDHEAP, DSMDHEAP, DOMHEAP, POMHEAP, DMMHEAP, DPMHEAP, DSMHEAP, DODHEAP, PODHEAP, DMDHEAP, DPDHEAP, DSDHEAP, DDAB, DODAC, DOEPC, DMEPC, DPEPC, DSEPC, POEPC, DORIE, DMRIE, DOMCAP, DOMGME, DOP5P, DOP6P, DC-Chol, TC-Chol, DAC-Chol, Chol-Bétaïne, N-méthyl-PipChol, CTAB, DOTMA, MoChol, HisChol, Chim, MoC3Chol, Chol-C3N-Mo3, Chol-C3N-Mo2, Chol-C4N-Mo2, Chol-DMC3N-Mo2, CholC4Hex-Mo2, DmC4Mo2, DmC3Mo2, C3Mo2, C3Mo3, C5Mo2, C6Mo2, C8Mo2, C4Mo4, PipC2-Chol, MoC2Chol, PyrroC2Chol, ImC3Chol, PyC2Chol, MoDO, MoDP, DOIM ou DPIM.

9. Liposome amphotère selon une quelconque revendication précédente, dans lequel ledit liposome amphotère a un point isoélectrique entre 4,5 et 6,5, ou a une taille dans la plage de 50 à 1000 nm.

10. Liposome amphotère selon une quelconque revendication précédente, dans lequel ledit liposome comprend :
des ligands de ciblage cellulaire sélectionnés à partir du groupe constitué de : anticorps ou leurs fragments, sucres, hormones, vitamines, peptides, facteurs de croissance, billirubine, transferrine et folate ; et/ou
des molécules membranogènes ou situées en membrane sélectionnées à partir du groupe constitué de : dextranes amphipathiques, acides polysialiques, amidons hydroxyéthylique, acides hyaluroniques, polyéthylèneglycols, Tween 80 ou gangliosides GM1.

11. Liposome amphotère selon une quelconque revendication précédente, dans lequel ledit liposomes encapsule au moins un agent actif, dans lequel de préférence ledit agent actif :
(i) comprend un acide nucléique qui est capable d'être transcrit dans une cellule vertébrée en un ou plusieurs ARNs, lesdits ARNs étant mARNs, shARNs, miARNs ou ribozymes, lesdits mARNs codant pour un(e) ou plusieurs protéines ou polypeptides, de préférence dans lequel ledit acide nucléique est un plasmide d'ADN circulaire, une construction d'ADN linéaire ou un mARN ; ou
(ii) est un oligonucléotide, de préférence un oligonucléotide leurre, un oligonucléotide antisens, un siARN, un agent influençant la transcription, un agent influençant l'épissage, des ribozymes, DNAzymes ou aptamères, optionnellement dans lequel ledit oligonucléotide comprend des nucléosides modifiés tels qu'ADN, ARN, acides nucléiques verrouillés (LNA), acides nucléiques peptidiques (PNA), ARN 2'O-méthyle (2'Ome), ARN 2'O-méthoxyéthyle (2'MOE) dans leurs formes phosphates ou phosphothioates.

12. Liposome amphotère selon la revendication 11, dans lequel au moins 80 % en poids dudit agent actif est disposé à l'intérieur desdits liposomes.

13. Composition pharmaceutique, comprenant des liposomes amphotères, chargés d'agent actif, selon la revendication 11 ou la revendication 12 et un véhicule pharmaceutiquement acceptable associé.

14. Liposomes amphotères selon l'une quelconque des revendications 1 à 12 pour l'utilisation dans la transfection in vitro, in vivo ou ex-vivo de cellules.
